# EUROPEAN PATENT SPECIFICATION

(11) **EP 4 457 235 B1**
(45) Date of publication and mention of the grant of the patent: **02.09.2026**
(21) Application number: 22854681.8
(22) Date of filing: 30.12.2022
(51) Int. Cl.: C07H 15/04, C07H 15/18, C07H 15/20, A61K 47/69, A61P 35/00, A61P 37/02

(54) **GLYCOMIMETIC LIGANDS**
GLYCOMIMETISCHE LIGANDEN
LIGANDS GLYCOMIMÉTIQUES

(30) Priority: 31.12.2021 US 202163295698 P
(43) Date of publication of application: 06.11.2024
(73) Proprietor: Aviceda Therapeutics, Inc., Cambridge, MA 02142 (US); University of Georgia Research Foundation, Inc., Athens, GA 30602 (US)
(72) Inventor: TOLENTINO, Michael, Lakeland, FL 33803 (US); GENEAD, Mohamed, A., Newton Center, MA 02459 (US); KRISHNAN, Anitha, Braintree, MA 02184 (US); BOONS, Gerardus, J.P.H., Athens, GA 30605 (US); PRUDDEN, Anthony, R., Athens, GA 30605 (US); LIU, Lin, Athens, GA 30602 (US); GREENE, Michelle, Belfast, Northern Ireland, BT88FT (IE); SCOTT, Christopher, Lisburn BT27 5TZ (GB); SHINDE, Rajesh, R., Lexington, MA 02421 (US)
(74) Representative: Snodin, Michael D.
(86) International application number: PCT/US2022/054381
(87) International publication number: WO 2023/129737

(56) References cited:
- WO-A1-2018/098342
- WO-A1-2022/060984
- WO-A2-02/09744
- WO-A2-2006/068758
- WO-A2-2007/056525

## Description

### BACKGROUND OF THE INVENTION

A need exists for improved compositions and methods for modulating sialic-acid binding self-associated pattern recognition receptors, known as sialic-acid-binding immunoglobulin-type lectins (Siglecs), for treatment of diseases resulting from acute, chronic, or aberrant immune system activation.

WO 2018/098342 A1, entitled "N-acetylated sialic acids and related sialosides", was published on 31 May 2018. WO 2006/068758, entitled "Detection, prevention and treatment of breast cancer", was published on 29 June 2006. WO 02/09744, entitled "Modified sialic acid vaccines", was published on 07 February 2002. WO 2007/056525, entitled "High affinity siglec ligands", was published on 18 May 2007. WO 2022/060984, entitled "Sialic-acid ligand decorated therapeutics", was published on 24 March 2022.

### SUMMARY OF THE INVENTION

The invention is defined in the appended claims. Any disclosure going beyond the extent of protection provided by said claims, even if indicated as being part of the invention, is intended only for illustrative purposes and should not be construed as being part of the invention. Furthermore, any reference to methods of treatment should be interpreted as a compound for use in a method of treatment. In an example embodiment, the present invention is a compound represented by any one of the following structural formulas: 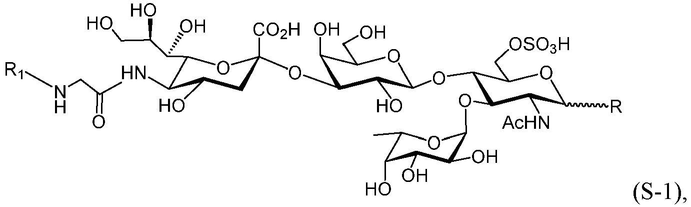 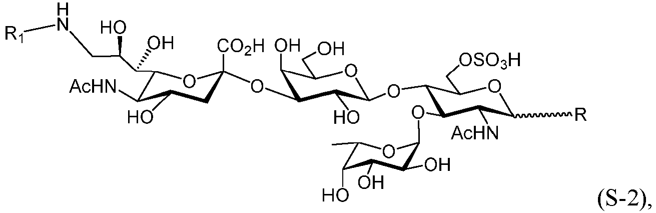 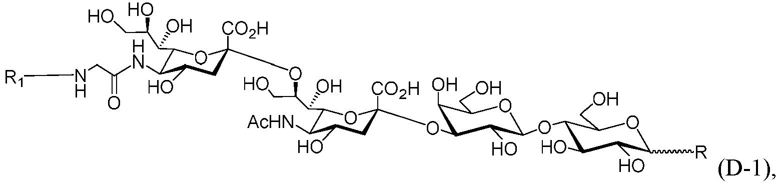 or 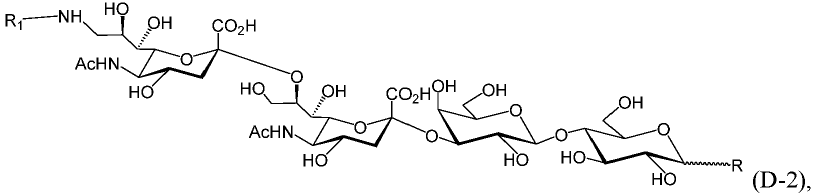 or a pharmaceutically acceptable salt thereof.

In formulas (S-1), (S-2), (D-1), and (D-2): R₁, for each occurrence independently, is -C(O)-A, wherein A is a C₁-C₆ alkyl, a C₆-C₁₈ aryl, a (C₆-C₁₈)aryl(C₁-C₃)alkyl, a 5-18-member heteroaryl, a (5-18-member)heteroaryl(C₁-C₃)alkyl, a C₃-C₈ cycloalkyl, a (C₃-C₈)cycloalkyl(C₁-C₃)alkyl, a 5-8-member heterocycloalkyl, or a (5-8-member)heterocycloalkyl(C₁-C₃)alkyl, wherein one or two carbon atoms within the alkyl portion of A is optionally, each independently, replaced with a heteroatom selected from N, O, or S, and wherein A is optionally substituted with 1 to 3 R¹¹ groups, each said R¹¹ group independently selected from a C₁-C₆ alkyl, a C₁-C₆ haloalkyl, a C₁-C₆ alkoxy, a halogen, a C₆₋C₁₂ aryl, a 5-12-member heteroaryl, cyano, or two groups R¹¹, taken together with the atoms to which they are attached, form a 5-7-member heterocyclyl having 1 to 3 heteroatoms selected from N, O, or S. R¹¹, each independently, is optionally substituted with 1 to 3 substituents selected from a halogen, a C₁-C₆ alkyl, a C₁-C₆ haloalkyl, a C₁-C₆ alkoxy, or cyano; and further wherein: R, for each occurrence independently, is -R^{L}-R^{F}, and wherein: R^{L} is, for each occurrence independently: -O-(C₁-C₁₂) alkylenyl-, -O-, -S-, -NR¹⁰⁰-, -S-(C₁-C₁₂) alkylenyl-, -NR¹⁰¹-(C₁-C₁₂) alkylenyl-, -NR^{101a}-O-(C₁-C₁₂)alkylenyl-; -O-(CH₂CH₂O)ₘ-, -O-(CH₂CH₂O)ₖ-(CH₂CH₂)-, -NR¹⁰²-X¹⁰⁰-(C₁-C₁₂) alkylenyl-, -NR^{102a}-NR^{102b}-C(O)-(C₁-C₁₂)alkylenyl, wherein R¹⁰⁰, R¹⁰¹, R^{101a}, R¹⁰², R^{102a}, and R^{102b}, each independently is H or a C₁-C₃ alkyl, and X¹⁰⁰ is -O- or -NH-, and wherein m and k, each independently, is an integer from 1 to 12; R^{F}, for each occurrence independently, is: H, a C₁-C₃ alkyl, -NH₂, -NH-Fmoc, -NH-Boc, -NH-CBz, -NH-Troc, -NH-TFA, a mono(C₁-C₃)alkylamino, a di(C₁-C₃) alkylamino; -C(O)-R¹⁰³, wherein R¹⁰³ is -H, -OH, or a (C₁-C₃) alkyl; -SH, a moiety represented by the following structural formula 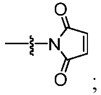 or a click chemistry reagent.

**In** another example embodiment, the present invention is a particle, comprising a compound represented by the following structural formula (2).

G-L-P (2).

**In** structural formula (2), P is a biocompatible polymer. For example, the biocompatible polymer comprises at least one of polyglycolic acid, poly(lactic acid), poly(lactic-co-glycolic acid), polycaprolactone, poly(3-hydroxybutyric acid ), poly(ethylene glycol), polyethylene oxide, poly(ethylene glycol)-block-poly(propylene glycol)-block-poly(ethylene glycol) (Pluronic F127), polyoxyethylene-polyoxypropylene block copolymer (Pluronic F68), poloxamer, poly(hydroxymethylmethacrylate), polyvinyl alcohol, poly(vinylpyrrolidone), hyaluronic acid, heparin, heparin sulfate, polysialic acid and chitosan; L is a covalent linker; and G is any one of the moieties represented by the following structural formulas: 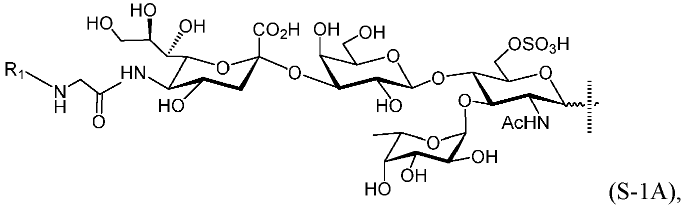 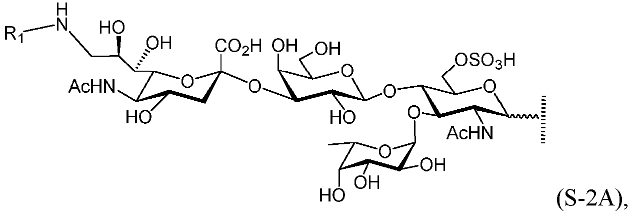 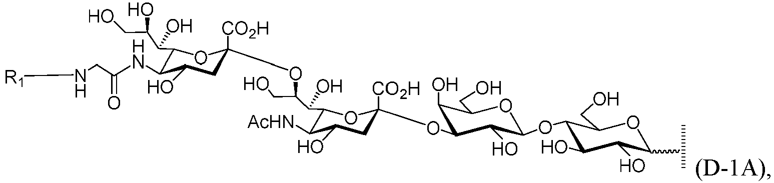 or 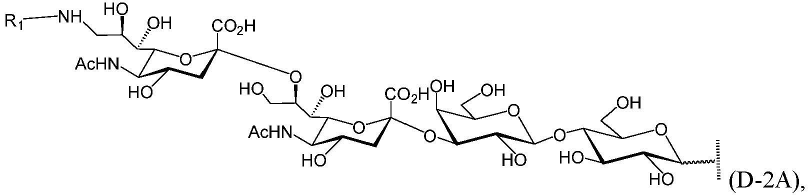 or a pharmaceutically acceptable salt thereof. Values and example values of variable R₁ are as defined above with respect to formulas (S-1), (S-2), (D-1), and (D-2).

In another example embodiment, the present invention is a method of making a particle comprising a compound represented by the following structural formula (2):

G-L-P (2),

wherein P is a biocompatible polymer. For example, the biocompatible polymer comprises at least one of polyglycolic acid, poly(lactic acid), poly(lactic-co-glycolic acid), polycaprolactone, poly(3-hydroxybutyric acid), poly(ethylene glycol), polyethylene oxide, poly(ethylene glycol)-block-poly(propylene glycol)-block-poly(ethylene glycol) (Pluronic F127), polyoxyethylene-polyoxypropylene block copolymer (Pluronic F68), poloxamer,
poly(hydroxymethylmethacrylate), polyvinyl alcohol, poly(vinylpyrrolidone), hyaluronic acid, heparin, heparin sulfate, polysialic acid and chitosan; L is a covalent linker; and G is any one of the moieties represented by the structural formulas (S-1A), (S-2A), (D-1A), and (D-2A), or a pharmaceutically acceptable salt thereof, wherein the symbol 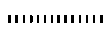 represents the point of attachment to L, and the values and example values of variable R₁ are as defined above with respect to formulas (S-1), (S-2), (D-1), and (D-2). The method comprises: reacting a compound represented by a structural formula (I)

G-R^{F1} (I)

with a compound represented by a structural formula (II)

P-R^{F2} (II),

wherein R^{F1} and R^{F2} are each a reactive moiety, under conditions suitable to cause moieties R^{F1} and R^{F2} to react with each other thereby producing a portion of the covalent -L-.

In various example embodiments, the compounds and particles described herein are useful in methods of treating diseases and disorders that are responsive to modulating (e.g., amplifying, reducing or eliminating) the activity of the Siglec receptors. Such diseases and disorders include, but are not limited to, cancer, immune-related and inflammatory-related diseases and disorders. For example, the compounds and particles described herein are useful in methods of treating a disorder selected from a cancer, an ophthalmic disease, a fibrotic disease, a parasitic inflammation, a fungal inflammation, a viral inflammation, an autoimmune inflammation, a neurological inflammation, a neurological degeneration, a dermatologic inflammation, a renal inflammation, a cardiovascular disease, a gastrointestinal inflammation, or a rheumatic disease.

In other embodiments, the compounds and particles described herein are useful for treating a disorder selected from cancer (e.g., a breast cancer, non-small cell lung cancer (NSCLC), prostrate cancer, colorectal cancer, melanoma, pancreatic cancer, and myelofibrosis), diabetic retinopathy, idiopathic pulmonary lung fibrosis, liver fibrosis, sickle cell anemia, and acute respiratory distress syndrome (ARDS).

In a further example embodiment, the present invention is a compound represented by the following structural formula: 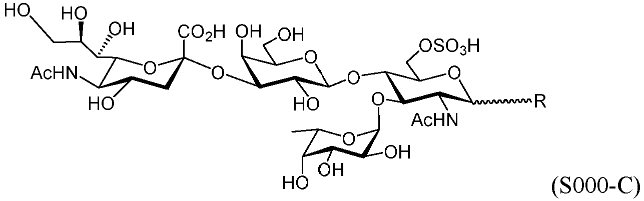 or a pharmaceutically acceptable salt thereof. In formula (S000-C), R, for each occurrence independently, is -R^{L}-R^{F}, and wherein: R^{L} is, for each occurrence independently: -O-, -S-, - NR¹⁰⁰-, -O-(C₁-C₁₂) alkylenyl-, -S-(C₁-C₁₂) alkylenyl-, -NR¹⁰¹-(C₁-C₁₂) alkylenyl-, -NR^{101a}-O-(C₁-C₁₂)alkylenyl-; -O-(CH₂CH₂O)ₘ-, -O-(CH₂CH₂O)ₖ-(CH₂CH₂)-, -NR¹⁰²-X¹⁰⁰-(C₁-C₁₂) alkylenyl-, -NR^{102a}-NR^{102b}-C(O)-(C₁-C₁₂)alkylenyl, wherein R¹⁰⁰, R¹⁰¹, R^{101a}, R¹⁰², R^{102a}, and R^{102b}, each independently is H or a C₁-C₃ alkyl, and X¹⁰⁰ is -O- or -NH-, and wherein m and k, each independently, is an integer from 1 to 12; R^{F}, for each occurrence independently, is: H, a C₁-C₃ alkyl, -NH₂, -NH-Fmoc, -NH-Boc, -NH-CBz, -NH-Troc, -NH-TFA, a mono(C₁-C₃)alkylamino, a di(C₁-C₃ alkyl)amino; -C(O)-R¹⁰³, wherein R¹⁰³ is -H, -OH, or a (C₁-C₃) alkyl; - SH, a moiety represented by the following structural formula 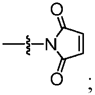 or a click chemistry reagent, provided that -R^{L}-R^{F} is not -OH.

In another embodiment, the present invention is a particle, compising a compound represented by the following structural formula:

G-L-P,

wherein: P is a biocompatible polymer. For example, the biocompatible polymer comprises at least one of polyglycolic acid, poly(lactic acid), poly(lactic-co-glycolic acid), polycaprolactone, poly(3-hydroxybutyric acid ), poly(ethylene glycol), polyethylene oxide, poly(ethylene glycol)-block-poly(propylene glycol)-block-poly(ethylene glycol) (Pluronic F127), polyoxyethylene-polyoxypropylene block copolymer (Pluronic F68), poloxamer, poly(hydroxymethylmethacrylate), polyvinyl alcohol, poly(vinylpyrrolidone), hyaluronic acid, heparin, heparin sulfate, polysialic acid and chitosan; L is a covalent linker; and G is represented by the following structural formula: 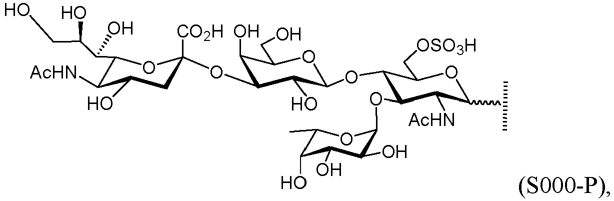 or a pharmaceutically acceptable salt thereof, wherein: the symbol 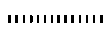 represents the point of attachment to L.

In another embodiment, the present invention is a method of treating a disorder in a subject in need thereof, the method comprising: administering to the subject a therapeutically effective amount of a compound of formula (S000-C) or a pharmaceutically acceptable salt thereof, or a composition comprising particles comprising a molecule represented by the following structural formula:

G-L-P,

or a pharmaceutically acceptable salt threof, wherein G is represented by structural formula (S000-P), wherein the disorder is influenza.

In another example embodiment, the present invention is any of the compounds listed in Table 4 or a pharmaceutically acceptable salt thereof.

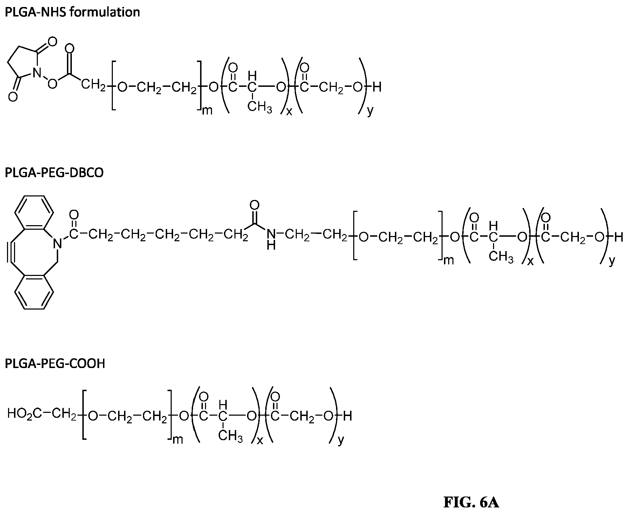

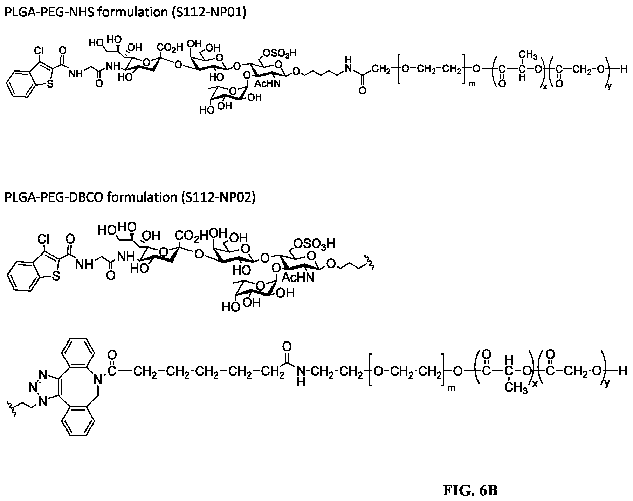

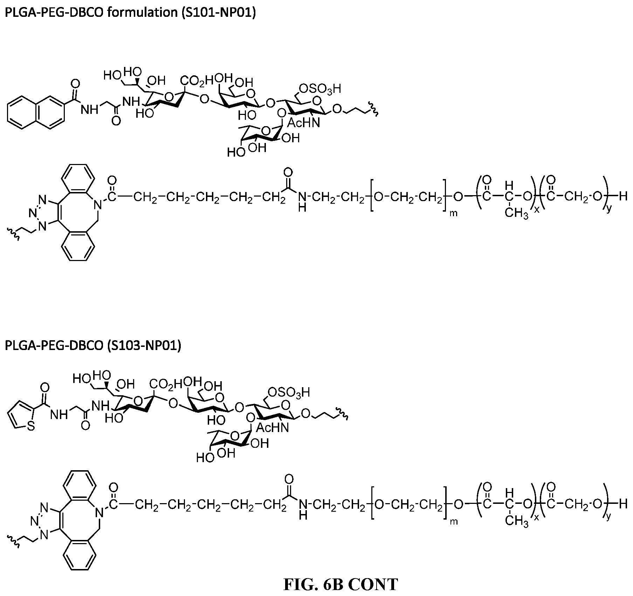

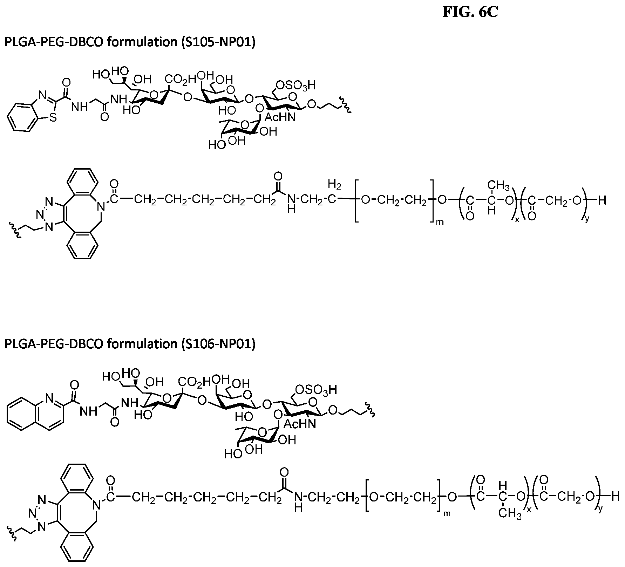

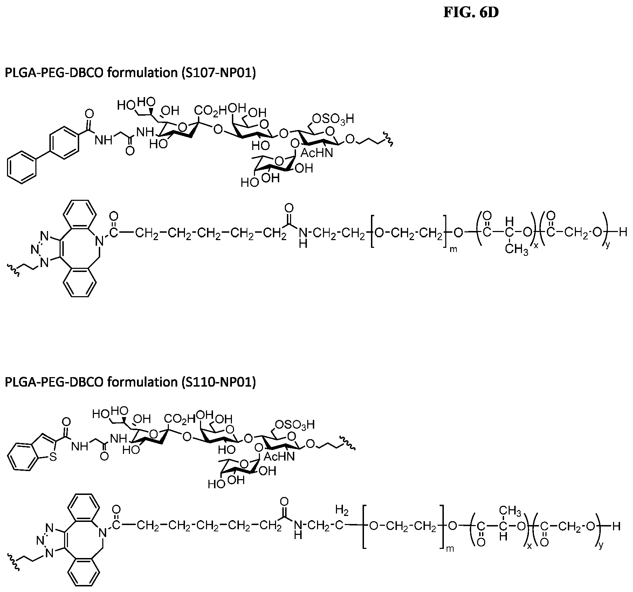

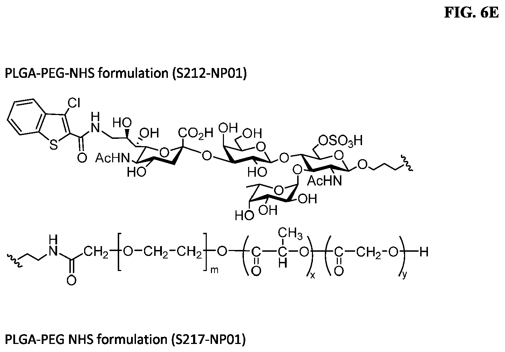

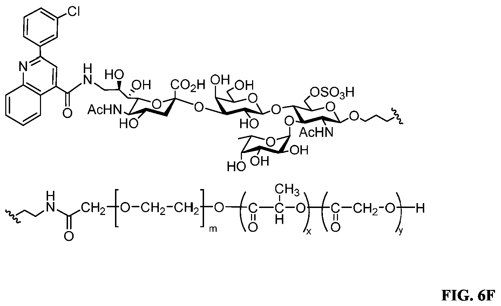

In another example embodiment, the present invention is a compound represented by any of the structural formulas listed in FIG. 6B through FIG. 6F or a pharmaceutically acceptable salt thereof.

In another embodiment, the present invention is a compound or a particle described herein for use in therapy. For example, for use in treating diseases and disorders that are responsive to modulating (e.g., amplifying, reducing or eliminating) the activity of a Siglec receptor. Such diseases and disorders include, but are not limited to, cancer, immune-related and inflammatory-related diseases and disorders, such as those described herein.

In another embodiment, the present invention is a compound or a particle described herein for the manufacture of a medicament for use in treating treating diseases and disorders that are responsive to modulating (e.g., amplifying, reducing or eliminating) the activity of a Siglec receptor. Such diseases and disorders include, but are not limited to, cancer, immune-related and inflammatory-related diseases and disorders, such as those described herein.

The compounds, particles, compositions and methods described herein can be used to treat the disorders described herein, providing addition therapies for many difficult to treat diseases and disorders.

### BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.
FIG. 1A and FIG. 1B are 2D ¹H-NMR spectra of compounds 22 and 23 of Scheme 1-6, which correspond to compounds 240 and 250 of Scheme 10-10.
FIG. 2 is a plot showing the results of tracing reactants and products of reaction Scheme 1-7 and reaction Scheme 10-11 using Hydrophilic interaction liquid chromatography/electrospray ionization mass spectrometry (HILIC-LC/ESI-MS).
FIGs. 3A through 3M are the bar plot showing the results of microarray screening of the compounds described herein for binding to the indicated Siglecs and viral particles (the vertical axis corresponds to fluorescent intensity measured in relative fluorescent unit (RFU)).
FIGs. 4A through 4E are sensograms generated by biolayer interferometry (BLI) measurements of binding constants of certain compounds of the invention, as described in Example 6.
FIG. 5 is an HSQC (600 MHz) 2D NMR spectrum of S106 Azide.
FIG. 6A through FIG. 6F shows structural formulas of the polymer-ligand conjugates.
FIG. 7 is a plot of the viability of the THP-1 monocyte derived macrophages in an MTT assay as a function of the nanoparticles concentration.
FIG. 8 is a bar plot showing suppression of TNF-alpha production in LPS-challenged THP-1 cells following incubation with nanoparticles described herein.
FIG. 9 is a bar plot showing suppression of IL-6 production in LPS-challenged THP-1 cells following incubation with nanoparticles described herein.
FIG. 10 is a bar plot showing suppression of VEGF production in LPS-challenged THP-1 cells following incubation with nanoparticles described herein.
FIG. 11(A) and FIG. 11(B) are bar plots showing the effect of the tested nanoparticle formulations on fibrocyte differentiation.
FIG. 12 is a plot of ROS production in neutrophils post 1 hr PMA treatment as a function of nanoparticle dose.
FIG. 13(A), FIG. 13(B), and FIG. 13(C) are plots of MPO (Myeloperoxidase) levels in neutrophil supernantants post PMA treatment on neutrophils at different time points in the presence of the indictaed nanoparticles.
FIG. 14(A) and FIG. 14(B) are bar plots showing a quantification of LysoBrite labeled BV-2 microglia cells by fluorescent spectrophotometer after treatment with the indicated nanoparticle formulation.
FIG. 15 is a schematic diagram of a cell surface binding assay of recombinant Siglec-9 Fc.
FIG. 16 shows the flow cytometry analysis of Siglec-9 Fc binding to PANC-1 cells +/- neuraminidase.
FIG. 17 shows the flow cytometry analysis of Siglec-9 Fc binding to PANC-1 cells +/- PLGA-based nanoparticles.
FIG. 18 shows the results of the flow cytometry analysis of Siglec-9 Fc binding to PANC-1 cells +/- PLGA-based nanoparticles.
FIG. 19 is a schematic depiction of exemplary S-series ligands coupled to FluoSpheres^{™} NeutrAvidin^{™}-labelled microspheres.
FIG. 20 shows the results of the flow cytometry analysis of Siglec-9 Fc binding to PANC-1 cells +/- FluoSpheres^{™} microspheres.
FIG. 21 shows a schematic of FLISA setup described herein.
FIG. 22 is a bar plot showing the results of the FLISA analysis of FluoSpheres^{™} microspheres binding to immobilised Siglec-9 Fc.
FIG. 23 is a schematic diagram of binding assay setup with Siglec-9-expressing HEK293T cells.
FIG. 24 is a histogram showing the results of the flow cytometry analysis of Siglec-9-PE binding to Siglec-9-expressing HEK293T cells +/- PLGA-based nanoparticles.

### DETAILED DESCRIPTION OF THE INVENTION

A description of example embodiments of the invention follows.

The present disclosure provides methods and compositions for modulating the activity of self-associated pattern recognition receptors such as, for example, Siglecs (sialic-acid-binding immunoglobulin-type lectins). The provided compositions include, for example, nanoparticles, microparticles, other polymer structures decorated with modified glycans that bind to, agonize or antagonize, self-associated molecular pattern recognition receptors and infectious associated sialic-acid binding moieties that allow entry, propagation and evasion of immune surveillance in the host.

The binding to such self-associated pattern recognition receptors, and/or agonizing or antagonizing their activity, can resolve innate, adaptive, multimodal, inflammatory, or complement-mediated immune responses, thereby providing treatment of diseases of: (1) acute inflammation such as viral, bacterial, allergen, transplant rejection, or autoimmune induced inflammation; (2) chronic inflammation such as chronic obstructive pulmonary disease, or rheumatic disorders; and (3) chronic non-resolving inflammation of the innate and adaptive form such as exudative or non- exudative macular degeneration, or Alzheimer's disease.

The provided compositions can also be used to block, or antagonize, self-associated molecular pattern recognition receptors, which allow cancer cells, infectious agents such as viral, bacterial, helminthic, parasitic or damaged-associated molecular patterns (DAMP) to evade immune surveillance, detection and clearance by the innate or adaptive immune system.

Agonizing Siglec 3, 5, 7, 8, 9, 10, 11, or 15 will dephosphorylate all the activated (phosphorylated) tyrosine kinases within a given cell resulting in intracellular shut down of activation of that particular cell. The modified oligosaccharide ligands and presentation of these ligands to the particular Siglec receptor determine its ability to agonize, antagonize, or block the receptor binding site.

Antagonizing Siglec 14 or 16, which activate inflammation via the immunoglobulin tyrosine kinase activation motif (ITAM) is another mechanism to deactivate inflammation. When Siglec 14 or 16 are agonized, ITAM is activated and the tyrosine residues within ITAM become phosphorylated by the SRC family of kinases, which creates a conformational change allowing the motif to become a docking site for proteins containing the SH2 domain. Agonizing Siglec 14 and 16 also can be used to activate inflammation for the treatment of infectious diseases or in the field of oncology.

Antagonizing or blocking the binding site of Siglec 3, 5, 7, 8, 9, 10, 11, or 15 is a method for treating conditions that agonize Siglec with self-associated molecular pattern (SAMP)-mimicking surface sialic-acid ligands to evade immune surveillance or immune activation. Conditions that use this method include cancer and infections. Cancers have been shown to express sialic-acid structures on their surface to evade immune activation of macrophages, natural killer (NK) cells, and monocytes. Streptococcus B also expresses a sialic-acid ligand on its surface that binds Siglec 7 to avoid immune attack.

Siglecs 9 has been extensively studies in both fibrocytes/Fibrosis. Siglec-9 is one of the main Siglecs in human blood monocytes/macrophages and modulates innate immunity. Siglec-9 expression in alveolar and peripheral blood neutrophil were increased in chronic obstructive pulmonary Disease (COPD) patients. These fibrocytes are unique cells possessing the proinflammatory properties of macrophages and the tissue remodeling properties of fibroblasts. Apart from fibrosis neutrophils including diabetic retinopathy models show role of Siglec 9.

Glycophorin is a sialoglycoprotein at the surface of erythrocytes that inhibits NET release/neutrophil activation via sialic acid binding to Siglec-9 within circulation. The interaction of lactoferrin with polySia increases the inhibition of NET releases. Glycophorin A, the most abundant sialoglycoproteinon erythrocytes, engaged neutrophil Siglec-9, a sialic acid-recognizing receptor known to dampen innate immune cell activation lung inflammatory models. The nanoparticles disclosed herein have been shown to be effective in suppressing the key pathways involved in both the Siglec 9 mediated disease pathogenesis.

Sialic-acid is also used as an entry point for several family of viruses such as Influenza A, Influenza B, Influenza C, SARS-CoV-1, or SARS-CoV-2. Binding receptors on these viruses can be hemagglutinin esterase (viral HE), Neuraminidase (viral N), or a viral capsid moiety such as spike protein (viral SP) that binds to sialic-acid ligands on the surface of host cells and facilitates viral entry into host cells, or CD147 which is a sialic-acid binding lectin used for infective entry by SARS-CoV-2 and Plasmodium falciparum. Binding these sialic-acid receptors with a decoy ligand can prevent virus from infecting host cells as well as prevent egress of viral particles from an infected cell.

A need exists for improved compositions and methods for agonizing sialic-acid binding self-associated pattern recognition receptors, for treatment of diseases resulting from acute, chronic, or aberrant immune system activation. A need also exists for blocking these SAMP receptors from being commandeered by cancer and infections to avoid immune surveillance and attack. The present invention provides for nanoparticles that can present ligands that will agonize, block, or antagonize a particular Siglec receptors specifically and profoundly.

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various stereoisomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions, Wiley Interscience, New York, 1981; Wilen et al., Tetrahedron 33:2725 (1977); Eliel, E.L. Stereochemistry of Carbon Compounds, McGraw-Hill, NY, 1962; and Wilen, S.H., Tables of Resolving Agents and Optical Resolutions p. 268, E.L. Eliel, Ed., Univ. of Notre Dame Press, Notre Dame, IN 1972. The invention additionally encompasses compounds as individual isomers substantially free of other isomers, and alternatively, as mixtures of various isomers.

In a formula, 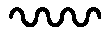 is a single bond where the stereochemistry of the moieties immediately attached thereto is not specified, --- is absent or a single bond, and-̅ -̅ -̅ -̅ or - - - - is a single or double bond.

When a range of values is listed, it is intended to encompass each value and sub-range within the range. For example, "C₁₋₆ alkyl" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The term "alkyl" refers to a radical of a straight-chain or branched saturated hydrocarbon group having a specified range of carbon atoms (e.g., a "C₁₋₁₆ alkyl" can have from 1 to 16 carbon atoms). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), propyl (C₃) *(e.g.,* n-propyl, isopropyl), butyl (C₄) *(e.g.,* n-butyl, tert-butyl, sec-butyl, iso-butyl), pentyl (C₅) *(e.g.,* n-pentyl, 3-pentanyl, amyl, neopentyl, 3- methyl-2-butanyl, tertiary amyl), and hexyl (C₆) *(e.g.,* n-hexyl). Additional examples of alkyl groups include n-heptyl (C₇), n-octyl (C₈), and the like. Unless otherwise specified, each instance of an alkyl group is independently unsubstituted (an "unsubstituted alkyl") or substituted (a "substituted alkyl") with one or more substituents (e.g., halogen, such as F). In certain embodiments, the alkyl group is an unsubstituted C₁₋₁₀ alkyl (such as unsubstituted C₁₋₆ alkyl, *e.g.,* -CH₃ (Me), unsubstituted ethyl (Et), unsubstituted propyl (Pr, e.g., unsubstituted n-propyl (n-Pr), unsubstituted isopropyl (i-Pr)), unsubstituted butyl (Bu, e.g., unsubstituted n-butyl (n-Bu), unsubstituted tert-butyl (tert-Bu or t-Bu), unsubstituted sec-butyl (sec-Bu), unsubstituted isobutyl (i-Bu)). In certain embodiments, the alkyl group is a substituted C₁₋₁₀ alkyl (such as substituted C₁₋₆ alkyl, *e.g.,* -CF₃, Bn).

The term "alkylenyl" refers to a divalent radical of a straight-chain, cyclic, or branched saturated hydrocarbon group having a specified range of carbon atoms (e.g., a "C₁₋₁₆ alkyl" can have from 1 to 16 carbon atoms). An example of alkylenyl is a methylene (-CH₂-). An alkylenyl can be substituted as described above for an alkyl.

The term "haloalkyl" is a substituted alkyl group, wherein one or more of the hydrogen atoms are independently replaced by a halogen, e.g., fluoro, bromo, chloro, or iodo. In some embodiments, the haloalkyl moiety has 1 to 8 carbon atoms ("C₁₋₈ haloalkyl"). In some embodiments, the haloalkyl moiety has 1 to 6 carbon atoms ("C₁₋₆ haloalkyl"). In some embodiments, the haloalkyl moiety has 1 to 4 carbon atoms ("C₁₋₄ haloalkyl"). In some embodiments, the haloalkyl moiety has 1 to 3 carbon atoms ("C₁₋₃ haloalkyl"). In some embodiments, the haloalkyl moiety has 1 to 2 carbon atoms ("C₁₋₂ haloalkyl"). Examples of haloalkyl groups include -CHF₂, -CH₂F, -CF₃, -CH₂CF₃, -CF₂CF₃, -CF₂CF₂CF₃, -CCl₃, -CFCl₂, - CF₂Cl, and the like.

The term "hydroxyalkyl" is a substituted alkyl group, wherein one or more of the hydrogen atoms are independently replaced by a hydroxyl. In some embodiments, the hydroxyalkyl moiety has 1 to 8 carbon atoms ("C₁₋₈ hydroxyalkyl"). In some embodiments, the hydroxyalkyl moiety has 1 to 6 carbon atoms ("C₁₋₆ hydroxyalkyl"). In some embodiments, the hydroxyalkyl moiety has 1 to 4 carbon atoms ("C₁₋₄ hydroxyalkyl"). In some embodiments, the hydroxyalkyl moiety has 1 to 3 carbon atoms ("C₁₋₃ hydroxyalkyl"). In some embodiments, the hydroxyalkyl moiety has 1 to 2 carbon atoms ("C₁₋₂ hydroxyalkyl").

The term "alkoxy" refers to an alkyl group, as defined herein, appended to the parent molecular moiety through an oxygen atom. In some embodiments, the alkoxy moiety has 1 to 8 carbon atoms ("C₁₋₈ alkoxy"). In some embodiments, the alkoxy moiety has 1 to 6 carbon atoms ("C₁₋₆ alkoxy"). In some embodiments, the alkoxy moiety has 1 to 4 carbon atoms ("C₁₋₄ alkoxy"). In some embodiments, the alkoxy moiety has 1 to 3 carbon atoms ("C₁₋₃ alkoxy"). In
some embodiments, the alkoxy moiety has 1 to 2 carbon atoms ("C₁₋₂ alkoxy"). Representative examples of alkoxy include, but are not limited to, methoxy, ethoxy, propoxy, 2-propoxy, butoxy and tert-butoxy.

The term "haloalkoxy" refers to a haloalkyl group, as defined herein, appended to the parent molecular moiety through an oxygen atom. In some embodiments, the alkoxy moiety has 1 to 8 carbon atoms ("C₁₋₈ haloalkoxy"). In some embodiments, the alkoxy moiety has 1 to 6 carbon atoms ("C₁₋₆ haloalkoxy"). In some embodiments, the alkoxy moiety has 1 to 4 carbon atoms ("C₁₋₄ haloalkoxy"). In some embodiments, the alkoxy moiety has 1 to 3 carbon atoms ("C₁₋₃ haloalkoxy"). In some embodiments, the alkoxy moiety has 1 to 2 carbon atoms ("C₁₋₂ haloalkoxy"). Representative examples of haloalkoxy include, but are not limited to, difluoromethoxy, trifluoromethoxy, and 2,2,2-trifluoroethoxy.

The term "alkoxyalkyl" is a substituted alkyl group, wherein one or more of the hydrogen atoms are independently replaced by an alkoxy group, as defined herein. In some embodiments, the alkoxyalkyl moiety has 1 to 8 carbon atoms ("C₁₋₈ alkoxyalkyl"). In some embodiments, the alkoxyalkyl moiety has 1 to 6 carbon atoms ("C₁₋₆ alkoxyalkyl"). In some embodiments, the alkoxyalkyl moiety has 1 to 4 carbon atoms ("C₁₋₄ alkoxyalkyl"). In some embodiments, the alkoxyalkyl moiety has 1 to 3 carbon atoms ("C₁₋₃ alkoxyalkyl"). In some embodiments, the alkoxyalkyl moiety has 1 to 2 carbon atoms ("C₁₋₂ alkoxyalkyl").

The term "heteroalkyl" refers to an alkyl group, which further includes at least one heteroatom *(e.g.,* 1, 2, 3, or 4 heteroatoms) selected from oxygen, nitrogen, or sulfur within (i.e., inserted between adjacent carbon atoms of) and/or placed at one or more terminal position(s) of the parent chain. In certain embodiments, a heteroalkyl group refers to a saturated group having from 1 to 20 carbon atoms and 1 or more heteroatoms within the parent chain ("heteroC₁₋₂₀ alkyl"). In some embodiments, a heteroalkyl group is a saturated group having 1 to 18 carbon atoms and 1or more heteroatoms within the parent chain ("heteroC₁₋₁₈ alkyl"). In some embodiments, a heteroalkyl group is a saturated group having 1 to 16 carbon atoms and1or more heteroatoms within the parent chain ("heteroC₁₋₁₆ alkyl"). In some embodiments, a heteroalkyl group is a saturated group having 1 to14 carbon atoms and 1 or more heteroatoms within the parent chain ("heteroC₁₋₁₄ alkyl"). In some embodiments, a heteroalkyl group is a saturated group having 1 to12 carbon atoms and 1 or more heteroatoms within the parent chain ("heteroC₁₋₁₂ alkyl"). In some embodiments, a heteroalkyl group is a saturated group having 1to 10 carbon atoms and 1or more heteroatoms within the parent chain ("heteroC₁₋₁₀ alkyl"). In some embodiments, a heteroalkyl group is a saturated group having 1 to 8 carbon atoms and 1 or more heteroatoms within the parent chain ("heteroC₁₋₈ alkyl"). In some embodiments, a heteroalkyl group is a saturated group having 1 to 6 carbon atoms and 1 or more heteroatoms within the parent chain ("heteroC₁₋₆ alkyl"). In some embodiments, a heteroalkyl group is a saturated group having 1 to 4 carbon atoms and 1 or 2 heteroatoms within the parent chain ("heteroC₁₋₄ alkyl"). In some embodiments, a heteroalkyl group is a saturated group having 1 to 3 carbon atoms and 1 heteroatom within the parent chain ("heteroC₁₋₃ alkyl"). In some embodiments, a heteroalkyl group is a saturated group having 1to 2 carbon atoms and 1 heteroatom within the parent chain ("heteroC₁₋₂ alkyl"). In some embodiments, a heteroalkyl group is a saturated group having 1carbon atom and 1heteroatom ("heteroC₁ alkyl"). In some embodiments, the heteroalkyl group defined herein is a partially unsaturated group having 1 or more heteroatoms within the parent chain and at least one unsaturated carbon, such as a carbonyl group. For example, a heteroalkyl group may comprise an amide or ester functionality in its parent chain such that one or more carbon atoms are unsaturated carbonyl groups. Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₂₀ alkyl. In certain embodiments, the heteroalkyl group is an unsubstituted heteroC₁₋₁₀ alkyl. In certain embodiments, the heteroalkyl group is a substituted heteroC₁₋₂₀ alkyl. In certain embodiments, the heteroalkyl group is an unsubstituted heteroC₁₋₁₀ alkyl.

The term "alkenyl" refers to a radical of a straight-chain or branched hydrocarbon group having from 2 to 10 carbon atoms and one or more carbon-carbon double bonds (e.g., 1, 2, 3, or 4 double bonds). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms

("C₂ alkenyl"). The one or more carbon-carbon double bonds can be internal (such as in 2- butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently unsubstituted (an "unsubstituted alkenyl") or substituted (a "substituted alkenyl") with one or more substituents. In certain embodiments, the alkenyl group is an unsubstituted C₂₋₁₀ alkenyl. In certain embodiments, the alkenyl group is a substituted C₂₋₁₀ alkenyl. In an alkenyl group, a C=C double bond for which the stereochemistry is not specified (e.g., -CH=CHCH₃ or 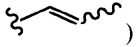 may be an (E)- or (Z)-double bond.

The term "heteroalkenyl" refers to an alkenyl group, which further includes at least one heteroatom (e.g., 1, 2, 3, or 4 heteroatoms) selected from oxygen, nitrogen, or sulfur within (i.e., inserted between adjacent carbon atoms of) and/or placed at one or more terminal position(s) of the parent chain. In certain embodiments, a heteroalkenyl group refers to a group having from 2 to 10 carbon atoms, at least one double bond, and 1 or more heteroatoms within the parent chain ("heteroC₂₋₁₀ alkenyl"). In some embodiments, a heteroalkenyl group has 2 to 9 carbon atoms at least one double bond, and 1 or more heteroatoms within the parent chain ("heteroC₂₋₉ alkenyl").

In some embodiments, a heteroalkenyl group has 2 to 8 carbon atoms, at least one double bond, and 1 or more heteroatoms within the parent chain ("heteroC₂₋₈ alkenyl"). In some embodiments, a heteroalkenyl group has 2 to 7 carbon atoms, at least one double bond, and 1 or more heteroatoms within the parent chain ("heteroC₂₋₇ alkenyl"). In some embodiments, a heteroalkenyl group has 2 to 6 carbon atoms, at least one double bond, and 1 or more heteroatoms within the parent chain ("heteroC₂₋₆ alkenyl"). In some embodiments, a heteroalkenyl group has 2 to 5 carbon atoms, at least one double bond, and 1 or 2 heteroatoms within the parent chain ("heteroC₂₋₅ alkenyl"). In some embodiments, a heteroalkenyl group has 2 to 4 carbon atoms, at least one double bond, and 1 or 2 heteroatoms within the parent chain ("heteroC₂₋₄ alkenyl"). In some embodiments, a heteroalkenyl group has 2 to 3 carbon atoms, at least one double bond, and 1 heteroatom within the parent chain ("heteroC₂₋₃ alkenyl"). In some embodiments, a heteroalkenyl group has 2 to 6 carbon atoms, at least one double bond, and 1 or 2 heteroatoms within the parent chain ("heteroC₂₋₆ alkenyl"). Unless otherwise specified, each instance of a heteroalkenyl group is independently unsubstituted (an "unsubstituted heteroalkenyl") or substituted (a "substituted heteroalkenyl") with one or more substituents. In certain embodiments, the heteroalkenyl group is an unsubstituted heteroC₂₋₁₀ alkenyl. In certain embodiments, the heteroalkenyl group is a substituted heteroC₂₋₁₀ alkenyl.

The term "alkynyl" refers to a radical of a straight-chain or branched hydrocarbon group having from 2 to 10 carbon atoms and one or more carbon-carbon triple bonds (e.g., 1, 2, 3, or 4 triple bonds) ("C2_ 10 alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C_{2_4} alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently unsubstituted (an "unsubstituted alkynyl") or substituted (a "substituted alkynyl") with one or more substituents. In certain embodiments, the alkynyl group is an unsubstituted C₂₋₁₀ alkynyl. In certain embodiments, the alkynyl group is a substituted C₂₋₁₀ alkynyl.

The term "heteroalkynyl" refers to an alkynyl group, which further includes at least one heteroatom (e.g., 1, 2, 3, or 4 heteroatoms) selected from oxygen, nitrogen, or sulfur within (i.e., inserted between adjacent carbon atoms of) and/or placed at one or more terminal position(s) of the parent chain. In certain embodiments, a heteroalkynyl group refers to a group having from 2 to 10 carbon atoms, at least one triple bond, and 1 or more heteroatoms within the parent chain ("heteroC₂₋₁₀ alkynyl"). In some embodiments, a heteroalkynyl group has 2 to 9 carbon atoms, at least one triple bond, and 1or more heteroatoms within the parent chain ("heteroC₂₋₉ alkynyl"). In some embodiments, a heteroalkynyl group has 2 to 8 carbon atoms, at least one triple bond, and 1or more heteroatoms within the parent chain ("heteroC₂₋₈ alkynyl"). In some embodiments, a heteroalkynyl group has 2 to 7 carbon atoms, at least one triple bond, and 1 or more heteroatoms within the parent chain ("heteroC₂₋₇ alkynyl"). In some embodiments, a heteroalkynyl group has 2 to 6 carbon atoms, at least one triple bond, and 1 or more heteroatoms within the parent chain ("heteroC₂₋₆ alkynyl"). In some embodiments, a heteroalkynyl group has 2 to 5 carbon atoms, at least one triple bond, and 1 or 2 heteroatoms within the parent chain ("heteroC₂₋₅ alkynyl"). In some embodiments, a heteroalkynyl group has 2 to 4 carbon atoms, at least one triple bond, and l or 2 heteroatoms within the parent chain ("heteroC₂₋₄ alkynyl"). In some embodiments, a heteroalkynyl group has 2 to 3 carbon atoms, at least one triple bond, and 1heteroatom within the parent chain ("heteroC₂₋₃ alkynyl"). In some embodiments, a heteroalkynyl group has 2 to 6 carbon atoms, at least one triple bond, and 1 or 2 heteroatoms within the parent chain ("heteroC₂₋₆ alkynyl"). Unless otherwise specified, each instance of a heteroalkynyl group is independently unsubstituted (an "unsubstituted heteroalkynyl") or substituted (a "substituted heteroalkynyl") with one or more substituents. In certain embodiments, the heteroalkynyl group is an unsubstituted heteroC₂₋₁₀ alkynyl. In certain embodiments, the heteroalkynyl group is a substituted heteroC₂₋₁₀ alkynyl.

The term "carbocyclyl" or "carbocyclic" refers to a radical of a non-aromatic cyclic hydrocarbon group having from 3 to 14 ring carbon atoms ("C₃₋₁₄ carbocyclyl") and zero heteroatoms in the non-aromatic ring system. In some embodiments, a carbocyclyl group has 3 to 10 ring carbon atoms ("C₃₋₁₀ carbocyclyl"). In some embodiments, a carbocyclyl group has 3 to 8 ring carbon atoms ("C₃₋₈ carbocyclyl"). In some embodiments, a carbocyclyl group has 3 to 7 ring carbon atoms ("C₃₋₇ carbocyclyl"). In some embodiments, a carbocyclyl group has 3 to 6 ring carbon atoms ("C₃₋₆ carbocyclyl"). In some embodiments, a carbocyclyl group has 4 to 6 ring carbon atoms ("C₄₋₆ carbocyclyl"). In some embodiments, a carbocyclyl group has 5 to 6 ring carbon atoms ("C₅₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (Cs), cyclopentenyl (C₅), cyclohexyl (C₆), cyclohexenyl (C₆), cyclohexadienyl (C₆), and the like.

Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl

(C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or polycyclic (e.g., containing a fused, bridged or spiro ring system such as a bicyclic system ("bicyclic carbocyclyl") or tricyclic system ("tricyclic carbocyclyl")) and can be saturated or can contain one or more carbon-carbon double or triple bonds. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently unsubstituted (an "unsubstituted carbocyclyl") or substituted (a "substituted carbocyclyl") with one or more substituents. In certain embodiments, the carbocyclyl group is an unsubstituted C₃₋₁₄ carbocyclyl. In certain embodiments, the carbocyclyl group is a substituted C₃₋₁₄ carbocyclyl.

In some embodiments, "carbocyclyl" is a monocyclic, saturated carbocyclyl group having from 3 to 14 ring carbon atoms ("C₃₋₁₄ cycloalkyl"). In some embodiments, a cycloalkyl group has 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 4 to 6 ring carbon atoms ("C₄₋₆ cycloalkyl"). In some embodiments, a cycloalkyl group has 5 to 6 ring carbon atoms ("C₅₋₆ cycloalkyl"). In some embodiments, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (C₆). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is an unsubstituted C₃₋₁₄ cycloalkyl. In certain embodiments, the cycloalkyl group is a substituted C₃₋₁₄ cycloalkyl.

As used herein, the term "heterocyclyl" refers to an aromatic (also referred to as a heteroaryl), unsaturated, or saturated cyclic hydrocarbon that includes at least one heteroatom in the cycle.

For example, the term "heterocyclyl" or "heterocyclic" refers to a radical of a 3- to 14-membered non-aromatic ring system having ring carbon atoms and 1 to 4 ring heteroatoms, wherein each heteroatom is independently selected from nitrogen, oxygen, and sulfur ("3-14 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or polycyclic *(e.g.,* a fused, bridged or spiro ring system such as a bicyclic system ("bicyclic heterocyclyl") or tricyclic system ("tricyclic heterocyclyl")), and can be saturated or can contain one or more carbon-carbon double or triple bonds. Heterocyclyl polycyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently unsubstituted (an "unsubstituted heterocyclyl") or substituted (a "substituted heterocyclyl") with one or more substituents. In certain embodiments, the heterocyclyl group is an unsubstituted 3-14 membered heterocyclyl. In certain embodiments, the heterocyclyl group is a substituted 3-14 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, and sulfur ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has 1 ring heteroatom selected from nitrogen, oxygen, and sulfur.

Exemplary 3-membered heterocyclyl groups containing 1 heteroatom include, without limitation, aziridinyl, oxiranyl, and thiiranyl. Exemplary 4-membered heterocyclyl groups containing 1 heteroatom include, without limitation, azetidinyl, oxetanyl, and thietanyl. Exemplary 5-membered heterocyclyl groups containing 1 heteroatom include, without limitation, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing 2 heteroatoms include, without limitation, dioxolanyl, oxathiolanyl and dithiolanyl. Exemplary 5-membered heterocyclyl groups containing 3 heteroatoms include, without limitation, triazolinyl, oxadiazolinyl, and thiadiazolinyl. Exemplary 6-membered heterocyclyl groups containing 1 heteroatom include, without limitation, piperidinyl, tetrahydropyranyl, dihydropyridinyl, and thianyl. Exemplary 6-membered heterocyclyl groups containing 2 heteroatoms include, without limitation, piperazinyl, morpholinyl, dithianyl, and dioxanyl. Exemplary 6-membered heterocyclyl groups containing 3 heteroatoms include, without limitation, triazinyl. Exemplary 7-membered heterocyclyl groups containing 1 heteroatom include, without limitation, azepanyl, oxepanyl and thiepanyl. Exemplary 8-membered heterocyclyl groups containing 1 heteroatom include, without limitation, azocanyl, oxecanyl and thiocanyl. Exemplary bicyclic heterocyclyl groups include, without limitation, indolinyl, isoindolinyl, dihydrobenzofuranyl, dihydrobenzothienyl, tetrahydrobenzothienyl, tetrahydrobenzofuranyl, tetrahydroindolyl, tetrahydroquinolinyl, tetrahydroisoquinolinyl, decahydroquinolinyl, decahydroisoquinolinyl, octahydrochromenyl, octahydroisochromenyl, decahydronaphthyridinyl, decahydro-1,8-naphthyridinyl, octahydropyrrolo[3,2-b]pyrrole, indolinyl, phthalimidyl, naphthalimidyl, chromanyl, chromenyl, IH-benzo[e][1,4]diazepinyl, 1,4,5,7-tetrahydropyrano[3,4-b]pyrrolyl, 5,6-dihydro-4H-furo[3,2-b]pyrrolyl, 6,7-dihydro-5H furo[3,2-b]pyranyl, 5,7-dihydro-4H-thieno[2,3-c]pyranyl, 2,3-dihydro-1H-pyrrolo[2,3-b]pyridinyl, 2,3-dihydrofuro[2,3-b]pyridinyl, 4,5,6,7 -tetrahydro-1H-pyrrolo[2,3-b ]pyridinyl, 4,5,6,7-tetrahydrofuro[3,2-c]pyridinyl, 4,5,6,7-tetrahydrothieno[3,2-b]pyridinyl, 1,2,3,4-tetrahydro-1,6-naphthyridinyl, and the like.

The term "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has 6 ring carbon atoms ("C₆ aryl"; *e.g.,* phenyl). In some embodiments, an aryl group has 10 ring carbon atoms ("C₁₀ aryl"; *e.g.,* naphthyl such as 1-naphthyl and 2-naphthyl). In some embodiments, an aryl group has 14 ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently unsubstituted (an "unsubstituted aryl") or substituted (a "substituted aryl") with one or more substituents. In certain embodiments, the aryl group is an unsubstituted C₆₋₁₄ aryl. In certain embodiments, the aryl group is a substituted C₆₋₁₄ aryl.

"Aralkyl" is a subset of "alkyl" and refers to an alkyl group substituted by an aryl group, wherein the point of attachment is on the alkyl moiety.

The term "heteroaryl" refers to a radical of a 5-14 membered monocyclic or polycyclic *(e.g.,* bicyclic, tricyclic) 4n+2 aromatic ring system *(e.g.,* having 6, 10, or 14 π electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl polycyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused polycyclic (aryl/heteroaryl) ring system. Polycyclic heteroaryl groups wherein one ring does not contain a heteroatom (*e.g*., indolyl, quinolinyl, carbazolyl, and the like) the point of attachment can be on either ring, *i.e.,* either the ring bearing a heteroatom (e.g., 2-indolyl) or the ring that does not contain a heteroatom (*e.g*., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently unsubstituted (an "unsubstituted heteroaryl") or substituted (a "substituted heteroaryl") with one or more substituents. In certain embodiments, the heteroaryl group is an unsubstituted 5-14 membered heteroaryl. In certain embodiments, the heteroaryl group is a substituted 5-14 membered heteroaryl.

Exemplary 5-membered heteroaryl groups containing 1 heteroatom include, without limitation, pyrrolyl, furanyl, and thiophenyl. Exemplary 5-membered heteroaryl groups containing 2 heteroatoms include, without limitation, imidazolyl, pyrazolyl, oxazolyl, isoxazolyl, thiazolyl, and isothiazolyl. Exemplary 5-membered heteroaryl groups containing 3 heteroatoms include, without limitation, triazolyl, oxadiazolyl, and thiadiazolyl. Exemplary 5-membered heteroaryl groups containing 4 heteroatoms include, without limitation, tetrazolyl. Exemplary 6-membered heteroaryl groups containing 1 heteroatom include, without limitation, pyridinyl. Exemplary 6-membered heteroaryl groups containing 2 heteroatoms include, without limitation, pyridazinyl, pyrimidinyl, and pyrazinyl. Exemplary 6-membered heteroaryl groups containing 3 or 4 heteroatoms include, without limitation, triazinyl and tetrazinyl, respectively. Exemplary 7-membered heteroaryl groups containing 1 heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl. Exemplary tricyclic heteroaryl groups include, without limitation, phenanthridinyl, dibenzofuranyl, carbazolyl, acridinyl, phenothiazinyl, phenoxazinyl, and phenazinyl.

"Heteroaralkyl" is a subset of "alkyl" and refers to an alkyl group substituted by a heteroaryl group, wherein the point of attachment is on the alkyl moiety.

Affixing the suffix "-ene" to a group indicates the group is a divalent moiety, e.g., alkylene is the divalent moiety of alkyl, alkenylene is the divalent moiety of alkenyl, alkynylene is the divalent moiety of alkynyl, heteroalkylene is the divalent moiety of heteroalkyl, heteroalkenylene is the divalent moiety of heteroalkenyl, heteroalkynylene is the divalent moiety of heteroalkynyl, carbocyclylene is the divalent moiety of carbocyclyl, heterocyclylene is the divalent moiety of heterocyclyl, arylene is the divalent moiety of aryl, and heteroarylene is the divalent moiety of heteroaryl.

A group is optionally substituted unless expressly provided otherwise. The term "optionally substituted" refers to being substituted or unsubstituted. In certain embodiments, alkyl, alkenyl, alkynyl, heteroalkyl, heteroalkenyl, heteroalkynyl, carbocyclyl, heterocyclyl, aryl, and heteroaryl groups are optionally substituted. "Optionally substituted" refers to a group which may be substituted or unsubstituted (e.g., "substituted" or "unsubstituted" alkyl, "substituted" or "unsubstituted" alkenyl, "substituted" or "unsubstituted" alkynyl, "substituted" or "unsubstituted" heteroalkyl, "substituted" or "unsubstituted" heteroalkenyl, "substituted" or "unsubstituted" heteroalkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted" means that at least one hydrogen present on a group is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds and includes any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety. The invention is not intended to be limited in any manner by the exemplary substituents described herein.

Exemplary carbon atom substituents include, but are not limited to, halogen, -CN, - NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, - SR^{aa}, -SSR^{cc}, -C(=O)R^{aa}, -CO₂H, -CHO, -C(OR^{cc})₃, -CO₂R^{aa}, -OC(=O)R^{aa}, -OCO₂R^{aa}, - C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂,-C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂,-OC(=NR^{bb})N(R^{bb})₂, -NR^{bb}C(=NR^{bb})N(R^{bb})₂, -C(=O)NR^{b}SO₂R^{aa}, -NR^{bb}SO₂R^{aa}, -SO₂N(R^{bb})₂,-SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)(R^{aa})₂, -P(=O)(OR^{cc})₂, -OP(=O)(R^{aa})₂, -OP(=O)(OR^{cc})₂, -P(=O)(N(R^{bb})₂)₂,-OP(=O)(N(R^{bb})₂)₂, -NR^{bb}P(=O)(R^{aa})₂, -NR^{bb}P(=O)(OR^{cc})₂, -NR^{bb}p(=O)(N(R^{bb})₂)₂, -P(R^{cc})₂,-P(OR^{cc})₂, -P(R^{cc})₃⁺X⁻, -P(OR^{cc})₃⁺X⁻, -P(R^{cc})₄, -P(OR^{cc})₂, -OP(R^{cc})₂, -OP(R^{cc})₃⁺X⁻, -OP(OR^{cc})₂, - OP(OR^{cc})₃⁺X⁻, -OP(R^{cc})₄, -OP(OR^{cc})₄, -B(R^{aa})₂, -B(OR^{cc})₂, -BR^{aa}(OR^{cc}), C₁₋₁₀ alkyl, C₁₋₁₀ perhaloalkyl, C₂₋₁₀ alkenyl, C_{2- 10} alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; wherein X⁻ is a counterion; or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc}; each instance of R^{aa} is, independently, selected from C₁₋₁₀ alkyl, C₁₋₁₀ haloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; each instance of R^{bb} is, independently, selected from hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, - SO₂R^{cc}, -SO₂OR^{cc}, -SOR^{aa}, -C(=S)N(R^{cc})₂, -C(=O)SR^{cc}, -C(=S)SR^{cc}, -P(=O)(R^{aa})₂, - P(=O)(OR^{cc})₂, -P(=O)(N(R^{cc})₂)₂, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; wherein X⁻ is a counterion; each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; each instance of R^{dd} is, independently, selected from halogen, -CN, - NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, - NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, - NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, - C(=S)N(R^{ff})₂, -C(=O)SR^{cc}, -C(=S)SR^{ee}, -SC(=S)SR^{ee}, -P(=O)(OR^{ee})₂, -P(=O)(R^{ee})₂, - OP(=O)(R^{ee})₂, -OP(=O)(OR^{ee})₂, C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, 3-10 membered heterocyclyl, C₆₋₁₀ aryl, 5-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S; wherein X⁻ is a counterion; each instance of R^{ee} is, independently, selected from C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups; each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, 3-10 membered heterocyclyl, C₆₋₁₀ aryl and 5-10 membered heteroaryl, or two R^{ff} groups are joined to form a 3-10 membered heterocyclyl or 5-10 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups; and each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(Cₗ₋₆ alkyl)₂, -N(Cₗ₋₆ alkyl)₃ , -NH(Cₗ₋₆ alkyl)2⁺ X⁻, - NH₂(C₁₋₆ alkyl)⁺X⁻, -NH₃⁺X⁻, -N(OC₁₋₆ alkyl)(Cₗ₋₆ alkyl), -N(OH)(Cₗ₋₆ alkyl), -NH(OH), -SH, - SC₁₋₆ alkyl, -SS(Cₗ₋₆ alkyl), -C(=O)(Cₗ₋₆ alkyl), -CO₂H, -CO₂(C₁₋₆ alkyl), -OC(=O)(Cₗ₋₆ alkyl), - OCO₂(C₁₋₆ alkyl), -C(=O)NH₂, -C(=O)N(C₁₋₆ alkyl)₂, -OC(=O)NH(C₁₋₆ alkyl), -NHC(=O)(Cₗ₋₆ alkyl), -N(Cₗ₋₆ alkyl)C(=O)( C₁₋₆ alkyl), -NHCO₂(C₁₋₆ alkyl), -NHC(=O)N(Cₗ₋₆ alkyl)₂, - NHC(=O)NH(Cₗ₋₆ alkyl), -NHC(=O)NH₂, -C(=NH)O(Cₗ₋₆ alkyl), -OC(=NH)(Cₗ₋₆ alkyl), - OC(=NH)OCₗ₋₆ alkyl, -C(=NH)N(Cₗ₋₆ alkyl)₂, -C(=NH)NH(Cₗ₋₆ alkyl), -C(=NH)NH₂, - OC(=NH)N(C₁₋₆ alkyl)₂, -OC(=NH)NH(C₁₋₆ alkyl), -OC(=NH)NH₂, -NHC(=NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, - SO₂(C₁₋₆ alkyl), -SO₂O(C₁₋₆ alkyl), -OSO₂(C₁₋₆ alkyl), -SO(C₁₋₆ alkyl), -Si(Cₗ₋₆ alkyl)₃, -OSi(Cₗ₋₆ alkyl)₃, -C(=S)N(Cₗ₋₆ alkyl)₂, -C(=S)NH(Cₗ₋₆ alkyl), -C(=S)NH₂, -C(=O)S(Cₗ₋₆ alkyl), - C(=S)SC₁₋₆ alkyl, -SC(=S)SC₁₋₆ alkyl, -P(=O)(OC₁₋₆ alkyl)₂, -P(=O)(C₁₋₆ alkyl)₂, -OP(=O)(Cₗ₋₆ alkyl)₂, -OP(=O)(OCₗ₋₆ alkyl)₂, C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

The term "halo" or "halogen" refers to fluorine (fluoro, -F), chlorine (chloro, -Cl), bromine (bromo, -Br), or iodine (iodo, -I).

The term "hydroxyl" or "hydroxy" refers to the group -OH. The term "substituted hydroxyl" or "substituted hydroxyl," by extension, refers to a hydroxyl group wherein the oxygen atom directly attached to the parent molecule is substituted with a group other than hydrogen, and includes groups selected from -OR^{aa}, -ON(R^{bb})₂, -OC(=O)SR^{aa}, -OC(=O)R^{aa}, - OCO₂R^{aa}, -OC(=O)N(R^{bb})₂, -OC(=NR^{bb})R^{aa}, -OC(=NR^{bb})OR^{aa}, -OC(=NR^{bb})N(R^{bb})₂, - OS(=O)R^{aa}, -OSO₂R^{aa}, -OSi(R^{aa})₃, -OP(R^{cc})₂, -OP(R^{cc})₃⁺X⁻, -OP(OR^{cc})₂, -OP(OR^{cc})₃⁺X⁻, - OP(=O)(R^{aa})₂, -OP(=O)(OR^{cc})₂, and -OP(=O)(N(R^{bb})₂)₂, wherein X⁻, R^{aa}, R^{bb} and R^{cc} are as defined herein.

The term "amino" refers to the group -NH₂. The term "substituted amino," by extension, refers to a monosubstituted amino, a disubstituted amino, or a trisubstituted amino. In certain embodiments, the "substituted amino" is a monosubstituted amino or a disubstituted ammino group.

The term "monosubstituted amino" refers to an amino group wherein the nitrogen atom directly attached to the parent molecule is substituted with one hydrogen and one group other than hydrogen, and includes groups selected from -NH(R^{bb}), -NHC(=O)R^{aa}, -NHCO₂R^{aa}, - NHC(=O)N(R^{bb})₂, -NHC(=NR^{bb})N(R^{bb})₂, -NHSO₂R^{aa}, -NHP(=O)(OR^{cc})₂,
and -NHP(=O)(N(R^{bb})₂)₂, wherein R^{aa}, R^{bb}, and R^{cc} are as defined herein, and wherein R^{bb} of the group -NH(R^{bb}) is not hydrogen.

The term "disubstituted amino" refers to an amino group wherein the nitrogen atom directly attached to the parent molecule is substituted with two groups other than hydrogen, and includes groups selected from -N(R^{bb})₂, -NR^{bb}C(=O)R^{aa}, -NR^{bb}CO₂R^{aa}, -NR^{bb}C(=O)N(R^{bb})₂, - NR^{bb}C(=NR^{bb})N(R^{bb})₂, -NR^{bb}SO₂R^{aa}, -NR^{bbp}(=O)(OR^{cc})₂, and -NR^{bb}P(=O)(N(R^{bb})₂)₂, wherein R^{aa}, R^{bb}, and R^{cc} are as defined herein, with the proviso that the nitrogen atom directly attached to the parent molecule is not substituted with hydrogen.

The term "trisubstituted amino" refers to an amino group wherein the nitrogen atom directly attached to the parent molecule is substituted with three groups, and includes groups selected from -N(R^{bb})₂ and -N(R^{bb})₃⁺X⁻, wherein R^{bb} and X are as defined herein.

The term "sulfonyl" refers to a group selected from -SO₂N(R^{bb})₂, -SO₂R^{aa}, and SO₂OR^{aa}, wherein R^{aa} and R^{bb} are as defined herein.

The term "sulfinyl" refers to the group -S(=O)R^{aa}, wherein R^{aa} is as defined herein.

The term "acyl" refers to a group having the general formula -C(=O)R^{X1}, - C(=O)OR^{X1}, -C(=O)-O-C(=O)R^{X1}, -C(=O)SR^{X1}, -C(=O)N(R^{X1})₂, -C(=S)R^{X1}, -C(=S)N(R^{X1})2, - C(=S)O(R^{X1}), -C(=S)S(R^{X1}), -C(=NR^{X1})R^{X1}, -C(=NR^{X1})OR^{X1}, -C(=NR^{X1})SR^{X1}, and - C(=NR^{X1})N(R^{X1})₂, wherein R^{X1} is hydrogen; halogen; substituted or unsubstituted hydroxyl; substituted or unsubstituted thiol; substituted or unsubstituted amino; substituted or unsubstituted acyl, cyclic or acyclic, substituted or unsubstituted, branched or unbranched aliphatic; cyclic or acyclic, substituted or unsubstituted, branched or unbranched heteroaliphatic; cyclic or acyclic, substituted or unsubstituted, branched or unbranched alkyl; cyclic or acyclic, substituted or unsubstituted, branched or unbranched alkenyl; substituted or unsubstituted alkynyl; substituted or unsubstituted aryl, substituted or unsubstituted heteroaryl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, mono- or di- aliphaticamino, mono- or di- heteroaliphaticamino, mono- or dialkylamino, mono- or di-heteroalkylamino, mono- or di-arylamino, or mono- or diheteroarylamino; or two R^{X1} groups taken together form a 5- to 6-membered heterocyclic ring.

Exemplary acyl groups include aldehydes (-CHO), carboxylic acids (-CO₂H), ketones, acyl halides, esters, amides, imines, carbonates, carbamates, and ureas. Acyl substituents include, but are not limited to, any of the substituents described herein, that result in the formation of a stable moiety (e.g., aliphatic, alkyl, alkenyl, alkynyl, heteroaliphatic, heterocyclic, aryl, heteroaryl, acyl, oxo, imino, thiooxo, cyano, isocyano, amino, azido, nitro, hydroxyl, thiol, halo, aliphaticamino, heteroaliphaticamino, alkylamino, heteroalkylamino, arylamino, heteroarylamino, alkylaryl, arylalkyl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, acyloxy, and the like, each of which may or may not be further substituted).

The term "carbonyl" refers a group wherein the carbon directly attached to the parent molecule is sp² hybridized, and is substituted with an oxygen, nitrogen or sulfur atom, *e.g.,* a group selected from ketones *(e.g.,* -C(=O)R^{aa}), carboxylic acids (*e.g.,* -CO₂H), aldehydes( CHO), esters *(e.g.,* -CO₂R^{aa}, -C(=O)SR^{aa}, -C(=S)SR^{aa}), amides (*e.g.,* -C(=O)N(R^{bb})₂, C(=O)NR^{bb}SO₂R^{aa}, -C(=S)N(R^{bb})₂, and imines (e.g., -C(=NR^{bb})R^{aa}, -C(=NR^{bb})OR^{aa}), C(=NR^{bb})N(R^{bb})₂, wherein R^{aa} and R^{bb} are as defined herein.

The term "oxo" refers to the group =O, and the term "thiooxo" refers to the group =S.

The term "cyano" refers to the group -CN.

The term "azide" refers to the group -N₃.

Nitrogen atoms can be substituted or unsubstituted as valency permits, and include primary, secondary, tertiary, and quaternary nitrogen atoms. Exemplary nitrogen atom substituents include, but are not limited to, hydrogen, -OH, -OR^{aa}, -N(R^{cc})₂, -CN, -C(=O)R^{aa}, - C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)(OR^{cc})₂, -P(=O)(R^{aa})₂, -P(=O)(N(R^{cc})₂)₂, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups attached to an N atom are joined to form a 3-14 membered heterocyclyl or a 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc}, and R^{dd} are as defined herein.

In certain embodiments, the substituent present on the nitrogen atom is an nitrogen protecting group (also referred to herein as an "amino protecting group"). Nitrogen protecting groups include, but are not limited to, -OH, -OR^{aa}, -N(R^{cc}h, -C(=O)R^{aa}, -C(=O)N(R^{cc})₂,

-CO₂R^{aa}, -SO₂R^{aa}, -C(=NR^{cc})R^{aa}, -C(=NR^{cc})OR^{aa}, -C(=NR^{cc})N(R^{cc})₂, -SO₂N(R^{cc})₂, - SO₂R^{cc}, -SO₂OR^{cc}, -SOR^{aa}, -C(=S)N(R^{cc})₂, -C(=O)SR^{cc}, -C(=S)SR^{cc}, C₁₋₁₀ alkyl (e.g., aralkyl, heteroaralkyl), C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl groups, wherein each alkyl, alkenyl, alkynyl, heteroalkyl, heteroalkenyl, heteroalkynyl, carbocyclyl, heterocyclyl, aralkyl, aryl, and heteroaryl is independently substituted with 0, 1, 2, 3, 4, or 5 R^{dd} groups, and wherein R^{aa}, R^{bb}, R^{cc} and R^{dd} are as defined herein. Nitrogen protecting groups are well known in the art and include those described in detail in *Protecting Groups in Organic Synthesis,* T. W. Greene and P. G. M. Wuts, 3^{rd} edition, John Wiley & Sons, 1999.

For example, nitrogen protecting groups such as amide groups *(e.g.,* -C(=O)R^{aa}) include, but are not limited to, formamide, acetamide, chloroacetamide, trichloroacetamide, trifluoroacetamide, phenylacetamide, 3-phenylpropanamide, picolinamide, 3-pyridylcarboxamide, N-benzoylphenylalanyl derivative, benzamide, p-phenylbenzamide, o nitrophenylacetamide, o-nitrophenoxyacetamide, acetoacetamide, (N'-dithiobenzyloxyacylamina)acetamide, 3-(p-hydroxypheny1)propanamide, 3-(o-nitrophen y1)propanamide, 2-methyl-2-(o-nitrophenoxy)propanamide, 2-methy1-2-(o-phenylazophenoxy )propanamide, 4-chlorobutanamide, 3-methyl-3-nitrobutanamide, onitrocinnamide, N-acetylmethionine derivative, o-nitrobenzamide and o(benzoyloxymethyl)benzamide.

Nitrogen protecting groups such as carbamate groups *(e.g.,* -C(=O)OR^{aa}) include, but are not limited to, methyl carbamate, ethyl carbamate, 9-fluorenylmethyl carbamate (Fmoc), 9-(2-sulfa)fluorenylmethy1 carbamate, 9-(2,7-dibromo)fluoroenylmethy1 carbamate, 2,7-di-t-buty1- [9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-t-BOC), 1,1-dimethyl-2,2,2-trichloroethyl carbamate (TCBOC), 1-methyl-1-(4-biphenylyl)ethyl carbamate (Bpoc), 1-(3,5-di-t-butylphenyl)-1-methylethyl carbamate (t-Bumeoc), 2-(2'- and 4'-pyridyl)ethyl carbamate (Pyoc), 2-(N,N dicyclohexylcarboxamido)ethyl carbamate, t-butyl carbamate (BOC or Boc), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, N hydroxypiperidinyl carbamate, alkyldithio carbamate, benzyl carbamate (Cbz), p-methoxybenzyl carbamate (Moz), p-nitrobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, m-chloro-p-acyloxybenzyl carbamate, p (dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, o-nitrobenzyl carbamate, 3,4-dimethoxy-6-nitrobenzyl carbamate, phenyl(o-nitrophenyl)methyl carbamate, t-amyl carbamate, S-benzyl thiocarbamate, p-cyanobenzyl carbamate, cyclobutyl carbamate, cyclohexyl carbamate, cyclopentyl carbamate, cyclopropylmethyl carbamate, p decyloxybenzyl carbamate, 2,2-dimethoxyacylvinyl carbamate, o-(N,N-dimethylcarboxamido )benzy1 carbamate, 1,1-dimethyl-3-(N,N-dimethylcarboxamido )propy1 carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, p-(p' - methoxyphenylazo )benzyl carbamate, 1-methylcyclobutyl carbamate, 1- methylcyclohexyl carbamate, 1-methyl-1-cyclopropylmethyl carbamate, 1-methyl-1-(3,5- dimethoxyphenyl)ethyl carbamate, 1-methyl-1-(p-phenylazophenyl)ethyl carbamate, 1-methyl-1- phenylethyl carbamate, 1-methyl-1-(4-pyridyl)ethyl carbamate, phenyl carbamate, p (phenylazo)benzyl carbamate, 2,4,6-tri-t-butylphenyl carbamate, 4-(trimethylammonium)benzyl carbamate, and 2,4,6-trimethylbenzyl carbamate.

Nitrogen protecting groups such as sulfonamide groups *(e.g.,* -S(=O)₂R^{aa}) include, but are not limited to, p-toluenesulfonamide (Ts), benzenesulfonamide, 2,3,6-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4- methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), -trimethylsilylethanesulfonamide (SES), 9-anthracenesulfonamide, 4-(4',8'-dimethoxynaphthylmethyl)benzenesulfonamide (DNMBS ), benzylsulfonamide, trifluoromethylsulfonamide, and phenacylsulfonamide.

Other nitrogen protecting groups include, but are not limited to, phenothiazinyl-(10) acyl derivative, N'-p-toluenesulfonylaminoacyl derivative, N'-phenylaminothioacyl derivative, N-benzoylphenylalanyl derivative, N-acetylmethionine derivative, 4,5-diphenyl-3-oxazolin-2-one, N-phthalimide, N-dithiasu^{cc}inimide (Dts), N-2,3-diphenylmaleimide, N-2,5-dimethylpyrrole, N-1,1,4,4-tetramethyldisilylazacyclopentane adduct (STABASE), 5-substituted 1,3-dimethy1-1,3,5-triazacyclohexan- 2-one, 5-substituted 1,3-dibenzyl-1 ,3,5-triazacyclohexan-2-one, 1-substituted 3,5-dinitro-4-pyridone, N-methylamine, N-allylamine, N-[2-(trimethylsilyl)ethoxy ]methylamine (SEM), N-3-acetoxypropylamine, N-(1-isopropyl-4-nitro-2-oxo-3-pyroolin-3-yl)amine, quaternary ammonium salts, N-benzylamine, N-di(4-methoxyphenyl)methylamine, N-5-dibenzosuberylamine, N-triphenylmethylamine (Tr), N-[(4-methoxyphenyl)diphenylmethyl]amine (MMTr), N-9-phenylfluorenylamine (PhF), N-2,7-dichloro-9-fluorenylmethyleneamine, N-ferrocenylmethylamino (Fern), N-2-picolylamino N'-oxide, N-1,1-dimethylthiomethyleneamine, N-benzylideneamine, N-p-methox ybenzy lideneamine, N-diphenylmethyleneamine, N-[(2-pyrid y1)mesity1]methyleneamine, N-(N' ,N'-dimethylaminomethylene)amine, N,N'-isopropylidenediamine, N-pnitrobenzylideneamine, N-salicylideneamine, N- 5-chlorosalicylideneamine, N-(5-chloro-2- hydrox yphen y1)phenylmethyleneamine, N -cyclohex ylideneamine, N-(5,5-dimethy1-3-oxo-1-cyclohexenyl)amine, N-borane derivative, N-diphenylborinic acid derivative, N [phenyl(pent^{aa}cylchromium- or tungsten)acyl]amine, N-copper chelate, N-zinc chelate, N nitroamine, N-nitrosoamine, amine N-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o nitrobenzenesulfenamide (Nps ), 2,4-dinitrobenzenesulfenamide, pentachlorobenzenesulfenamide, 2-nitro-4-methoxybenzenesulfenamide, triphenylmethylsulfenamide, and 3-nitropyridinesulfenamide (Npys). In certain embodiments, a nitrogen protecting group is benzyl (Bn), tert-butyloxycarbonyl (BOC), carbobenzyloxy (Cbz), 9-flurenylmethyloxycarbonyl (Fmoc), trifluoroacetyl, triphenylmethyl, acetyl (Ac), benzoyl (Bz), p-methoxybenzyl (PMB), 3,4-dimethoxybenzyl (DMPM), p-methoxyphenyl (PMP), 2,2,2-trichloroethyloxycarbonyl (Troc), triphenylmethyl (Tr), tosyl (Ts), brosyl (Bs), nosyl (Ns), mesyl (Ms), triflyl (Tf), or dansyl (Ds).

In certain embodiments, the substituent present on an oxygen atom is an oxygen protecting group (also referred to herein as an "hydroxyl protecting group"). Oxygen protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, -Si(R^{aa})₃, -P(R^{cc})₂, -P(R^{cc})₃⁺ X⁻, -P(OR^{cc})₂, -P(OR^{cc})3⁺X⁻, -P(=O)(R^{aa})₂, -P(=O)(OR^{cc})₂, and - P(=O)(N(R^{bb})₂)₂, wherein X⁻, R^{aa}, R^{bb}, and R^{cc} are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), methylthiomethyl (MTM), t-butylthiomethyl, (phenyldimethylsilyl)methoxymethyl (SMOM), benzyloxymethyl (BOM), p-methoxybenzyloxymethyl (PMBM), (4-methoxyphenoxy)methyl (p-AOM), guaiacolmethyl (GUM), t-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymeth yl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4- methoxytetrahydrothiopyranyl S,S-dioxide, 1-[(2-chloro-4-meth yl)phenyl]-4-methox ypiperidin-4-yl (CTMP), 1,4-dioxan-2-yl, tetrahydrofuranyl, tetrahydrothiofuranyl, 2,3,3a,4,5,6,7,7aoctahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, t-butyl, allyl, p-chlorophenyl, p-methoxyphenyl, 2,4-dinitrophenyl, benzyl (Bn), p-methoxybenzyl, 3,4-dimethoxybenzyl, onitrobenzyl, p-nitrobenzyl, p-halobenzyl, 2,6-dichlorobenzyl, p-cyanobenzyl, p-phenylbenzyl, 2- picolyl, 4-picolyl, 3-methyl-2-picolyl N-oxido, diphenylmethyl, p,p'-dinitrobenzhydryl, 5-dibenzosuberyl, triphenylmethyl, a-naphthyldiphenylmethyl, p-methoxyphenyldiphenylmethyl, di(p-methoxyphenyl)phenylmethyl, tri(p-methoxyphenyl)methyl, 4-(4'-bromophenacy loxyphen y1)diphen ylmethy1, 4,4',4"-tris(4,5-dichlorophthalimidopheny1)methy1, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9- (9-phenyl)xanthenyl, 9-(9-phenyl-10-oxo)anthryl, 1,3-benzodithiolan-2-yl, benzisothiazolyl S,S dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, t butyldimethylsilyl (TBDMS), t-butyldiphenylsilyl (TBDPS), tribenzylsilyl, tri-p-xylylsilyl, triphenylsilyl, diphenylmethylsilyl (DPMS), t-butylmethoxyphenylsilyl (TEMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3- phenylpropionate, 4-oxopentanoate (levulinate), 4,4-(ethylenedithio )pentanoate (levulinoyldithioacetal), pivaloate, adamantoate, crotonate, 4-methoxycrotonate, benzoate, p phenylbenzoate, 2,4,6-trimethylbenzoate (mesitoate), methyl carbonate, 9-fluorenylmethyl carbonate (Fmoc), ethyl carbonate, 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), isobutyl carbonate, vinyl carbonate, allyl carbonate, t-butyl carbonate (BOC or Boc), p-nitrophenyl carbonate, benzyl carbonate, p-methoxybenzyl carbonate, 3,4- dimethoxybenzyl carbonate, o-nitrobenzyl carbonate, p-nitrobenzyl carbonate, S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2- formylbenzenesulfonate, 2-(methylthiomethox y)ethy1, 4-(meth ylthiomethox y)butyrate, 2-(methylthiomethox ymethy1)benzoate, 2,6-dichloro-4-methylphenoxyacetate, 2,6-dichloro-4- ( 1,1,3,3-tetramethylbutyl)phenox yacetate, 2,4-bis( 1,1-dimethylpropy1)phenox yacetate, chlorodiphenylacetate, isobutyrate, monosu^{cc}inoate, (E)-2-methyl-2-butenoate, o(methoxyacyl)benzoate, a-naphthoate, nitrate, alkyl N,N,N',N'-tetramethylphosphorodiamidate, alkyl N-phenylcarbamate, borate, dimethylphosphinothioyl, alkyl2,4-dinitrophenylsulfenate, sulfate, methanesulfonate (mesylate), benzylsulfonate, and tosylate (Ts). In certain embodiments, an oxygen protecting group is silyl. In certain embodiments, an oxygen protecting group is t butyldiphenylsilyl (TBDPS), t-butyldimethylsilyl (TBDMS), triisoproylsilyl (TIPS), triphenylsilyl (TPS), triethylsilyl (TES), trimethylsilyl (TMS), triisopropylsiloxymethyl (TOM), acetyl (Ac), benzoyl (Bz), allyl carbonate, 2,2,2-trichloroethyl carbonate (Troc), 2- trimethylsilylethyl carbonate, methoxymethyl (MOM), 1-ethoxyethyl (EE), 2-methyoxy-2-propyl (MOP), 2,2,2-trichloroethoxyethyl, 2-methoxyethoxymethyl (MEM), 2-trimethylsilylethoxymethyl (SEM), methylthiomethyl (MTM), tetrahydropyranyl (THP), tetrahydrofuranyl (THF), p-methoxyphenyl (PMP), triphenylmethyl (Tr), methoxytrityl (MMT), dimethoxytrityl (DMT), allyl, p-methoxybenzyl (PMB), t-butyl, benzyl (Bn), allyl, or pivaloyl (Piv).

In certain embodiments, the substituent present on an oxygen atom is an oxygen protecting group (also referred to herein as an "hydroxyl protecting group"). Oxygen protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, -Si(R^{aa})₃, -P(R^{cc})₂, -P(R^{cc})₃⁺ X⁻, -P(OR^{cc})₂, -P(OR^{cc})3⁺X⁻, -P(=O)(R^{aa})₂, -P(=O)(OR^{cc})₂, and - P(=O)(N(R^{bb})₂)₂, wherein X⁻, R^{aa}, R^{bb}, and R^{cc} are as defined herein. Oxygen protecting groups are well known in the art and include those described in detail in *Protecting Groups in Organic*

*Synthesis,* T. W. Greene and P. G. M. Wuts, 3rd edition, John Wiley & Sons, 1999.

Exemplary oxygen protecting groups include, but are not limited to, methyl, methoxylmethyl (MOM), methylthiomethyl (MTM), t-butylthiomethyl, (phenyldimethylsilyl)methoxymethyl (SMOM), benzyloxymethyl (BOM), p-methoxybenzyloxymethyl (PMBM), (4-methoxyphenoxy)methyl (p-AOM), guaiacolmethyl (GUM), t-butoxymethyl, 4-pentenyloxymethyl (POM), siloxymethyl, 2-methoxyethoxymethyl (MEM), 2,2,2-trichloroethoxymethy1, bis(2-chloroethoxy)methy1, 2-(trimethylsilyl)ethoxymeth yl (SEMOR), tetrahydropyranyl (THP), 3-bromotetrahydropyranyl, tetrahydrothiopyranyl, 1-methoxycyclohexy1, 4-methoxytetrahydropyrany1 (MTHP), 4-methoxytetrahydrothiopyrany1, 4- methoxytetrahydrothiopyranyl S,S-dioxide, 1-[(2-chloro-4-meth y1)pheny1]-4-methox ypiperidin-4-yl (CTMP), 1,4-dioxan-2-yl, tetrahydrofuranyl, tetrahydrothiofuranyl, 2,3,3a,4,5,6,7,7aoctahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, t-butyl, allyl, p-chlorophenyl, p-methoxyphenyl, 2,4-dinitrophenyl, benzyl (Bn), p-methoxybenzyl, 3,4-dimethoxybenzyl, onitrobenzyl, p-nitrobenzyl, p-halobenzyl, 2,6-dichlorobenzyl, p-cyanobenzyl, p-phenylbenzyl, 2- picolyl, 4-picolyl, 3-methyl-2-picolyl N-oxido, diphenylmethyl, p,p'-dinitrobenzhydryl, 5-dibenzosuberyl, triphenylmethyl, a-naphthyldiphenylmethyl, p-methoxyphenyldiphenylmethyl, di(p-methoxyphenyl)phenylmethyl, tri(p-methoxyphenyl)methyl, 4-(4'-bromophenacy loxyphen y1)diphen ylmethy1, 4,4',4"-tris(4,5-dichlorophthalimidopheny1)methy1, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9- (9-phenyl)xanthenyl, 9-(9-phenyl-10-oxo)anthryl, 1,3-benzodithiolan-2-yl, benzisothiazolyl S,S dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, t butyldimethylsilyl (TBDMS), t-butyldiphenylsilyl (TBDPS), tribenzylsilyl, tri-p-xylylsilyl, triphenylsilyl, diphenylmethylsilyl (DPMS), t-butylmethoxyphenylsilyl (TEMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3- phenylpropionate, 4-oxopentanoate (levulinate), 4,4-(ethylenedithio )pentanoate (levulinoyldithioacetal), pivaloate, adamantoate, crotonate, 4-methoxycrotonate, benzoate, p phenylbenzoate, 2,4,6-trimethylbenzoate (mesitoate), methyl carbonate, 9-fluorenylmethyl carbonate (Fmoc), ethyl carbonate, 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), isobutyl carbonate, vinyl carbonate, allyl carbonate, t-butyl carbonate (BOC or Boc), p-nitrophenyl carbonate, benzyl carbonate, p-methoxybenzyl carbonate, 3,4- dimethoxybenzyl carbonate, o-nitrobenzyl carbonate, p-nitrobenzyl carbonate, S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2- formylbenzenesulfonate, 2-(methylthiomethox y)ethy1, 4-(meth ylthiomethox y)butyrate, 2-(methylthiomethox ymethy1)benzoate, 2,6-dichloro-4-methylphenoxyacetate, 2,6-dichloro-4- ( 1,1,3,3-tetramethylbuty1)phenox yacetate, 2,4-bis( 1,1-dimethylpropy1)phenox yacetate, chlorodiphenylacetate, isobutyrate, monosu^{cc}inoate, (E)-2-methyl-2-butenoate, o(methoxyacyl)benzoate, a-naphthoate, nitrate, alkyl N,N,N',N'-tetramethylphosphorodiamidate, alkyl N-phenylcarbamate, borate, dimethylphosphinothioyl, alkyl2,4-dinitrophenylsulfenate, sulfate, methanesulfonate (mesylate), benzylsulfonate, and tosylate (Ts). In certain embodiments, an oxygen protecting group is silyl. In certain embodiments, an oxygen protecting group is t butyldiphenylsilyl (TBDPS), t-butyldimethylsilyl (TBDMS), triisoproylsilyl (TIPS), triphenylsilyl (TPS), triethylsilyl (TES), trimethylsilyl (TMS), triisopropylsiloxymethyl (TOM), acetyl (Ac), benzoyl (Bz), allyl carbonate, 2,2,2-trichloroethyl carbonate (Troc), 2- trimethylsilylethyl carbonate, methoxymethyl (MOM), 1-ethoxyethyl (EE), 2-methyoxy-2-propyl (MOP), 2,2,2-trichloroethoxyethyl, 2-methoxyethoxymethyl (MEM), 2-trimethylsilylethoxymethyl (SEM), methylthiomethyl (MTM), tetrahydropyranyl (THP), tetrahydrofuranyl (THF), p-methoxyphenyl (PMP), triphenylmethyl (Tr), methoxytrityl (MMT), dimethoxytrityl (DMT), allyl, p-methoxybenzyl (PMB), t-butyl, benzyl (Bn), allyl, or pivaloyl (Piv).

In certain embodiments, the substituent present on a sulfur atom is a sulfur protecting group (also referred to as a "thiol protecting group"). Sulfur protecting groups include, but are not limited to, -R^{aa}, -N(R^{bb})₂, -C(=O)SR^{aa}, -C(=O)R^{aa}, -CO₂R^{aa}, -C(=O)N(R^{bb})₂, -C(=NR^{bb})R^{aa}, - C(=NR^{bb})OR^{aa}, -C(=NR^{bb})N(R^{bb})₂, -S(=O)R^{aa}, -SO₂R^{aa}, -Si(R^{aa})₃, -P(R^{cc})₂, -P(R^{cc})₃⁺X⁻, - P(OR^{cc})₂, -P(OR^{cc})₃⁺X⁻, -P(=O)(R^{aa})₂, -P(=O)(OR^{cc})₂, and -P(=O)(N(R^{bb})₂)₂, wherein R^{aa}, R^{bb} and R^{cc} are as defined herein. Sulfur protecting groups are well known in the art and include those described in detail in Protecting Groups in Organic Synthesis, T. W. Greene and P. G. M. Wuts, 3rd edition, John Wiley & Sons, 1999. In certain embodiments, a sulfur protecting group is acetamidomethyl, t-Bu, 3-nitro-2-pyridine sulfenyl, 2-pyridine-sulfenyl, or triphenylmethyl.

Additionally, the following terms are used herein:
- "Ac" refers to an acyl group;
- "Fmoc" refers to a fluorenylmethoxycarbonyl protecting group;
- "tBoc" refers to a tert-butyloxycarbonyl protecting group;
- "Cbz" refers to a benzyl chlorocarbonate protecting group;
- "Troc" refers to a trichloroethyl chloroformate protetcting group;
- "TFA" refers to a trifluoroacetamide protecting group;
- "[1,3]dioxolo group" refers to a moiety having the following structural formula

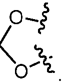

As used herein, the term "a click chemistry reagent" (interchangeably used with "click reagent" or "click moiety") refers to any of a chemical moiety that reacts with its counterpart click moiety (the two reacting moieties referred to as "a click reagent pair") via an electrocyclic addition reaction ("a click chemistry reaction" or "a click reaction") to produce a "click reaction product." Typically, click reactions take place under mild conditions in aqueous solvents, and can include any one of the following: neutral pH, ambient temperature, and low reactant concentrations. Exemplary click reagents pairs include, but are not limited to: Cu(I)-catalyzed Azide-Alkyne Click Chemistry reaction (CuAAC); Strain-promoted Azide-Alkyne Click Chemistry reaction (SPAAC), and tetrazine and alkene (trans-cyclooctene).

Any suitable click reagent pairs can be used in the present invention. Examples of click reagents include:
- reagents that include bicyclo[6.1.0]nonyne (BCN) group, for example: 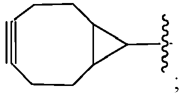
- reagents that include an alkyne, for example: 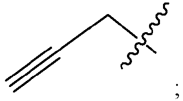
- reagents that include an azide, for example: N₃-CH₂-;
- reagents that include a Dibenzocyclooctyne (DBCO) group, for example: 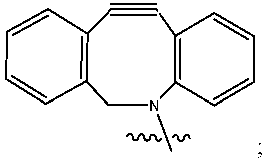
- reagents that include a trans-cyclooctene, for example: 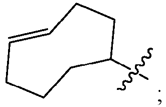
- reagents that include a tetrazine, for example:

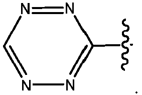

A "sialic acid" refers to neuraminic acid or any chemical modification of neuraminic acid, either naturally occurring or synthetically derived. The structural formula of neuraminic acid is reproduced below:

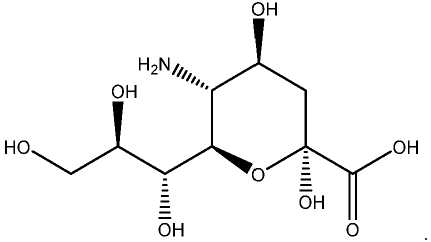

As used herein, the terms "poly(ethylene glycol)", "polyethylene oxide", and "polyoxyethylene" are used interchangeably and refer to a polymer having the following structural formula H-(O-CH₂CH₂)ₘ-OH, where *m* is the number of repeat units. The polymer molecular weight can vary from 300 g/mol to 10,000,000 g/mol.

As used herein, the term "particle" includes a microparticle and a nanoparticle, as defined herein.

As used herein, the term "particle" includes a microparticle and a nanoparticle, as defined herein.

As used herein, "subject" refers to the subject being treated according to the provided treatment methods. A subject can be human, a primate, canine, feline, bovine, equine, murine, etc. Subject also refers to those animals being used for laboratory testing.

As used herein, "nanoparticle" refers to a particle, composed of one or more polymers, whose size in nanometers (nm) includes a range of linear dimensions between 10 nanometers to 2000 nanometers. As used herein, "linear dimension" refers to the distance between any two points on the surface of a nanoparticle measured in a straight line. Nanoparticles of the present disclosure can be irregular, oblong, spindle, rod, cylindrical, pancake, discoid, spherical, biconcave, or red blood cell shaped. Linear dimension can be measured using multiple methods including but not exclusive to transmission electron microscopy or tunable resistive pulse sensing which are some of the standard means of determining nanoparticle size. One of the widely used techniques for measuring the size of nanoparticles is dynamic light scattering (DLS) that can provide the diameter and polydispersity of the nanoparticles. DLS assumes that the nanoparticles are spherical in nature, and the size of the nanoparticles are the average diameter (or radius) of such assumed spheres. In such measurements, the nanoparticles can be described to have a size range of 10 nm to 1000 nm or 1 nm to 500 nm.

As used herein, "microparticle", refers to a microscopic particle, composed of one or more polymers, whose size in micrometers (µm) includes a greatest cross-sectional width less than 1000 µm and which is greater than or equal to 1 µm.

As used herein, a "polymer" refers to a molecule(s) composed of a plurality of repeating structural units connected by covalent bonds. As used herein, a "polymer particle" refers to a solid or porous particle in contrast to the shell-like structure of liposomes and polymersomes and the relatively open structures of hydrogel particles. As used herein, a "hydrogel particle" refers to a cross-linked network of polymer chains that is absorbent but stable in an aqueous environment.

As used herein, the term "biocompatible polymer" refers to a polymer that does not undesirably interfere with biological function of tissues. The biocompatible polymer can in some instances be biodegradable, bioabsorbable, and bioerodible. Biodegradable, bioabsorbable and bioerodible, as well as degraded, eroded, and absorbed, are used interchangeably (unless the context shows otherwise) and refer to polymers and materials that are capable of being degraded or absorbed when exposed to bodily fluids such as blood, and components thereof such as enzymes, and that can be gradually resorbed, absorbed, and/or eliminated by the body.

The term "treatment" or "treating" as used herein to characterize a method or process that is aimed at (1) delaying or preventing the onset of a disease, disorder, or condition; (2) slowing down or stopping the progression, aggravation, or deterioration of one or more symptoms of the disease, disorder, or condition; (3) bringing about ameliorations of the symptoms of the disease, disorder, or condition; (4) reducing the severity or incidence of the disease, disorder, or condition; or (5) curing the disease, disorder, or condition. A treatment may be administered prior to the onset of the disease, disorder, or condition, for a prophylactic or preventive action. Alternatively, or additionally, the treatment may be administered after initiation of the disease, disorder, or condition, for a therapeutic action.

As used herein, an "effective amount" means that amount of active compound agent that elicits the desired biological response in a subject, e.g., the amount that results in treatmenmt as defined hereinabove. In one embodiment, the effective amount of a compound of the invention is from about 0.01 mg/kg/day to about 1000 mg/kg/day, from about 0.1 mg/kg/day to about 100 mg/kg/day, or from about 0.5 mg/kg/day to about 50 mg/kg/day.

### Particles

In one aspect, the present disclosure provides nanoparticles comprising polymers that provide for teathering via covalent chemical conjugation of ligands (e.g., compounds represented by structural formulas S-1, S-2, D-1, D-2, S-1-1, S-2-1, D-1-1, D-2-1, or the moieties represented by S-1A, S-2A, D-1A, D-2A also referred to herein as sialic acid-containing ligands) for presentation on the nanoparticle surface. The nanoparticles can be used to contact immune cells expressing sialic-acid-binding immunoglobulin-type lectins (Siglecs) in order to modulate inflammatory processes. It has been determined that providing ligands capable of targeting and binding to immune cells expressing sialic-acid-binding immunoglobulin-like lectins (Siglecs) can be used to modulate an inflammatory response in the targeted cells and associated environment.

Presentation of a ligand on a nanoparticle surface means that the ligands are decorated on the nanoparticle such that they are available to be bound by a Siglec receptor on a target cell, or organism. Suitably they may be provided to bind, activate or block the receptor.

A single nanoparticle may be decorated with multivalent ligands, which will allow for multivalent binding of different Siglec receptors by this single nanoparticle resulting in modulation of the inflammatory response. Nanoparticles decorated with a unique ligand can also be mixed with other ligand-decorated nanoparticles that may target different Siglec receptors, again enabling desired modulation of the inflammatory response. In some embodiments, the presentation of the ligand on the surface of a nanoparticle, or microparticle, can provide for an increased uptake of the particle by a cell of at least about two-fold, at least about three-fold, at least about four-fold, at least about five-fold, at least about six-fold, or at least about 10-fold. In some embodiments, the presentation of a ligand on the surface of nanoparticle or microparticle can decrease an inflammatory response. In a non-limiting embodiment, the decrease in an inflammatory response is over about two-fold, over about three-fold, over about four-fold, over about five-fold, over about 10-fold, over about 20-fold, over about 50-fold, over about 100-fold, over about 500-fold or over about 1000-fold.

The nanoparticle or microparticles may be used for systemic delivery or local delivery to target diseased tissues in a subject in need of treatment resulting in modulation of an inflammatory response in said subject to resolve innate and adaptive inflammation, activate innate and/or adaptive immunity when enhanced immune surveillance is desired, or reduce infectivity of infectious organisms. The targeted immune cells or virus should possess Siglec receptors or viral sialic-ligand binding regions, respectively. The activity of the innate immune system includes, for example, the cellular response of the innate immune system; the non-cellular / humoral response of the innate immune system, the complement system, the alternative complement pathway, the amplification loop of the alternative complement pathway, and/or the amplification loop of the alternative complement pathway activated by complement factor H. The activity of the adaptive immune system involves dendritic cell maturation and presentation to T cells, T-cell activation, T-cell modulation, T-cell checkpoint inhibition or activation, neutrophil NETosis, and B-cell activation. The reduction of infectivity includes reduction of viral ingress into host cells, reduction in reproduction of viral particles, or reduction in inflammatory response to the viral infection.

Several types and configurations of nanoparticles are encompassed by the present disclosure. For example, nanoparticles may be composed of a range of materials including, but not limited to, a biodegradable polymer, biocompatible polymer, a bioabsorbable polymer, or a combination thereof.

The polymer backbone of the nanoparticle, upon which the sialic-acid-containing ligands are linked, may be composed of naturally occurring polymers, such as carbohydrates or proteins, or may be composed of synthetic polymers. The polymer backbone will have a unique terminal functional group to provide for tethering of the sialicacid-containing ligand to the nanoparticle surface. The polymer backbone may first be joined with a plurality of sialic-acid ligands prior to forming the nanoparticle via chemical conjugation methods, or the polymer backbone may first be formed into a nanoparticle and then the functional groups displayed on the surface of the nanoparticle can be joined with sialicacid-containing ligands via chemical conjugation methods. Suitable nanoparticles include polymer particles and hydrogel particles.

Polymers suitable for preparing nanoparticles include, but are not limited to, Poly(lactide-*co*-glycolide)- poly(ethylene glycol) or Poly(lactide-*co*-glycolide)-*block-*poly(ethylene glycol) or Poly(lactide-co-glycolide) comprising a click chemistry functional group (e.g., an azide or an alkyne, such as DBCO, etc.) or an amine reactive ester such as succinimidyl ester. Such polymers include, but are not limited to PLGA-PEG-DBCO and PLGA-PEG-NHS). Other polymers include poly(N-acetylglucosamine) (Chitin), Chitosan, poly(3-hydroxyvalerate), poly(lactide-co-gycolide (e.g,, poly(D,L-lactide-co-glycolide), poly(L-lactide-co-glycolide)) poly(3-hydroxybutyrate), poly(4-hydroxybutyrate), poly(3-hydroxybutyrate-co-3-hydroxyvalerate), polyorthoester, polyanhydride, poly(glycolic acid), poly(glycolide), poly(lactic acid) (e.g., poly(L-lactic acid), poly(D,L-lactic acid)), poly(lactide) (e.g., poly(D,L-lactide)) , poly((D,L)Lactide)-b-Poly(ethylene glycol)-Azide, Poly(DL-lactide)-b-poly(ethylene glycol)-methyltetrazine, poly(L-lactide-co-D,L-lactide), Poly(D,L-lactide)-b-poly(ethylene glycol)-carboxylic acid, Poly(ethylene glycol) methyl ether-block-poly(lactide-coglycolide), Poly(lactide-co-glycolide)-b-poly(ethylene glycol)-b-poly(lactide-co-glycolide), Poly(lactide-co-glycolide)-b-poly(ethylene glycol)-azide, Poly(lactide-co-glycolide)-b-poly(ethylene glycol)-alkyne, Poly((D,L)Lactic acid)-b-Poly(ethylene glycol)-Azide, Poly((D,L)Lactic acid)-b-Poly(ethylene glycol)-alkyne, poly(caprolactone), Poly(caprolactone)-b-Poly(ethylene glycol), Polycaprolactone-b-poly(ethylene glycol), Poly(lactide-cocaprolactone)-b-poly(ethylene glycol)-b-poly(lactide-co-caprolactone), poly(L-lactide-cocaprolactone), poly(L-lactide-co-caprolactone), poly(D,L-lactide-co-caprolactone), poly(glycolide-co-caprolactone), Poly(DL-lactide)-b-Poly(ethylene glycol)-b-Poly(DL-lactide), poly(trimethylene carbonate), polyester amide, poly(glycolic acid-co-trimethylene carbonate), acrylate-poly(caprolactone)-b-poly(ethylene glycol)-alkyne, co-poly(ether-esters) (e.g. PEO/PLA), poly(N-isopropylacrylamide-co-acrylic acid), poly(N-isopropylacrylamide-comethoxy poly(ethylene glycol) methacrylate), polyphosphazenes, biomolecules (such as fibrin, fibrin glue, fibrinogen, cellulose, starch, collagen and hyaluronic acid, elastin and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (such as polyvinyl chloride), polyvinyl ethers (such as polyvinyl methyl ether), polyvinylidene halides (such as polyvinylidene chloride), poly(vinylidene fluoride), poly(vinylidene fluoride-co-hexafluoropropylene), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates including tyrosine-based polycarbonates, polyoxymethylenes, polyimides, polyethers, polyurethanes, rayon, rayon-triacetate, cellulose, cellulose acetate, cellulose butyrate, cellulose acetate butyrate, cellophane, cellulose nitrate, cellulose propionate, cellulose ethers, carboxymethyl cellulose, and fullerenes.

In one aspect, the nanoparticles are formed from a polycaprolactone, and in other embodiments formed of a polymer comprising polyglycolic acid, poly(lactic acid), poly(lactic-co-glycolic acid), polycaprolactone, poly(3-hydroxybutyric acid), In some embodiments, the nanoparticle may be formed from poly(lactide) (PLA), poly(glycolide)(PGA), poly lacticglycolic acid (PLGA), poly(butyl cyanoacrylate) (PBCA), or N-(2-hydroxypropyl)methacrylamide (HPMA) copolymers. In another aspect, the nanoparticles are formed from polymer such as poly(ethylene glycol), polyethylene oxide, Pluronic F127, Pluronic F68, poloxamer, poly(hydroxymethylmethacrylate), polyvinyl alcohol and poly(vinylpyrrolidone).

In some embodiments, the nanoparticles are formed from mixtures of biodegradable and nonbiodegradable polymers as block copolymers (BCPs), including a preferred embodiment of PLGA-block-PEG. A block copolymer comprises a polymer having two or more different polymer subunits linked by covalent bonds. In some embodiments the nanoparticles are formed from mixtures of biodegradable and nonbiodegradable polymers as block copolymers, including a preferred embodiment of PLGA-block-PEG. In yet another aspect, the nanoparticles are formed from naturally derived polymers in the form of hydrogel nanoparticles including formed from collagen, hyaluronic acid, heparin, heparin sulfate, chitosan, and alginate.

Methods for synthesis of nanoparticles are well known to those of skill in the art. (see, for example, Spence et al., Science Translational Medicine, 2015, 7: 303 303ra140 and references cited therein), for example, methods for synthesis of nanoparticles with known degradation rates are known to those skilled in the art, as described in U.S. Pat. No. 6,451,338 to Gregoriadis et al., U.S. Pat. No. 6,168,804 to Samuel et al. and U.S. Pat. No. 6,258,378 to Schneider et al.

Chemical linkage of the ligands described herein to the nanoparticle surface may be achieved through the use of click chemistry reactions. In such reactions, a chemical reaction occurs between a terminal functional group of a nanoparticle polymer and a terminal functional group of a ligand (referred to herein as "terminal functional group conjugate pairs") resulting in linkage of the polymer and sialicacid-containing ligand. The types of terminal functional groups found on the surface of the polymer, and its binding partner ligand, will determine the type of click chemistry reaction that is to be used to chemically link the ligand to the surface of the nanoparticles. Additionally, the selection of polymers having specific terminal functional groups can be used to control the types, density and spatial arrangement of ligand conjugate partners to be presented on the surface of the nanoparticle. In embodiments, the polymers have specific functional groups that provide chemical conjugation sites on the formed nanoparticle surface including azide, alkyne, aryl ester, amide, amine, aryl amide, aldehyde, acetyl, substituted aryl ester, alkyl ester, alkyl ketone, aryl ketone, substituted aryl ketone, ketone, alkyl halide, amnioxy, alcohols, aza-ylide, carboxylic acid, ester, amide, bicyclononyne, dihydrazide, halo-carbonyl, halosulfonyl, hydrazide, N-hydroxysuccinimide, succinimidyl ester, monofluorinated and difluorinated cyclooctynes, isothiocyanate, iodoacetamide, maleimide, methylcyclopropene, hydrazine, nitrile, nitro, phosphine, phosphazide, tertazine, methyl-tetrazine, trans-cyclooctene, strained alkynes, dibenzocyclooctyne, biarylazacyclooctynone, azadibenzylcyclooctyne, vinyl, sulfonyl ester, thioester, thiocarboxylate, thioester a sulfonyl halide, thiol, and thiolene. In a particular embodiment, the polymer is a Poly(lactide-co-glycolide)-poly(ethylene glycol) or Poly(lactide-*co*-glycolide)-*block*-poly(ethylene glycol) or Poly(lactide-co-glycolide) having at least one of the functional groups listed directly above.

Such conjugation sites provide a position for linkage of the ligands to the surface of the nanoparticle through performance of click chemistry reactions. In one example, the nanoparticle is formed of a PLGA-PEG polymer with an azide or alkyne terminal functional group. In a non-limiting embodiment, blends of different polymers having different terminal functional groups may be used. Such polymers include, for example, PLGA-PEG-alkyne, PLGA-PEG-ester and PLGA-PEG-DBCO. In a specific aspect, a blend of PLGA-PEG-alkyne and PLGA-PEG-carboxylic acid may be prepared as nanoparticles. In another specific aspect, a blend of PLGA-PEG-akyne and PLGA-PEG-ester may be prepared as nanoparticles. In another specific embodiment, a blend of PLGA-PEG-DBCO and PLGA-PEG-carboxylic acid may be prepared as nanoparticles. In another specific aspect, a blend of PLGA-PEG-DBCO and PLGA-PEG-ester may be prepared as nanoparticles.

Although PLGA polymers can possess free terminal alkyne groups, many of these can be buried in the particle matrix and not be available for binding on the surface of the particle. In some embodiments, more alkyne groups may be introduced to the particle by providing a second polymer or copolymer surfactant or coating in addition to the first PGLA polymer or copolymer of the particle. Suitably the second polymer or copolymer can be branched or linear and can have a plurality of terminal alkyl groups wherein an alkyl group contains only carbon and hydrogen and forms the homologous series with the general formula CnH2n+1. In other embodiments, the sialic-acid-containing ligands can be attached to the particle, for example a polymeric nanoparticle, via a covalent linkage.

In other embodiments, the ligands comprise terminal functional groups (i.e., conjugation sites) that provide for tethering at the nanoparticle surface. Such terminal functional groups include azide, alkyne, aryl ester, amide, amine, aryl amide, aldehyde, acetyl, substituted aryl ester, alkyl ester, alkyl ketone, aryl ketone, substituted aryl ketone, ketone, alkyl halide, amnioxy, alcohols, aza-ylide, carboxylic acid, ester, amide, bicyclononyne, dihydrazide, halo-carbonyl, halosulfonyl, hydrazide, N-hydroxysuccinimide, norbornene, oxanorbornadiene, succinimidyl ester, isothiocyanate, iodoacetamide, monofluorinated and difluorinated cyclooctynes, maleimide, methylcyclopropene, isocyanopropanoate, hydrazine, nitrile, nitro, phosphine, phosphazide, tertazine, methyl-tetrazine, trans-cyclooctene, strained alkynes, dibenzocyclooctyne, biarylazacyclooctynone, propargyl, isocyanide, azadibenzylcyclooctyne, vinyl, sulfonyl ester, thioester, thiocarboxylate, thioester a sulfonyl halide, thiol, and thiolene. Such conjugation sites provide a position for linkage of the ligands to the surface of the nanoparticle through performance of click chemistry reactions.

In one embodiment, click chemistry reactions are employed for linkage of the ligands to the nanoparticle surface. Such click chemistry reactions are characterized as a class of biocompatible small molecule reactions commonly used for bioconjugation, which is employed in chemical ligation to modify other molecules, biomolecules, nanoparticles, and other surfaces. In general, click chemistry reactions possess the following properties: modularity, insensitivity to solvent parameters, high chemical yields, insensitivity towards oxygen and water, regiospecificity and stereospecificity, and a large thermodynamic driving force (>20 kcal/mol) to favor a reaction with a single reaction product. Click chemistry reactions provide a high reaction specificity giving control of both regio- and stereo- specificity. The reaction specificity is of particular usefulness for achieving the desired presentation of the sialic-acid ligands on the surface of the nanoparticle, thereby permitting optimal binding of the nanoparticle to immune cell Siglec receptors. The bonds formed by the click reactions during conjugation provide accessibility to highly stable covalent bonds between the sialic-acid ligand and the nanoparticle, which do not undergo rearrangement or reaction or result in degradation or hydrolysis in biological conditions.

A variety of different click chemistry reactions may be used to link the ligand to the surface of the nanoparticle. The use of such click chemistry reactions provides a controlled reaction medium for generation of nanoparticles with desired sialic-acid ligand density and spatial arrangement. The density and spatial arrangements of the sialic-acid ligands on the nanoparticle surface can be controlled, for example, by controlling the amounts of polymer with functional groups used forming the nanoparticles, the polymer molecular weight, polymer density, the number of functional groups per polymer, solvent, types of functional groups, concentration of ligands, types of click chemistry employed and type of click chemistry conjugate pairs.

In various embodiments, an average molecular weight of a polymer, e.g., PEG, PLGA, PEG-PLGA block copolymer, can be determined by any of the methods known in the art, such as anion-exchaneg chromatography, gel permeation chromatography, viscosity measurements, among others.

Click chemistry reactions used for tethering of the ligand to the polymer are well known to those of skill in the art and include, for example, Huisgen 1,3-dipolar cycloaddition, copper(I)-catalyzed azide-alkyne cycloaddition (CuAAC) yielding 1,3-substitued products, ruthenium-catalyzed alkyne-azide cycloaddition (RuAAC) yielding 1,5-substituted triazoles, strain promoted alkyne azide cycloaddition (SPAAC) yielding 1,4-substituted products, strain-promoted alkyne-nitrone cycloaddition, alkene and tetrazine inverse-demand Diels-Alder, tetrazine trans-cyclooctene ligation, thiol-ene reaction, thiol-yne reaction, Staudinger reaction, [4+1] cycloaddition, quadricyclane ligation [2+2+2] cycloaddition, norbornene cycloaddition, and alkene tetrazole photoclick reaction.

The tethering of ligands to the surface of the nanoparticles is performed in such a way so as to provide presentation of the ligand for maximum binding affinity to the Siglec receptors expressed on the surface of immune cells or sialic-acid ligand receptor expressed on the surface of viral particles. The ligand density can be controlled to provide the desired multivalent or polyvalent ligand interactions with the Siglec receptors when contacting the immune cells, as such interactions are correlated with a desired cellular immune response. Multivalent or polyvalent ligand-receptor interactions may be controlled based upon the density of the ligands provided on the nanoparticle surface, and this density can influence the response elicited by the immune cells upon contact.

As disclosed, the use of click chemistry reactions requires that both polymers of the nanoparticle and the sialic-acid ligand be functionalized to permit the desired conjugation of the ligand to the nanoparticle surface. In one embodiment, the terminal alkyne is presented on the nanoparticle surface for covalent chemical conjugation with an azide-presenting sialic-acid ligand through performance of a copper(I)- catalyzed azide - alkyne (CuAAC) reaction; a copper-free reaction; a atrain-promoted azide-alkyne (SPAAC) reaction; a tetrazine-alkene ligation reaction, or a trans-cyclooctene (TCO)-tetrazine reaction. The azide functional group can be added to the ligand using for example, sialytransferase ST8SIA4.

In one aspect, Ligand/Nanoparticle conjugate pairs can be prepared with copper(I) azide-alkyne cycloaddition with ligands with azide and nanoparticles with alkyne functional groups, or with ligands with alkynes and nanoparticles with azide functional groups. Ligands with dibenzylcyclooctyne, difluorooctyne, or biarylazacyclooctynone can be reacted with azides via SPACC. Ligands with trans-cyclooctene can be reacted with nanoparticles having tetrazine functional groups.

The nanoparticles can be prepared by mixing Poly(D,L-lactide-co-glycolide-COOH)-PEG-COOH(PLGA 10,000 Da-PEG-COOH 5000Da) and Poly(lactide-co-glycolide)-b-Poly(ethylene glycol)-alkyne (PLGA-PEG-alkyne; 10,000 Da PLGA : 1,000 PEG Da) at a 75:25 (w/w) ratio of PLGA-PEG-COOH:PLGA-alkyne (DBCO). This 75:25 ratio is one embodiment of the density of alkyne functional groups on the nanoparticle surface. Other ratios of PLGA-PEG-COOH to PLGA-PEG-Alkyne (DBCO) used for nanoparticle preparation are 95:5, 90:10, 85:15, 80:20, 70:30, 65:35, 60:40, 55:45, 50:50. The ratio of PLGA-PEG-COOH to PLGA-PEG-Alkyne (DBCO) is designed to provide sufficient space between the functional groups to permit efficient conjugation of the polymer ligands and allow for the desired ligand density to be achieved.

Nanoparticles can be prepared comprising one or more polymers possessing different click chemistry functional groups for pairing with their sialic acid-containing ligand conjugation partner thereby allowing for the presentation of one or more types of ligands, with different densities and/or spatial arrangement, on the nanoparticle surface. Use of different conjugate pairs can be designed into the nanoparticle by employing one or more nanoparticle polymer/ligand pair.

By having different click chemistry functional groups available on the nanoparticle surface, different types of ligands can be conjugated to the surface of the nanoparticles. The density of the different functional groups can be controlled by the ratio of the different polymers to one another, the concentration of the polymers, and the type of click chemistry conjugate pairs, the type of click chemistry reactions, and the size and shape of the sialic acid-containing ligand. The number of different ligands that can be presented on the surface can be determined by those skilled in the art. In a non-limiting embodiment, the number of different ligands present on the nanoparticle surface is in the range of 1 to 20. The number of different ligands include, for example, is 1, 2, 3, 4, 5, 6, 7, 8, 9, 10, 11, 12, 13, 14, 15, 16, 17, 18, 19, or 20. In another embodiment, the number of different ligands present on the nanoparticle surface is in the range of 2 to 20. The number of different ligands include, for example, is 2, 3, 4, 5, 6, 7, 8, 9, 10, 11, 12, 13, 14, 15, 16, 17, 18, 19, or 20. In another embodiment, the nanoparticle will comprise at least two different ligands. In another embodiment, the nanoparticle will comprise at least three different ligands. In another embodiment, the nanoparticle will comprise at least four different ligands. In another embodiment, the nanoparticle will comprise at least five different ligands.

In general, the density of the functional groups on the nanoparticle surface dictates the maximum ligand density that can be tethered to the surface of the nanoparticle via covalent chemical conjugation via click chemistry. The ligand density can be controlled and quantified in terms of the number of functional groups per square nanometer of surface area.

The density of the ligands on the nanoparticle surface can be controlled by several methods including chemical conjugation techniques, ligand density on the polymer, ligand type, solvent, pH, and ionic strength. Ligand density on the surface of the nanoparticles can be tuned such that contacting immune cells with such nanoparticles results in an immune-modulating response, including an anti-inflammatory biological response. Control of the ligand density can also be used to modulate the magnitude of the desired anti-inflammatory response.

In some embodiments, the sialic acid-containing ligands can be presented on the nanoparticle in groups of at least 2, at least 5, at least 10, at least 15, at least 20 or at least 25, at least 50, at least 100, at least 200, or at least 400. In some embodiments, the sialic acid - containing ligands can be spaced on the surface of the nanoparticle such that they or the nanoparticle can bind to more than one Siglec receptor. In some embodiments, the sialic acid - containing ligandcan be spaced on the surface of the nanoparticle such that they or the nanoparticle can bind to multiple Siglec receptors presented on individual cell types, which may vary in the quantity of Siglec receptors presented on their plasma membrane.

In some embodiments the nanoparticle can comprise a polymer that includes a sialic acid-containing ligand at a concentration in the range 0.05 nmol/mg of sialic acid-containing ligand to nanoparticles to 250 nmol/mg of sialicacid-containing ligand to nanoparticles, preferably 0.5 nmol/mg to 25 nmol/mg, and most preferably 0.5 to 15 nmol of sialicacid-containing ligand per mg of nanoparticle. In embodiments, a device can be coated with such a nanoparticle. In alternative embodiments a device can be formed from a polymer, for example wherein the device is a microparticle or nanoparticle, wherein sialicacid-containing ligand is provided in the polymer at a concentration in the range 0.05 nmol/mg of sialicacid-containing ligand to nanoparticle to 250 nmol /mg of sialic-acid to nanoparticle, preferably 1 nmol/mg to 25 nmol/mg, and most preferably 2 to 15 nmol of sialicacid-contaning ligand per mg of nanoparticle.

In some embodiments, a nanoparticle can have a greatest cross-sectional width or diameter of less than about 1000 nm, less than about 500 nm, less than about 250 nm or less than about 200 nm. In embodiments, a nanoparticle can have a width greater than about 1 nm, greater than about 10 nm, greater than about 50 nm, or greater than about 100 nm. In embodiments, a nanoparticle coated with sialic-acid or a sialic-acid analog can have a greatest cross-sectional width or diameter in the range of about 130 nm to about 170 nm, more preferably a width of about 150 nm. In embodiments these range of sizes can be average widths of nanoparticles. In embodiments, at least 80% of the nanoparticles live within a disclosed range.

Suitably, in some embodiments around at least 80%, more preferably at least 90% of the particles have a greatest cross-sectional width between 130 nm to 170 nm. In embodiments, the particles can have an average greatest cross-sectional width of 150 nm with the particles having no width greater or less than a value not within one standard deviation of 150 nm. In some embodiments, the nanoparticle can have a volume equal to that of a sphere with a diameter between 10 nm to 500 nm, suitably between 50 nm to 250 nm, or 100 nm to 200 nm, or 130 nm to 170 nm.

In one example, the nanoparticle can have a volume equal to that of a sphere with a diameter of about 100 nm.

In another aspect of the invention, the linkage of the nanoparticles with ligands provides a means for the nanoparticles to evade the immune system, i.e., opsonization and phagocytosis via the reticuloendothelial system (RES). It is known that PEGylation of nanoparticles, i.e., the coating of nanoparticles with polyethylene glycol, provides barrier of protection from detection by immune cells. However, PEG has disadvantages of toxicity, immunogenicity, reduced cellular uptake, reduced binding, and nonbiodegradable or bioresorbable properties. Coating of nanoparticles with sialic acid-containing ligands overcomes the disadvantages of PEG, and provides for a natural, non-immunogenic nanoparticle coating that can evade the RES and immune detection. Therefore, the nanoparticles disclosed herein possess the ability to evade immune detection and mitigate immunogenic response.

The nanoparticles, or microparticles, disclosed herein may further comprise a bioactive agent encapsulated within, adhered to the surface of, or integrated into the structure of said nanoparticles. For example, the nanoparticle can further comprise at least one of an antibiotic, an anti-viral agent, an anti-inflammatory, a cytokine, a cytokine inhibitor, an immunomodulator, an immunotoxin, an anti-angiogenic agent, an anti-hypertensive agent, an anti-edema agent, a radiosensitizer, an oligonucleotide comprising DNA or RNA, a peptide, an anti-cancer agent, or any combination thereof. Methods of preparing nanoparticles that include a bioactive agent encapsulated within, adhered to a surface of, or integrated into the structure of the nanoparticle are known to those skilled in the art.

### Modified Oligosaccharides

Disclosed herein are modified oligosaccharides generally referred to as S-series (or S-1XX and S-2XX) compounds and D-series (or D-1XX and D-2XX) compounds, and also referred to herein as sialic acid-containing ligands. The S-sereis compounds are the compounds represented by the following general structural formulas (S-1) (or S-1XX compounds), or (S-2) (or S-2XX compounds), or a pharmaceutically acceptable salt thereof. The D-series compounds are the compounds represented by the general structural formulas (D-1) (or D-1XX compounds) or (D-2) (or D-2XX compounds) or a pharmaceutically acceptable salt thereof.

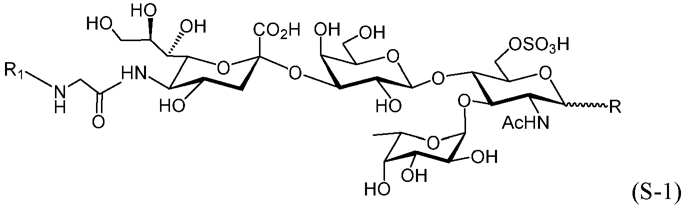

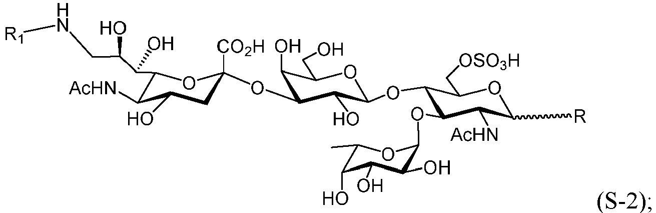

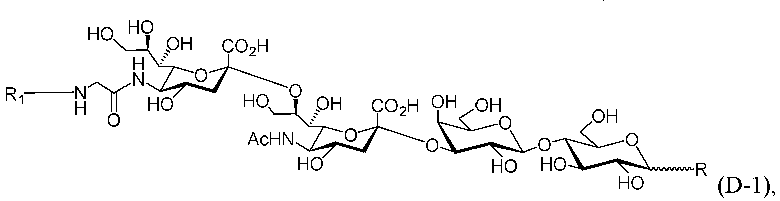

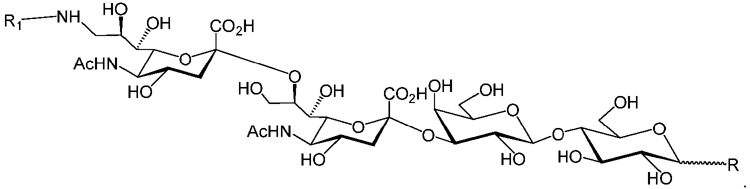

The values and example values of variables R and R₁ in formulas (S-1), (S-2), (D-1), and (D-2) are defined hereinbelow.

Example compounds of the S- and D-series and the synthesis thereof are described below in Examples 1A, 1B and 2.

### Conjugation of Oligosaccharides to Polymers and Production of Nanoparticles

Also described herein are nanoparticles comprising a polymer conjugated to an oligosaccharide described above, having a general structural formula

G-L-P,

wherein P is a biocompatible polymer and G is any one of the moieties (also referred to herein as sialic acid-containing ligands) represented by the following structural moieties: 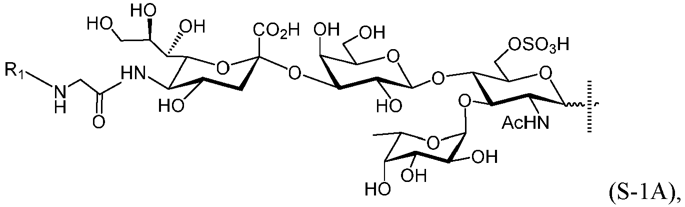 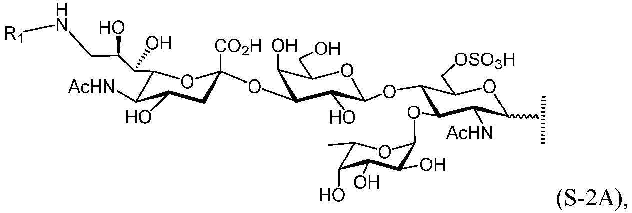 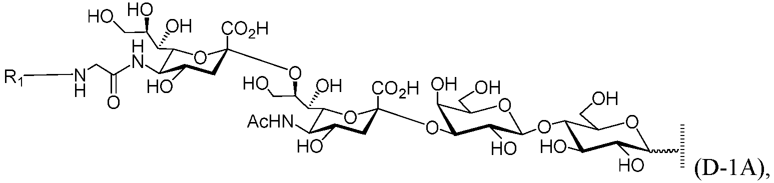 or 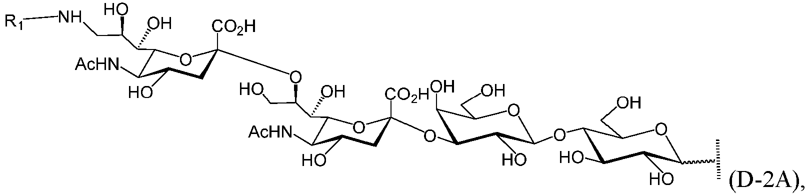 or a pharmaceutically acceptable salt thereof. The values and example values of variables R, R₁, and L in formulas (S-1A), (S-2A), (D-1A), and (D-2A) are described hereinbelow.

Example compounds of the conjugated oligoglycans of formulas (S-1A), (S-2A), (D-1A), (D-2A), and the synthesis thereof are described below in Example 3.

### Indications

In various example embodiments, the compounds and particles described herein are useful in methods of treating diseases and disorders that are responsive to modulating (e.g., amplifying, reducing or eliminating) the activity of a Siglec receptor. Such diseases and disorders include, but are not limited to, cancer, immune-related and inflammatory-related diseases and disorders. Such diseases and disorders include, but are not limited to a breast cancer, non-small cell lung cancer (NSCLC), prostate cancer, colorectal cancer, melanoma, pancreatic cancer, and myelofibrosis, diabetic retinopathy, idiopathic pulmonary lung fibrosis, liver fibrosis, sickle cell anemia, and acute respiratory distress syndrome (ARDS).

In further embodiments, the present disclosure provides for a method of treating immune-related and inflammatory-related diseases, including, but not limited to, dry and wet macular degeneration, retinal vascular disease, diabetic retinopathy, diabetic macular edema, cystoid macular edema, proliferative diabetic retinopathy, proliferative vitreoretinopathy, dry eye, allergic conjunctivitis, rheumatoid arthritis, inflammatory arthritis, lupus, nephritis, immune complex nephropathy, allergic esophagitis, allergic gastritis, hepatitis, fibrotic diseases of the liver, idiopathic pulmonary fibrosis, acute respiratory distress syndrome, sepsis, bacterial and viral infections, influenza, SARS-CoV-1 and SARS-CoV-2, HIV/AIDS, Group B streptococcal infection, Neisseria infection, cancers involving solid organs, Non-small cell lung cancer, Colorectal cancer, prostate cancer, uveal melanoma, malignant melanoma-skin, Myleofibrosis or hematopoietic cancers, in each case in an afflicted subject through the administration of such pharmaceutical compositions. The present disclosure provides a method of modulating an inflammatory response in a cell, the method comprising: providing sialic acid-containing ligands to a cell, wherein the sialic acid-containing ligands are presented on a nanoparticle such that a pro-inflammatory response in a cell is suppressed or an anti-inflammatory response in increased in the cell. In certain embodiments, the method provides for the suppression of a proinflammatory response. In alternative embodiments, the method provides for the increase in an anti-inflammatory response. In some embodiments, the method provides for the enhancement of a pro-inflammatory response in situations such as infections, or cancer.

Accordingly, a method of treating an inflammatory disease, in a subject in need thereof, is provided, said method comprising administering to a subject sialic-acid or an analog thereof, wherein the sialic acid-containing ligand is presented on a nanoparticle such that a proinflammatory immune response is suppressed or an anti-inflammatory immune response is increased in the subject.

Said method may comprise: identifying a subject having a pro-inflammatory immune response and/or suffering from a disorder associated with or caused by a pro-inflammatory immune response or at risk of developing a pro-inflammatory immune response or a disorder associated with or caused by a pro-inflammatory immune response; administering to a subject sialic acid-containing ligands, wherein the sialic-acid-containing ligandsare presented on a nanoparticle.

In specific embodiments, the method can be used to treat a subject with pulmonary disease, including inflammatory and non-inflammatory conditions of the lung, but not exclusive to tuberculosis, chronic obstructive pulmonary disorder (COPD), asthma, acute lung injury, acute respiratory distress syndrome, cystic fibrosis, bronchiectasis, pulmonary fibrosis interstitial lung disease, pulmonary vascular disease, influenza, viral pneumonia, bacterial pneumonia, allergic bronchitis, nonallergic bronchitis, rhinitis, and fibro sing alveolitis.

In some embodiments, the method can be used for the treatment of rheumatic diseases including but not exclusive to rheumatoid arthritis, fibromyalgia, systemic lupus erythematosus, systemic sclerosis (scleroderma), psoriatic arthritis, ankylosing spondylitis, sjogrens syndrome, polymyalgia rehumatica, gout, osteoarthritis, infectious arthritis, and juvenile idiopathic arthritis.

In some embodiments, the method can be used for the treatment of gastrointestinal inflammation including but not exclusive to Crohn's disease, ulcerative colitis, irritable bowel syndrome, celiac disease, diverticulitis, gastroesophageal reflux, lactose intolerance, peptic ulcer, cholecystitis, gastritis, colitis, pancreatitis, autoimmune hepatitis, hepatitis, infectious hepatitis, and pancreatitis.

In some embodiments, the method can be used for the treatment of cardiovascular diseases including but not exclusive to septic shock, atherosclerosis, diastolic dysfunction, heart failure, cardiac fibrosis, coxsackie myocarditis, congenital heart block, autoimmune myocarditis, giant cell myocarditis, and inflammation.

In some embodiments, the method can be used for the treatment of renal inflammation including but not exclusive to kidney transplant rejection, glomerulonephritis, acute nephritis, cystitis, prostatitis diabetic nephritis, diabetic kidney disease, and urinary tract infections.

In some embodiments, the method can be used for the treatment of dermatologic inflammation including but not exclusive to dermatitis, eczema, inflammatory rashes, scleroderma, keloid, acne, sarcoidosis, tinea cruris, tinea corporis, tinea pedis, tinea capitis, tinea unguium, rosacea, vitiligo, lichen sclerosis, autoimmune urticaria, dermatomyositis, and hidradenitis suppurativa.

In some embodiments, the method can be used for the treatment of neurological inflammation and degeneration including but not exclusive to neuromyelitis, multiple sclerosis, encephalitis, neuro sarcoid, Alzheimer's, amyotrophic lateral sclerosis, and Huntington's chorea

In some embodiments, the method can be used for the treatment of autoimmune inflammation including but not exclusive to diabetes, SLE, multiple sclerosis, sjogrens syndrome, Addison's disease, Graves Disease, Hashimotos thyroiditis, myasthenia gravis, autoimmune vasculitis, celiac disease, pernicious anemia, alopecia areata, autoimmune hepatitis, autoimmune angioedema, autoimmune encephalomyelitis, autoimmune inner ear disease, Guillain barre, Kawasaki disease, lambert-eaton syndrome, Vogt-Koyanagi- Harada Syndrome, systemic vasculitis, giant cell arteritis, sarcoidosis, and polyarteritis nodosa,

In some embodiments, the method can be used for the treatment of viral inflammation including but not exclusive to influenza A, B, C, SARS-CoV1, SARS-CoV2, Newcastle Disease, Sendai virus, Polyomavirus, HIV, Flavivirus, Caclivirus, Herpes virus, Picoronovirus, and Coronavirus.

In some embodiments, the method can be used for the treatment of fungal inflammation including but not exclusive to fungemia, fungal abscess, fungal keratitis, candidiasis, tinea pedis, and tinea cruris.

In some embodiments, the method can be used for the treatment of parasitic inflammation including but not exclusive to amoebiasis, giardiasis, toxoplasmosis, and toxocara.

In some embodiments, the method can be used for the treatment of a fibrotic disease including but not exclusive to idiopathic pulmonary fibrosis, myelofibrosis, hepatic fibrosis, cardiac fibrosis with dystolic dysfunction and CHF, kidney fibrosis, retinal fibrosis, dermal fibrosis, and scarring,

In some embodiments, the method can be used for the treatment of acute lifethreatening inflammation including but not exclusive to sepsis and cytokine storm. In a specific embodiment, provided are methods of treating a plurality of ocular inflammatory diseases such as macular degeneration, uveitis, optic neuritis, neuromyelitis, and inflammation arising from infections of the eye, eye exposure to drugs and toxins, and general immune disorders including autoimmune disorders. In a non-limiting embodiment, provides useful methods are provided for preventing, treating, or ameliorating a macular degeneration such as dry macular degeneration, wet macular degeneration, geographic atrophy, intermediate macular degeneration, and age-related macular degeneration in a patient. The methods of treating, preventing, or ameliorating ocular inflammation, including macular degeneration, comprise administering a composition of sialic-acid ligand nanoparticles to a patient suffering from, or a risk of developing, ocular inflammation such as macular degeneration.

In some embodiments, an ophthalmic preparation is provided as an eye drop, an eye ointment or an ophthalmic injection. For an ophthalmic injection, intravitreous or subconjunctival injection, may be used to administer the nanoparticles.

Co-administration of additional compounds having applications in methods to treat, prevent or ameliorate a macular degeneration may be co-administered in conjunction with the nanoparticle containing pharmaceutical compositions used for treating macular degeneration. For example, anti-angiogenic pharmaceuticals for the treatment of wet age-related macular degeneration such as pegaptanib sodium, ranibizumab, bevacizumab, aflibrecept and brolucizumab can be used as a combination. While particular embodiments of the present disclosure have been shown and described, it will be obvious to those skilled in the art that changes and modifications can be made without departing from the disclosure in its broader aspects. Therefore, the appended claims are to encompass within their scope all such changes and modifications as fall within the true spirit and scope of this disclosure.

In example embodiments, the compounds described herein can be used in methods of treatment of the following disorders and conditions:

Ophthalmic Diseases, such as Age-macular degeneration, Dry Eyes, Diabetic Retinopathy, Allergic Ocular Conditions, and Ocular Fibrosis.

Neurological diseases, such as Alzheimer "Neuroinflammation/degeneration, ALS, Multiple Sclerosis, Neuropsychiatry "Schizophrenia."

Cancers such as Solid Tumors (Breast, Lung, Colon, Prostate, Brain "Glioblastoma), Lymphomas and Leukemia.

Infections such as viral infections including Influenza, Avian Bird Flu, and SARS-COV-2, bacterial infections, such as Meningococcal, Strep Pneumonia and Group-B Strep.

Allergic Conditions such as anaphylactic shock, food allergies, and contact allergies.

Fibrosis such as pulmonary fibrosis, Hepatitis induced fibrosis, and Myelofibrosis.

Autoimmune conditions, such as Uveitis, Arthritis, Systemic Lupus Erythematosus, Sepsis.

Kidney diseases, such as AHUS or Glomerulonephritis.

GI conditions, such as Crohn's Disease or Ulcerative colitis.

Cardiac Diseases, such as Atherosclerosis "Macrophages driven," Cardiac Fibrosis, Ischemia induced cardiomyopathy, Cardiac Heart Failure.

### Inflammatory conditions.

In other embodiments, the compositions disclosed herein can provide for the suppression of a pro-inflammatory response and an increase in an anti-inflammatory response.

In one aspect, the pro-inflammatory response can be suppressed by at least 10%, at least 20%, at least 30%, at least 40%, at least 50%, at least 60%, at least 70%, at least 80%, at least 90%, or at least 99%. In alternative embodiments, the anti-inflammatory response can be increased by at least 10%, at least 20%, at least 30%, at least 40%, and at least 50%, at least 60%, at least 70%, at least 80%, at least 90%, at least 99%.

Suitably pro-inflammatory cytokines can be measured to determine the efficacy of nanoparticle drug treatment. Such measurements can be made during the actual treatment of a subject, or alternatively, during animal testing of the nanoparticles disclosed herein. In embodiments, pro-inflammatory cytokines can include, for example, TNF-α and IL-6. Suitably anti-inflammatory cytokines can also be measured, for example IL-10. The skilled person would know of suitable assay methods to measure such cytokines. For example, the Bio-Plex^{™} Cytokine Assay (Bio-Rad) may be used. To determine whether a cell produces greater or less proinflammatory cytokines, a suitable method which can be used is that cells are resuspended and seeded at 2×10⁵ cells/ml and 200 µl per well in a 96 well plate. They can then be left to adhere to the plate overnight and be treated with LPS and ligands for 24 hours at range of concentrations. Supernatant can then be removed and stored at -70° C. Cytokine levels can then be assessed by ELISA (R&D systems). As will be appreciated a similar method can be applied to determine anti-inflammatory cytokines.

In an embodiment, TNF-α levels can be suitably determined by coating a 96 well plate with TNF-α capture antibody diluted in 1× phosphate buffered saline (PBS) overnight. All steps can be carried out at room temperature. The wells can be washed three times in 1×PBS/0.1% Polyoxyethylene sorbitan monolaurate (Tween 20) before being blocked for one hour with 1% BSA (BDH) dissolved in 1 ×PBS. The washing step can be repeated and 50 µl of treated cell supernatants or standards ranging from 2000 pg/ml to 0 pg/ml can be added to the wells and left for 2 hours. Subsequently supernatant can be aspirated out, the wells washed 3 times and 50 µl of TNF-α detection antibody diluted in 1% BSA/1×PBS can be added for 2 hours. Again, wells can be washed three times and Horse Radish Peroxidase (HRP) conjugated antibody can be added at 1 in 200 dilution in 1% BSA/1 ×PBS for 20 minutes. At this stage, the plate can be covered in aluminum foil. Once wells have been washed 3,3',5,5'-tetramethylbenzidine (TMB) can be added for 20 minutes and again protected from light. 1M hydrochloric acid can be added to halt the reaction and absorbance read on a plate reader at 450 nM. TNF-α concentrations can then be extrapolated from the standard curve. As will be appreciated, a similar methodology can be applied to determine other cytokine levels, substituting the TNF-α detection antibody for a detection antibody or other agent specific for the applicable cytokine.

In an embodiment, IL-10 levels can be suitably determined by coating a 96 well plate with IL-10 capture antibody diluted in 1× phosphate buffered saline (PBS) overnight. All steps can be carried out at room temperature. The wells can be washed three times in 1×PBS/0.1% Polyoxyethylene sorbitan monolaurate (Tween 20) before being blocked for one hour with 1% BSA (BDH) dissolved in 1 ×PBS. The washing step can be repeated and 50 µl of treated cell supernatants or standards ranging from 2000 pg/ml to 0 pg/ml can be added to the wells and left for 2 hours. Subsequently supernatant can be aspirated out, the wells washed 3 times and 50 µl of IL-10 detection antibody diluted in 1% BSA/1 ×PBS can be added for 2 hours. Again, wells can be washed three times and Horse Radish Peroxidase (HRP) conjugated antibody can be added at 1 in 200 dilution in 1% BSA/1 ×PBS for 20 minutes. At this stage, the plate can be covered in aluminum foil. Once wells have been washed 3,3',5,5'-tetramethylbenzidine (TMB) can be added for 20 minutes and again protected from light. 1M hydrochloric acid can be added to halt the reaction and absorbance read on a plate reader at 450 nM. IL-10 concentrations can then be extrapolated from the standard curve. As will be appreciated, a similar methodology can be applied to determine other cytokine levels, substituting the IL-10 detection antibody for a detection antibody or other agent specific for the applicable cytokine.

To determine whether the treated subject or test animal produces a greater or lesser pro-inflammatory response, a method that may be used is analysis of serum cytokine levels. For example, this may be achieved by the collection of 50 µl blood from the treated subject using a capillary tube. This blood is allowed to clot at room temperature for 30 minutes prior to centrifugation at 1300 rpm to pellet red blood cells. Serum is decanted to a clean microcentrifuge tube and analyzed by ELISA. For more extensive analysis, a larger volume of blood (approximately 600 µl-1 ml) may be taken by direct cardiac puncture, thus allowing for a greater volume of serum to be collected and analyzed by ELISA or such other technique. Other suitable techniques to determine whether the treated subject or test animal produces a greater or lesser pro-inflammatory response will be known in the art, particularly to detect and measure cytokines.

### Pharmaceutical Compositions

As used herein, "pharmaceutically acceptable carrier or excipient" may include any and all medically appropriate inactive ingredients and/or solvents, diluents, or other liquid vehicle, dispersion or suspension aids, surface active agents, isotonic agents, thickening or emulsifying agents, preservatives, solid binders, fillers, bulking agents, lubricants and the like, as suited to the particular dosage form desired. Remington's Pharmaceutical Sciences, Eighteenth Edition, A. R. Geunaro (Mack Publishing Co., Easton, Pa., 1990) discloses various carriers used in formulating pharmaceutical compositions and known techniques for the preparation thereof.

The present disclosure further provides pharmaceutical or veterinary compositions comprising the sialic-acid-containing ligand linked nanoparticles disclosed herein. Such a pharmaceutical composition is formulated to be compatible with its intended route of administration. Examples of routes of administration include both parenteral and non-parenteral administration methods including, for example, intravenous, intravitreal, oral, intraocular, subretinal, subtenons, intrascleral, periocular, intravenous, inhalational nasal and oral, intramuscular, intra-areterial, intraspinal, intrathecal, intracranial, intradermal, transdermal (topical), transmucosal, subcutaneous, pulmonary lavage, gastric lavage, intrahepatic, subcutaneous, and rectal administration.

Suitably, in some embodiments nanoparticles can be parenterally administered. After parenteral administration, nanoparticles can selectively accumulate in particular tissues or body locations. In some embodiments, nanoparticles can deliver a therapeutic payload to the cell or tissue. In some embodiments, nanoparticles can access diseased tissue through an enhanced permeability and retention effect.

In general, pharmaceutical compositions are provided comprising an effective amount of a nanoparticle with pharmaceutically acceptable diluents, preservatives, solubilizers, emulsifiers, adjuvants, and/or carriers. Such compositions include diluents of various buffer content (e.g., Tris-HCl, acetate, phosphate), pH and ionic strength; additives such as detergents and solubilizing agents (e.g., tween 80, polysorbate 80), antioxidants (e.g., ascorbic acid, sodium metabisulfite), preservatives (e.g., thimersol, benzyl alcohol) and bulking substances (e.g., lactose, mannitol). Such compositions may influence the physical state, stability, rate of *in vivo* release, and rate of *in vivo* clearance of the nanoparticle. See, e.g., Remington's Pharmaceutical Sciences, 18th Ed. (1990, Mack Publishing Co., Easton, Pa. 18042) pages 1435-1712. The compositions may be prepared in liquid form, or may be formulated into a dried powder, such as lyophilized form.

The term "pharmaceutically acceptable salt" also refers to a salt prepared from a compound disclosed herein or any other compound delineated herein, having an acidic functional group, such as a carboxylic acid functional group, and a pharmaceutically acceptable inorganic or organic base.

Pharmaceutically acceptable salts of the compounds of the present invention are also included. For example, an acid salt of a compound of the present invention containing an amine or other basic group can be obtained by reacting the compound with a suitable organic or inorganic acid, resulting in pharmaceutically acceptable anionic salt forms. Examples of anionic salts include the acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylsulfate, mucate, napsylate, nitrate, pamoate, pantothenate, phosphate/diphosphate, polygalacturonate, salicylate, stearate, subacetate, succinate, sulfate, tannate, tartrate, teoclate, tosylate, and triethiodide salts.

Salts of the compounds of the present invention containing a carboxylic acid or other acidic functional group can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, N,N'-bisdehydroabietylamine, glucamine, N-methylglucamine, collidine, quinine, quinoline, and basic amino acids such as lysine and arginine.

Accordingly, in a 1^{st} example embodiment, the present invention is a compound represented by any one of the following structural formulas: 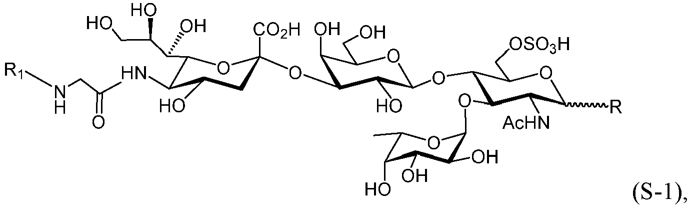 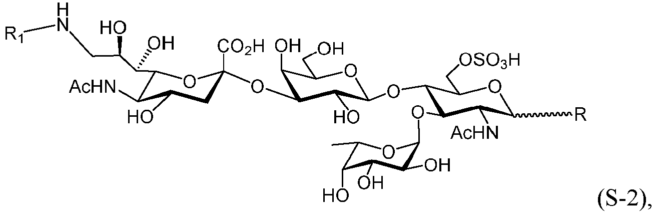 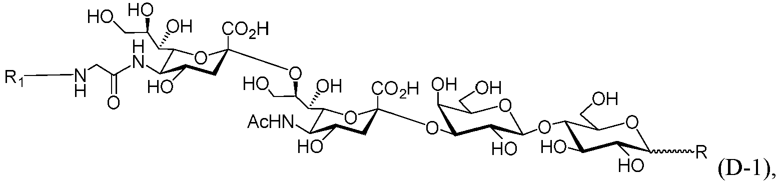 or 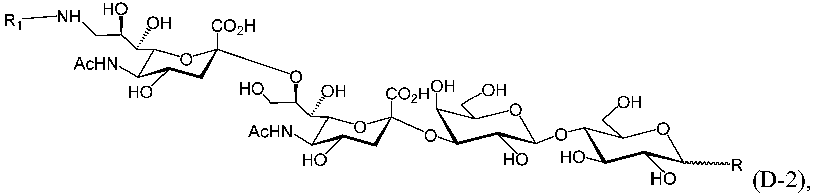 or a pharmaceutically acceptable salt thereof.

In a 1^{st} aspect of the 1^{st} example embodiment, R₁, for each occurrence independently, is -C(O)-A, wherein A is a C₁-C₆ alkyl, a C₆-C₁₈ aryl, a (C₆-C₁₈)aryl(C₁-C₃)alkyl, a 5-18-member heteroaryl, a (5-18-member)heteroaryl(C₁-C₃)alkyl, a C₃-C₈ cycloalkyl, a (C₃-C₈)cycloalkyl(C₁-C₃)alkyl, a 5-8-member heterocycloalkyl, or a (5-8-member)heterocycloalkyl(C₁-C₃)alkyl; wherein one or two carbon atoms within the alkyl portion of A is optionally, each independently, replaced with a heteroatom selected from N, O, or S. (Examples of an alkyl portion of A that includes a heteroatom are CH₃-CH₂-O-CH₂-CH₂-, CH₃-NH-CH₂-, CH₃-N(CH₃)-CH₂-, CH₃-S-CH₂-CH₂-, etc.). Further, A is optionally substituted with 1 to 3 R¹¹ groups, each said R¹¹ group independently selected from a C₁-C₆ alkyl, a C₁-C₆ haloalkyl, a C₁-C₆ alkoxy, a halogen, a C₆-C₁₂ aryl, a 5-12-member heteroaryl, cyano, or two groups R¹¹, taken together with the atoms to which they are attached, form a 5-7-member heterocyclyl having 1 to 3 heteroatoms selected from N, O, or S; wherein R¹¹, each independently, is optionally substituted with 1 to 3 substituents selected from a halogen, a C₁-C₆ alkyl, a C₁-C₆ haloalkyl, a C₁-C₆ alkoxy, or cyano; and further wherein: R, for each occurrence independently, is -R^{L}-R^{F}, and wherein: R^{L} is, for each occurrence independently: -O-(C₁-C₁₂) alkylenyl-, -O-, -S-, -NR¹⁰⁰-, -S-(C₁-C₁₂) alkylenyl-, -NR¹⁰¹-(C₁-C₁₂) alkylenyl-, -NR^{101a}-O-(C₁-C₁₂)alkylenyl-; -O-(CH₂CH₂O)ₘ-, -O-(CH₂CH₂O)ₖ-(CH₂CH₂)-, -NR¹⁰²-X¹⁰⁰-(C₁-C₁₂) alkylenyl-, -NR^{102a}-NR^{102b}-C(O)-(C₁-C₁₂)alkylenyl, wherein R¹⁰⁰, R¹⁰¹, R^{101a}, R¹⁰², R^{102a}, and R^{102b}, each independently is H or a C₁-C₃ alkyl, and X¹⁰⁰ is -O- or -NH-, and wherein m and k, each independently, is an integer from 1 to 12; R^{F}, for each occurrence independently, is: H, a C₁-C₃ alkyl, -NH₂, -NH-Fmoc, -NH-Boc, -NH-CBz, -NH-Troc, -NH-TFA, a mono(C₁-C₃)alkylamino, a di(C₁-C₃ alkyl)amino;
- (O)-R¹⁰³, wherein R¹⁰³ is -H, -OH, or a (C₁-C₃) alkyl;
-SH, a moiety represented by the following structural formula 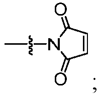 or a click chemistry reagent.

In a 2^{nd} aspect of the 1^{st} example embodiment, the compound is represented by any one of the following structural formulas: 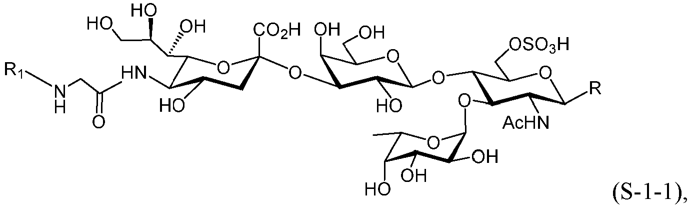 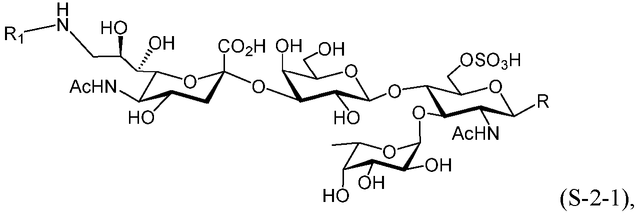 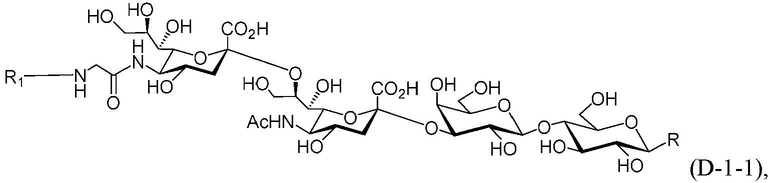 or 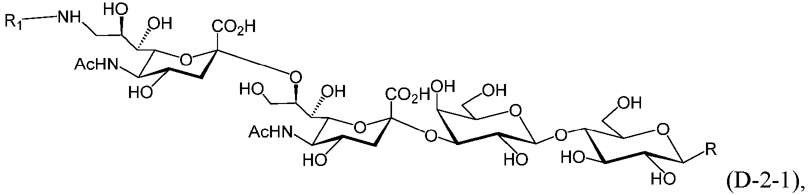 or a pharmaceutically acceptable salt thereof. Values and example values of the varuiables in formulas (S-1-1), (S-2-1), (D-1-1), and (D-2-1) are defined above with resepct to the 1^{st} aspect.

In a 3^{rd} aspect of the 1^{st} example embodiment, the click chemistry reagent comprises an azide, a C₂-C₃ alkyne, a tetrazine, a trans-cyclooctene, or a cyclooctyne. The remainder of the values and example values are as defined above with respect to the 1^{st} and 2^{nd} aspects.

In a 4^{th} aspect of the 1^{st} example embodiment, the click chemistry reagent is an azide, a C₂-C₃ alkyne, or a moiety represented by any one of the following structural formulas: 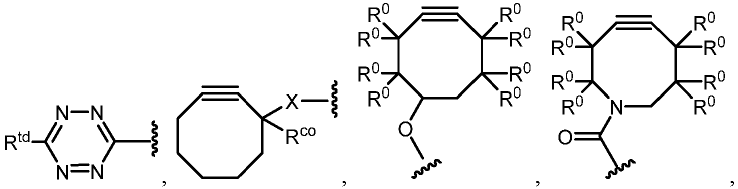 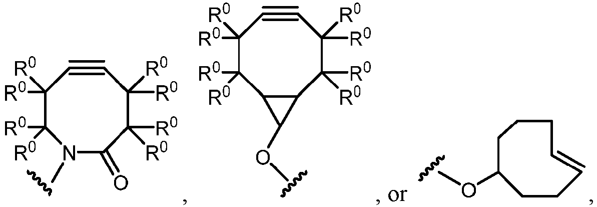 wherein: R^{co} is hydrogen or halogen; X is null or O; and R⁰ is in each case independently selected from hydrogen, halogen, C₁₋₈alkyl, C₁₋₈alkoxy, a C₆-C₁₂ aryl, 5-8 member heteroaryl, C₃₋₈cycloalkyl, or 3-8-member heterocyclyl; wherein any two or more R⁰ groups can together with the atoms to which they are attached optionally form an unsaturated, saturated, or aromatic 5-8-member ring; and R^{td} is hydrogen, C₁₋₈alkyl, C₁₋₈alkoxy, a C₆-C₁₂ aryl, a 5-8-member heteroaryl, C₃₋₈cycloalkyl, or a 3-8 member heterocyclyl. The remainder of the values and example values are as defined above with respect to the 1^{st} through 3^{rd} aspects.

In a 5^{th} aspect of the 1^{st} example embodiment, the click reagent is an azide, a C₂-C₃ alkyne, or any one of the moieties represented by the following structural formulas: 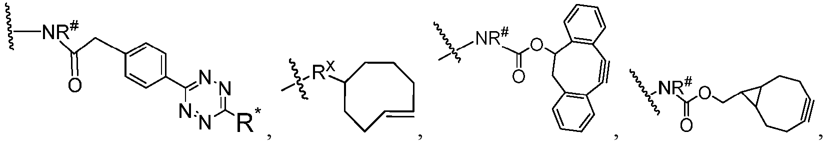 or 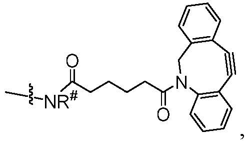 wherein R* is H or methyl, R^{#}, for each occurrence independently, is H or a C₁-C₃ alkyl, and R^{X} is -NH-C(O)O-. The remainder of the values and example values are as defined above with respect to the 1^{st} through 4^{th} aspects.

In a 6^{th} aspect of the 1^{st} example embodiment, R, for each occurrence independently, is -O-(CH₂)ₓ-NH₂ or -O-(CH₂)ₓ-NH-Fmoc, wherein x, for each occurrence independently, is an integer from 1 to 10, for example x is 5. The remainder of the values and example values are as defined above with respect to the 1^{st} through 5^{th} aspects.

In an additional aspect, R, for each occurrence independently, is -O-(CH₂)ₓ-N₃, wherein x, for each occurrence independently, is an integer from 1 to 10, for example x is 5.

In a 7^{th} aspect of the 1^{st} example embodiment, the cycloalkyl portion of moiety A is selected from a C₆-C₇ cycloalkyl; the heterocycloalkyl portion of moiety A is selected from a 5-6-member heterocycloalkyl having 1 or 2 heteroatoms selected from N, O, or S; the aryl portion of moiety A is selected from a phenyl or a naphthalenyl; and the heteroaryl portion of moiety A is selected from a moiety represented by any of the following structural formulas: 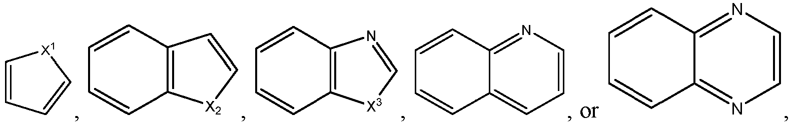 wherein X¹, X² and X³, each independently, is selected from NR^{H}, O, or S, wherein R^{H} is H or a C₁-C₃ alkyl; and wherein, if present, each R¹¹ is independently selected from cyano, a halogen, a phenyl, a halophenyl, a C₁-C₆ alkyl, a C₁-C₆ haloalkyl, a C₁-C₃ alkoxy, or two groups R¹¹, taken together with the atoms to which they are attached, form a [1,3]dioxolo group, optionally substituted with one or two methyl or ethyl groups. The remainder of the values and example values are as defined above with respect to the 1^{st} through 6^{th} aspects.

In an 8^{th} aspect of the 1^{st} example embodiment, R₁, for each occurrence independently, is selected from the moiety represented by the following structural formulas, wherein the wavy line represents the point of attachment of R₁ to the nitrogen atom:

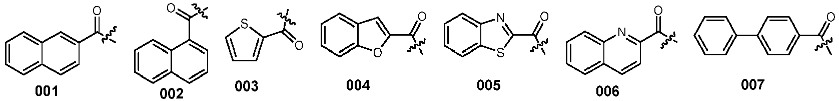

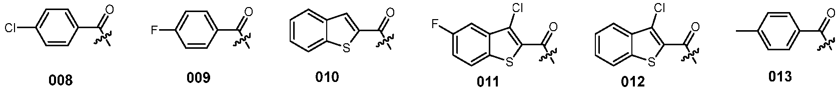

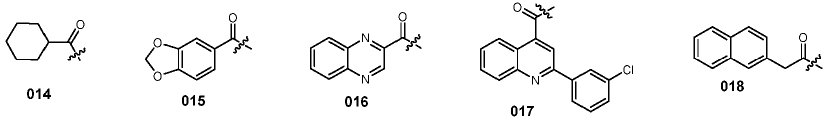

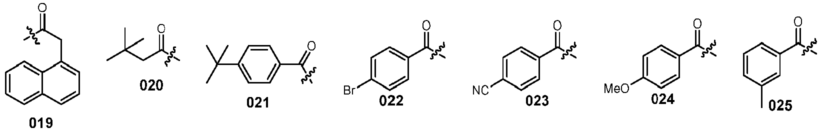

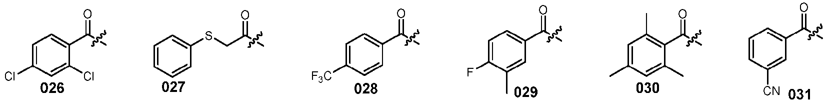

The remainder of the values and example values are as defined above with respect to the 1^{st} through 7^{th} aspects.

In a 9^{th} aspect of the 1^{st} example embodiment, the compound is any one of the compounds S-101 through S-131, or D-101 through D-131, wherein the moiety R is -O-(CH₂)₅-NH-Fmoc.

In an additional aspect, the compound is any one of the compounds S-101 through S-131, or D-101 through D-131, wherein the moiety R is -O-(CH₂)₅-NH₂ or -O-(CH₂)₅-N₃.

In a 2^{nd} example embodiment, the present inventipon is a particle, comprising a compound represented by the following structural formula:

G-L-P.

In a 1^{st} aspect of the 2^{nd} example embodment, P is a biocompatible polymer; L is a covalent linker; and G is any one of the moieties represented by the following structural formulas: 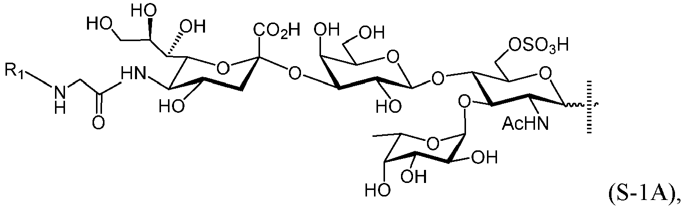 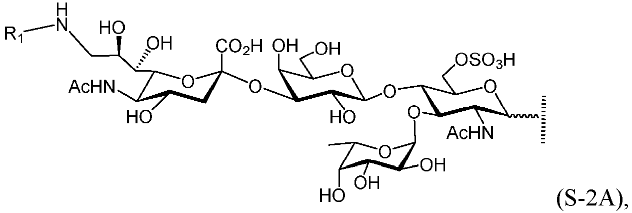 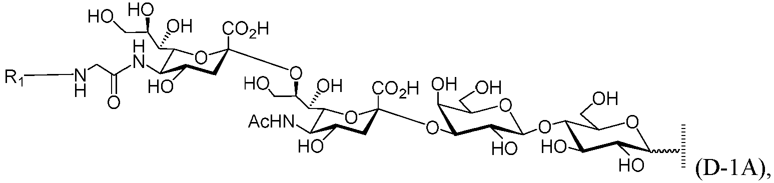 or 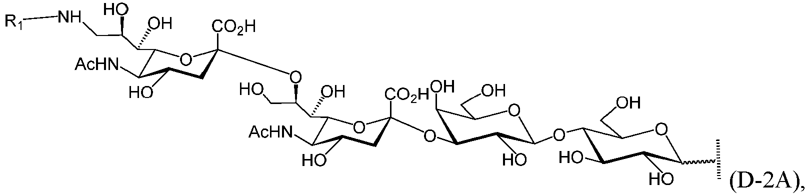 or a pharmaceutically acceptable salt thereof.

In formulas (S-1A), (S-2A), (D-1A), and (D-2A): the symbol 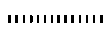 represents the point of attachment to L; R₁, for each occurrence independently, is -C(O)-A, wherein A is a C₁-C₆ alkyl, a C₆-C₁₈ aryl, a (C₆-C₁₈)aryl(C₁-C₃)alkyl, a 5-18-member heteroaryl, a (5-18-member)heteroaryl(C₁-C₃)alkyl, a C₃-C₈ cycloalkyl, a (C₃-C₈)cycloalkyl(C₁-C₃)alkyl, a 5-8-member heterocycloalkyl, or a (5-8-member)heterocycloalkyl(C₁-C₃)alkyl; wherein one or two carbon atoms within the alkyl portion of A is optionally, each independently, replaced with a heteroatom selected from N, O, or S. (Examples of an alkyl portion of A that includes a heteroatom are CH₃-CH₂-O-CH₂-CH₂-, CH₃-NH-CH₂-, CH₃-N(CH₃)-CH₂-, CH₃-S-CH₂-CH₂-, etc.) A is optionally substituted with 1 to 3 R¹¹ groups, each said R¹¹ group independently selected from a C₁-C₆ alkyl, a C₁-C₆ haloalkyl, a C₁-C₆ alkoxy, a halogen, a C₆-C₁₂ aryl, a 5-12-member heteroaryl, cyano, or two groups R¹¹, taken together with the atoms to which they are attached, form a 5-7-member heterocyclyl having 1 to 3 heteroatoms selected from N, O, or S; wherein R¹¹, each independently, is optionally substituted with 1 to 3 substituents selected from a halogen, a C₁-C₆ alkyl, a C₁-C₆ haloalkyl, a C₁-C₆ alkoxy, or cyano.

In a 2^{nd} aspect of the 2^{nd} example embodment, moiety G is represented by any one of the following structural formulas: 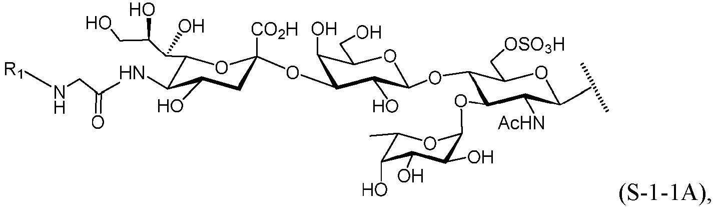 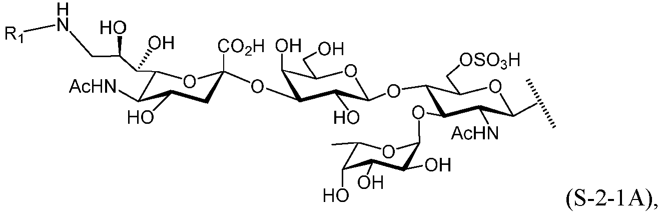 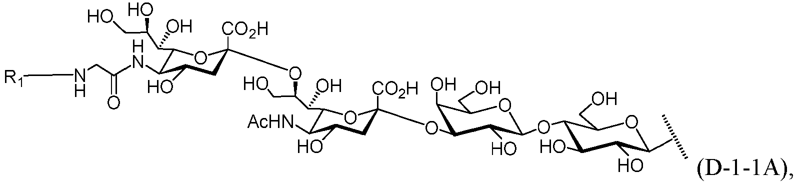 or 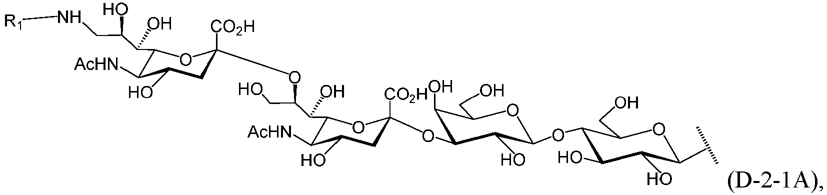 or a pharmaceutically acceptable salt thereof. The remainder of the values and example values are as defined above with respect to the 1^{st} aspect.

In some aspects, the biocompatible polymer can comprise at least one biocompatible polymer selected from the group consisting of polyglycolic acid, poly(lactic acid), poly(lactic-co-glycolic acid), polycaprolactone, poly(3-hydroxybutyric acid ), poly(ethylene glycol), polyethylene oxide, poly(ethylene glycol)-block-poly(propylene glycol)-block-poly(ethylene glycol) (Pluronic F127), polyoxyethylene-polyoxypropylene block copolymer (Pluronic F68), poloxamer, poly(hydroxymethylmethacrylate), polyvinyl alcohol, poly(vinylpyrrolidone), hyaluronic acid, heparin, heparin sulfate, polysialic acid and chitosan. For example, the polymer can comprise a copolymer PLGA-PEG. The remainder of the values and example values are as defined above with respect to the 1^{st} and 2^{nd} aspects.

In a 3^{rd} aspect, the biocompatible polymer comprises at least one of polyglycolic acid, poly(lactic acid), poly(lactic-co-glycolic acid), polycaprolactone, poly(3-hydroxybutyric acid), polyethylene oxide, polyoxyethylene-polyoxypropylene block copolymer, poly(hydroxymethylmethacrylate), polyvinyl alcohol, poly(vinylpyrrolidone), hyaluronic acid, heparin, heparin sulfate, polysialic acid and chitosan. The remainder of the values and example values are as defined above with respect to the 1^{st} and 2^{nd} aspects.

In a 4^{th} aspect, the biocompatible polymer comprises poly(L-lactic acid), poly(D-lactic acid), poly(D/L-lactic acid), a copolymer thereof, or a combination thereof. The remainder of the values and example values are as defined above with respect to the 1^{st} and 2^{nd} aspects.

In a 5^{th} aspect, the biocompatible polymer comprises polyethylene oxide, polyoxyethylene-polyoxypropylene block copolymer, a copolymer thereof, or a combination thereof. The remainder of the values and example values are as defined above with respect to the 1^{st} and 2^{nd} aspects.

In a 6^{th} aspect of the 2^{nd} example embodment, the polymer P is represented by the following structural formula: 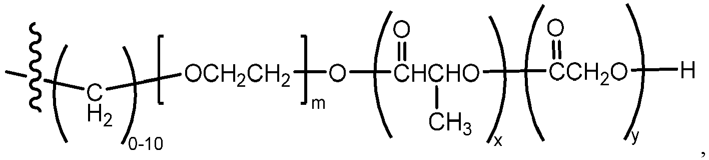 wherein the symbol 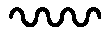 represents the point of attachment of the polymer to the linker L, and further wherein: y is an integer from 0 to 1000, x is an integer from 0 to 1000, and m is an integer from 0 to 450, provided that x and y are not simultaneously 0. The remainder of the values and example values are as defined above with respect to the 1^{st} through 5^{th} aspects.

In certain examples of the 6^{th} aspect, y is an integer from 0 to 500, x is an integer from 0 to 500, and m is an integer from 0 to 250. For example, in various aspects, m is 50-200, or m is 75-175, or m is 90-150, or m is 90-140, or m is 100-130. In various aspects, x is 50-200, or x is 75-175, or x is 90-150, or x is 90-140, or x is 100-130. In additional aspects, y is 5-100, or y is 10-75, or y is 15-50. Any combination of values for x, y and m based on the various aspects above are also contemplated. For example, m can be 100-130, x can be 100-130 and y can be 15-50.

In another example of the 6^{th} aspect, x is an integer from 90 to 140; y is an ineteger from 10 to 75; and m is an integer from 90 to 140.

The remainder of the values and example values are as defined above with respect to the 1^{st} through 5^{th} aspects.

In a 7^{th} aspect, the particle further comprises one or more of: an acid-terminated PLGA (PLGA-COOH); an acid-terminated PLGA-PEG copolymer, wherein the acid moiety terminates the PEG block (PLGA-PEG-COOH); an PLGA-PEG copolymer, wherein the PEG block is terminated with the moiety represented by the following structural formula: 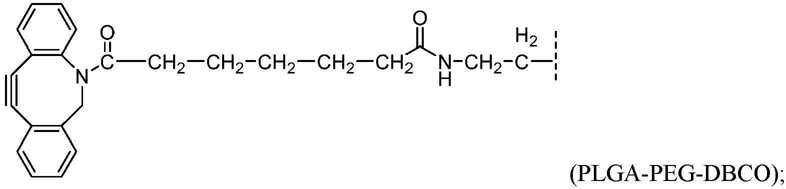 or
an PLGA-PEG copolymer, wherein the PEG block is terminated by the the moiety represented by the following structural formula: 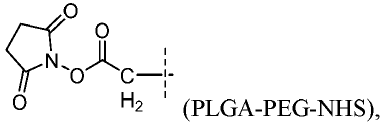
wherein the dash line denotes the point of attachment of the terminating moiety to PEG block.

In an example of the 7^{th} aspect, the particle comprises at least one of: a blend of 75% by weight of PLGA-COOH and 25% by weight of PLGA-PEG-DBCO; a blend of 75% by weight of PLGA-COOH and 25% by weight of PLGA-PEG-NHS; a blend of 90% by weight of PLGA-PEG-COOH and 10% by weight of PLGA-PEG-DBCO; and PLGA-PEG-NHS.

The remainder of the values and example values are as defined above with respect to the 1^{st} through 6^{th} aspects.

In an 8^{th} aspect of the 2^{nd} example embodment, the linker L includes a portion that is a product of a click chemistry reaction. The remainder of the values and example values are as defined above with respect to the 1^{st} through 7^{th} aspects.

In a 9^{th} aspect of the 2^{nd} example embodment, the linker L comprises a portion represented by any one of the following structural formulas, wherein the symbols ------ and 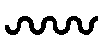 , each indepednently, represents the point of attachment to additional portions of the linker L, to P, or to G: 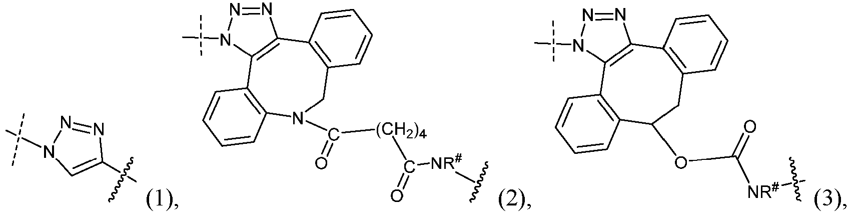 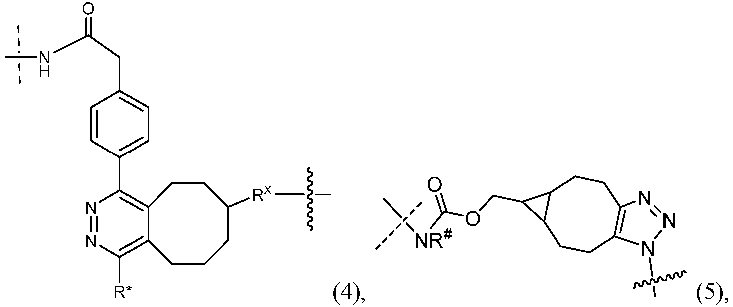 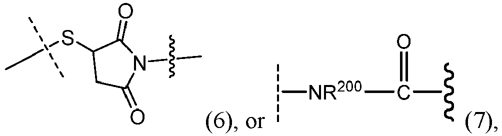 wherein R* is H or methyl, R^{#}, for each occurrence independently, is H or a C₁-C₃ alkyl, and R^{X} is -NH-C(O)O-, and R²⁰⁰ is an -H or a C₁-C₃ alkyl. An example of the additional portion of the linker is the moiety R^{L} defined hereinabove with respect to the 1^{st} example embodiment or any aspects thereof. One example of the additional portion of the linker is a -O-(C₁-C₁₂)alkylenyl, such as the moiety -O-(CH₂)₅. The remainder of the values and example values are as defined above with respect to the 1^{st} through 8^{th} aspects.

In an 10^{th} aspect of the 2^{nd} example embodment, the polymer P is a PLGA(10k)-PEG(5k). The remainder of the values and example values are as defined above with respect to the 1^{st} through 9^{th} aspects.

In a 11^{th} aspect of the 2^{nd} example embodment, the weight of G per unit weight of P (ligand density) is from 10 to 75 µg/mg. The remainder of the values and example values are as defined above with respect to the 1^{st} through 10^{th} aspects.

In a 12^{th} aspect of the 2^{nd} example embodiment, the weight of G (also referred to herein as the sialic acid-containing ligand) per weight of total solids is from about 1 µg/mg to about 1000 µg/mg.

In a 3^{rd} example embodiment, the present invention is a method of treating a disorder in a subject in need thereof.

In a 1^{st} aspect of the 3^{rd} example embodment, the method comprises administering to a subject an effective amount of a compound or a composition comprising particles described herein, or a pharmaceutically acceptable salt threof, wherein the disorder is selected from a cancer, an ophthalmic disease, a fibrotic disease, a parasitic inflammation, a fungal inflammation, a viral inflammation, an autoimmune inflammation, a neurological inflammation, a neurological degeneration, a dermatologic inflammation, a renal inflammation, a cardiovascular disease, a gastrointestinal inflammation, or a rheumatic disease.

In a 4^{th} example embodiment, the present invention is a method of treating a disorder in a subject in need thereof.

In a 1^{st} aspect of the 4^{th} example embodment, the method comprises administering to a subject an effective amount of a compound or a composition comprising particles described herein, , or a pharmaceutically acceptable salt threof, wherein the disorder is selected from a breast cancer, non-small cell lung cancer (NSCLC), prostrate cancer, colorectal cancer, melanoma, pancreatic cancer, myelofibrosis, diabetic retinopathy, idiopathic pulmonary lung fibrosis, liver fibrosis, sickle cell anemia, and acute respiratory distress syndrome (ARDS).

In a 5^{th} example embodiment, the present invention is a pharmaceutical composition, comprising a compound or a particle as described herein with respect to the 1^{st} and 2^{nd} example embodiments and their various aspects and example aspects, or a pharmaceutically acceptable salt thereof, in a pharmaceutically acceptable carrier.

In a 6^{th} example embodiment, the present invention is a method of making a particle comprising a molecule represented by the following structural formula:

G-L-P.

The values and example values of the variables G, L, and P of the 6^{th} example embodiment are defined hereinabove with respect to the 1^{st} and 2^{nd} example embodiments and various aspects thereof.

In a 1^{st} aspect of the 6^{th} example embodiment, the method comprises reacting a compound represented by a structural formula (I)

G-R^{F1} (I)

with a compound represented by a structural formula (II)

P-R^{F2} (II),

wherein R^{F1} and R^{F2} are each a reactive moiety, under conditions suitable to cause moieties R^{F1} and R^{F2} to react with each other thereby producing a portion of the covalent -L-.

In a 2^{nd} aspect of the 6^{th} example embodiment, R^{F1} and R^{F2}, is a pair of click reagent, and wherein the conditions are suitable to cause a click reaction between R^{F1} and R^{F2}, wherein the the linker L includes a portion that is a product of a click chemistry reaction between R^{F1} and R^{F2}. Value and example values of the remainder of the variable are as defined with respect to the 1^{st} aspect.

In a 3^{rd} aspect, the biocompatible polymer comprises at least one biocompatible polymer selected from the group consisting of polyglycolic acid, poly(L-lactic acid), poly(lactic-co-glycolic acid), polycaprolactone, poly(3-hydroxybutyric acid ), polyethylene oxide, polyoxyethylene-polyoxypropylene block copolymer, poly(hydroxymethylmethacrylate), polyvinyl alcohol, poly(vinylpyrrolidone), hyaluronic acid, heparin, heparin sulfate, polysialic acid and chitosan. The remainder of the values and example values are as defined above with respect to the 1^{st} and 2^{nd} aspects.

In a 7^{th} example embodiment, the present invention is a compound represented the following structural formulas: 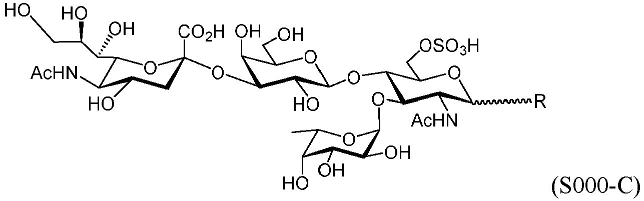 or a pharmaceutically acceptable salt thereof.
In a first aspect of the 7^{th} example embodiment, R, for each occurrence independently, is -R^{L}-R^{F}, and wherein: R^{L} is, for each occurrence independently: -O-, -S-, -NR¹⁰⁰-, -O-(C₁-C₁₂) alkylenyl-, -S-(C₁-C₁₂) alkylenyl-, -NR¹⁰¹-(C₁-C₁₂) alkylenyl-, -NR^{101a}-O-(C₁-C₁₂)alkylenyl-; -O-(CH₂CH₂O)ₘ-, -O-(CH₂CH₂O)ₖ-(CH₂CH₂)-, -NR¹⁰²-X¹⁰⁰-(C₁-C₁₂) alkylenyl-, -NR^{102a}-NR^{102b}-C(O)-(C₁-C₁₂)alkylenyl, wherein R¹⁰⁰, R¹⁰¹, R^{101a}, R¹⁰², R^{102a}, and R^{102b}, each independently is H or a C₁-C₃ alkyl, and X¹⁰⁰ is -O- or -NH-, and wherein m and k, each independently, is an integer from 1 to 12; R^{F}, for each occurrence independently, is:
H, a C₁-C₃ alkyl, -NH₂, -NH-Fmoc, -NH-Boc, -NH-CBz, -NH-Troc, -NH-TFA, a mono(C₁-C₃)alkylamino, a di(C₁-C₃ alkyl)amino;
-C(O)-R¹⁰³, wherein R¹⁰³ is -H, -OH, or a (C₁-C₃) alkyl;
-SH, a moiety represented by the following structural formula 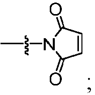
or a click chemistry reagent.

In one aspect of the 7^{th} example embodiment,-R^{L}-R^{F} is not -OH.

In various additional aspect, the values and example values of variable R are described above with repsect to the 1^{st} to 6^{th} example embodiments and their various aspects.

In an 8^{th} example embodiment, the present invention is a particle, comprising a molecule represented by the following structural formula:

G-L-P.

In a 1^{st} aspect of the 8^{th} example embodiment, P is a biocompatible polymer, L is a covalent linker; and G is represented by the following structural formula: 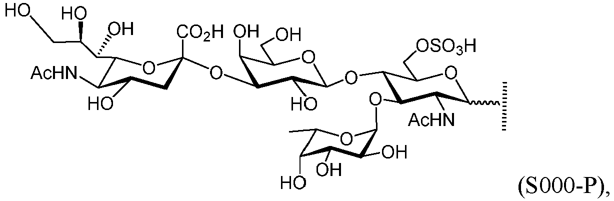 or a pharmaceutically acceptable salt thereof, wherein the symbol 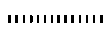 represents the point of attachment to L.

In additional aspect, the values and example values of variables L and P are described above with repsect to the 2^{nd} example embodiments and its various aspects.

In a 2^{nd} aspect, the biocompatible polymer comprises at least one of polyglycolic acid, poly(lactic acid), poly(lactic-co-glycolic acid), polycaprolactone, poly(3-hydroxybutyric acid), polyethylene oxide, polyoxyethylene-polyoxypropylene block copolymer, poly(hydroxymethylmethacrylate), polyvinyl alcohol, poly(vinylpyrrolidone), hyaluronic acid, heparin, heparin sulfate, polysialic acid and chitosan. The remainder of the values and example values are as defined above with respect to the 1^{st} and additional aspects of the 8^{th} embodiment.

In a 2^{nd} aspect, the biocompatible polymer comprises poly(L-lactic acid), poly(D-lactic acid), poly(D/L-lactic acid), a copolymer thereof, or a combination thereof. The remainder of the values and example values are as defined above with respect to the 1^{st} and additional aspects of the 8^{th} embodiment.

In a 3^{rd} aspect, the biocompatible polymer comprises polyethylene oxide, polyoxyethylene-polyoxypropylene block copolymer, a copolymer thereof, or a combination thereof. The remainder of the values and example values are as defined above with respect to the 1^{st} and additional aspects of the 8^{th} embodiment.

In a 4^{th} aspect, the polymer scaffold comprises a block copolymer PLGA-PEG. The remainder of the values and example values are as defined above with respect to the 1^{st} and additional aspects of the 8^{th} embodiment.

In a 5^{th} aspect, the polymer P is represented by the following structural formula: 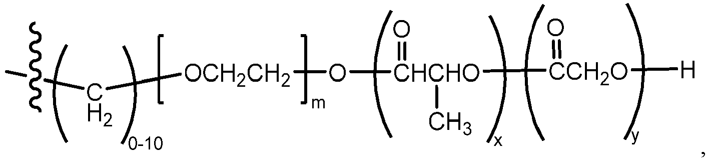 wherein the symbol 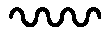 represents the point of attachment of the polymer to the linker L, and further wherein: y is an integer from 0 to 1000, x is an integer from 0 to 1000, and m is an integer from 0 to 450, provided that x and y are not simultaneously 0. The remainder of the values and example values are as defined above with respect to the 1^{st} through 4^{th} and the additional aspects of the 8^{th} embodiment.

In certain examples of the 5^{th} aspect, y is an integer from 0 to 500, x is an integer from 0 to 500, and m is an integer from 0 to 250. For example, in various aspects, m is 50-200, or m is 75-175, or m is 90-150, or m is 90-140, or m is 100-130. In various aspects, x is 50-200, or x is 75-175, or x is 90-150, or x is 90-140, or x is 100-130. In additional aspects, y is 5-100, or y is 10-75, or y is 15-50. In an example of the 5^{th} aspect, x is an integer from 90 to 140; y is an ineteger from 10 to 75; and m is an integer from 90 to 140. The remainder of the values and example values are as defined above with respect to the 1^{st} through 4^{th} and the additional aspects of the 8^{th} embodiment.

In a 6^{th} aspect, the particle further comprises one or more of: an acid-terminated PLGA (PLGA-COOH); an acid-terminated PLGA-PEG copolymer, wherein the acid moiety terminates the PEG block (PLGA-PEG-COOH); an PLGA-PEG copolymer, wherein the PEG block is terminated with the moiety represented by the following structural formula: 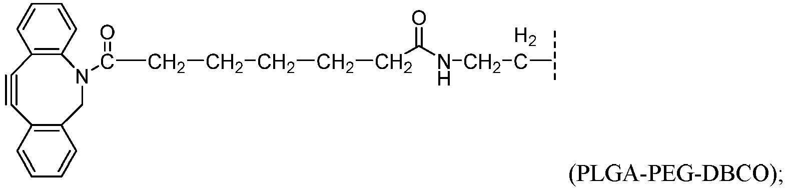 or
an PLGA-PEG copolymer, wherein the PEG block is terminated by the the moiety represented by the following structural formula: 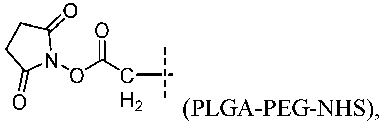
wherein the dash line denotes the point of attachment of the terminating moiety to PEG block.

The remainder of the values and example values are as defined above with respect to the 1^{st} through 5^{th} and the additional aspects of the 8^{th} embodiment.

In a 7^{th} aspect, the particle comprises at least one of: a blend of 75% by weight of PLGA-COOH and 25% by weight of PLGA-PEG-DBCO; a blend of 75% by weight of PLGA-COOH and 25% by weight of PLGA-PEG-NHS; a blend of 90% by weight of PLGA-PEG-COOH and 10% by weight of PLGA-PEG-DBCO; and PLGA-PEG-NHS. The remainder of the values and example values are as defined above with respect to the 1^{st} through 4^{th} and the additional aspects of the 8^{th} embodiment.

In a 9^{th} example embodiment, the present invention is a pharmaceutical composition, comprising a compound as described with respect to the 7^{th} example embodiment or any of its aspects or a particle as described with respect to the 8^{th} example embodiment or any of its aspects, or a pharmaceutically acceptable salt thereof, in a pharmaceutically acceptable carrier.

In a 10^{th} example embodiment, the present invention is a method of treating a disorder in a subject in need thereof. The method comprises administering to the subject a therapeutically effective amount of a compound as described above with respect to the 7^{th} example embodiment and various aspects thereof or a pharmaceutically acceptable salt thereof, or a composition comprising particles of as described above with respect to the 8^{th} example embodiment and various aspects thereof or a pharmaceutically acceptable salt threof, or a pharmaceutical composition of the 9^{th} example embodiment. In one aspect, the disorder is influenza. Influenza can be human influenza or avian influenza.

In an 11^{th} example embodiment, the present invention is any of the compounds represented by the structural formulas listed in Table 4 or a pharmaceutically acceptable salt thereof, wherein the variable R is -O-(CH₂)₅-NH₂, -O-(CH₂)₅-NH-Fmoc, -O-(CH₂)₅-N₃, or -O-(CH₂)₅-biotin.

In a 12^{th} example embodiment, the present invention is a compound represented by any of the following structural formulas of a pharmaceutically acceptable salts thereof: 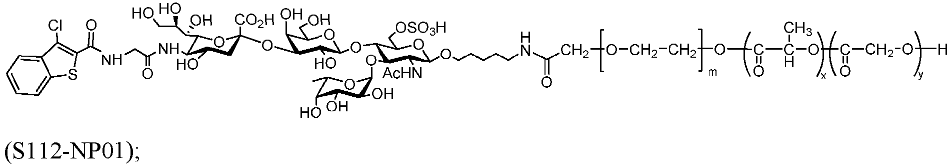 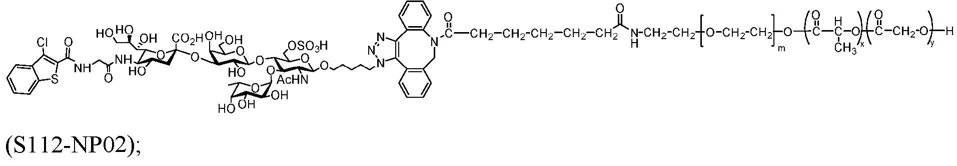 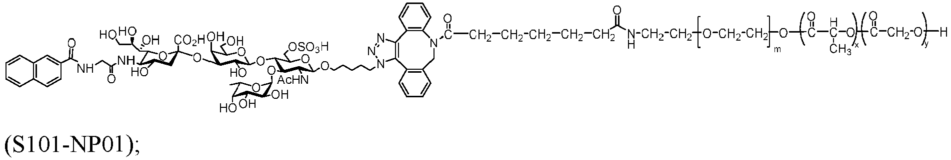 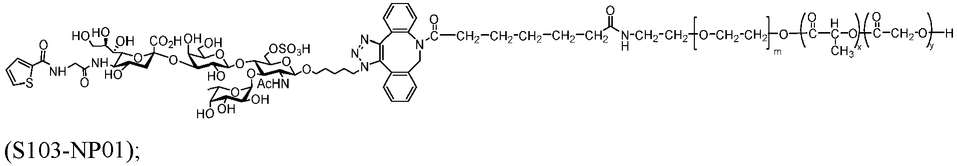 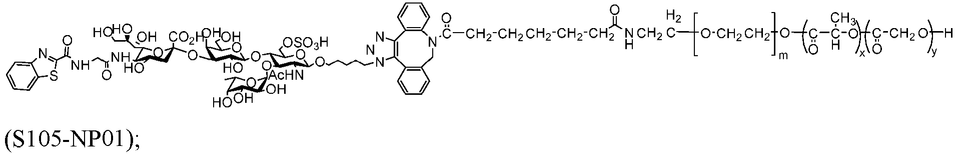 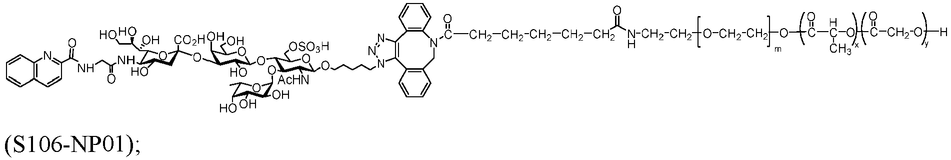 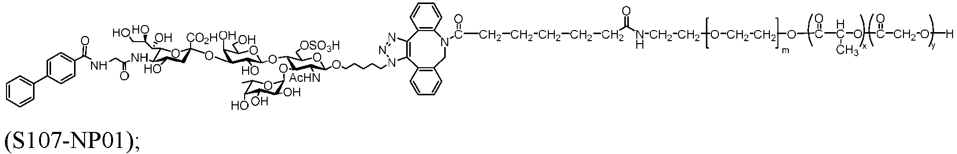 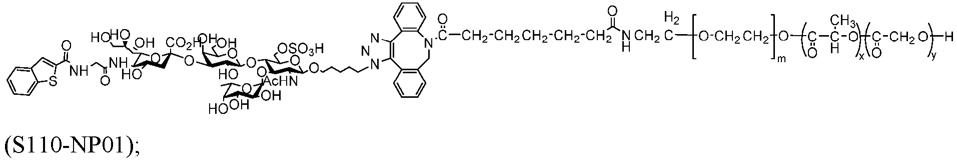 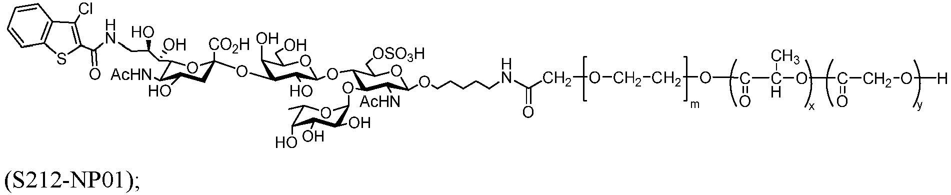 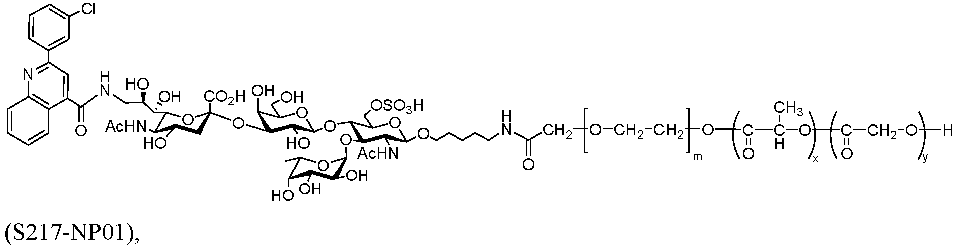 wherein, for each occurrence independently, x is an integer from 90 to 140; y is an ineteger from 10 to 75; and m is an integer from 90 to 140.

### EXEMPLIFICATION

### Example 1A: Synthesis of the S-Compounds - Procedure A

The following reaction schemes were used for synthesizing 6-sulfo-sialyl Lewis X derivatives (S-series of compounds).

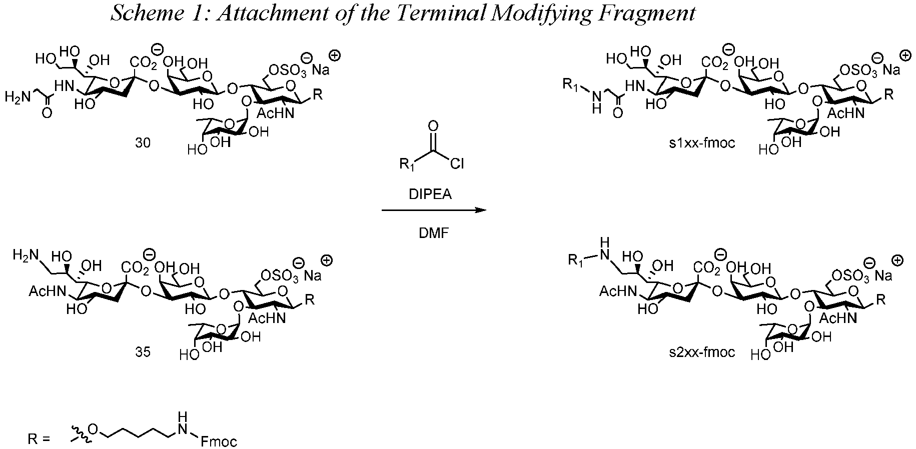

In Scheme 1, notation S1XX denotes compounds in which the substituent at position 5 of the terminal sialic acid is one of the fragments described below in Table 1, and notation S2XX denotes compounds in which position 9 of the terminal sialic acid is substituted with one of the fragments described below in Table 1.

### I. Synthesis of Precursors 30 and 35

Precursor compounds 30 and 35 of Scheme 1 were synthesized as follows.

### 1. Synthesis of galactose building block.

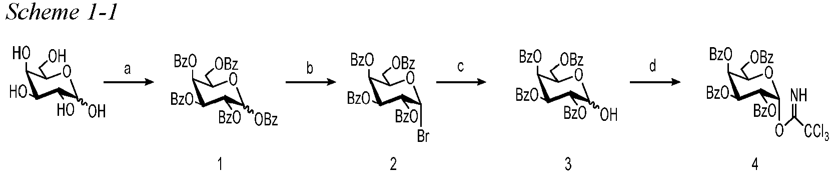

The following conditions were used in the reactions shown in Scheme 1-1: a) BzCl, pyridine; b) AcOH, Ac₂O, HBr; c) Ag₂CO₃, Acetone:H₂O; d) CNCCl₃, DBU.

More specifically, to a 1L round bottom in an ice bath, galactose (30g, 166 mmol) was dissolved in 400 mL pyridine. To the cooled solution was added benzoyl chloride (154 mL, 1.33 mol) dropwise over 1 h. After all benzy chloride has been added, the reaction was taken out of the ice bath and mixed at room temperature for 2 hrs which results in significant salt formation. The reaction was monitored by TLC (30% EtOAC/Hex, *R_{f}* = 0.8) and when complete, salt was removed by vacuum filtration. Excess pyridine was evaporated under reduced pressure, the crude material was dissolved in EtOAc (1L) successively washed with 5% H₂SO₄ (5 x 100mL), saturated CuSO₄ (2 x 100mL), 1M HCl (2 x 100 mL), brine (2 x 100mL), dried over MgSO₄, and solvents were evaporated under reduced pressure. The resulting oil was transferred to a 2L round bottom equipped with a condenser and a magnetic stir bar and placed in an oil bath. With vigorous stirring, the round bottom was heated to reflux with addition of MeOH (~1L in total). This process results in the formation of the desired per-benzoylated galactose **1,** which was collected by vacuum filtration and used in the next step without further purification. In an ice bath, **1** was suspended in acetic anhydride (50 mL) and acetic acid (80 mL) with vigorous stirring. To this suspension was added hydrogen bromide in acetic acid (204mL, 833 mmol) dropwise over 1 h; the mixture was brought to room temperature. After ~ 2 hrs the reaction mixture turns a translucent orange. When total conversion was observed by TLC (20% EtOAc/Hex, *R_{f} =* 0.50), the reaction was diluted with EtOAc (1L), extracted with DI water (5 x 200 mL), quenched with sodium bicarbonate (100 mL of DI water, then solid sodium bicarbonate was added), brine (2 x 100 mL), dried over MgSO₄, and reduced volume to yield 2 as a white solid (104 g, 95% over two-steps). To a solution of 2 (104 g, 158 mmol) in acetone (300 mL) and water (14.2 mL) was added freshly prepared Ag₂CO₃ (22 g, 79 mmol). The reaction was kept in the dark at room temperature and monitored by TLC (30% EtOAc/Hex, *R_{f} =* 0.45). When all starting material has been consumed, the reaction was filtered over celite, reduced *en vacuo,* dissolved in EtOAc (500 mL), washed with saturated sodium bicarbonate (3 x 100 mL), brine (2 x 100 mL), dried over MgSO₄, and volume reduced to yield the desired product **3.** Per-benzoylated galactol **3** was dissolved in DCM (316 mL) containing trichloroacetonitrile (48 mL, 470 mmol). To this solution was added DBU dropwise until the reaction's pH reaches ~ 10. The progress of the reaction was monitored by TLC (15% EtOAc/Hex, *R_{f} =* 0.2) and when all starting material has was consumed, the reaction volume was reduced. The desired product **4** was purified by flash chromatography (15% EtOAc/Hex) to yield the desired α-imidate as a white solid (79.05 g, 68% over two-steps) with ¹H data agreeing with that reported in the literature.

### 2. Synthesis of N-trifluoroacetyl-glucosamine building block.

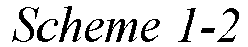

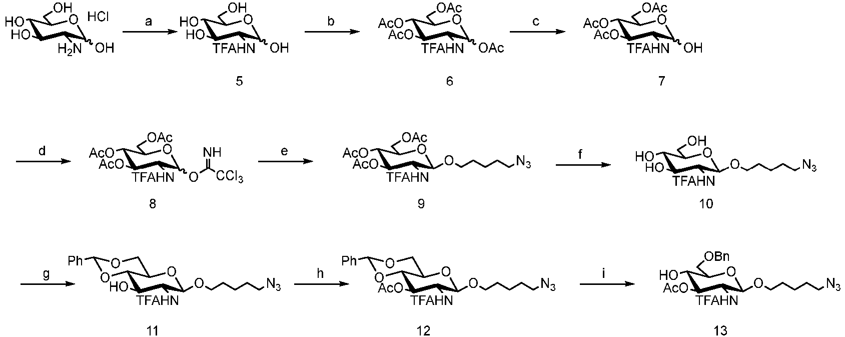

The following conditions were used in the reactions shown in Scheme 1-2: a)Ethyl trifluoroacetate, sodium methoxide; b) Ac₂O; c) Hydrozonium acetate; d) CNCCl, DBU; e) 5-azidopentan-1-ol, TMSOTf; f) Sodium methoxide; g) Benzaldehdye dimethyl acetal; CSA; h) Ac₂O; i) TFA, triethylsilane.

More specifically, to a 500 mL round bottom containing MeOH (130 mL) and sodium methoxide (51 mL of a 1M stock solution) was added glucosamine hydrochloride (GlcNH₂, 10 g, 46.3 mmol). The solution was stirred at room temperature until GlcNAH₂ has dissolved at which time, ethyl trifluoroacetate (6.2 mL, 51 mmol) was added in one portion. The mixture was stirred at room temperature and monitored by TLC (20% MeOH/DCM, *R_{f} =* 0.5). Upon completion, the solvent was reduced *en vacuo* to provide crude **5. 5** was dissolved in pyridine (70 mL) in an ice bath. To the solution was added acetic anhydride (22 mL, 231.5 mmol) dropwise over 30 mins at which, the reaction was warmed to room temperature. Evaluation by TLC (40% EtOAC/hex, *R_{f} =* 0.48) shows the reaction complete after 2 hrs. The crude reaction was diluted with EtOAc (500 mL) and water-soluble impurities were extracted with HCl (5 x 100 mL) and brine (3 x 100 mL). The organic layer was dried over MgSO₄ and dried under reduced pressure to yield tetra-acetate intermediate 6. Crude intermediate 6 was dissolved in THF (156 mL) and to the solution was added freshly prepared methanolic solution of 2M hydrazonium acetate (27.8 mL, 55.5 mmol). Anomeric acetate deprotection was monitored by TLC (1:1 EtOAc/Hex, *R_{f} =* 0.47) and when all starting material was consumed, the reaction volume was reduced and crude 7 was dissolved in EtOAc (500 mL), washed with 1 M HCl (3 x 100 mL), brine (2 x 100 mL), dried over MgSO₄, and dried under reduced pressure. Intermediate **7** was dissolved in DCM (176 mL) and to this solution was added trichloroacetonitrile (9.2 mL, 96.6 mmol). The reaction was mixed at room temperature and the pH was adjusted to 9 by addition of DBU. Product formation was observed by TLC (40% EtOAc/Hex, *R_{f} =* 0.51) after 1 hr. The reaction volume was reduced *en vacuo* and the product was purified by flash chromatography (20 → 40% EtOAc/Hex) to provide the desired imidate **8** as a white foam (15.2 g, 52% over 4-steps). To a 500 mL round bottom was added **7** (12.7 g, 23.2 mmol), 5-azidopentan-1-ol (3.6 g, 28 mmol), and 4Å molecular sieve (5 g). The components were dissolved in DCM (232 mL) and stirred at room temperature for 30 mins after which the reaction was put in an ice bath for 30 mins. To the chilled solution was added TMSOTf (840 µL, 4.64 mmol) and the reaction was monitored by TLC (40% EtOAc/Hex, *R_{f} =* 0.4). Upon conversion of starting material to the glycosylated product, the reaction was quenched by the addition of triethylamine (970 µL, 6.96 mmol) and the product, **9,** was purified by flash chromatography (35% EtOAc/Hex) to yield a white solid (10 g, 85%). Protected advanced intermediate **9** (10 g, 19.5 mmol) was suspended in MeOH (195 mL) and the pH was adjusted to 8 by the addition of sodium methoxide. Desired triol was observed with TLC (10% MeOH/DCM, *R_{f} =* 0.45) and reaction was neutralized with Dowex 50WX8 H⁺ resin. The reaction was filtered and the MeOH was removed under reduced pressure to yield a **10** as a light, yellow wax which was advanced to the next step without further purification. **10** (7.5 g, 19.4 mmol) was dissolved in ACN (190 mL) and to this solution was added benzaldehyde dimethyl acetal (3.2 mL, 21.3 mmol), and camphorsulfonic acid until the pH reached 3. The reaction mixture was moved to a rotary evaporator where it was stilled at 50°C under reduced pressure. The reaction was monitored by TLC (40% Acetone/Hex, *R_{f} =* 0.6) and after all starting material was consumed, the pH of the solution was adjusted to 7 with triethylamine. The solvent was removed to yield benzylidene intermediate **11** as brown solid which was dissolved in DCM (87 mL). To the reaction was added pyridine (6 mL, 77.6 mmol) and acetic anhydride (3.6 mL, 38.8 mmol) which was stirred at room temperature. TLC (40% Acetone/Hex, *R_{f} =* 0.65) was used to monitor the reaction progress. After all starting material was consumed, the reaction was reduced, resuspended in EtOAc (300 mL), washed with 1 M HCl (3 x 50 mL), brine (2 x 50 ml), dried over MgSO₄, and dried *en vacuo.* The resulting solid was recrystallized using EtOAc and Hex to yield the 3-O-Ac product **12** (6.2 g, 67% over 3-steps). Selective benzylidene ring opening was achieved by dissolving **12** (2g, 3.88 mmol) and DCM (33 mL). The reaction was placed in an ice bath, and to the chilled solution was added triethylsilane (3.2 mL, 19.4 mmol) and trifluoroacetic acid (1.48 mL, 19.4 mmol). The reaction was monitored by TLC (40% Acetone/Hex, *R_{f} =* 0.62) and when all **12 was** consumed, the reaction was diluted with EtOAc (200 mL) and extracted with sodium bicarbonate (3 x 50 mL), brine (2 x 50 mL), and dried over MgSO₄, The crude reaction mixture was purified by flash chromatography (30% Acetone/Hex) to provide 13 (1.52 g, 76%) as a clear oil which upon standing turned to a white solid.

### 3. Disaccharide glycosylation

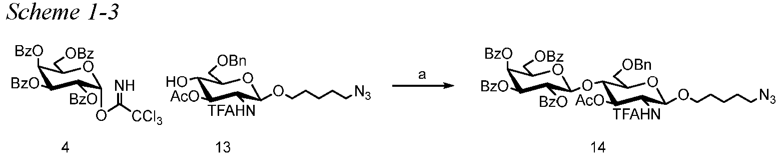

The following conditions were used in the reaction shown in Scheme 1-3: TMSOTf, 4A MS.

More specifically, acceptor **13** (1.52 g, 3 mmol) and donor **4** (2.8 g, 3.75 mmol) were dissolved in DCM (30 mL) and to this solution was added 4Å MS (2g). The solution was stirred at room temperature for 30 mins then placed in an ice bath for 30 mins. To the cooled solution was added TMSOTf (135 µL, 0.75 mmol) in one portion. The reaction was kept on ice with vigorous stirring. After 30 mins, TLC (30% EtOAc/Hex, *R_{f} =* 0.35) indicates complete disappearance **4** and **13** with the creation of the desired disaccharide **14.** The reaction was quenched with triethylamine (167 µL, 1.2 mmol) and purified by flash chromatography (30% EtOAc/Hex) to produce pure **14** (1.3 g, 40%) as a white foam.

### 4. 6-O-Benzyl deprotection

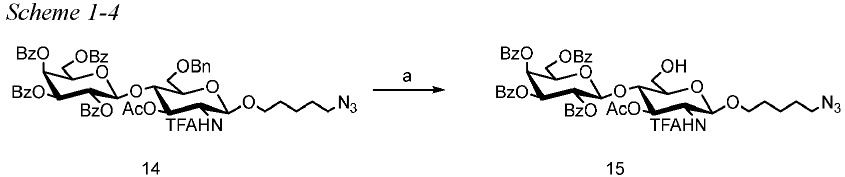

The following conditions were used in the reaction shown in Scheme 1-4: sodium dithionite / sodium bromate.

More specifically, full protected disaccharide **14** (1.1 g, 1 mmol) was dissolved in EtOAc (20 mL) and H₂O (20 mL) with vigorous mixing. To the biphasic solution was added sodium dithionite (870 mg, 5 mmol). The reaction was placed in an ice bath and sodium bromate (150 mg, 1 mmol) was added resulting in the *in-situ* formation of bromine radicals (solution turns yellow / orange). Reaction was monitored every 20 mins by TLC (40% Actone/Hex, *R_{f}* = 0.56). Additional sodium bromate (50 mg, 0.3 mol) was added as necessary to convert all **14 → 15.** Reaction was quenched by the addition of saturated sodium thiosulfate and purified by flash chromatography (30% acetone/hex) to yield the desired 3-OH product (0.62 g, 62%) as a white solid foam.

### 5. 6-O-sulfation, global deprotection, and amine acetylation.

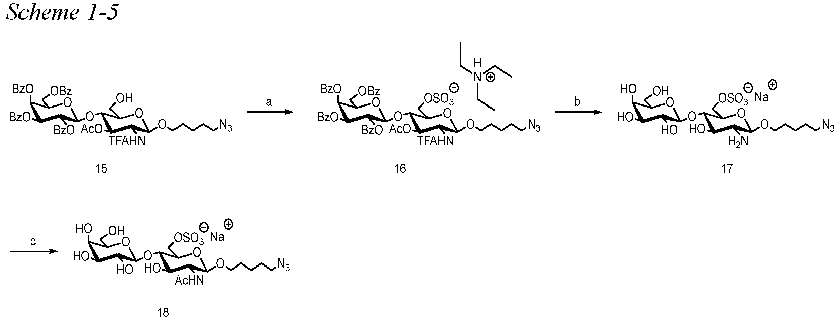

The following conditions were used in the reactions shown in Scheme 1-5: a) Chlorosulfonic acid, TEA; b) NaOH; c) Ac₂O.

More specifically, **15** (620 mg, 0.61 mmol) was dissolved in THF (5 mL) with triethylamine (1.3 mL, 9.2 mmol) in an ice bath to which was added chlorosulfonic acid (405 µL, 6.1 mmol). The reaction was mixed on ice for 1 hr, then brough to room temperature and mixed overnight. The reaction was monitored by TLC (10% MeOH/DCM, *R_{f}* = 0.58) desalted by LH-20 size-exclusion chromatography (eluting with 1:1 MeOH/DCM). Recovered 3-O-sulfated **16** was suspended in MeOH (8 mL) and H₂O (4 mL). To the reaction mixture was added NaOH (1.1 mL, 5.49 mmol, 5 M stock solution) and the solution was vigorously stirred at room temperature resulting in global deprotection to provide **17** which was observed by TLC (EtOH:NH₄OH:H₂O - 7:3:2, *R_{f}* = 0.82). The reaction pH was adjusted with conc. AcOH and the solvents were reduced by rotary evaporation. Crude deprotected **17** was redissolved in MeOH (10mL) and to the solution was added acetic anhydride (115 µL, 1.22 mmol) and triethylamine (170 µL, 1.22 mmol). Amine acetylation was achieved by mixing at room temperature. 6-sulfo-N-acetyllactosamine **(18)** was purified by Bio-Gel P-2 size chromatography with ammonium bicarbonate (100 mM) elution. Product containing fractions were freeze-dried to yield the desired product as a white fluffy solid (203 mg, 55% over 3-steps).

### 6. Chemical synthesis of 6-sulfo-N-acetylglucosamine

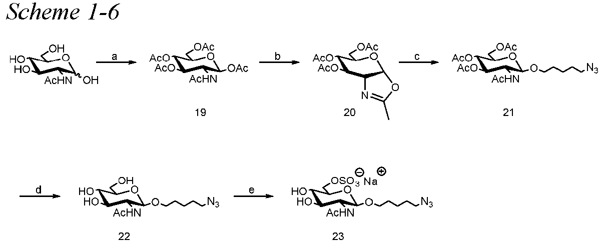

The following conditions were used in the reactions shown in Scheme 1-6: a) Ac₂O, sodium acetate; b) TMSOTf; c) 5-azidopentan-1-ol, TMSOTf; d) sodium methoxide; e) sulfur trioxide-pyridine.

More specifically, N-acetylglucosamine (100 g, 450 mmol) was added to a solution of refluxing of acetic anhydride (340 mL, 3375 mmol) and sodium acetate (37.2 g, 450 mmol) in portions. The mixture was stirred at reflux until all material was dissolved. TLC (100% EtOAc, *R_{f}* = 0.47) indicates reaction was complete. Excess acetic anhydride was removed under reduced-pressure and the crude material was dissolved in EtOAc (1 L) and washed with saturated sodium bicarbonate (10 x 150 mL), brine (3 x 100), dried over MgSO₄, and reduced *en vacuo* to provide per-acetylated N-acetyl glucosamine (**19,** 129 g, 74%) as a white solid. **19** (5.4 g, 13.9 mmol) was dissolved in DCE (69 mL) and to this solution was added TMSOTf (2.5 mL, 13.87 mmol). The solution was warmed to 60°C to facilitate oxazoline formation which was monitored by TLC (40% acetone/hex, *R_{f}* = 0.3). The reaction was quenched with TEA (2.2 mL, 15.18 mmol), the solvent was reduced, and the product was purified by flash chromatography (40% acetone/hex) to yield **20** (3.9 g, 85%) as a clear oil. Tri-acetylated oxazoline 20 (4.36 g, 13.24 mmol) was dissolved in DCE (66 mL). To this solution was added 5-azidopentan-1-ol (3.4 g, 26.48 mmol) and 4Å MS (3 g). The combination was mixed at room temperature after which, TMSOTf (2.4 mL, 13.24 mmol) was added and the reaction was warmed to 60°C to facilitate glycosylation. The reaction was monitored by TLC (90% EtOAc/Hex, *R_{f}* = 0.55) and when all starting material was converted to product, the reaction was quenched with TEA (1.84 mL, 13.24 mmol) and cooled to room temperature. The solvent was removed under reduced pressure and glycosylated N-acetylglucosamine was recovere by flash chromatography (80→90% EtOAc/Hex) to provide **21** (5 g, 80%) as a white solid. **21** (14.38, 31.37 mmol) was dissolved in MeOH (156 mL) and the solution was adjusted to pH 9 with sodium methoxide. The reaction was monitored with TLC (20% MeOH/DCM, *Rᵣ* = 0.76) and when complete, the pH was neutralized with Dowex 50WX8 H⁺ resin. The resin was removed by filtration and the MeOH was removed by reduced pressure to yield triol **22** (9.3 g, 90%) as a white solid. 6-O-sulfation was added by dissolving **22** (600 mg, 1.8 mmol) in DMF (36 mL) and placing the mixture in an ice bath. To the cooled solution was added sulfur trioxide-pyridine complex (287 mg, 1.8 mmol). The reaction was stirred overnight at 4°C for three days with additional of sulfur trioxide-pyridine (287 mg, 1.8 mmol) daily. The reaction was monitored by TLC (20% MeOH/DCM, *Rf* = 0.23) and quenched with aliquots of NaOH (5M, pH → 8). The solvent was removed, and the reaction was purified by Bio-Gel size-exclusion chromatography. Product containing fractions were freeze-dried to yield **23** (514 mg, 69%) as a white powder.

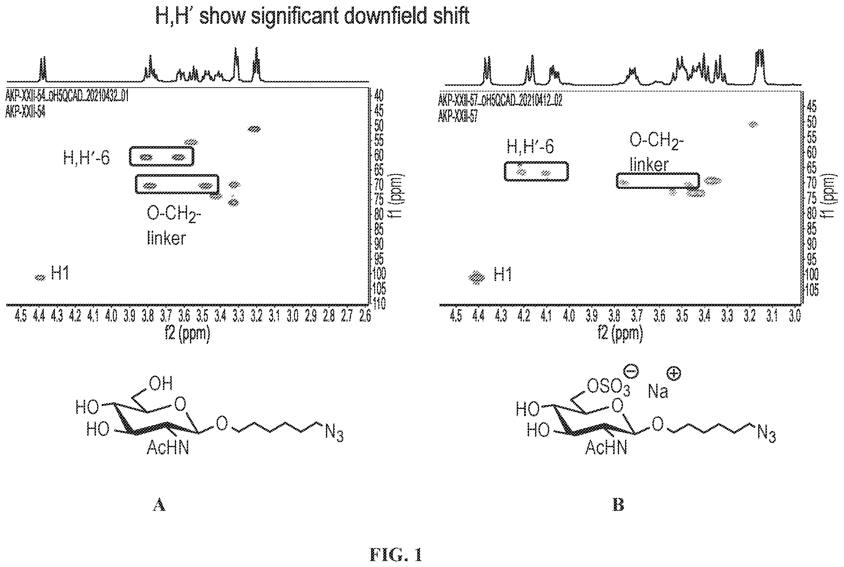

The 2D ¹H-NMR spectra of compounds 22 and 23 of Scheme 1-6 are shown in FIGs. 1A and 1B.

### 7. Enzymatic installation of β1,4-Gal for 6-sulfo-N-acetyllactosamine formation

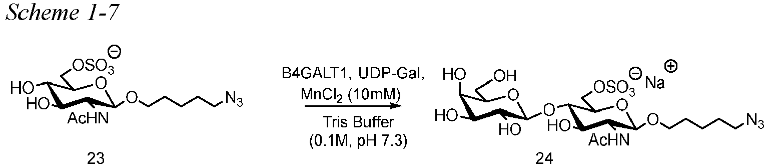

Installation of the β1,4-Gal was achieved by dissolving 5 mg 6S-GlcNAc (**23, 5** mg, 11.7 µmol) and UDP-Galactose (10 mg, 17.6 µmol) in Tris Buffer (585 µL, pH 7.3, 0.1M) containing MnCl₂ (10 mM). To this solution was added B4GALT1 (50 µg, 1% *wt*/*wt*) and calf intestine alkaline phosphatase (CIAP, 5.85 µL, 1kU stock). The reaction was incubated at 37°C for three hours. Reaction progress was monitored by LC-ESI-MS equipped with a Waters XBridge BEH, Amide column, 2.5 µm, 130 Å, 2.1 × 150 mm (Flow rate 0.25 mL/min, A = 10 mM ammonium formate, B = ACN. Linear gradient 80%→60% B over 18 mins).

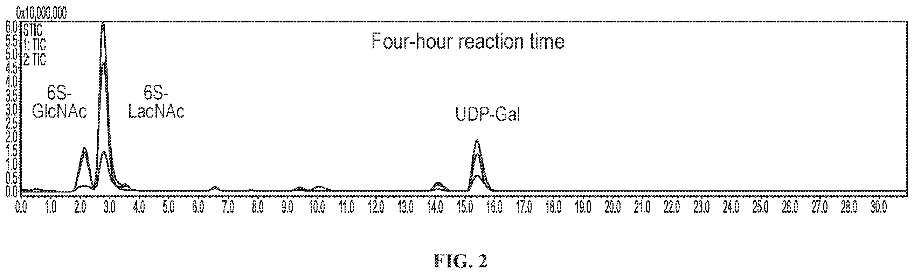

The reaction was monitored by tracing the reactants and products using Hydrophilic interaction liquid chromatography/electrospray ionization mass spectrometry (HILIC-LC/ESI-MS). The results of such tracing are shown in FIG. 2.

Residual starting material was converted to product via the addition of excess UDP-Gal (5 mg, 5.85 µmol) and B4GALT1 (25 µg). The reaction mixture was filtered through a PALL Nanosep^{®} Centrifuge spin filter (3k MWCO) and the filtrate purified by Bio-Gel P2 size-exclusion chromatography. Product containing fractions were freeze-dried to provide desired disaccharide **24** (4.8 mg, 70%).

### 8. 6S-LacNAc azide reduction and Fmoc installation

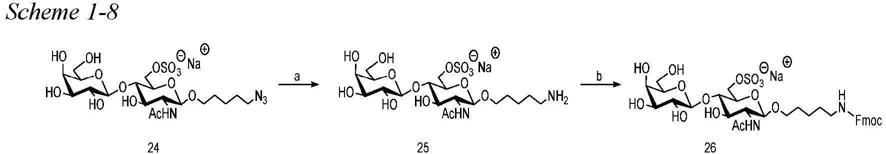

The following conditions were used in the reactions shown in Scheme 1-8: a) Pd/C, H2, b) Fmoc-OSu.

More specifically, **24** (100 mg, 0.17 mmol) was dissolved in a 1:1 mixture of H₂O/*t-*BuOH (8 mL) containing 5% *wt*/*wt* Pd/C. The reaction vessel was evacuated thrice and kept under an atmosphere of H₂ to facilitate the reduction of the azide to a primary amine. Upon complete reduction of the azide, the reaction was filtered through a 0.22 µm syringe filter and the filtrate was freeze-dried to provide **25** which was subsequently dissolved in H₂O (8 mL) containing NaHCO₃ (0.51 mmol). In a separate vessel was dissolved FmocOSu (114 mg, 0.34 mmol) in ACN (8 mL). To the solution ACN was added, dropwise, the aqueous solution containing **25.** The mixed solutions were vigorously vortexed to yield **26** whose formation was monitored via C18 TLC (30% ACN/water; *R_{f}* = 0.3). The mixture was reduced *en vacuo* and the product was purified by Bio-Gel P2 size-exclusion chromatography to yield **26** (103 mg, 77% over 2-steps).

¹H NMR (600 MHz, D₂O) δ 7.23-7.21 (m, 4H, Fmoc H1, H1', H4, H4'), 6.95 (m, 4H, Fmoc H2, H2', H3, H3'), 4.40-4.39 (d, J = Hz, 1H, Gal H1), 4.21-4.16 (m, 3H, GlcNAc H1,H6), 4.06-4.02 (m, 2H, Fmoc H8), 3.79-3.78 (m, 1H, Gal H4), 3.73-3.40 (m, 11H, Gal, H2, H3, H5, H6, GlcNAc H2, H3, H4, H5, Fmoc H7, Lipid Chain H5a), 3.11-3.10 (m, 1H, H5b), 2.67-2.65 (m, 1.5H, H1), 2.32 (m, 0.5H, H1), 1.79 (s, 3H, GlcNAc, Ac, CH₃), 1.16-1.12 (m, 1.5H, H2), 1.00-0.98 (m, 2H, H4), 0.87-0.83 (m, 1.5H, H3), 0.69 (m, 1H, H2, H3). ¹³C NMR (151 MHz, D₂O) δ 173.90 (GlcNAc, -CO-), 157.49 (Fmoc, -CO-), 143.62 (Fmoc C6, C6'), 140.76 (Fmoc C5, C5'), 127.60 (Fmoc C3, C3'), 127.09 (Fmoc C2, C2'), 124.86 (Fmoc C1, C1'), 119.75 (Fmoc C4, C4'), 102.37 (Gal, C1), 101.03 (GlcNAc, C1), 77.19 (Gal, C3), 75.22 (GlcNAc, C4), 72.47 (GlcNAc, C5), 72.42 (Gal, C5), 72.15 (GlcNAc, C3), 70.95 (Gal, C2), 70.01 (C5), 68.58 (Gal, C4), 66.11 (GlcNAc, C6), 65.88 (Fmoc, C8), 60.94 (Gal, C6), 54.99 (GlcNAc, C2), 46.77 (Fmoc, C7), 40.27 (C1), 28.50 (C4), 28.20 (C2), 22.21 (C3, GlcNAc, CH₃).

### 9. Enzymatic installation of α2,3 Neu5Az

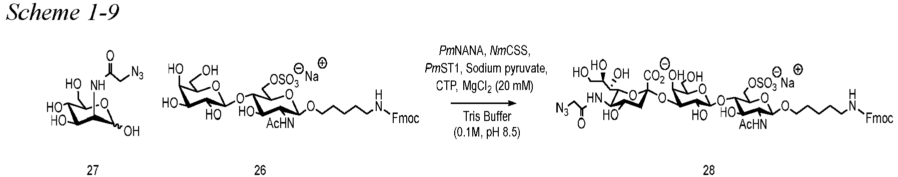

Amine protected 6-sulfo-LacNAc (**26**, 50 mg, 63 µmol) and ManNAz (**27,** 33 mg, 126 µmol) were dissolved in Tris Buffer (0.1 M, pH 8.5, 6.3 mL) containing MgCl₂(20 mM), sodium pyruvate (630 µmol), and cytidine-5'-triphosphate (CTP, 252 µmol). To this mixture was added sialic acid aldolase¹ (*Pm*NANA, 660 µg), CMP-sialic acid synthetase² (*NmCSS,* 660 µg), and Sialyltransferse 1 ³⁻⁵ (*Pm*ST1, 500 µg). The reaction was incubated at 37°C with mild agitation for 2 hrs. The reaction was quenched by the addition of EtOH (8 mL) and placed in the freeze. Protein precipitation was removed via centrifugation and the supernatant was reduced by rotary evaporation. The crude reaction mixture was purified by preparative HPLC (Agilent 1200, Eclipse XDB-C18, 21.2 x 250 mm, 7 µm. A solvent = 50 mM ammonium bicarbonate; B = ACN, λ = 262 m) with a linear gradient of 10→60% B over 30 mins, flow rate 20 mL/min. Compound containing fractions were freeze-dried to trisaccharide **28** (59 mg, 83%)

¹H NMR (600 MHz, D₂O) δ 7.57-7.52 (m, 2H, Fmoc H4, H4'), 7.41-7.35 (m, 2H, Fmoc H1, H1'), 7.22-7.14 (m, 4H, Fmoc H2, H2', H3, H3'), 4.46-4.45 (d, 1H, Gal H1), 4.27-4.15 (m, 5H, Fmoc H8, GlcNAc H1, H6), 4.00-3.99 (m, 1H, GlcNAc H4), 3.93 (m, 3H, Fmoc H7, Neu5Az, Az, -CH₂), 3.83-3.75 (m, 4H, Gal H4, Neu5Az H5, H6, H9a), 3.62-3.46 (m,12H, H5a, Gal H3, H5, H6, GlcNAc H2, H3, H5, Neu5Az H4, H7, H8, H9b), 3.44-3.41 (t, 1H, Gal H2), 3.26-3.14 (m, 1H, H5b), 2.74 (m, 1.5H, H1), 2.64-2.62 (dd, 1H, Neu5Az H3a), 2.29 (m, 0.5H, H1), 1.81 (s, 3H, GlcNAc Ac, -CH₃), 1.71-1.67 (t, 1H, Neu5Az H3b), 1.24 (m, 1.5H, H2), 1.08 (m, 2H, H4), 0.94 (m, 1.5H, H3), 0.67 (m, 1H, H2, H3). ¹³C NMR (151 MHz, D₂O) δ 174.12 (Neu5Az, C1), 173.72 (GlcNAc, Ac, -CO-), 170.98 (Neu5Az, Az, -CO-), 157.94 (Fmoc, C9, -CO-), 143.67 (Fmoc, C6), 140.86 (Fmoc, C5), 127.85 (Fmoc, C3), 127.29 (Fmoc, C2), 124.87 (Fmoc, C1), 119.96 (Fmoc, C4), 102.07 (Gal, C1), 101.01 (GlcNAc, C1), 99.56 (Neu5Az, C2), 77.18 (Gal, C3), 75.27 (GlcNAc, C4), 74.95 (Neu5Az, C7), 72.46 (GlcNAc, C5), 72.43 (GlcNAz, C3), 72.18 (Gal, C5), 71.39 (Gal, C4), 70.14 (C5), 69.38 (Gal, C2), 68.15 (Neu5Az, C4), 67.96 (Neu5Az, C8), 67.38 (Neu5Az, C6), 66.17 (GlcNAz, C6), 65.74 (Fmoc, C8), 62.46 (Neu5Az, C9), 60.93 (Gal, C6), 54.96 (GlcNAz, C2), 51.83 (Neu5Az, Az, CH₂), 51.74 (Neu5Az, C5), 46.98 (Fmoc, C7), 40.13 (C1), 39.53 (Neu5Az, C3), 28.26 (C4), 28.10 (C2), 22.11 (GlcNAc, Ac, CH₃ and C3).

### 10. Enzymatic installation of α1,3 Fucose

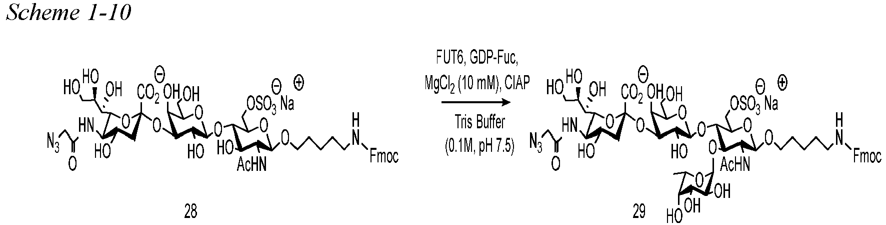

Trisaccharide **28** (59 mg, 52 µmol) was dissolved in Tris buffer (5.2 mL, 0.1 M, pH 7.5) containing MgCl₂ (10 mM) and GDP-L-fucose (78 µmol). To this solution was added fucosyltransferase 6 (FUT6, 1.7 mg) and CIAP (52 µL, 1kU stock solution). The mixture was incubated at 37°C overnight. The reaction was quenched with addition of EtOH (5.2 mL) and the solution was kept at 0°C for 1 hr. The insoluble protein was removed by centrifugation and the supernatant was reduced *en vacuo* and purified by preparatory HPLC as outlined for **28.** Compound containing fractions were freeze-dried to yield tetrasaccharide **29** (52 mg, 78%) as a white powder.

¹H NMR (600 MHz, D₂O) δ 7.75-7.74 (d, 2H, Fmoc H4, H4'), 7.54-7.52 (d, 2H, Fmoc H1, H1'), 7.35-7.33 (t, 2H, Fmoc H3, H3'), 7.28-7.25 (t, 2H, Fmoc H2, H2'), 4.95-4.94 (d, 1H, Fuc H1), 4.67-4.61 (m, 1H, Fuc H5), 4.58 (m, 0.5H, Fmoc H8), 4.48-4.44 (m, 2.5H, Gal H1, Fmoc H8), 4.36 (m, 1H, GlcNAc H1), 4.24-4.19 (m, 2H, GlcNAc H6), 4.12 (m, 1H, Fmoc H7), 3.98-3.96 (m, 1H, Gal H3), 3.93 (s, 2H, Neu5Az, Az, -CH₂), 3.87-3.80 (m, 4H, Fuc H4, Gal H4, Neu5Az H5, H6), 3.76-3.61 (m, 9H, H5a, Fuc H3, Gal H5, GlcNAc H2, H3, H5, Neu5Ac H4, H7,H9a), 3.54-3.51 (m, 4H, Fuc H2, Gal H6, Neu5Ac H9b), 3.47-3.44 (m, 2H, GlcNAc H4, Neu5Ac H8), 3.39-3.36 (m, 2H, H5b, Gal H2), 2.80 (m, 1.5H, H1), 2.64-2.61 (dd, 1H, Neu5Az H3a), 2.33 (m, 0.5H, H1), 1.81 (s, 3H, GlcNAc Ac,-CH₃), 1.71-1.67 (t, 1H, Neu5Ac H3b), 1.32 (m, 1.5H, H2), 1.15 (m, 2H, H4), 1.04-0.99 (m, 4.5H, H3, Fuc H6), 0.71 (m, 1H, H2, H3). ¹³C NMR (151 MHz, d2o) δ 173.98 (Neu5Az, C1), 173.40 (GlcNAc, Ac, -CO-), 171.01(Neu5Az, Az, -CO-), 158.18 (Fmoc, C9, -CO-), 143.77 (Fmoc, C6), 140.94 (Fmoc, C5), 127.91 (Fmoc, C3), 127.36 (Fmoc, C2), 124.88 (Fmoc, C1), 120.03 (Fmoc, C4), 101.09 (Gal, C1), 100.75 (GlcNAc, C1), 99.15 (Neu5Az, C2), 98.47 (Fuc, C1), 75.35 (Gal, C3), 74.69 (GlcNAc, C4), 74.63 (Neu5Az, C7), 72.81 (GlcNAc, C5), 72.62 (GlcNAc, C3), 72.53 (Fuc, C4), 71.82 (Gal, C5), 71.11 (Gal, C4), 70.21 (C5), 69.30 (Gal, C2), 69.10 (Fuc, C3), 67.97 (Neu5Az, C4), 67.94 (Neu5Az, C8), 67.70 (Fuc, C2), 67.10 (Neu5Az, C6), 66.62 (Fuc, C5), 65.80 (GlcNAc, C6), 65.61 (Fmoc, C8), 62.54 (Neu5Az, C9), 61.31 (Gal, C6), 55.60 (GlcNAc, C2), 51.82 (Neu5Az, Az, CH₂), 51.73 (Neu5Az, C5), 47.19 (Fmoc, C7), 40.04 (C1), 39.58 (Neu5Az, C3), 28.18 (C4), 28.06 (C2), 22.11 (GlcNAc, Ac, CH₃), 22.07 (C3), 15.22 (Fuc, C6).

### 11. Azide reduction; preparation of handle for library generation.

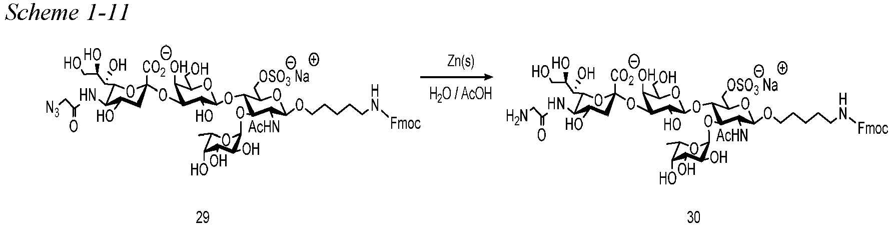

Tetrasaccharide 29 (52 mg, 41 µmol) was dissolved in H₂O (2 mL) and to this solution was added HCl washed Zn (266.5 mg, 4.1 mmol) and AcOH (820 µmol). The reaction was placed in an ice-bath and vigorously mixed for 1 hr. Reaction was monitored by ESI-MS when full-conversion to 30 was achieved, the reaction was filtered through a pad of celite and dried under reduced-pressure. The crude reaction mixture was purified by Bio-Gel P2 size-exclusion chromatography. Compound containing fractions were freeze-dried to yield reduced tetrasaccharide **30** (42 mg, 82%).

¹H NMR (600 MHz, D₂O) δ 7.77-7.76 (d, 2H, Fmoc H4, H4'), 7.55-7.54 (d, 2H, Fmoc H1, H1'), 7.36-7.34 (t, 2H, Fmoc H3, H3'), 7.29-7.27 (t, 2H, Fmoc H2, H2'), 4.95 (d, 1H, Fuc H1), 4.67-4.61 (m, 1.5H, Fuc H5, Fmoc H8), 4.48-4.46 (m, 2.5H, Gal H1, Fmoc H8), 4.37 (m, 1H, GlcNAc H1), 4.22-4.15 (m, 3H, GlcNAc H6, Fmoc H7), 3.97-3.96 (m, 1H, Gal H3), 3.87-3.36 (m, 23H, H5, Fuc H2, H3, H4, Gal H2, H4, H5, H6, GlcNAc H2, H3, H4, H5, Neu5Az, H4, H5, H6, H7, H8, H9, Az, -CH₂), 2.81 (m, 1.5H, H1), 2.64-2.61 (dd, 1H, Neu5Az H3a), 2.34 (m, 0.5H, H1), 1.82 (s, 3H, GlcNAc Ac,-CH₃), 1.70-1.66 (t, 1H, Neu5Ac H3b), 1.33 (m, 1.5H, H2), 1.15 (m, 2H, H4), 1.04-1.00 (m, 4.5H, H3, Fuc H6), 0.72 (m, 1H, H2, H3). ¹³C NMR (151 MHz, D₂O) δ 174.00 (Neu5Ac, C1), 173.91 (GlcNAc, Ac, -CO-), 167.38 (Neu5Az, Az, -CO-), 158.16 (Fmoc, C9, -CO-), 143.79 (Fmoc, C6), 140.94 (Fmoc, C5), 127.92 (Fmoc, C3), 127.37 (Fmoc, C2), 124.89 (Fmoc, C1), 120.03 (Fmoc, C4), 101.18 (Gal, C1), 100.75 (GlcNAc, C1), 99.43 (Neu5Ac, C2), 98.50 (Fuc, C1), 75.24 (Gal, C3), 74.71 (GlcNAc, C4), 74.65(Neu5Ac, C7), 72.84 (GlcNAc, C5), 72.75 (GlcNAc, C3), 72.53 (Fuc, C4), 71.82 (Gal, C5), 71.14 (Gal, C4), 70.22 (C5), 69.29 (Gal, C2), 69.10 (Fuc, C3), 68.36 (Neu5Ac, C4), 67.91 (Neu5Ac, C8), 67.70 (Fuc, C2), 67.11 (Neu5Ac, C6), 66.62 (Fuc, C5), 65.82 (GlcNAc, C6), 65.59 (Fmoc, C8), 62.32 (Neu5Ac, C9), 61.35 (Gal, C6), 55.62 (GlcNAc, C2), 51.74 (Neu5Ac, C5), 47.22 (Fmoc, C7), 40.44 (Neu5Az, Az, CH₂), 40.04 (C1), 39.69 (Neu5Ac, C3), 28.17 (C4), 28.06 (C2), 22.12 (GlcNAc, Ac, CH₃), 22.06(C3), 15.24 (Fuc, C6).

Synthesis of 6-Sulfo-9-amino-Sialyl Lewis X was performed using the same protocol as described above with respect to Schemes 1-8 through 1-11.

### 12. 6-Sulfo-9-amino-Sialyl Lewis X Construction

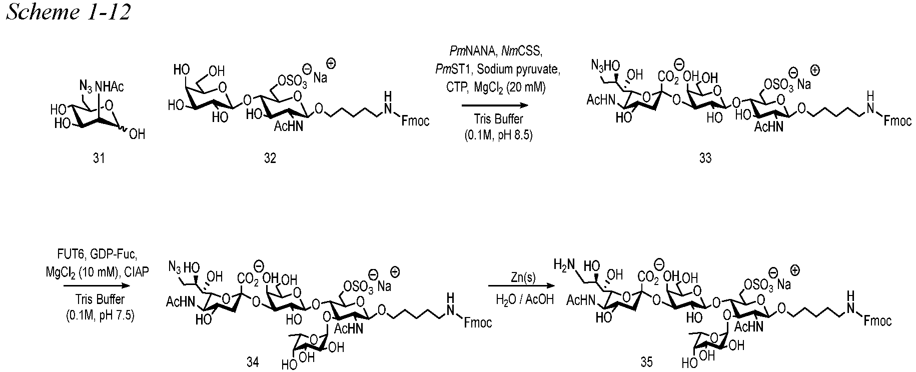

¹H-NMR spectra of compounds **33** through **35** were obtained:

### Compound 33:

¹H NMR (600 MHz, D₂O) δ 7.48-7.43 (m, 2H, Fmoc H4, H4'), 7.36-7.28 (m, 2H, Fmoc H1, H1'), 7.15-7.10 (m, 4H, Fmoc H2, H2', H3, H3'), 4.46-4.45 (d, 1H, Gal H1), 4.24-4.19 (m, 5H, Fmoc H8, GlcNAc H1, H6), 3.98-3.96 (m, 2H, Gal H4, GlcNAc H4), 3.87-3.82 (m, 2H, Fmoc H7, Neu5Az H6), 3.74-3.71 (t, 1H, Neu5Az H5), 3.62-3.46 (m,12H, H5a, Gal H3, H5, H6, GlcNAc H2, H3, H5, Neu5Az H4, H7, H8, H9a), 3.43-3.38 (m, 2H, Gal H2, Neu5Az H9b), 3.21-3.09 (m, 1H, H5b), 2.72 (m, 1.5H, H1), 2.63-2.60 (dd, 1H, Neu5Az H3a), 2.29 (m, 0.5H, H1), 1.90 (s, Neu5Az, Az, -CH₃), 1.80 (s, 3H, GlcNAc Ac, -CH₃), 1.69-1.65 (t, 1H, Neu5Az H3b), 1.21 (m, 1.5H, H2), 1.06 (m, 2H, H4), 0.91 (m, 1.5H, H3), 0.67 (m, 1H, H2, H3). ¹³C NMR (151 MHz, D₂O) δ 174.77 (Neu5Ac, Ac, -CO-), 174.07 (Neu5Ac, C1), 173.69 (GlcNAc, Ac, -CO-), 157.84 (Fmoc, C9, -CO-), 143.65 (Fmoc, C6), 140.83 (Fmoc, C5), 127.79 (Fmoc, C3), 127.23 (Fmoc, C2), 124.86 (Fmoc, C1), 119.92 (Fmoc, C4), 102.06 (Gal, C1), 101.03 (GlcNAc, C1), 99.50 (Neu5Ac, C2), 77.12 (Gal, C3), 75.34 (GlcNAc, C4), 74.96 (Neu5Ac, C7), 72.59 (GlcNAc, C5), 72.44 (GlcNAc, C3), 72.14 (Gal, C5), 70.10 (C5), 69.84 (Gal, C4), 69.37 (Gal, C2), 68.58 (Neu5Ac, C4), 68.31 (Neu5Ac, C8), 67.32 (Neu5Ac, C6), 66.05 (GlcNAc, C6), 65.79 (Fmoc, C8), 60.93 (Gal, C6), 54.96 (GlcNAc, C2), 52.97 (Neu5Ac, C9), 51.65 (Neu5Ac, C5), 46.90 (Fmoc, C7), 40.15 (C1), 39.64 (Neu5Ac, C3), 28.32 (C4), 28.12 (C2), 22.13 (GlcNAc, Ac, CH3 and C3), 22.01 (Neu5Ac, Ac, CH₃).

### Compound 34:

¹H NMR (600 MHz, D₂O) δ 7.75-7.74 (d, 2H, Fmoc H4, H4'), 7.53-7.52 (d, 2H, Fmoc H1, H1'), 7.35-7.33 (t, 2H, Fmoc H3, H3'), 7.28-7.25 (t, 2H, Fmoc H2, H2'), 4.95-4.94 (d, 1H, Fuc H1), 4.67-4.61 (m, 1H, Fuc H5), 4.58 (m, 0.5H, Fmoc H8), 4.50-4.48 (d, 1H, Gal H1), 4.37 (m, 1.5H, Fmoc H8), 4.36 (m, 1H, GlcNAc H1), 4.25-4.18 (m, 2H, GlcNAc H6), 4.12 (m, 1H, Fmoc H7), 3.97-3.95 (m, 2H, Gal H4, GlcNAc H4), 3.87-3.80 (m, 2H, Fuc H4, Neu5Az H6), 3.76-3.52 (m, 13H, H5a, Fuc H2, H3, Gal H5, H6, GlcNAc H2, H3, H5, Neu5Ac H4, H5, H7,H9a), 3.47-3.36 (m, 5H, H5b, Gal H2, GlcNAc H4, Neu5Ac H8, H9b), 2.80 (m, 1.5H, H1), 2.63-2.60 (dd, 1H, Neu5Az H3a), 2.33 (m, 0.5H, H1), 1.90 (s, Neu5Az, Az, -CH₃), 1.81 (s, 3H, GlcNAc Ac,-CH₃), 1.69-1.65 (t, 1H, Neu5Ac H3b), 1.32 (m, 1.5H, H2), 1.15 (m, 2H, H4), 1.14-0.99 (m, 4.5H, H3, Fuc H6), 0.71 (m, 1H, H2, H3). ¹³C NMR (151 MHz, D₂O) δ 174.78 (Neu5Ac, Ac, -CO-, 173.99 (Neu5Ac, C1), 173.36 (GlcNAc, Ac, -CO-), 158.16 (Fmoc, C9, - CO-), 143.77 (Fmoc, C6), 140.94 (Fmoc, C5), 127.91 (Fmoc, C3), 127.36 (Fmoc, C2), 124.89 (Fmoc, C1), 120.03 (Fmoc, C4), 101.06 (Gal, C1), 100.77 (GlcNAc, C1), 99.05 (Neu5Ac, C2), 98.48 (Fuc, C1), 75.37 (Gal, C3), 74.70 (GlcNAc, C4), 74.65 (Neu5Ac, C7), 72.83 (GlcNAc, C5), 72.65 (GlcNAc, C3), 72.62 (Fuc, C4), 71.83 (Gal, C5), 70.21 (C5), 69.56 (Gal, C4), 69.29 (Gal, C2), 69.11 (Fuc, C3), 68.48 (Neu5Ac, C4), 68.12 (Neu5Ac, C8), 67.71 (Fuc, C2), 67.04 (Neu5Ac, C6), 66.62 (Fuc, C5), 65.70 (GlcNAc, C6), 65.62 (Fmoc, C8), 61.31 (Gal, C6), 55.60 (GlcNAc, C2), 53.05 (Neu5Ac, C9), 51.65 (Neu5Ac, C5), 47.19 (Fmoc, C7), 40.05 (C1), 39.68 (Neu5Ac, C3), 28.18 (C4), 28.06 (C2), 22.12 (GlcNAc, Ac, CH3, C3), 21.99 (Neu5Ac, Ac, CH3), 15.22 (Fuc, C6).

### Compound 35:

¹H NMR (600 MHz, D₂O) δ 7.76-7.75 (d, 2H, Fmoc H4, H4'), 7.55-7.54 (d, 2H, Fmoc H1, H1'), 7.36-7.33 (t, 2H, Fmoc H3, H3'), 7.29-7.26 (t, 2H, Fmoc H2, H2'), 4.95 (d, 1H, Fuc H1), 4.67-4.61 (m, 1.5H, Fuc H5, Fmoc H8), 4.47-4.46 (m, 2.5H, Gal H1, Fmoc H8), 4.37 (m, 1H, GlcNAc H1), 4.29-4.15 (m, 3H, GlcNAc H6, Fmoc H7), 3.99-3.93 (m, 2H, Gal H3, Neu5Az H8), 3.88-3.85 (m, 1H, GlcNAc H3), 3.76-3.30 (m, 19H, H5, Fuc H2, H3, H4, Gal H2, H4, H5, H6, GlcNAc H2, H4, H5, Neu5Az, H4, H5, H6, H7, H8, H9a), 2.90-2.87 (t, 1H, Neu5Ac H9b), 2.80 (m, 1.5H, H1), 2.67-2.64 (dd, 1H, Neu5Az H3a), 2.34 (m, 0.5H, H1), 1.89 (s, Neu5Az, Az, -CH₃), 1.82 (s, 3H, GlcNAc Ac,-CH₃), 1.64-1.60 (t, 1H, Neu5Ac H3b), 1.32 (m, 1.5H, H2), 1.15-0.98 (m, 4.5H, H3, Fuc H6), 0.71 (m, 1H, H2, H3). ¹³C NMR (151 MHz, D₂O) δ 174.89 (Neu5Ac, Ac, -CO-), 174.03 (Neu5Ac, C1), 173.56 (GlcNAc, Ac, -CO-), 158.19 (Fmoc, C9, -CO-), 143.80 (Fmoc, C6), 140.96 (Fmoc, C5), 127.92 (Fmoc, C3), 127.37 (Fmoc, C2), 124.89 (Fmoc, C1), 120.03 (Fmoc, C4), 101.10 (Gal, C1), 100.77 (GlcNAc, C1), 98.99 (Neu5Ac, C2), 98.52 (Fuc, C1), 75.47 (Gal, C3), 74.80 (GlcNAc, C4), 74.58 (Neu5Ac, C7), 72.70 (GlcNAc, C5), 72.47 (GlcNAc, C3), 72.40 (Fuc, C4), 71.82 (Gal, C5), 70.23 (C5), 70.08 (Gal, C4), 69.28 (Gal, C2), 69.11 (Fuc, C3), 68.05 (Neu5Ac, C4), 67.83 (Neu5Ac, C8), 67.68 (Fuc, C2), 66.73 (Neu5Ac, C6), 66.61 (Fuc, C5), 65.78 (GlcNAc, C6), 65.59 (Fmoc, C8), 61.34 (Gal, C6), 55.67 (GlcNAc, C2), 51.55 (Neu5Ac, C5), 47.23 (Fmoc, C7), 42.24 (Neu5Ac, C9), 40.24 (C1), 40.04 (Neu5Ac, C3), 28.18 (C4), 28.07 (C2), 22.12 (GlcNAc, Ac, CH₃, C3), 21.96 (Neu5Ac, Ac, CH₃), 15.23 (Fuc, C6).

### 13. General protocol for Fmoc deprotection

The deprotection of the S-series compounds was generally carried out according to Scheme 1-13. Following deprotection, the compounds S1XX and S2XX shown in Scheme 13 are also referred to herein as S1XX-amine and S2XX-amine (see Example 8 for instance where S212-amine and S217-amine are conjugated to nanoparticles).

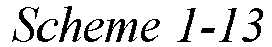

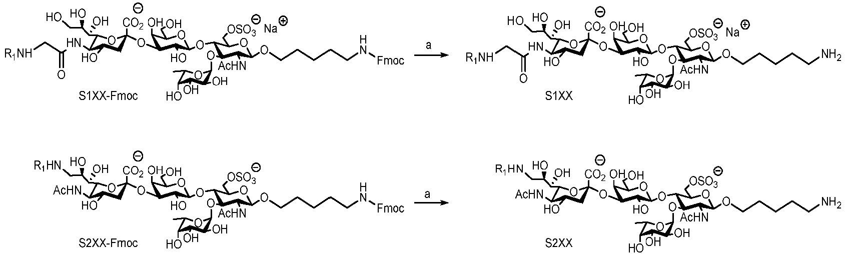

The following conditions were used for the reaction shown in Scheme 1-13: H₂O / Triethylamine.

Specifically, compound S1XX or S2XX (0.15 µmol) was dissolved in a solution of 4:1 *(v*/*v)* H₂O / Triethylamine (150 µL). The solution was periodically mixed using a vortex over a 3 hrs reaction time. Progress was monitored by ESI-MS, and when starting material was converted to product, the organic material was extracted EtOAc (500 µL). The aqueous was separated and freeze-dried to produce the desired deprotected amine target as a white amorphous solid. The material was used without further purification for microarray analysis.

### II. The Library of S-Compounds According to Example 1A - Procedure A

Tetrasaccharide (**30** or **35,** 1mg, 0.8 µmol) was dissolved in DMF (100 µL) containing diisopropylethylamine (DIPEA, 4 µmol). To this solution was added acyl chloride (1.6 µmol) and the reaction was mixed using a vortex for 30 mins. The reaction was monitored by ESI, and when complete conversion to the product was achieved, the product was purified by HPLC chromatography under the following conditions: Agilent 1200 HPLC, Eclipse XDB-C8, 4.6 x 250 mm, 5 µm analytical column, A solvent = 50 mM ammonium bicarbonate; B = ACN, λ = 262 m) with a linear gradient of 10→60% B over 30 mins, flow rate 1 mL/min. Fractions containing the desired product were freeze-dried to yield the desired product as a white, fluffy solid. Table 1 shows 31 R₁-Acyl chloride fragments used to synthesize the library of the S-series compounds.

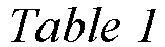

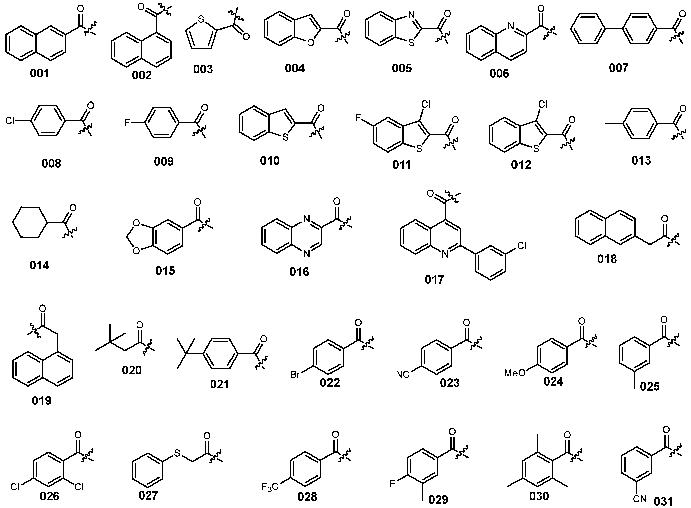

The following compounds were synthesized and their identities confirmed by the ¹H-NMR. For the compound below the naming convention is such that R has the following formula:

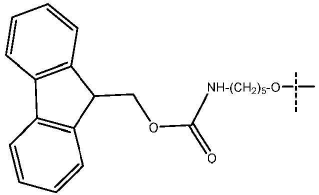

The general procedure referenced in the SXX-Fmoc examples below is general procedure A.

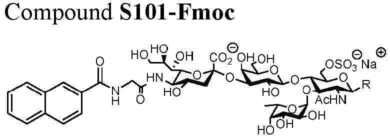

(0.15 µmol) was prepared by using the general procedure. ¹H NMR (600 MHz, D₂O) δ 8.29 (m, 1H), 7.94-7.87 (m, 3H), 7.78-7.73 (m, 3H), 7.56-7.50 (m, 4H), 7.37-7.35 (t, 2H), 7.30-7.27 (t, 2H), 4.95-4.95 (d, 1H), 4.67-4.61 (m, 1.5H), 4.48-4.47 (m, 2.5H), 4.39-4.38 (m, 1H), 4.22-4.17 (m, 3H), 3.97-3.95 (m, 1H), 3.88-3.36 (m, 23H), 2.81 (m, 1.5H), 2.64-2.61 (dd, 1H), 2.34 (m, 0.5H), 1.82 (s, 3H), 1.71-1.67 (t, 1H), 1.34 (m, 1.5H), 1.17 (m, 2H), 1.04-1.00 (m, 4.5H), 0.73 (m, 1H).

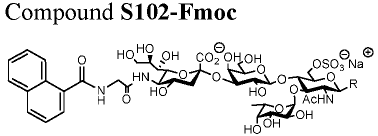

(0.15 µmol) was prepared by using the general procedure. ¹H NMR (600 MHz, dmso) δ 8.74-8.72 (t, 1H), 8.30-8.29 (dd, 1H), 8.14 (m, 1H), 8.00-7.99 (m, 1H), 7.96-7.94 (dd, 1H), 7.89-7.86 (m, 3H), 7.68-7.63 (m, 3H), 7.55-7.52 (m, 3H), 7.41-7.38 (t, 2H), 7.32-7.30 (t, 2H), 7.25-7.24 (t, 1H), 4.82 (d, 1H), 4.44-4.36 (m, 4H), 4.26-4.17 (m, 4H), 4.12-3.96 (m, 6H), 3.90-3.88 (m, 1H), 3.75 (m, 1H), 3.69-3.46 (m, 12H), 3.38 (m, 1H), 3.25-3.19 (m, 3H), 2.94 (m, 1H), 2.60-2.59 (m, 1H), 1.77 (s, 3H), 1.44-1.38 (m, 2H), 1.37-1.36 (m, 2H), 1.24-1.21 (m, 2H), 0.99-0.97 (d, 3H).

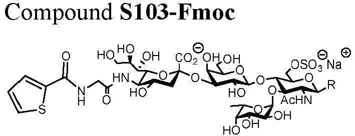

(0.15 µmol) was prepared using the general procedure. ¹H NMR (600 MHz, dmso) δ 8.75-8.73 (t, 1H), 8.06-8.05 (m, 1H), 7.87-7.86 (m, 3H), 7.79-7.78 (d, 1H), 7.75-7.74 (d, 1H), 7.68-7.67 (d, 2H), 7.41-7.38 (t, 2H), 7.32-7.30 (t, 2H), 7.25-7.23 (t, 1H), 7.15-7.13 (t, 1H), 4.82-4.81 (d, 1H), 4.58-4.35 (m, 4H), 4.26-4.17 (m, 4H), 4.11-3.82 (m, 7H), 3.73 (m, 1H), 3.69-3.46 (m, 12H), 3.38 (m, 1H), 3.25-3.18 (m, 3H), 2.93 (m, 1H), 2.71-2.60 (m, 1H), 1.76 (s, 3H), 1.44-1.36 (m, 4H), 1.24-1.21 (m, 2H), 0.98-0.96 (d, 3H). 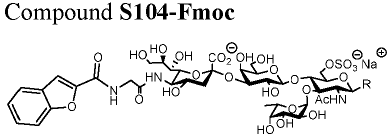 (0.15 µmol) was prepared using the general procedure. ¹H NMR (600 MHz, dmso) δ 8.82-8.80 (t, 1H), 8.07-8.06 (m, 1H), 7.87-7.86 (m, 3H), 7.77-7.76 (d, 1H), 7.68-7.66 (m, 3H), 7.56 (s, 1H), 7.47-7.44 (t, 1H), 7.41-7.38 (t, 2H), 7.33-7.30 (m, 3H), 7.25-7.23 (t, 1H), 4.82-4.81 (d, 1H), 4.55-4.35 (m, 3H), 4.26-4.10 (m, 5H), 4.07-3.87 (m, 5H), 3.73 (m, 1H), 3.68-3.37 (m, 13H), 3.24-3.18 (m, 3H), 2.93 (m, 1H), 2.70-2.60 (m, 1H), 1.76 (s, 3H), 1.44-1.35 (m, 4H), 1.24-1.21 (m, 2H), 0.98-0.97 (d, 3H).

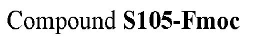

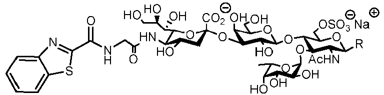

(0.15 µmol) was prepared using the general procedure. ¹H NMR (600 MHz, dmso) δ 9.13-9.11 (t, 1H), 8.23-8.22 (d, 1H), 8.16-8.15 (d, 1H), 8.10-8.08 (m, 1H), 7.89-7.86 (m, 3H), 7.68-7.67 (d, 2H), 7.64-7.56 (m, 2H), 7.40-7.38 (t, 2H), 7.32-7.30 (t, 2H), 7.25-7.23 (t, 1H), 4.82-4.81 (d, 1H), 4.58-4.35 (m, 3H), 4.26-4.03 (m, 10H), 3.94-3.90 (m, 2H), 3.73 (m, 1H), 3.68-3.37 (m, 13H), 3.25-3.18 (m, 3H), 2.93 (m, 3H), 2.71-2.60 (m, 1H), 1.76 (s, 3H), 1.44-1.34 (m, 4H), 1.25-1.21 (m, 2H), 0.98-0.97 (d, 3H).

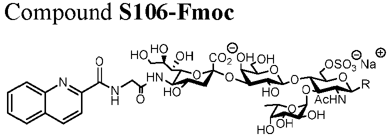

(0.15 µmol) and was prepared using the general procedure. ¹H NMR (600 MHz, dmso) δ 9.02-9.00 (t, 1H), 8.58-8.56 (d, 1H), 8.17-8.14 (m, 2H), 8.10-8.07 (m, 2H), 7.88-7.86 (m, 4H), 7.73-7.67 (m, 3H), 7.40-7.38 (t, 2H), 7.32-7.30 (t, 2H), 7.25-7.23 (t, 1H), 4.82-4.81 (d, 1H), 4.57-4.35 (m, 3H), 4.26-3.98 (m, 11H), 3.89-3.87 (m, 1H), 3.74 (m, 1H), 3.68-3.34 (m, 13H), 3.25-3.18 (m, 3H), 2.93 (m, 3H), 2.71-2.60 (m, 1H), 1.76 (s, 3H), 1.45-1.35 (m, 4H), 1.24-1.22 (m, 2H), 0.98-0.97 (d, 3H).

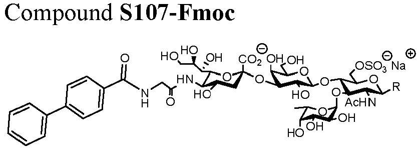

(0.15 µmol) was prepared using the general procedure. ¹H NMR (600 MHz, dmso) δ 8.78-8.76 (t, 1H), 8.05-8.04 (m, 2H), 7.97-7.96 (d, 2H), 7.87-7.86 (m, 3H), 7.78-7.76 (d, 2H), 7.73-7.72 (d, 2H), 7.68-7.67 (d, 2H), 7.49-7.46 (t, 2H), 7.40-7.38 (t, 3H), 7.32-7.30 (t, 2H), 7.25-7.23 (t, 1H), 4.82-4.81 (d, 1H), 4.58-4.36 (m, 3H), 4.26-3.98 (m, 10H), 3.90-3.87 (m, 2H), 3.74 (m, 1H), 3.69-3.37 (m, 13H), 3.25-3.18 (m, 3H), 2.93 (m, 3H), 2.71-2.60 (m, 1H), 1.77 (s, 3H), 1.44-1.34 (m, 4H), 1.24-1.21 (m, 2H), 0.98-0.97 (d, 3H).

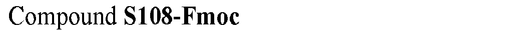

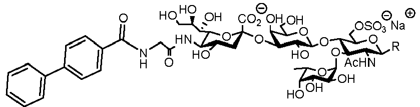

(0.15 µmol) was prepared using the general procedure. ¹H NMR (600 MHz, dmso) δ 8.82-8.80 (t, 1H), 8.04-8.03 (m, 1H), 7.89-7.86 (m, 5H), 7.68-7.67 (d, 2H), 7.54-7.53 (d, 2H), 7.41-7.38 (t, 2H), 7.32-7.30 (t, 2H), 7.25-7.23 (t, 1H), 4.82-4.81 (d, 1H), 4.57-4.35 (m, 3H), 4.26-3.95 (m, 10H), 3.89-3.84 (m, 2H), 3.73 (m, 1H), 3.68-3.37 (m, 13H), 3.25-3.18 (m, 3H), 2.93 (m, 3H), 2.70-2.60 (m, 1H), 1.76 (s, 3H), 1.45-1.34 (m, 4H), 1.24-1.21 (m, 2H), 0.98-0.96 (d, 3H).

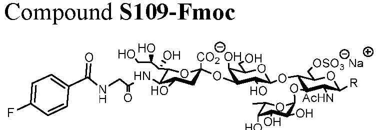

(0.135 µmol) was prepared using the general procedure. ¹H NMR (600 MHz, dmso) δ 8.76-8.74 (t, 1H), 8.04-8.03 (m, 1H), 7.95-7.92 (dd, 1H), 7.89-7.86 (m, 3H), 7.68-7.67 (d, 2H), 7.41-7.38 (t, 2H), 7.32-7.28 (m, 4H), 7.25-7.23 (t, 1H), 4.82-4.81 (d, 1H), 4.58-4.35 (m, 3H), 4.26-3.95 (m, 10H), 3.88-3.84 (m, 2H), 3.73 (m, 1H), 3.69-3.36 (m, 13H), 3.24-3.17 (m, 3H), 2.93 (m, 3H), 2.70-2.60 (m, 1H), 1.76 (s, 3H), 1.45-1.35 (m, 4H), 1.24-1.22 (m, 2H), 0.98-0.96 (d, 3H).

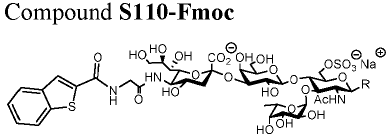

(0.135 µmol) was prepared using the general procedure. ¹H NMR (600 MHz, dmso) δ 9.01-8.99 (t, 1H), 8.12,-8.08 (m, 2H), 8.02-8.00 (d, 1H), 7.95-7.94 (d, 1H), 7.89-7.86 (m, 3H), 7.68-7.67 (d, 2H), 7.46-7.38 (m, 4H), 7.32-7.30 (t, 2H), 7.25-7.23 (t, 1H), 4.82-4.81 (d, 1H), 4.58-4.35 (m, 3H), 4.26-3.98 (m, 10H), 3.91-3.87 (m, 2H), 3.74 (m, 1H), 3.68-3.36 (m, 13H), 3.24-3.19 (m, 3H), 2.94-2.60 (m, 4H), 1.76 (s, 3H), 1.45-1.34 (m, 4H), 1.25-1.21 (m, 2H), 0.98-0.96 (d, 3H).

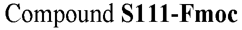

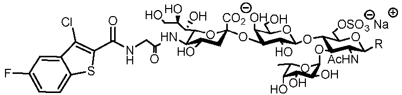

(0.15 µmol) was prepared using the general procedure. ¹H NMR (600 MHz, dmso) δ 8.46-8.44 (t, 1H), 8.11-8.05 (m, 2H), 7.94-7.92 (dd, 1H), 7.89-7.86 (m, 3H), 7.68-7.67 (d, 2H), 7.48-7.44 (td, 1H), 7.41-7.38 (t, 2H), 7.32-7.30 (t, 2H), 7.25-7.23 (t, 1H), 4.82-4.81 (d, 1H), 4.57-4.36 (m, 3H), 4.26-4.01 (m, 10H), 3.97-3.87 (m, 2H), 3.74 (m, 1H), 3.69-3.36 (m, 13H), 3.25-3.18 (m, 3H), 2.93-2.60 (m, 4H), 1.77 (s, 3H), 1.44-1.36 (m, 4H), 1.24-1.21 (m, 2H), 0.98-0.97 (d, 3H).

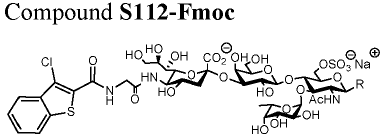

(0.15 µmol) was prepared using the general procedure. ¹H NMR (600 MHz, dmso) δ 8.48-8.46 (t, 1H), 8.12-8.10 (m, 2H), 7.91-7.86 (m, 4H), 7.68-7.57 (m, 4H), 7.41-7.38 (t, 2H), 7.32-7.30 (t, 2H), 7.25-7.23 (t, 1H), 4.82-4.81 d, 1H), 4.58-4.36 (m, 3H), 4.26-3.87 (m, 12H), 3.74 (m, 1H), 3.68-3.37 (m, 13H), 3.24-3.18 (m, 3H), 2.96-2.59 (m, 4H), 1.77 (s, 3H), 1.45-1.36 (m, 4H), 1.24-1.21 (m, 2H), 0.98-0.97 (d, 3H).

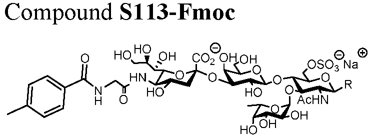

(0.15 µmol) was prepared using the general procedure. ¹H NMR (600 MHz, dmso) δ 8.62-8.60 (t, 1H), 8.03-8.02 (m, 1H), 7.89-7.86 (m, 3H), 7.77-7.76 (d, 2H), 7.68-7.67 (d, 2H), 7.41-7.38 (t, 2H), 7.32-.30 (t, 2H), 7.27-7.23 (m, 3H), 4.82-4.81 (d, 1H), 4.57-4.35 (m, 3H), 4.26-3.83 (m, 12H), 3.73 (m, 1H), 3.69-3.38 (m, 13H), 3.25-3.18 (m, 3H), 2.96-2.60 (m, 4H), 2.34 (s, 3H), 1.76 (s, 3H), 1.44-1.35 (m, 4H), 1.24-1.21 (m, 2H), 0.98-0.96 (d, 3H).

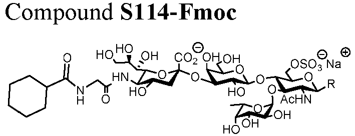

(0.15 µmol) was prepared using the general procedure. ¹H NMR (600 MHz, dmso) δ 7.91-7.86 (m, 5H), 7.68-7.67 (d, 2H), 7.41-7.38 (t, 2H), 7.33-7.30 (t, 2H), 7.25-7.23 (t, 1H), 4.82-4.81 (d, 1H), 4.58-4.36 (m, 3H), 4.26-3.98 (m, 10H), 3.88-3.87 (m, 1H), 3.74-3.71 (m, 2H), 3.68-3.38 (m, 13H), 3.23-3.15 (m, 3H), 2.96-2.59 (m, 4H), 2.17-2.13 (m, 1H), 1.77 (s, 3H), 1.69-1.67 (m, 4H), 1.59-1.57 (m, 1H), 1.45-1.36 (m, 4H), 1.32-1.11 (m, 7H), 0.98-0.97 (d, 3H).

Compound **S115-Fmoc** (0.15 µmol) was prepared using the general procedure.

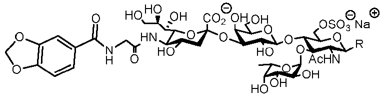

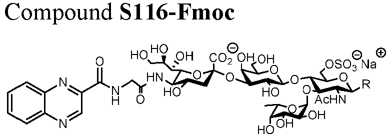

(0.15 µmol) was prepared using the general procedure. ¹H NMR (600 MHz, dmso) δ 9.48 (s, 1H), 9.11-9.09 (t, 1H), 8.24-8.19 (m, 2H), 8.07 (m, 1H), 8.00-7.97 (m, 2H), 7.89-7.86 (m, 3H), 7.68-7.67 (d, 2H), 7.40-7.38 (t, 2H), 7.32-7.30 (t, 2H), 7.25-7.23 (t, 1H), 4.82-4.81 (d, 1H), 4.58-4.35 (m, 3H), 4.26-4.07 (m, 10H), 4.05-3.98 (m, 2H), 3.89-3.87 (m, 1H), 3.73 (m, 1H), 3.69-3.42 (m, 12H), 3.26-3.18 (m, 3H), 2.96-2.92 (m, 3H), 2.71-2.59 (m, 1H), 1.76 (s, 3H), 1.44-1.35 (m, 4H), 1.24-1.21 (m, 2H), 0.98-0.97 (d, 3H).

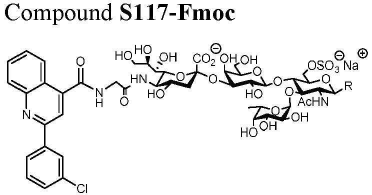

(0.525 µmol) was prepared using the general procedure. ¹H NMR (600 MHz, dmso) δ 9.16-9.14 (t, 1H), 8.36 (s, 1H), 8.33-8.32 (d, 1H), 8.27-8.25 (td, 1H), 8.22-8.20 (m, 2H), 8.14-8.13 (d, 1H), 7.92-7.90 (d, 1H), 7.87-7.86 (d, 2H), 7.85-7.82 (t, 1H), 7.68-7.58 (m, 5H), 7.41-7.38 (t, 2H), 7.32-7.30 (t, 2H), 7.25-7.23 (t, 1H), 4.82-4.81 (d, 1H), 4.63-4.62 (d, 1H), 4.47 (m, 1H), 4.37-4.36 (d, 2H), 4.27-4.23 (m, 4H), 4.19-4.17 (m, 3H), 4.08-4.01 (m, 3H), 3.98-3.97 (m, 1H), 3.90-3.88 (m, 1H), 3.75-3.37 (m, 16H), 3.21-3.19 (m, 1H), 2.95-2.93 (m, 3H), 2.71-2.59 (m, 1H), 1.77 (s, 3H), 1.45-1.36 (m, 4H), 1.24-1.21 (m, 2H), 0.98-0.97 (d, 3H).

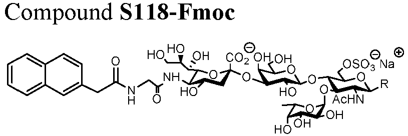

(0.15 µmol) was prepared using the general procedure.

1H NMR (600 MHz, D₂O) δ 7.80 (dt, J = 14.0, 6.4 Hz, 5H), 7.71 (s, 1H), 7.56 (d, J = 7.5 Hz, 2H), 7.43 (p, J = 6.9 Hz, 2H), 7.39 - 7.31 (m, 3H), 7.29 (t, J = 7.5 Hz, 2H), 4.95 (d, J = 4.1 Hz, 1H), 4.46 (d, J = 7.8 Hz, 1H), 4.38 (d, J = 7.5 Hz, 1H), 4.20 (d, J = 23.9 Hz, 3H), 3.94 (dd, J = 9.6, 3.2 Hz, 1H), 3.90 - 3.33 (m, 28H), 2.81 (s, 1H), 2.59 (dd, J = 12.6, 4.9 Hz, 1H), 1.81 (s, 3H), 1.65 (t, J = 12.1 Hz, 1H), 1.34 (s, 1H), 1.21 - 1.14 (m, 0H), 1.02 (d, J = 6.6 Hz, 5H), 0.73 (s, 1H)

Compound S119-Fmoc (0.15 µmol) was prepared using the general procedure. 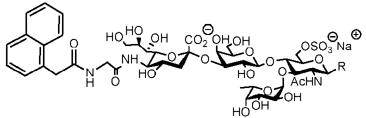 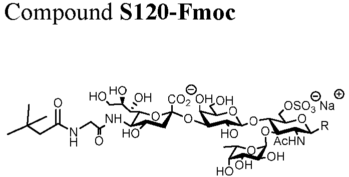
(0.15 µmol) was prepared using the general procedure
1H NMR (600 MHz, D₂O) δ 7.78 (d, J = 7.6 Hz, 2H), 7.56 (d, J = 7.6 Hz, 2H), 7.36 (t, J = 7.5 Hz, 2H), 7.29 (t, J = 7.5 Hz, 2H), 4.95 (d, J = 3.4 Hz, 1H), 4.46 (d, J = 7.8 Hz, 1H), 4.38 (d, J = 7.7 Hz, 1H), 4.25 - 4.16 (m, 3H), 3.95 (d, J = 10.0 Hz, 1H), 3.86 (t, J = 8.8 Hz, 1H), 3.82 - 3.35 (m, 26H), 2.81 (s, 2H), 2.61 (dd, J = 12.4, 4.6 Hz, 1H), 2.05 (s, 2H), 1.81 (s, 1H), 1.66 (t, J = 12.1 Hz, 1H), 1.33 (s, 1H), 1.21 - 1.18 (m, 2H), 1.02 (d, J = 6.6 Hz, 5H), 0.85 (s, 8H), 0.72 (s, 1H).

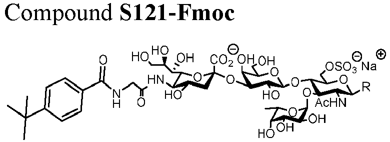

(0.15 µmol) was prepared using the general procedure.

1H NMR (600 MHz, D₂O) δ 7.77 (d, J = 7.6 Hz, 2H), 7.65 (dd, J = 8.6, 1.8 Hz, 2H), 7.56 (d, J = 7.5 Hz, 2H), 7.48 (d, J = 8.7 Hz, 2H), 7.36 (t, J = 7.5 Hz, 2H), 7.28 (t, J = 7.5 Hz, 2H), 4.94 (d, J = 3.9 Hz, 1H), 4.46 (d, J = 7.9 Hz, 1H), 4.38 (d, J = 7.7 Hz, 1H), 4.28 - 4.12 (m, 3H), 4.03 - 3.92 (m, 3H), 3.86 (t, J = 8.8 Hz, 1H), 3.84 - 3.34 (m, 23H), 2.81 (s, 2H), 2.61 (dd, J = 12.4, 4.5 Hz, 1H), 1.81 (s, 1H), 1.67 (t, J = 12.2 Hz, 1H), 1.33 (s, 1H), 1.19 (s, 9H), 1.02 (d, J = 6.6 Hz, 5H), 0.72 (s, 1H).

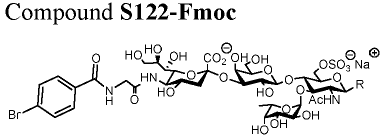

(0.15 µmol) was prepared using the general procedure.

1H NMR (600 MHz, D₂O) δ 7.77 (d, J = 7.6 Hz, 2H), 7.61 - 7.51 (m, 6H), 7.36 (t, J = 7.5 Hz, 2H), 7.28 (t, J = 7.5 Hz, 2H), 4.94 (d, J = 4.0 Hz, 1H), 4.46 (d, J = 7.8 Hz, 1H), 4.37 (s, 1H), 4.27 - 4.14 (m, 3H), 4.03 - 3.91 (m, 3H), 3.86 (t, J = 9.3 Hz, 1H), 3.83 - 3.31 (m, 23H), 2.81 (s, 2H), 2.61 (dd, J = 12.4, 4.5 Hz, 1H), 1.81 (s, 2H), 1.67 (t, J = 12.2 Hz, 1H), 1.33 (s, 1H), 1.17 (d, J = 20.2 Hz, 2H), 1.02 (d, J = 6.5 Hz, 5H), 0.72 (s, 1H).

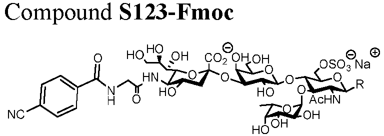

(0.15 µmol) was prepared using the general procedure.

1H NMR (600 MHz, D₂O) δ 7.88 - 7.83 (m, 1H), 7.80 - 7.77 (m, 4H), 7.72 - 7.68 (m, 1H), 7.56 (d, J = 7.6 Hz, 2H), 7.36 (t, J = 7.5 Hz, 2H), 7.28 (t, J = 7.5 Hz, 2H), 4.94 (d, J = 4.0 Hz, 1H), 4.46 (d, J = 8.1 Hz, 1H), 4.38 (d, J = 7.5 Hz, 1H), 4.28 - 4.12 (m, 3H), 4.01 (s, 2H), 3.96 (d, J = 10.0 Hz, 1H), 3.86 (t, J = 9.1 Hz, 1H), 3.84 - 3.32 (m, 22H), 3.06 (q, J = 8.0, 7.4 Hz, 1H), 2.81 (s, 2H), 2.61 (dd, J = 12.3, 4.7 Hz, 1H), 1.81 (s, 3H), 1.67 (t, J = 12.2 Hz, 1H), 1.33 (s, 1H), 1.19 (d, J = 6.4 Hz, 2H), 1.02 (d, J = 6.6 Hz, 5H), 0.72 (s, 1H).

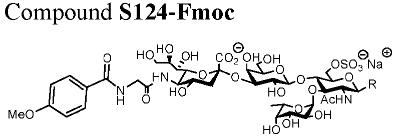

(0.15 µmol) was prepared using the general procedure.

1H NMR (600 MHz, D₂O) δ 7.77 (d, J = 7.6 Hz, 2H), 7.68 (d, J = 9.0 Hz, 2H), 7.56 (d, J = 7.5 Hz, 2H), 7.36 (t, J = 7.5 Hz, 2H), 7.28 (t, J = 7.5 Hz, 2H), 6.94 (d, J = 9.0 Hz, 2H), 4.94 (d, J = 4.0 Hz, 1H), 4.46 (d, J = 8.1 Hz, 1H), 4.38 (d, J = 7.4 Hz, 1H), 4.26 - 4.13 (m, 3H), 4.01 - 3.92 (m, 3H), 3.85 (t, J = 8.8 Hz, 1H), 3.83 - 3.30 (m, 25H), 2.81 (s, 2H), 2.61 (dd, J = 12.6, 4.7 Hz, 1H), 1.81 (s, 3H), 1.67 (t, J = 12.2 Hz, 1H), 1.33 (s, 1H), 1.23 - 1.10 (m, 6H), 1.02 (d, J = 6.6 Hz, 5H), 0.72 (s, 1H).

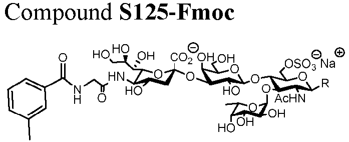

(0.15 µmol) was prepared using the general procedure.

1H NMR (600 MHz, D₂O) δ 7.77 (d, J = 7.6 Hz, 2H), 7.56 (d, J = 7.5 Hz, 2H), 7.52 (s, 1H), 7.47 (d, J = 8.0 Hz, 1H), 7.39 - 7.32 (m, 3H), 7.31 - 7.25 (m, 3H), 4.94 (d, J = 4.0 Hz, 1H), 4.46 (d, J = 7.9 Hz, 1H), 4.38 (d, J = 7.7 Hz, 1H), 4.26 - 4.16 (m, 3H), 4.03 - 3.91 (m, 3H), 3.86 (t, J = 8.9 Hz, 1H), 3.83 - 3.22 (m, 22H), 2.81 (s, 2H), 2.61 (dd, J = 12.5, 4.7 Hz, 1H), 2.26 (s, 3H), 1.81 (s, 1H), 1.67 (t, J = 12.2 Hz, 1H), 1.33 (s, 1H), 1.21 - 1.11 (m, 4H), 1.02 (d, J = 6.6 Hz, 5H), 0.72 (s, 1H).

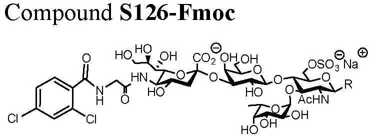

(0.15 µmol) was prepared using the general procedure.

1H NMR (600 MHz, D₂O) δ 7.78 (d, J = 7.6 Hz, 2H), 7.56 (d, J = 7.5 Hz, 2H), 7.49 (s, 0H), 7.41 - 7.25 (m, 6H), 4.95 (d, J = 3.9 Hz, 1H), 4.47 (d, J = 8.1 Hz, 1H), 4.38 (d, J = 4.4 Hz, 1H), 4.27 - 4.14 (m, 3H), 4.03 - 3.92 (m, 6H), 3.86 (t, J = 8.9 Hz, 1H), 3.84 - 3.33 (m, 23H), 2.81 (s, 2H), 2.62 (dd, J = 12.5, 4.6 Hz, 1H), 1.81 (s, 3H), 1.68 (t, J = 12.3 Hz, 1H), 1.33 (s, 1H), 1.20 - 1.15 (m, 2H), 1.03 (d, J = 6.6 Hz, 5H), 0.72 (s, 1H).

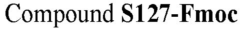

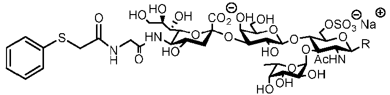

(0.075 µmol) was prepared using the general procedure.

1H NMR (600 MHz, D₂O) δ 7.78 (d, J = 7.6 Hz, 2H), 7.56 (d, J = 7.6 Hz, 2H), 7.36 (t, J = 7.5 Hz, 2H), 7.33 - 7.23 (m, 5H), 7.20 (t, J = 7.2 Hz, 1H), 4.94 (d, J = 4.0 Hz, 1H), 4.46 (d, J = 7.9 Hz, 1H), 4.38 (d, J = 7.8 Hz, 1H), 4.25 - 4.13 (m, 3H), 3.94 (d, J = 9.8 Hz, 1H), 3.85 (t, J = 9.3 Hz, 1H), 3.81 (d, J = 3.2 Hz, 1H), 3.78 - 3.35 (m, 50H), 2.81 (t, J = 6.0 Hz, 2H), 2.59 (dd, J = 12.4, 4.6 Hz, 1H), 1.81 (s, 3H), 1.64 (t, J = 12.2 Hz, 1H), 1.33 (s, 1H), 1.22 - 1.17 (m, 2H), 1.02 (d, J = 6.6 Hz, 5H), 0.72 (s, 1H).

Compound **S128-Fmoc** (0.15 µmol) was prepared using the general procedure.

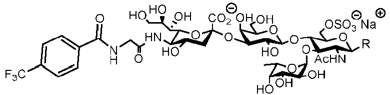

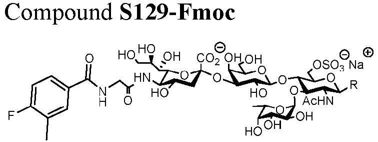

(0.15 µmol) was prepared using the general procedure.

1H NMR (600 MHz, D₂O) δ 7.78 (d, J = 7.6 Hz, 2H), 7.57 (dd, J = 17.6, 7.3 Hz, 3H), 7.36 (t, J = 7.5 Hz, 2H), 7.28 (t, J = 7.5 Hz, 2H), 7.04 (t, J = 9.1 Hz, 1H), 4.94 (d, J = 4.0 Hz, 1H), 4.46 (d, J = 8.1 Hz, 1H), 4.38 (d, J = 5.7 Hz, 2H), 4.25 - 4.14 (m, 7H), 4.01 - 3.92 (m, 6H), 3.86 (t, J = 8.7 Hz, 1H), 3.83 - 3.32 (m, 23H), 2.81 (t, J = 5.4 Hz, 3H), 2.61 (dd, J = 12.1, 4.4 Hz, 1H), 2.17 (s, 2H), 1.81 (s, 2H), 1.67 (t, J = 12.2 Hz, 1H), 1.33 (s, 1H), 1.20 - 1.13 (m, 6H), 1.02 (d, J = 6.6 Hz, 5H), 0.72 (s, 1H).

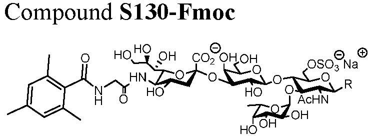

(0.105 µmol) was prepared using the general procedure.

1H NMR (600 MHz, D₂O) δ 7.78 (d, J = 7.6 Hz, 2H), 7.56 (d, J = 7.6 Hz, 2H), 7.36 (t, J = 7.5 Hz, 2H), 7.29 (t, J = 7.5 Hz, 2H), 6.84 (s, 1H), 4.95 (d, J = 2.9 Hz, 2H), 4.47 (d, J = 8.0 Hz, 1H), 4.38 (d, J = 5.6 Hz, 2H), 4.27 - 4.14 (m, 4H), 4.02 - 3.91 (m, 1H), 3.91 - 3.21 (m, 24H), 2.81 (t, J = 4.9 Hz, 3H), 2.66 - 2.59 (m, 1H), 2.16 - 2.07 (m, 7H), 1.81 (s, 2H), 1.68 (t, J = 12.6 Hz, 1H), 1.33 (s, 1H), 1.22 - 1.12 (m, 1H), 1.03 (d, J = 6.5 Hz, 5H), 0.72 (s, 1H).

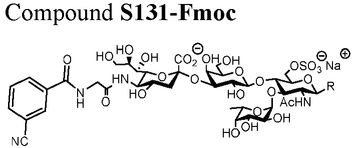

(0.15 µmol) was prepared using the general procedure.

1H NMR (600 MHz, D₂O) δ 8.06 (s, 1H), 7.99 - 7.93 (m, 1H), 7.86 - 7.81 (m, 1H), 7.77 (d, J = 7.6 Hz, 2H), 7.59 - 7.53 (m, 3H), 7.36 (t, J = 7.5 Hz, 2H), 7.28 (t, J = 7.5 Hz, 2H), 4.94 (d, J = 4.0 Hz, 1H), 4.46 (d, J = 7.9 Hz, 1H), 4.38 (d, J = 6.3 Hz, 1H), 4.27 - 4.13 (m, 6H), 4.01 (s, 2H), 3.96 (d, J = 10.1 Hz, 1H), 3.86 (t, J = 8.9 Hz, 1H), 3.85 - 3.25 (m, 23H), 2.81 (t, J = 6.3 Hz, 2H), 2.61 (dd, J = 12.6, 4.7 Hz, 1H), 1.81 (s, 1H), 1.67 (t, J = 12.2 Hz, 1H), 1.33 (s, 1H), 1.23 - 1.16 (m, 2H), 1.02 (d, J = 6.6 Hz, 5H), 0.72 (s, 1H).

The following compounds were synthesized according to the general procedure:
Compound **S201-Fmoc** (0.15 µmol) was prepared using the general procedure. 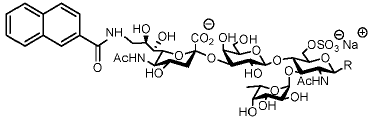
Compound **S202-Fmoc** (0.135 µmol) was prepared using the general procedure. 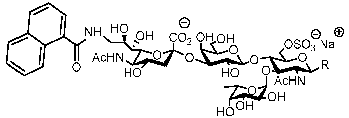
Compound **S203-Fmoc** (0.135 µmol) was prepared using the general procedure. 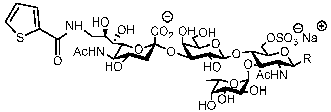
Compound **S204-Fmoc** (0.15 µmol) was prepared using the general procedure. 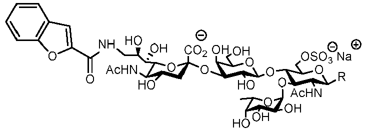
Compound **S205-Fmoc** (0.15 µmol) was prepared using the general procedure. 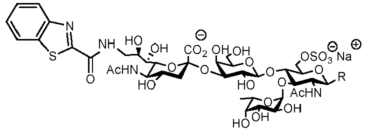
Compound **S206-Fmoc** (0.15 µmol) was prepared using the general procedure. 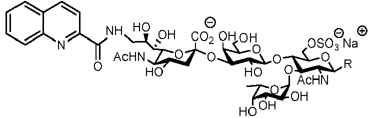
Compound **S207-Fmoc** (0.15 µmol) was prepared using the general procedure. 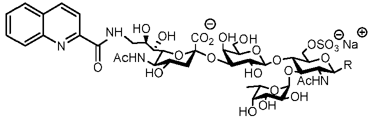
Compound **S208-Fmoc** (0.075 µmol) was prepared using the general procedure. 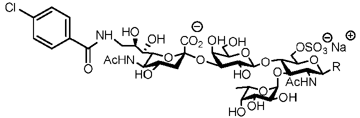
Compound **S209-Fmoc** (0.15 µmol) was prepared using the general procedure. 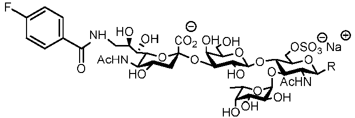
Compound **S210-Fmoc** (0.15 µmol) was prepared using the general procedure. 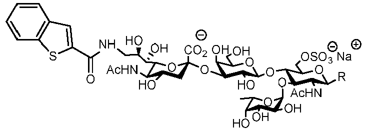
Compound **S211-Fmoc** (0.15 µmol) was prepared using the general procedure. 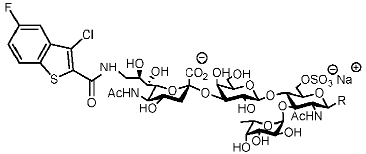
Compound **S212-Fmoc** (0.15 µmol) was prepared using the general procedure.

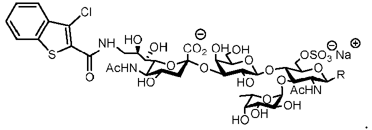

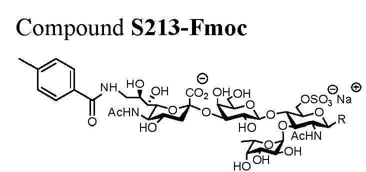

(0.075 µmol) was prepared using the general procedure.

1H NMR (600 MHz, D₂O) δ 7.74 (d, J = 7.5 Hz, 2H), 7.53 (d, J = 8.4 Hz, 5H), 7.34 (t, J = 7.5 Hz, 2H), 7.30 - 7.24 (m, 2H), 7.13 (d, J = 6.4 Hz, 3H), 4.93 (s, 1H), 4.43 (t, J = 7.2 Hz, 4H), 4.34 (s, 1H), 4.24 - 4.04 (m, 2H), 3.95 - 3.36 (m, 26H), 2.90 - 2.75 (m, 4H), 2.62 (d, J = 10.4 Hz, 1H), 2.18 (s, 3H), 1.89 - 1.78 (m, 10H), 1.63 (t, J = 12.3 Hz, 1H), 1.35 (s, 1H), 1.20 - 1.19 (m, 2H), 1.02 (d, J = 6.6 Hz, 5H), 0.73 (s, 1H).

The following compounds were synthesized according to the general procedure:
Compound **S214-Fmoc** (0.075 µmol) was prepared using the general procedure. 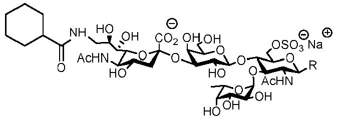
Compound **S215-Fmoc** (0.15 µmol) was prepared using the general procedure. 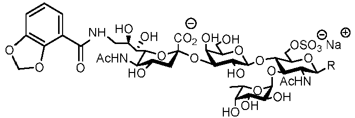
Compound **S216-Fmoc** (0.15 µmol) was prepared using the general procedure. 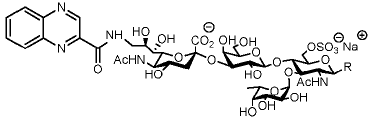
Compound **S217-Fmoc** (0.15 µmol) was prepared using the general procedure. 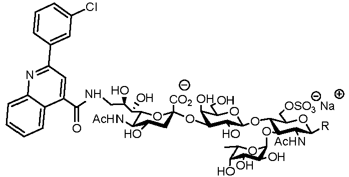
Compound **-Fmoc** (0.15 µmol) was prepared using the general procedure. 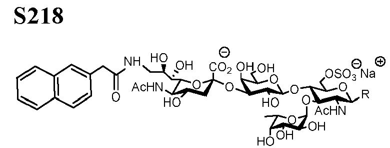
Compound **S219-Fmoc** (0.15 µmol) was prepared using the general procedure.

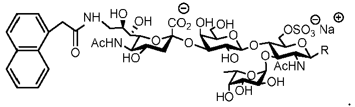

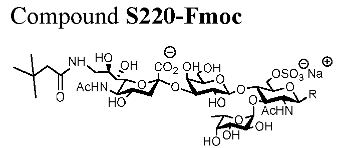

(0.15 µmol) was prepared using the general procedure.

1H NMR (600 MHz, D₂O) δ 7.78 (d, J = 7.6 Hz, 2H), 7.56 (d, J = 7.5 Hz, 2H), 7.36 (t, J = 7.5 Hz, 2H), 7.28 (t, J = 7.5 Hz, 2H), 4.94 (d, J = 3.9 Hz, 1H), 4.47 (d, J = 8.1 Hz, 1H), 4.39 (d, J = 5.6 Hz, 1H), 4.27 (d, J = 10.5 Hz, 1H), 4.19 (t, J = 13.0 Hz, 2H), 3.92 (dd, J = 9.6, 2.9 Hz, 2H), 3.90 - 3.82 (m, 2H), 3.81 - 3.20 (m, 22H), 2.81 (s, 2H), 2.61 (dd, J = 12.5, 4.8 Hz, 1H), 2.12 - 1.95 (m, 2H), 1.90 - 1.85 (m, 3H), 1.82 (s, 2H), 1.62 (t, J = 12.2 Hz, 1H), 1.34 (s, 1H), 1.19 (d, J = 6.6 Hz, 2H), 1.03 (d, J = 6.5 Hz, 4H), 0.84 (s, 5H), 0.73 (s, 2H).

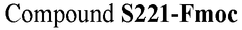

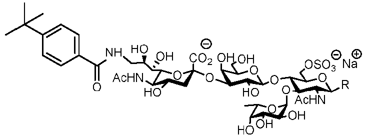

(0.15 µmol) was prepared using the general procedure.

1H NMR (600 MHz, D₂O) δ 7.81 - 7.67 (m, 2H), 7.63 - 7.50 (m, 4H), 7.43 - 7.30 (m, 4H), 7.31 - 7.22 (m, 2H), 4.93 (d, J = 4.0 Hz, 1H), 4.42 (d, J = 8.2 Hz, 1H), 4.38 - 4.30 (m, 1H), 4.08 (d, J = 9.6 Hz, 2H), 4.05 - 3.13 (m, 26H), 2.84 (q, J = 9.0, 6.9 Hz, 2H), 2.62 (dd, J = 12.4, 4.6 Hz, 1H), 1.86 (s, 3H), 1.82 (s, 3H), 1.63 (t, J = 12.2 Hz, 1H), 1.36 - 1.33 (m, 1H), 1.19 - 1.18 (m, 2H), 1.12 (s, 11H), 1.02 (d, J = 6.7 Hz, 5H), 0.73 (s, 1H).

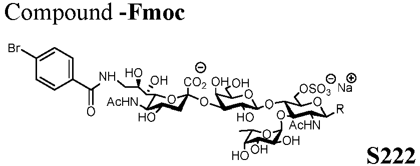

(0.15 µmol) was prepared using the general procedure.

1H NMR (600 MHz, D₂O) δ 7.79 - 7.68 (m, 2H), 7.59 - 7.40 (m, 6H), 7.33 (t, J = 7.6 Hz, 2H), 7.26 (t, J = 7.5 Hz, 2H), 4.93 (d, J = 4.0 Hz, 1H), 4.47 - 4.38 (m, 1H), 4.35 (d, J = 5.6 Hz, 1H), 4.22 - 4.05 (m, 2H), 3.97 - 3.31 (m, 26H), 2.84 (s, 2H), 2.62 (dd, J = 12.4, 4.5 Hz, 1H), 1.87 (s, 3H), 1.82 (s, 3H), 1.62 (t, J = 12.2 Hz, 1H), 1.35 (s, 1H), 1.19 (d, J = 7.0 Hz, 2H), 1.02 (d, J = 6.5 Hz, 5H), 0.74 (s, 1H).

Compound **S223-Fmoc** (0.15 µmol) was prepared using the general procedure.

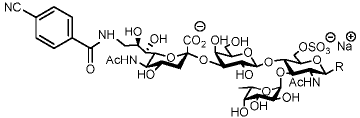

1H NMR (600 MHz, D₂O) δ 7.93 - 7.42 (m, 8H), 7.37 - 7.29 (m, 2H), 7.26 (t, J = 7.5 Hz, 2H), 4.93 (d, J = 4.0 Hz, 1H), 4.48 - 4.40 (m, 1H), 4.37 (d, J = 6.0 Hz, 1H), 4.21 - 4.06 (m, 2H), 4.00 - 3.32 (m, 26H), 2.91 - 2.74 (m, 2H), 2.63 (dd, J = 12.5, 4.6 Hz, 1H), 1.87 (s, 3H), 1.82 (s, 3H), 1.63 (t, J = 12.2 Hz, 1H), 1.34 (s, 1H), 1.20 - 1.19 (m, 2H), 1.02 (d, J = 6.6 Hz, 5H), 0.73 (s, 1H).

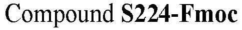

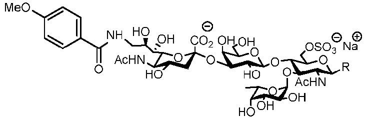

(0.15 µmol) was prepared using the general procedure.

1H NMR (600 MHz, D₂O) δ 7.71 (d, J = 7.7 Hz, 2H), 7.67 - 7.47 (m, 4H), 7.32 (q, J = 8.6, 8.1 Hz, 2H), 7.26 (q, J = 9.2, 7.5 Hz, 2H), 6.97 - 6.75 (m, 2H), 4.93 (d, J = 3.9 Hz, 1H), 4.50 - 4.38 (m, 1H), 4.37 - 4.28 (m, 1H), 4.18 - 4.04 (m, 2H), 4.03 - 3.21 (m, 28H), 2.90 - 2.77 (m, 2H), 2.62 (dd, J = 12.4, 4.5 Hz, 1H), 1.86 (s, 3H), 1.82 (s, 3H), 1.63 (t, J = 12.2 Hz, 1H), 1.35 (s, 1H), 1.20 - 1.17 (m, 2H), 1.02 (d, J = 6.6 Hz, 5H), 0.74 (s, 1H).

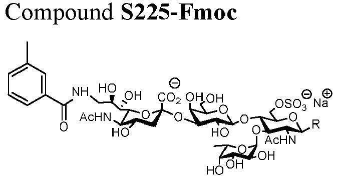

(0.15 µmol) was prepared using the general procedure.

1H NMR (600 MHz, D₂O) δ 7.79 - 7.70 (m, 2H), 7.56 - 7.49 (m, 2H), 7.45 - 7.20 (m, 8H), 4.93 (d, J = 4.1 Hz, 1H), 4.42 (d, J = 9.3 Hz, 1H), 4.32 (d, J = 6.3 Hz, 1H), 4.23 - 4.04 (m, 2H), 3.98 - 3.47 (m, 23H), 3.36 (d, J = 9.3 Hz, 3H), 2.88 - 2.76 (m, 2H), 2.62 (dd, J = 12.5, 4.5 Hz, 1H), 2.19 (s, 3H), 1.86 (s, 3H), 1.81 (s, 3H), 1.63 (t, J = 12.2 Hz, 1H), 1.36 - 1.33 (m, 1H), 1.19 (d, J = 6.5 Hz, 2H), 1.02 (d, J = 6.6 Hz, 5H), 0.73 (s, 1H).

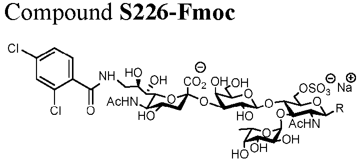

(0.15 µmol) was prepared using the general procedure.

1H NMR (600 MHz, D₂O) δ 7.76 (d, J = 7.9 Hz, 2H), 7.55 (d, J = 7.5 Hz, 2H), 7.40 - 7.32 (m, 3H), 7.32 - 7.18 (m, 4H), 4.93 (d, J = 4.1 Hz, 1H), 4.45 - 4.33 (m, 2H), 4.24 - 4.13 (m, 2H), 4.05 - 3.31 (m, 26H), 2.82 (s, 2H), 2.63 (dd, J = 12.4, 4.6 Hz, 1H), 1.89 (s, 3H), 1.81 (s, 3H), 1.62 (t, J = 12.2 Hz, 1H), 1.34 (s, 1H), 1.20 - 1.19 (m, 2H), 1.02 (d, J = 6.6 Hz, 5H), 0.73 (s, 1H).

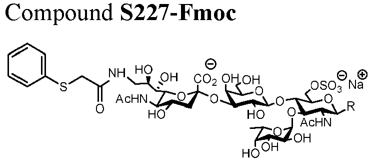

(0.15 µmol) was prepared using the general procedure.

1H NMR (600 MHz, D₂O) δ 7.76 (d, J = 7.8 Hz, 2H), 7.54 (d, J = 7.8 Hz, 2H), 7.35 (t, J = 7.1 Hz, 2H), 7.31 - 7.09 (m, 7H), 4.94 (d, J = 4.1 Hz, 1H), 4.49 - 4.34 (m, 2H), 4.25 - 4.08 (m, 3H), 3.90 (dd, J = 9.8, 3.2 Hz, 1H), 3.86 - 3.51 (m, 19H), 3.45 - 3.23 (m, 6H), 3.19 (dd, J = 14.1, 8.8 Hz, 1H), 2.87 - 2.74 (m, 2H), 2.62 (dd, J = 12.5, 4.6 Hz, 1H), 1.84 (d, J = 20.1 Hz, 6H), 1.61 (t, J = 12.2 Hz, 1H), 1.33 (s, 1H), 1.19 (d, J = 6.6 Hz, 2H), 1.03 (d, J = 6.5 Hz, 5H), 0.72 (s, 1H).

Compound S228-Fmoc (0.15 µmol) was prepared using the general procedure.

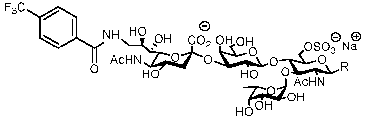

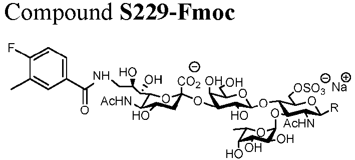

(0.15 µmol) was prepared using the general procedure.

1H NMR (600 MHz, D₂O) δ 7.75 (dd, J = 32.6, 7.7 Hz, 1H), 7.62 - 7.39 (m, 4H), 7.30 (dq, J = 43.3, 9.1, 7.4 Hz, 4H), 7.08 - 6.87 (m, 1H), 4.93 (d, J = 4.0 Hz, 1H), 4.51 - 4.29 (m, 2H), 4.26 - 4.06 (m, 2H), 4.02 - 3.24 (m, 26H), 2.91 - 2.76 (m, 1H), 2.62 (dd, J = 12.4, 4.5 Hz, 1H), 2.08 (s, 3H), 1.84 (d, J = 28.4 Hz, 6H), 1.63 (t, J = 12.2 Hz, 1H), 1.38 - 1.32 (m, 1H), 1.20 (d, J = 6.5 Hz, 2H), 1.02 (d, J = 6.6 Hz, 5H), 0.73 (s, 1H).

Compound S230-Fmoc (0.15 µmol) was prepared using the general procedure.

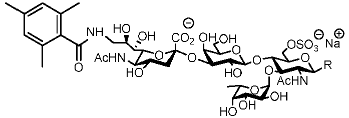

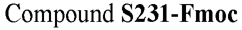

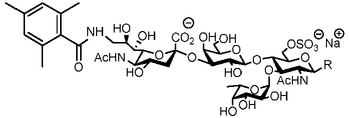

(0.15 µmol) was prepared using the general procedure.

1H NMR (600 MHz, D₂O) δ 8.00 - 7.79 (m, 2H), 7.69 (dd, J = 31.0, 7.9 Hz, 3H), 7.59 - 7.39 (m, 3H), 7.37 - 7.20 (m, 4H), 4.93 (d, J = 4.0 Hz, 1H), 4.47 - 4.40 (m, 1H), 4.39 - 4.33 (m, 1H), 4.22 - 4.06 (m, 2H), 4.03 - 3.15 (m, 26H), 2.90 - 2.75 (m, 1H), 2.63 (dd, J = 12.4, 4.6 Hz, 1H), 1.88 (s, 3H), 1.82 (s, 3H), 1.63 (t, J = 12.2 Hz, 1H), 1.34 (s, 1H), 1.19 (d, J = 6.6 Hz, 2H), 1.02 (d, J = 6.7 Hz, 5H), 0.74 (s, 1H).

### Example 1B - Synthesis of the S-Compounds - Procedure B

### I. Synthesis

The following reaction schemes were used for synthesizing 6-sulfo-sialyl Lewis X derivatives (S-series of compounds).

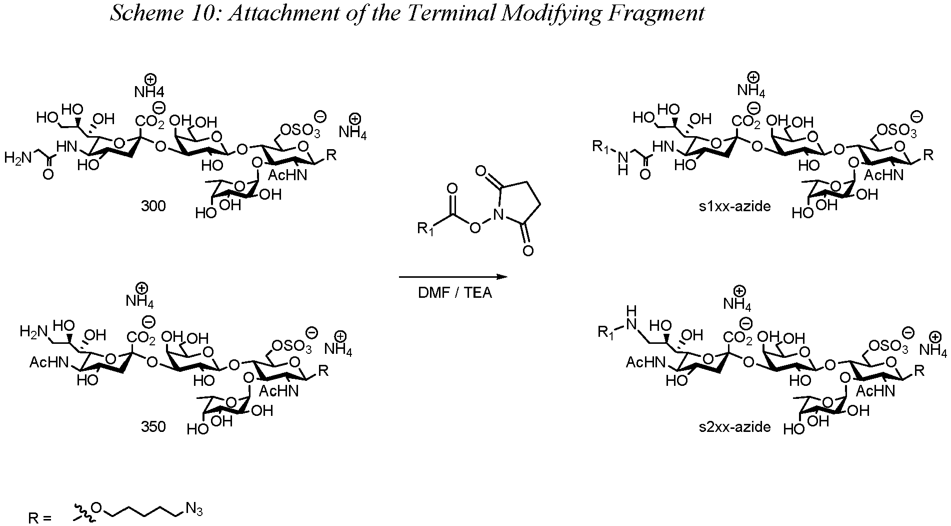

In Scheme 10, notation S1XX denotes compounds in which the substituent at position 5 of the terminal sialic acid is one of the fragments described herein in Table 1, and notation S2XX denotes compounds in which position 9 of the terminal sialic acid is substituted with one of the fragments described in Table 1. The compounds made according to Scheme 10, all have 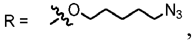 and are referred to herein as S1XX-Azide etc.

### I. Synthesis of Precursors 300 and 350

Precursor compounds 300 and 350 of Scheme 10 were synthesized as follows.

### 1. Synthesis of galactose building block.

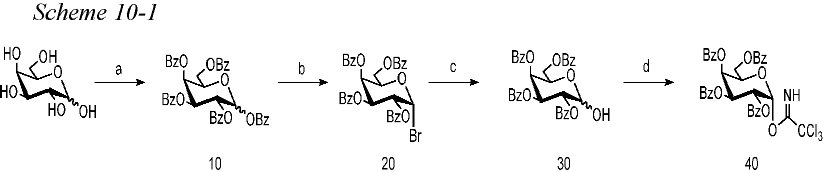

The following conditions were used in the reactions shown in Scheme 10-1: a) BzCl, pyridine; b) AcOH, Ac₂O, HBr; c) Ag₂CO₃, Acetone:H₂O; d) CNCCl₃, DBU. It is noted that Scheme 10-1 shows the same transformation as Scheme 1-1 of PROCEDURE A. The compound numbering in Scheme 10-1 is different from Scheme 1-1, but the compounds are the same.

More specifically, to a 1L round bottom in an ice bath, galactose (30g, 166 mmol) was dissolved in 400 mL pyridine. To the cooled solution was added benzoyl chloride (154 mL, 1.33 mol) dropwise over 1 h. After all benzoyl chloride has been added, the reaction was taken out of the ice bath and mixed at room temperature for 2 hrs which resulted in significant salt formation. The reaction was monitored by TLC (30% EtOAC/Hex, *R_{f}* = 0.8) and when complete, salt was removed by vacuum filtration. Excess pyridine was evaporated under reduced pressure, the crude material was dissolved in EtOAc (1L) successively washed with 5% H₂SO₄ (5 x 100mL), saturated CuSO₄ (2 x 100mL), 1M HCl (2 x 100 mL), brine (2 x 100mL), dried over MgSO₄, and solvents were evaporated under reduced pressure. The resulting oil was transferred to a 2L round bottom equipped with a condenser and a magnetic stir bar and placed in an oil bath. With vigorous stirring, the round bottom was heated to reflux with addition of MeOH (~1L in total). This process resulted in the formation of the desired per-benzoylated galactose **10,** which was collected by vacuum filtration and used in the next step without further purification. In an ice bath, **10** was suspended in acetic anhydride (50 mL) and acetic acid (80 mL) with vigorous stirring. To this suspension was added hydrogen bromide in acetic acid (204mL, 833 mmol) dropwise over 1 h; the mixture was brought to room temperature. After ~ 2 hrs the reaction mixture turned a translucent orange. When total conversion was observed by TLC (20% EtOAc/Hex, *R_{f} =* 0.50), the reaction was diluted with EtOAc (1L), extracted with DI water (5 x 200 mL), quenched with sodium bicarbonate (100 mL of DI water, then solid sodium bicarbonate was added), brine (2 x 100 mL), dried over MgSO₄, and reduced volume to yield **20** as a white solid (104 g, 95% over two-steps). To a solution of **20** (104 g, 158 mmol) in acetone (300 mL) and water (14.2 mL) was added freshly prepared Ag₂CO₃ (22 g, 79 mmol). The reaction was kept in the dark at room temperature and monitored by TLC (30% EtOAc/Hex, *R_{f}* = 0.45). When all starting material has been consumed, the reaction was filtered over celite, reduced *en vacuo,* dissolved in EtOAc (500 mL), washed with saturated sodium bicarbonate (3 x 100 mL), brine (2 x 100 mL), dried over MgSO₄, and volume reduced to yield the desired product 3. Per-benzoylated galactol **30** was dissolved in DCM (316 mL) containing trichloroacetonitrile (48 mL, 470 mmol). To this solution was added DBU dropwise until the reaction's pH reached ~ 10. The progress of the reaction was monitored by TLC (15% EtOAc/Hex, *R_{f}* = 0.2) and when all starting material was consumed, the reaction volume was reduced. The desired product **40** was purified by flash chromatography (15% EtOAc/Hex) to yield the desired α-imidate as a white solid (79.05 g, 68% over two-steps) with ¹H data agreeing with that reported in the literature.

### 2. Synthesis of Nphthalimido-glucosamine building block.

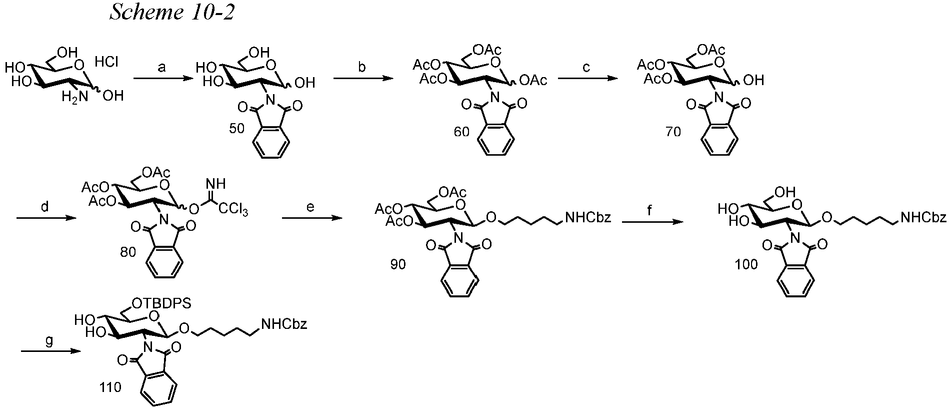

The following conditions were used in the reactions shown in Scheme 10-2: a) Phthalic anhydride, sodium methoxide; b) acetic anhydride, pyridine; c) ethylenediamine / acetic acid; d) trichloroacetonitrile, DBU; e) 5-amino-Cbz-pentan-1-ol, TMSOTf; f) sodium methoxide; g). TBDPS-Cl, imidazole.

Glucosamine hydrochloride (20 g, 93 mmol) was dissolved in MeOH (100 mL) with sodium methoxide (1M, 110 mL) and mixed at room temperature for 30 mins. The reaction mixture was filtered to remove NaCl and the filtrate was added to a 500 mL round bottom flask. To this solution was added phthalic anhydride (15.1 g, 102 mmol) and the mixture was vigorously mixed under reflux for 2 hrs. During this time, the product formed as a white insoluble material. After 2 hrs, the reaction was cooled in an ice bath and the insoluble material **(50)** was filtered and washed with cold MeOH and dried under reduced pressure. Solid material **50** was suspended in pyridine (130 mL) at 4°C and to this solution was added acetic anhydride (52 mL, 560 mmol) dropwise and catalytic DMAP. The mixture was kept on ice until all acetic anhydride was added and after, the reaction was warmed to room temperature and mixed overnight. The reaction progress was monitored with TLC (1:1 EtOAc/Hex) and upon completion, excess pyridine and acetic anhydride was removed under reduced pressure. The resulting crude material was dissolved in EtOAc (1 L), washed exhaustively with 1 M HCl (10 x 150 mL), sodium bicarbonate (5 x 100 mL), brine (2 x 100 mL), and dried over MgSO₄. The solvent was removed under reduced pressure to yield a white solid, **60** (35 g), which was carried to the next step without additional purification. Ethylenediamine (5.88 mL, 88 mmol) and acetic acid (5 mL, 88 mmol) were dissolved in THF (366 mL) at room temperature and mixed for 30 mins. To this solution was added **60** (35 g) and the solution was allowed to stir overnight. The reaction was monitored by TLC (1:1 EtOAc/Hex) and upon completion, THF was removed by reduced pressure, the crude material was redissolved in EtOAc (500 mL), washed with 1 M HCl (5 x 75 mL), saturated sodium bicarbonate (3 x 50 mL), brine (2 x 50 mL), dried over MgSO₄, and reduced *en vacuo* to provide **70** as a white solid. **70** (27.1 g) was dissolved in DCM (311 mL) containing trichloroacetonitrile (7.5 mL, 75 mmol). To this mixture was added DBU dropwise until the reaction pH → 10. Reaction progress was monitored by TLC (1:1 EtOAc/Hex) and when complete, the solvent was removed by reduced pressure and the product was purified by flash chromatography (1:1 EtOAc / Hex containing 0.1% TEA) to yield **80** as a clear oil (25.2 g, 46% yield over 4-steps). ¹H NMR and mass spectrometry data agreed with those details reported in the literature. **80** (12.6 g, 21.7 mmol) and 5-amino-Cbz-pentan-1-ol (5.15 g, 21.7 mmol) were dissolved in DCM (217 mL) containing flame dried 4Å molecular sieves (5 g, Sigma-Aldrich 208590). The solution was mixed at room temperature for 30 mins before being placed in a 4°C ice bath for 30 mins. To the ice-cooled solution was added TMSOTf (786 µL, 4.35 mmol) and progress was monitored by TLC (40% EtOAc / Hex). When all starting imidate had been consumed, the reaction was quenched by addition of triethylamine (1.5mL, 10.85 mmol). The reaction was filtered through a pad of celite, reduced *en vacuo,* and purified by flash chromatography (30→40% EtOAc / Hex) to yield **90** (8 g, 57%) as a viscous oil with ¹H NMR and mass spectrometry data agreed with those details reported in the literature. **90** (8 g, 12.2 mmol) was dissolved in MeOH (120 mL) and to this solution was added sodium methoxide in methanol (1 M stock solution) dropwise until the reaction pH steadied ~ 9. *Careful attention was be paid to not make the pH more basic than 9 as there is a risk of phalimido ring opening.* The reaction was monitored by TLC (10% MeOH / DCM) and confirmed by MALDI-MS. When all acetates had been removed, the reaction pH was neutralized using Amberchrom 50WX8 hydrogen form, 200-400 mesh (Sigma #217514). The resin was separated by vacuum filtration and the reaction solvent was removed under reduced pressure to yield the desired triol **100** as a sticky-white foam. To **100** was added DMF (120 mL) and imidazole (1.66 g, 24.4 mmol). This solution was placed in an ice bath and to the mixture was added TBDPS-Cl (4.7 mL, 18.3 mmol) dropwise over 1 hr. The reaction was allowed to warm to room temperature overnight and the following day, the reaction was monitored by TLC (100% EtOAc). DMF was removed via rotary evaporation and the crude product was redissolved in EtOAc (500 mL). The organic layer was washed thrice with 1 M HCl (3 x 50 mL), brine (2 x 50 mL), dried over MgSO₄, reduced, and purified by flash chromatography (40% EtOAc / Hex) to provide 110 (6.53 g, 70% over two steps) as a white foam.¹H NMR and mass spectrometry data agreed with those details reported in the literature.

### 3. Disaccharide glycosylation

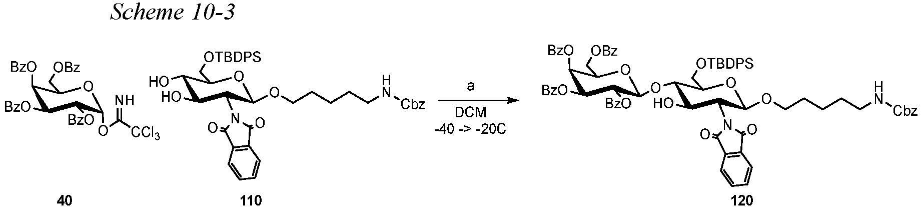

The following conditions were used in the reaction shown in Scheme 10-3: TMSOTf, 4A MS.

More specifically, acceptor **110**(6.53 g, 8.5 mmol) and donor **40** (6.3 g, 8.5 mmol) were dissolved in DCM (85 mL) containing 4Å MS and mixed at room temperature for 30 mins. The reaction mixture was cooled to -40°C in a dry ice bath and to this mixture was added TMSOTf (308µL, 1.7 mmol). The reaction was slowly warmed to -20°C over a 1 hr period. During this time, the reaction was monitored by TLC (40% EtOAc / Hex) and when complete, quenched with TEA (474 µL, 3.4 mmol). *Note: This reaction is a regioselective glycosylation. If the reaction temperature was increased too much, or excess donor (**40**) was used, there exists a possibility of over glycosylation at the 3-position.* The reaction was filtered through celite and purified by flash chromatography (40% EtOAc / Hex) to yield the product **120** as a white foam (5.1 g, 44% yield).

¹H (600 MHz, CDCl₃), δ (ppm): GlcNPhth: 5.13 (H1), 4.18 (dd, *J =* 10.8, 8.6 Hz, H2), 4.60 (H3), 4.09 (H4), 3.46 (H5), 3.79 (H6a, H6b); Gal: 5.11 (H1), 5.85 (dd, *J =* 10.5, 8.1 Hz, H2), 5.57 (dd, *J* = 10.5, 3.5 Hz, H3), 5.95 (d, *J* = 3.3 Hz, H4), 4.29 (H5), 4.69 (ddd, *J* = 11.7, 4.3, H6a), 4.37 (ddd, *J* = 11.6, 8.3 Hz, H6b).

¹³C from HSQC (75 MHz, CDCl₃), δ (ppm): GlcNPhth: 97.8 (C1), 56.1 (C2), 69.8 (C3) 80.2 (C4), 75.3 (C5), 61.7 (C6); Gal: 101.5 (C1), 69.6 (C2), 71.6 (C3), 67.9 (C4), 72.5 (C5), 62.6 (C6).

MALDI-TOF MS *m*/*z* calcd for C₇₇H₇₆N₂O₁₈SiNa (M+Na)⁺= 1367.4755, found = 1367.6501

### 4. Debenzoylation, phthalimdo deprotection, global acetylation

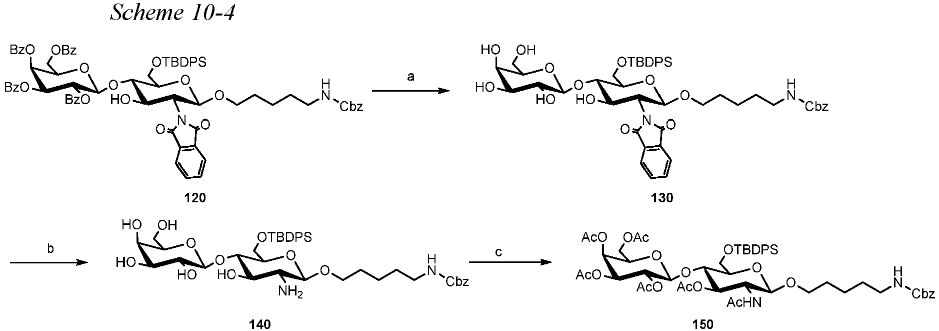

The following conditions were used in the reaction shown in Scheme 10-4: a) sodium methoxide in methanol; b) hydrazine hydrate; c) acetic anhydride and pyridine.

More specifically, disaccharide **120** (4.7 g, 3.5 mmol) was dissolved in MeOH (175 mL) and to this reaction was added aliquots of sodium methoxide (1 M stock solution) until the reaction pH was ~9. *Careful attention was paid to not increase the pH above 9 as the increased basicity could cause undesired imido ring opening.* The reaction progress was monitored by MALDI-MS, and when all benzoyl groups had been removed, the reaction pH was neutralized with Amberchrom 50WX8 hydrogen form, the resin was removed by vacuum filtration, and the MeOH was reduced under reduced pressure to yield **130** which was not purified before moving to the next step. **130** was dissolved in EtOH (175 mL) and to this solution was added hydrazine hydrate (50-60%, 1.6 mL, 17.5 mmol). The reaction was stirred under reflux until phalimido deprotection had been achieved (monitored by MALDI-MS). Upon successful phalimido deprotection, the reaction was cooled, any insoluble material was filtered through Celite, and the filtrate was reduced to yield crude **140** which was carried forward without additional purification. **140** was dissolved in pyridine (65 mL) and to this reaction was added catalytic DMAP. The reaction was placed in an ice bath and acetic anhydride (6.6 mL, 70 mmol) was added dropwise. After all anhydride was added, the reaction was warmed to room temperature. The reaction was monitored by TLC (40% EtOAc / Hex). Upon conversion to the product, the reaction was quenched with MeOH (20 mL), solvent was reduced *en vacuo,* and the crude material was redissolved in EtOAc (500 mL), washed with 1 M HCl (10 x 50 mL), saturate copper sulfate (2 x 50 mL), saturated sodium bicarbonate (5 x 50), brine (2 x 50), dried over MgSO₄, reduced, and purified by flash chromatography (40% EtOAc / Hex) to yield **150** (2.03 g, 55% over three steps) as a white foam.

¹H (600 MHz, CDCl₃), δ (ppm): GlcNAc: 4.35 (d, *J* = 7.8 Hz, H1), 4.07 (H2), 5.01 (H3), 4.11 (H4), 3.31 (H5), 3.94 (H6a), 3.89 (H6b); Gal: 4.76 (d, *J =* 8.0 Hz, H1), 5.06 (H2), 4.92 (dd, *J =* 3.5 Hz, H3), 5.31 (dd, *J =* 3.2 Hz, H4), 3.76 (dd, *J =* 6.6 Hz, H5), 4.11 (H6a, H6b)

¹³C from HSQC (75 MHz, CDCl₃): δ (ppm): GlcNAc: 101.1 (C1), 53.6 (C2), 72.3 (C3), 73.9 (C4), 75.4 (C5), 61.3 (C6); Gal: 100.5 (C1), 69.3 (C2), 71.0 (C3), 67.2 (C4), 70.7 (C5), 61.3 (C6).

MALDI-TOF MS *m*/*z* calcd for C₅₃H₇₀N₂NaO₁₈SiNa (M+Na)⁺= 1073.4285, found = 1073.5575

### 5. 6-O-TBDPS deprotection.

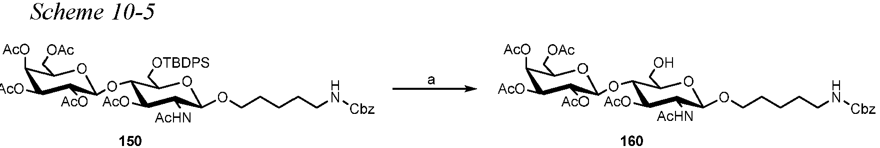

The following conditions were used in the reactions shown in Scheme 10-5: a) HF-Pyridine
More specifically, **150** (2.03 g, 1.93 mmol) was dissolved in pyridine (15 mL) and placed in an ice bath. To this solution was added HF-Pyridine (70%, 2.3 mL, 19.3 mmol). When all HF-pyridine had been added the reaction was kept on ice for 30 mins before warming to room temperature. The reaction was monitored with TLC (40% acetone / hex) and upon completion the contents were transferred into EtOAc (300 mL) and extracted with 1 M HCl, brine, and dried over MgSO₄. The crude reaction was purified by flash chromatography using 1:1 acetone / hex to yield **160** (1.12 g, 72%) as a white foam.

¹H (600 MHz, CDCl₃), δ (ppm): GlcNAc: 4.40 (d, *J =* 8.2 Hz, H1), 3.96 (H2), 5.06 (H3), 3.91 (H4), 3.35 (H5), 3.86 (H6a), 3.72 (H6b); Gal: 4.62 (d, *J* = 7.9 Hz, H1), 5.11 (H2), 4.99 (H3), 5.34 (dd, *J =* 2.5 Hz, H4), 3.90 (H5), 4.09 (ddd, *J =* 11.1, 5.9 Hz, H6a and H6b).

¹³C from HSQC (75 MHz, CDCl₃), δ (ppm): GlcNAc: 101.3 (C1), 54.1 (C2), 73.0 (C3), 74.9 (C4), 75.0 (C5), 60.7 (C6); Gal: 100.9 (C1), 69.8 (C2), 70.9 (C3), 68.0 (C4), 70.4 (C5), 61.2 (C6).

MALDI-TOF MS *m*/*z* calcd for C₃₇H₅₂N₂O₁₈Na (M+Na)⁺= 835.3113, found = 835.4063

### 6. Installation of 6-O sulfate

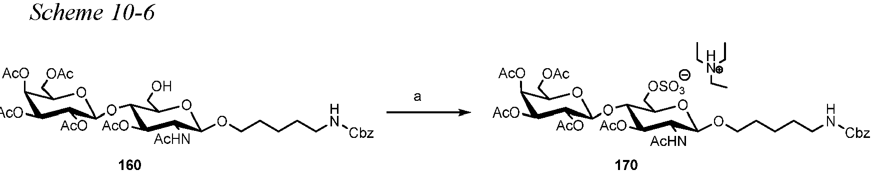

The following conditions were used in the reactions shown in Scheme 10-6: a) sulfurtrioxide pyridine complex in DMF.

More specifically, **160** (1.34 g, 1.65 mmol) was dissolved in 33 mL DMF at room temperature. To this mixture was added sulfur trioxide pyridine complex (787 mg, 4.9 mmol) and the reaction was stirred overnight. Product formation was monitored by TLC (10% MeOH / DCM). When all starting material had been consumed the reaction was quenched by adding 3 mL of a 1:1 mixture of MeOH/ TEA. The solvent was removed under reduced pressure and the product was desalted using LH-20 using size-exclusion chromatography (2.5 x 100 cm, eluting with 1:1 MeOH/DCM, 4 mins / fraction, 4 - 5 mL / fraction). The product was found to elute in fraction 66 - 76 and upon drying, yielded a white foam. The product **170** was moved to the next step without additional purification

### 7. Global deacetylation

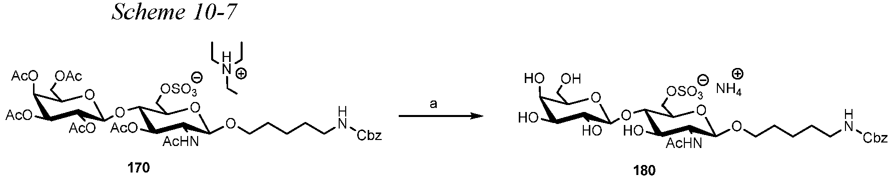

The following conditions were used in the reactions shown in Scheme 10-7: a) Sodium methoxide in methanol.

More specifically, **170** (1.25 g, 12.8 mmol) was dissolved in MeOH (50 mL) and the pH was adjusted with sodium methoxide in methanol (1 M stock) to ~ 9. The reaction progress was monitored by TLC (7:3:2 EtOH:NH₄OH:H₂O) and ESI-MS. When all acetate groups had been removed, the reaction pH was neutralized with Amberchrom H⁺ resin as previously described and then the resin was removed by vacuum filtration. The filtrate was dried under reduced pressure and the corresponding solid was redissolved in minimum volume of 100 mM Ammonium bicarbonate. The material was loaded onto a Bio-Rad P2 size-exclusion column (60 x 5 cm) and eluted with 100 mM ammonium bicarbonate with each fraction representing 15 mins of elution (~ 40 mL / fraction). Fractions were stained with 5% sulfuric acid in ethanol and confirmed by ESI. Fractions containing the product were collected, concentrated under reduced pressure, and placed on the freeze-dryer to yield **180** (726 mg, 80% yield) as a white fluffy solid.

¹H (600 MHz, CDCl₃), δ (ppm): GlcNAc: 4.38 (H1), 3.58 (H2), 3.63 (H5), 4.26 (H6a), 4.17 (H6b), 1.85 (NHAc); Gal: 4.38 (H1), 3.38 (H2), 3.55 (H3), 3.79 (H4), 3.62 (H6a and H6b).

¹³C from HSQC (75 MHz, CDCl₃), δ (ppm): GlcNAc: 102.1 (C1), 55.2 (C2), 72.6 (C5), 66.0 (C6), 22.1 (NHAc); Gal: 102.1 (C1), 71.2 (C2), 72.3 (C3), 68.8 (C4), 61.0 (C6)

ESI-TOF MS *m*/*z* calcd for C₂₇H₄₁N₂O₁₆S (M-H)⁻ = 681.2182, found = 681.0994

### 8. Cbz deprotection

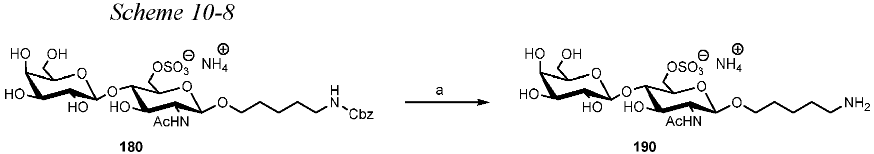

The following conditions were used in the reactions shown in Scheme 10-8: a) Pd/C, H₂, 1:1 *t*-BuOH/H₂O.

More specifically, **180** (100 mg, 0.14 mmol) was dissolved in a 1:1 mixuture of *t-*BuOH / H₂O (1.5 mL) and to this solution was added catalytic ~ 5% (*wt*/*wt*) Pd/C. The reaction was mixed under a hydrogen atmosphere overnight. Reaction progress was monitored by TLC (7:3:2 EtOH:NH₄OH:H₂O) and when complete the reaction was filtered through a 0.2 µm Whatman syringe filter and freeze-dried to yield a white solid **190** (73 mg, 91%).

### 9. Azide installation

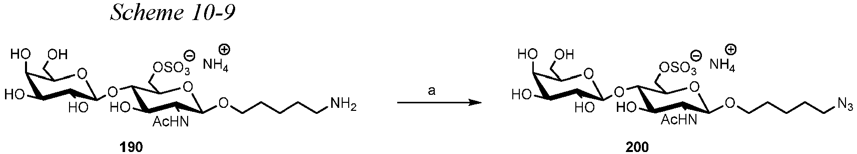

The following condition was used in the reaction shown in Scheme 10-9: a) 1-(azideosulfonyl)-1H-imidazol-3-ium chloride, copper(II) sulfate, potassium carbonate.

More specifically, **190** (72 mg, 0.13 mmol), 1-(azideosulfonyl)-1H-imidazol-3-ium chloride (80 mg, 0.38 mmol), copper sulfate (10 umol), and potassium carbonate (79 mg, 0.57 mmol, 579 uL of 1 M stock solution) were dissolved in water (1.96 mL). The reaction was mixed at room temperature and monitored by TLC (7:3:2 FtOH:NH₄OH:H₂O). When all starting material had been consumed, Cuprisorb^{™} was added to the reaction which was incubated at room temperature for 2 hrs to remove copper from the solution. The resin was removed by filtration, the reaction volume was reduced *en vacuo,* and the product was purified by **P2** size exclusion (70 x 2.5 cm column, 100 mM ammonium bicarbonate, 8 mins / fraction, ~ 5 mL / fraction). Product containing fractions (26 - 32) were pooled and dried under reduced pressure to yield a white solid **200** (67 mg, 89% yield).

¹H (600 MHz, CDCl₃), δ (ppm): GlcNAc: 4.39 (H1), 3.59 (H2), 3.64 (H5), 4.26 (ddd, *J* = 10.9 Hz, H6a), 4.17 (ddd, *J =* 11.0, 3.7 Hz, H6b); Gal: 4.41 (H1), 3.38 (H2), 3.53 (H3), 3.77 (H4), 3.60 (H6a and H6b)

¹³C from HSQC (75 MHz, CDCl₃), δ (ppm): GlcNAc: 102.5 (C1), 55.1 (C2), 65.9 (C6), 21.9 (NHAc); Gal: 101.3 (C1), 71.1 (C2), 72.1 (C3), 68.7 (C4), 61.0 (C6)

ESI-TOF MS *m*/*z* calcd for C₁₉H₃₃N₄O₁₄S (M-H)⁻ = 573.1719, found = 573.0749

### 10. Chemical synthesis of 6-sulfo-N-acetylglucosamine

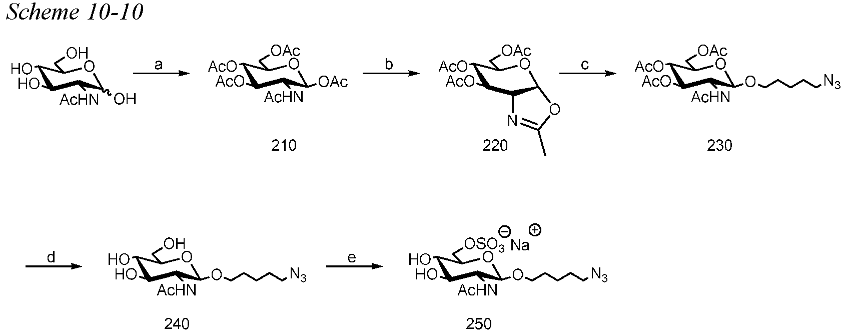

The following conditions were used in the reactions shown in Scheme 10-10: a) Ac₂O, sodium acetate; b) TMSOTf; c) 5-azidopentan-1-ol, TMSOTf; d) sodium methoxide; e) sulfur trioxide-pyridine. It is noted that Scheme 10-10 shows the same transformation as Scheme 1-6 of PROCEDURE A. The compound numbering in Scheme 10-10 is different from Scheme 1-6, but the compounds are the same.

More specifically, N-acetylglucosamine (100 g, 450 mmol) was added to a solution of refluxing of acetic anhydride (340 mL, 3375 mmol) and sodium acetate (37.2 g, 450 mmol) in portions. The mixture was stirred at reflux until all material was dissolved and TLC analysis (100% EtOAc, *R_{f}* = 0.47) indicated reaction was complete. Excess acetic anhydride was removed under reduced-pressure and the crude material was dissolved in EtOAc (1 L) and washed with saturated sodium bicarbonate (10 x 150 mL), brine (3 x 100), dried over MgSO₄, and reduced *en vacuo* to provide per-acetylated N-acetyl glucosamine (**210**, 129 g, 74%) as a white solid. **210** (5.4 g, 13.9 mmol) was dissolved in DCE (69 mL) and to this solution was added TMSOTf (2.5 mL, 13.87 mmol). The solution was warmed to 60°C to facilitate oxazolidone formation which was monitored by TLC (40% acetone/hex, *R_{f}* = 0.3). The reaction was quenched with TEA (2.2 mL, 15.18 mmol), the solvent was reduced, and the product was purified by flash chromatography (40% acetone/hex) to yield **220** (3.9 g, 85%) as a clear oil. Tri-acetylated oxazolidone **220** (4.36 g, 13.24 mmol) was dissolved in DCE (66 mL). To this solution was added 5-azidopentan-1-ol (3.4 g, 26.48 mmol) and 4Å MS (3 g). The combination was mixed at room temperature after which, TMSOTf (2.4 mL, 13.24 mmol) was added. The reaction was warmed to 60°C to facilitate glycosylation with progress monitored by TLC (90% EtOAc/Hex, *R_{f}* = 0.55). When all starting material was converted to product, the reaction was quenched with TEA (1.84 mL, 13.24 mmol) and cooled to room temperature. The solvent was removed under reduced pressure and glycosylated N-acetylglucosamine was recovered by flash chromatography (80→90% EtOAc/Hex) to provide **230** (5 g, 80%) as a white solid. **230** (14.38 g, 31.37 mmol) was dissolved in MeOH (156 mL) and the solution was adjusted to pH 9 with sodium methoxide in methanol (1 M stock solution). The reaction was monitored with TLC (20% MeOH/DCM, *Rᵣ* = 0.76) and when complete, the pH was neutralized with Dowex 50WX8 H⁺ resin. The resin was removed by filtration and the MeOH was removed by reduced pressure to yield triol **240** (9.3 g, 90%) as a white solid. 6-O-sulfation was achieved by dissolving **240** (600 mg, 1.8 mmol) in DMF (36 mL) and placing the mixture in an ice bath. To the cooled solution was added sulfur trioxide-pyridine complex (287 mg, 1.8 mmol). The reaction was stirred overnight at 4°C for three days with additional of sulfur trioxide-pyridine added (287 mg, 1.8 mmol) daily. The reaction was monitored by TLC (20% MeOH/DCM, *Rf* = 0.23) and quenched with aliquots of NaOH (5M, pH → 8). The solvent was removed, and the reaction was purified by Bio-Gel size-exclusion chromatography. Product containing fractions were freeze-dried to yield **250** (514 mg, 69%) as a white powder.

The 2D ¹H-NMR spectra of compounds **240** and **250** of Scheme 10-10are shown in FIGs. 1A and 1B.

### 11. Enzymatic installation of β1,4-Gal for 6-sulfo-N-acetyllactosamine formation

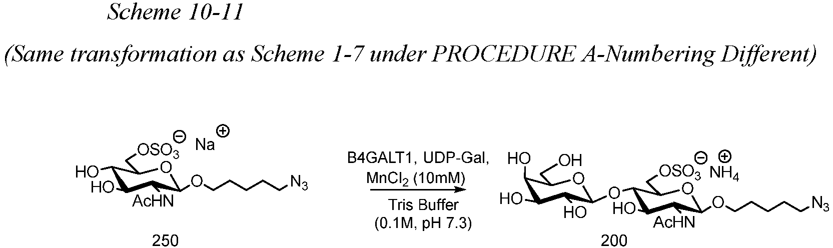

Installation of the β1,4-Gal was achieved by dissolving **250** (5 mg, 11.7 µmol) and UDP-Galactose (10 mg, 17.6 µmol) in Tris Buffer (585 µL, pH 7.3, 0.1M) containing MnCl₂ (10 mM). To this solution was added B4GALT1 (50 µg, 1% *wt*/*wt*) and calf intestine alkaline phosphatase (CIAP, 5.85 µL, 1kU stock). The reaction was incubated at 37°C for three hours. Reaction progress was monitored by LC-ESI-MS equipped with a Waters XBridge BEH, Amide column, 2.5 µm, 130 Å, 2.1 × 150 mm (Flow rate 0.25 mL/min, A = 10 mM ammonium formate, B = ACN. Linear gradient 80%→60% B over 18 mins).

The reaction was monitored by tracing the crude reaction using Hydrophilic interaction liquid chromatography/electrospray ionization mass spectrometry (HILIC-LC/ESI-MS). The results of such tracing are shown in FIG. 2. In the event the reaction was not complete, excess UDP-Gal (5 mg, 5.85 µmol) and B4GALT1 (25 µg) was added to the reaction. The reaction mixture was filtered through a PALL Nanosep^{®} Centrifuge spin filter (3k MWCO) and the filtrate purified by Bio-Gel P2 size-exclusion chromatography. Product containing fractions were freeze-dried to provide desired disaccharide **200** (4.8 mg, 70%).

### 12. Chemoenzymatic synthesis of CMP-Neu5-Glycine

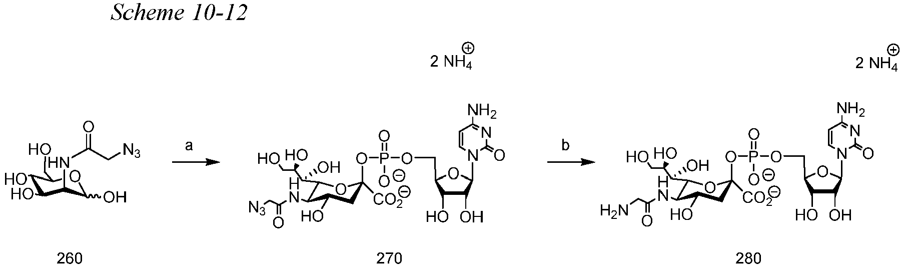

The following conditions were used in the reactions shown in Scheme 10-12: a) sodium pyruvate, cytidine triphosphate disodium salt; PmNanA; *NmCSS, PmPPA;* b) Lindlar Catalyst, H₂.

More specifically, ManAz (**260,** 100 mg, 0.38 mmol), cytidine triphosphate disodium (219 mg, 0.42 mmol), and sodium pyruvate (210 mg, 1.91 mmol) were dissolved in Tris Buffer (100 mM, pH 8.5, 19 mL) containing MgCl₂ (10 mM). To this reaction was added **sialic acid aldolase** (1 mg) from *Pasteurella multocida* (PmNanA)(See: Li, Y.; Yu, H.; Cao, H.; Lau, K.; Muthana, S.; Tiwari, V. K.; Son, B.; Chen, X., Pasteurella multocida sialic acid aldolase: a promising biocatalyst. Appl Microbiol Biot 2008, 79 (6), 963.), **CMP-sialic acid synthetase** (1 mg) from *Neisseria meningitidis* group B (*Nm*CSS) (See: Yu, H.; Yu, H.; Karpel, R.; Chen, X., Chemoenzymatic synthesis of CMP-sialic acid derivatives by a one-pot two-enzyme system: comparison of substrate flexibility of three microbial CMP-sialic acid synthetases. Bioorg Med Chem 2004, 12 (24), 6427-6435.) and inorganic **pyrophosphatase** (1 mg) from *Pasteurella multocida* (*PmPPA*) (See: Lau, K.; Thon, V.; Yu, H.; Ding, L.; Chen, Y.; Muthana, M. M.; Wong, D.; Huang, R.; Chen, X., Highly efficient chemoenzymatic synthesis of [small beta]1-4-linked galactosides with promiscuous bacterial [small beta]1-4-galactosyltransferases. Chem Commun 2010, 46 (33), 6066-6068.) and the reaction was incubated overnight at 37°C. When all **260** had been consumed as determined by TLC, Calf Intestine Alkaline Phosphate (CIAP, 1000 units) was added to the reaction and the mixture was again incubated at 37°C overnight. On the third day, cold ethanol (17 mL) was added to the mixture and all insoluble material was removed by centrifugation. The supernatant was concentrated, and CMP-Neu5Az **(270)** was purified by P2 size-exclusion chromatography (5 x 60 cm, eluting with 100 mM ammonium bicarbonate, 15 mins / fraction or ~35 mL). Product positive fractions were pooled to yield a white, fluffy solid (185 mg, 74%) with analytical details matching those found in the literature. **270** (185 mg, 0.28 mmol) was dissolved in 20 mM ammonium hydroxide (28 mL) and to this mixture was added Lindlar's catalyst (9 mg). The mixture was stirred under a hydrogen atmosphere until no starting material could be observed by TLC (7:3:2 EtOH:NH₄OH:H₂O). The catalyst was removed with a 0.2 µm syringe filter and the filtrate was dried by lyophilization to yield a white solid **280** (219 mg, 92%) that was used without additional purification.

Compound **280** was analyzed by LC-MS using a Waters XBridge BEH, Amide column, 2.5 µm, 130 Å, 2.1 x 150 mm using ESI as detector at a flow rate of 0.25 mL/min; 80% → 60% A over 18 mins (A = 10 mM ammonium formate, pH 3.4, B = Acetonitrile). The peak for Compound **280** eluted at 19.0 minutes. The molecular mass of Compound **280** was determined using electrospray ionization-time-of-flight mass spectrometry (ESI-TOF MS); *m*/*z* calcd for C₂₀H₃₁N₅O₁₆P (M-H)⁻ = 628.1509, found = 628.0397.

### 13. Enzymatic installation of α2,3-Neu5-Glycine

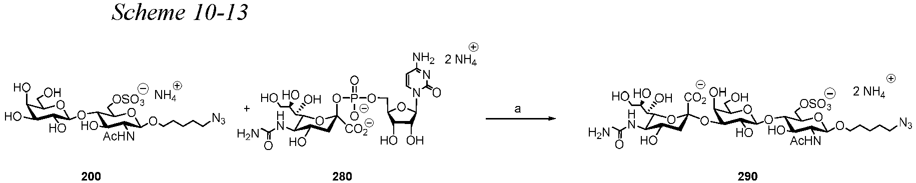

The following condition was used in the reaction shown in Scheme 10-13: a) PmST1, CIAP.

More specifically, **200** (70 mg, 0.12 mmol) and **280** (115 mg, 183 mmol) were dissolved in Tris buffer (100 mM, pH 8.5, 12 mL) containing sialyltransferase-I (700 µg) from *Pasteurella multocida* (*Pm*St1) (Yu H, Chokhawala H, Karpel R, Yu H, Wu B, Zhang J, Zhang Y, Jia Q, Chen X. A multifunctional Pasteurella multocida sialyltransferase: A powerful tool for the synthesis of sialoside libraries. J Am Chem Soc. 2005;127:17618-17619.) and CIAP (120 U). The reaction was incubated at 30°C until all **200** was consumed as determined by TLC (7:3:2 FtOH:NH₄OH:H₂O). After all starting disaccharide had been consumed, cold EtOH was added to the mixture (12 mL) and the insoluble material was removed by centrifugation. The supernatant was concentrated and purified by P2 size-exclusion chromatography (2.5 x 70 cm, eluting with 100 mM ammonium bicarbonate, 8 mins / fraction). Fractions containing the desired product were pooled and concentrated by lyophilization to yield **290** as a white solid (82 mg, 77% yield).

Compound 290 was analyzed by LC-MS HPLC using aWaters XBridge BEH, Amide column, 2.5 µm, 130 Å, 2.1 x 150 mm using ESI as detector at a flow rate of 0.25 mL/min; 80% → 60% A over 18 mins (A = 10 mM ammonium formate, pH 3.4, B = Acetonitrile). The peak for Compound 290 eluted at 11.2 minutes. The molecular mass of Compound 290 was determined using ESI-TOF MS: m/z calcd for C₃₀H₅₁N₆O₂₂S (M-H)⁻ = 879.2783, found = 879.1189.

### 14. Enzymatic installation of α1,3-fucose with FUT6

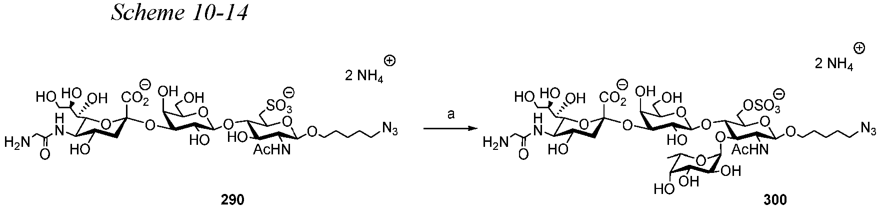

The following conditions were used in the reaction shown in Scheme 10-14: a) FUT6, GDP-fucose.

More generally, **290** (71 mg, 80.8 µmol) and GDP-Fucose (102 mg, 161 µmol) were dissolved in sodium cacodylate buffer (100 mM, pH 6.5, 4 mL) containing MnCl₂ (10 mM). To this solution was added FUT6 (50 µg) and CIAP (40 units). The reaction was incubated overnight at 37°C and the following day the progress was monitored by Shimadzu LC-ESI-IT-TOF with a Waters XBridge BEH, Amide column, 2.5 µm, 130 Å, 2.1 x 150 mm using ESI as detector at a flow rate of 0.25 mL/min; 20% → 40% A over 18 mins (A = 10 mM ammonium formate, pH 3.4, B = Acetonitrile). In the event the reaction was incomplete, an additional aliquot of FUT6 was added until the reaction reached completion. When complete, cold EtOH (4 mL) was added and the insoluble material was removed by centrifugation. The product was purified by P2 chromatography (70 x 2.5 cm, 100 mM ammonium bicarbonate, 8 mins / fraction). Product containing fractions were pooled to yield **300** (65 mg, 79%) as a white fluffy solid.

¹H (600 MHz, CDCl₃), δ (ppm): GlcNAc: 4.42 (d, *J* = 7.9 Hz, H1), 3.75 (H2), 3.73 (H3), 3.86 (H4), 3.64 (H5), 4.23 (H6a and H6b); Gal: 4.47 (d, *J* = 7.8 Hz, H1), 3.36 (H2), 3.97 (dd, *J* = 9.9, 3.1 Hz, H2), 3.82 (H3), 3.46 (H4), 3.55 (H6a and H6b); Fuc: 4.96 (d, *J* = 3.9 Hz, H1), 3.52 (H2), 3.75 (H3), 3.64 (H4), 4.68 (H5), 1.04 (d, *J =* 6.6 Hz, CH₃); Neu5Glycine: 2.61 (dd, *J* = 12.4, 6.0 Hz, H3-equitorial), 1.68 (dd, *J* = 12.2 Hz, H3-axial), 3.57 (H4), 3.75 (H9a and H9b).

¹³C from HSQC (75 MHz, CDCl₃), δ (ppm): GlcNAc: 100.9 (C1), 55.8 (C2), 74.7 (C3), 73.0 (C4), 72.9 (C5), 65.9 (C6); Gal: 101.3 (C1), 69.6 (C2), 75.4 (C3), 67.2 (C4), 67.9 (C5), 61.3 (C6); Fuc: 98.8 (C1), 67.0 (C2), 69.2 (C3), 71.8 (C4), 66.9 (C5) 15.9 (CH₃); Neu5Glcyine: 39.5 (C3), 72.6 (C4), 51.7 (C5), 62.7 (C9)

Compound **300** was analyzed by HPLC LC-MS using aWaters XBridge BEH, Amide column, 2.5 µm, 130 Å, 2.1 x 150 mm using ESI as detector at a flow rate of 0.25 mL/min; 80% → 60% A over 18 mins (A = 10 mM ammonium formate, pH 3.4, B = Acetonitrile). The peak for Compound **300** eluted at 13 minutes. The molecular mass of Compound 300 was determined using ESI-TOF MS: m/z calcd for C₃₆H₆₁N₆O₂₆S (M-H)⁻ = 1025.3362found = 1025.1684.

### 15. Enzymatic synthesis of 9-amino CMP-Neu5Ac

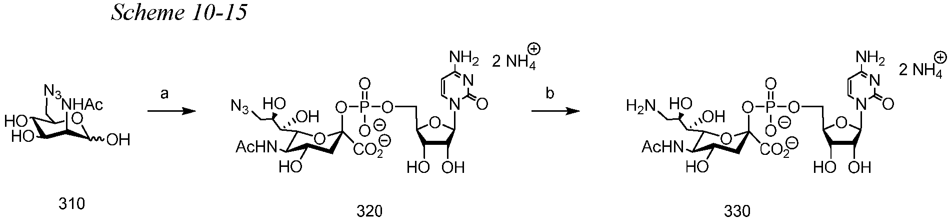

The following conditions can be used in the reaction shown in Scheme 10-15: a) sodium pyruvate, cytidine triphosphate disodium salt; PmNanA; *Nm*CSS, *Pm*PPA; b) Lindler Catalyst, H₂.

More specifically, **330** can be synthesized following the same protocol as shown for the synthesis of **280.**

### 16. Enzymatic installation of α2,3-Neu5Ac-9-amino

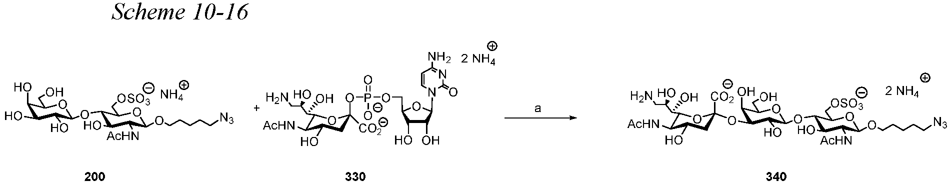

The following condition can be used in the reaction shown in Scheme 1-16: a) *Pm*ST1, CIAP.

More specifically, **340** can be synthesized following the same protocol as shown for the synthesis of **290.**

### 17. Enzymatic installation of α1,3-fucose with FUT6

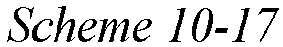

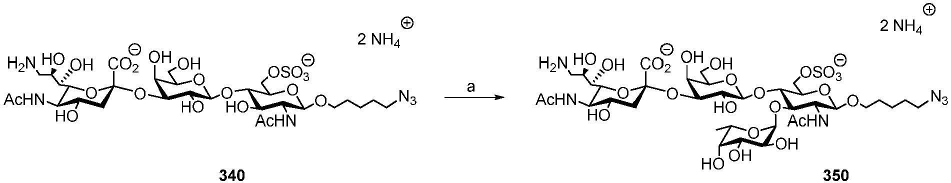

The following condition can be used in the reaction shown in Scheme 10-17: a) FUT6, GDP-fucose.

More specifically, **350** can be synthesized following the same protocol as the synthesis of **300.**

### II. The Library of the S-Compounds According to Procedure B

Exemplary S-compounds were prepared according to Procedure B as exemplified below.

### Synthesis of S106-Azide

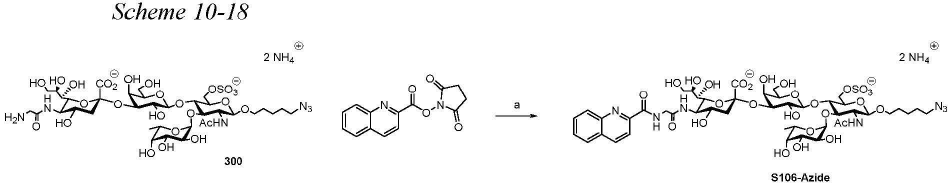

The following condition was used in the reaction shown in Scheme 10-18: a) Triethylamine.

More specifically, **300** (14 mg, 13.66 µmol) and quinoline-2-carbonyl-NHS ester (11 mg, 54.6 µmol) was dissolved in a 1:1 mixture of H₂O/DMF (683 µL) and to this solution was added triethylamine (11 µL, 81.9 µmol). The reaction mixture was incubated overnight at 37°C and the following day, the progress was monitored LC-ESI-MS equipped with a Shimadzu C18 5µm, 50x4.6 mm column with a flow rate of 0.25 mL/min with a gradient of 0→100% Acetonitrile over 10 mins (Solvent A = 10 mM ammonium formate, pH 3.4). When all starting material had been consumed, the product was purified by P2 chromatography (1.5 x 80 cm, 100 mM ammonium bicarbonate, 15 drops / fraction) and the product containing fractions were pooled and lyophilized to yield **S106-Azide** (11 mg, 68%) as a white fluffy solid.

LC-MS Traceof **S106-Azide** was conducted using the same conditions as those described above for monitoring the progress of the reaction. The product, **S106-Azide** eluted at 6 minutes. The molecular mass of Compound **S106-Azide** was determined using ESI-TOF MS: m/z calcd for C₄₆H₆₆N₇O₂₇S (M-H⁻) = 1180.3733found = 1180.1682.

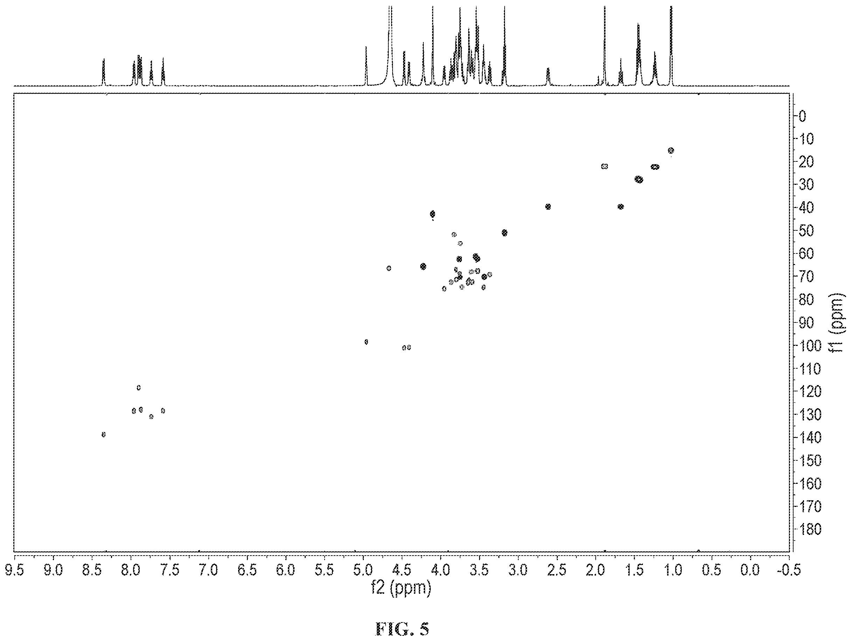

HSQC (600 MHz) 2D NMR spectrum of S106 Azide is shown in FIG. 5.

The following representative compounds were made in accordance with Scheme 10 generally, and Scheme10-18 specifically. All of the compounds were analyzed by LC-ESI-MS equipped with a Shimadzu C18 5µm, 50x4.6 mm column with a flow rate of 0.25 mL/min with a gradient of 0→100% Acetonitrile over 10 mins (Solvent A = 10 mM ammonium formate, pH 3.4).

### S101-Azide:

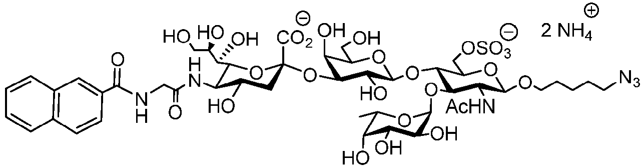

**S101-Azide:** The product eluted at 6.75 mins._The molecular mass of **S101-Azide** was determined using ESI-TOF MS: m/z calcd for C₄₇H₆₇N₆O₂₇S (M-H⁻) = 1179.3780, found = 1179.1732.

### S103-Azide:

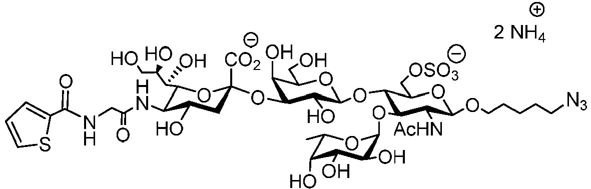

**S103-Azide:** The product eluted at 5.6 mins. The molecular mass of **S103-Azide** was determined using ESI-TOF MS: m/z calcd for C₄₁H₆₃N₆O₂₇S₂ (M-H⁻) = 1135.3188, found = 1135.1208.

### S105-Azide:

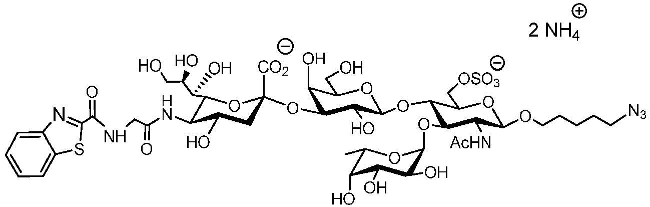

**S105-Azide:** The product eluted at 6.5 mins. The molecular mass of **S105-Azide** was determined using ESI-TOF MS: m/z calcd for C₄₄H₆₄N₇O₂₇S₂ (M-H⁻) = 1186.3297 found = 1186.0807.

### S107-Azide

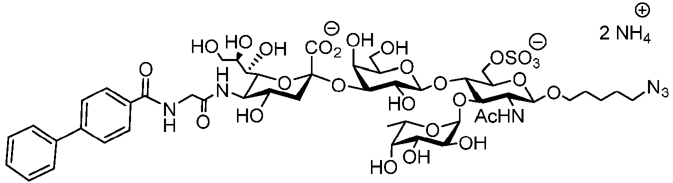

**S107-Azide:** The product eluted at 7 mins. The molecular mass of **S107-Azide** was determined using ESI-TOF MS: m/z calcd for C₄₉H₆₉N₆O₂₇S (M-H⁻) = 1205.3937, found = 1205.1807.

### S110-Azide:

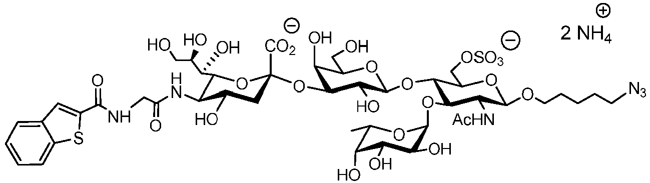

The product eluted at 6.6 mins. The molecular mass of **S110-Azide** was determined using ESI-TOF MS: m/z calcd for C₄₅H₆₅N₆O₂₇S₂ (M-H⁻) = 1185.3345, found = 1185.1246.

### S112-Azide:

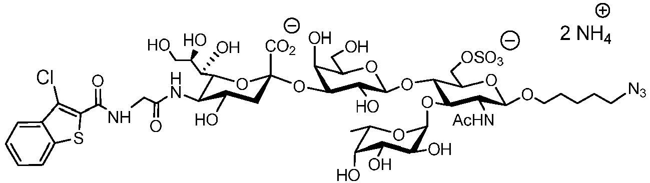

The molecular mass of **S112-Azide** was determined using ESI-TOF MS: m/z calcd for C₄₅H₆₄ClN₆O₂₇S₂ (M-H⁻) = 1219.2955, found = 1219.1099.

### Example 2: Synthesis of the Library of D-Compounds

The following reaction schemes were used for synthesizing the D-series of compounds.

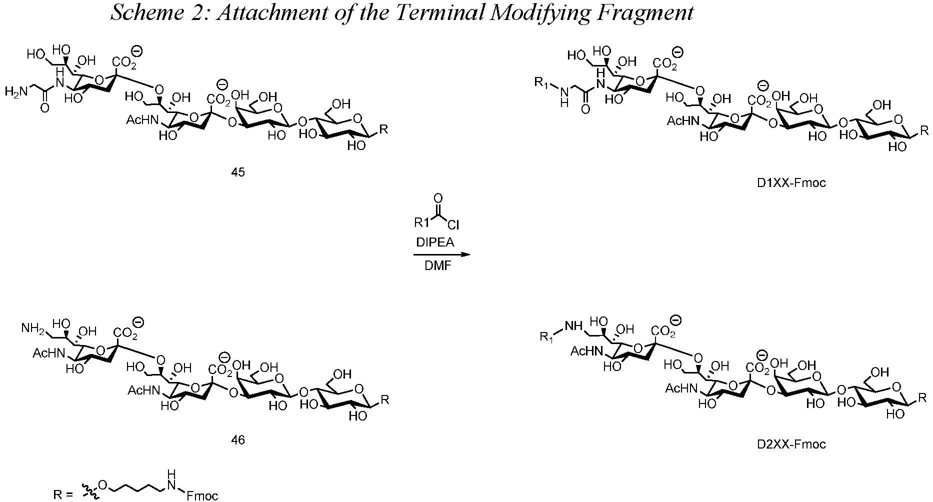

In Scheme 2, notation D1XX denotes compounds in which the N-acetyl at position 5 of the terminal sialic acid was substituted with one of the fragments described below, and notation D2XX denotes compounds in which the position 9 of the terminal sialic acid was substituted with one of the fragments described below.

### I. Synthesis of Precursors 45 and 46

Precursor compounds **45** and **46** of Scheme 2 were synthesized as follows.

### 1. Chemical synthesis of anomeric linkered lactose

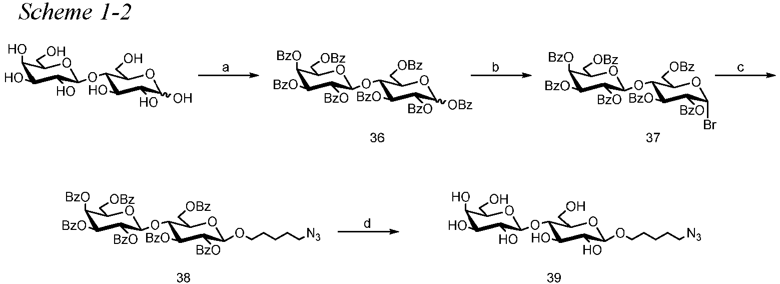

The following conditions were used in the reactions shown in in Scheme 1-2: a) BzCl, pyridine; b) HBr-AcOH / Ac₂O; c) 5-azidopentan-1ol, AgOTf; d) sodium methoxide.

More specifically, lactose (25 g, 73 mmol) was suspended in pyridine (300 mL) and the mixture was placed in an ice bath. To the chilled solution was added benzoyl chloride (100 mL, 876 mmol) dropwise over 30 mins. Upon addition of all benzoyl chloride, the reaction mixture was brought to room temperature and vigorously stilled until lactose was per-benzoylated as determined by TLC (30% EtOAc/Hex, *R_{f}*= 0.30). To an ice / water mixture was added the contents of the reaction mixture which results in the formation of an oil layer upon standing. The aqueous phase was decanted leaving the bottom oil material which was dissolved in boiling EtOH and left overnight at -20°C to yield the product **36** as white/yellow solid which was carried forward without additional purification. Per-benzylated lactose was suspended in Acetic acid (142 mL) and acetic anhydride (47 mL) in an ice bath. To this suspension was added HBr-AcOH (176 mL, 10 eq) dropwise over a 1 hr period. Upon addition of all HBr-AcOH, the reaction was brough to room temperature and vigorously stirred. The reaction progress was monitored by TLC (30% EtOAc/Hex, *R_{f} =* 0.44). The reaction was worked up by dilution in EtOAc (2 L), acid was extracted with water (5 x 250 mL), quenched with sodium bicarbonate (reaction added to beaker, and the stirred solution was added solid sodium bicarbonate), brine (2 x 200 mL), dried over MgSO₄, and reduced to yield the anomeric bromide product (3,74.9 g, 91% 2-steps) as a white solid. **37** (75 g, 66 mmol) was dissolved in toluene (660 mL) containing 20 g of 4Å MS and 5-azidopentan-1-ol (9.3 g, 72 mmol). The mixture was stirred at room temperature for 30 mins then cooled to -80°C in a dry-ice bath. To the cooled reaction was added silver trifluoromethansulfonate (21g, 82.5 mmol). The reaction was monitored by TLC (30% EtOAc/Hex, *R_{f}* = 0.34) upon complete conversion, the reaction mixture was filtered over a pad of celite, reduced, and purified by flash chromatography (30% EtOAc / Hex) to yield **38** (53.8 g, 69%) as a white solid. **38** (53.8 g, 45.5 mmol) was dissolved in MeOH (455 mL) and the pH was adjusted to 9 via the addition of a 1 M solution of sodium methoxide. The reaction was monitored by TLC (20% MeOH/DCM, *R_{f}* = 0.57), quenched with Dowex 50WX8 H⁺ resin (pH adjusted to neutral), solvent was reduced to yield a brown solid which was washed with acetone (acetone removed Benzylmethylester) to provide a light-brown solid (**39**, 17.3 g, 84%).

### 2. Chemo-enzymatic synthesis of Fmoc protected alpha2,3-sialyllactose

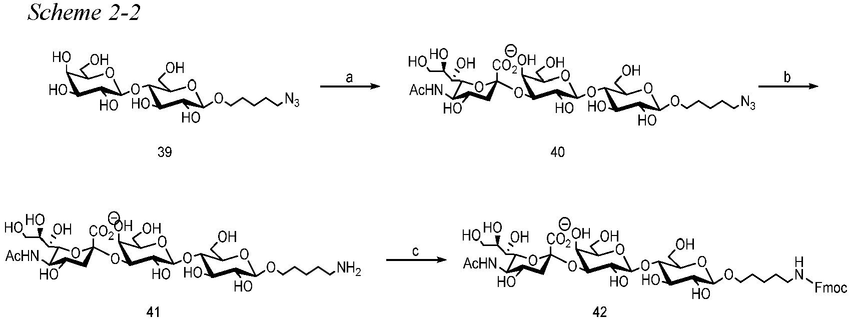

The following conditions were used in the reactions shown in Scheme 2-2: a) PmST1, NmCSS, PmPPA, Neu5Ac, CTP; b) Pd/C, H₂, c) Fmoc-OSu.

More specifically, lactose containing an anomeric linker **39** (100 mg, 0.22 mmol) was dissolved in Tris Buffer (11mL, 100 mM, pH -8.5) containing MgCl₂ (10 mM), N-acetylneuraminic acid (102 mg, 0.33 mmol) and cytidine-5'-triphosphate (159 mg, 0.33 mmol). The solution was adjusted to pH 8.5 using 5M NaOH. To the solution was added *Pm*ST1 (1 mg), *Nm*CSS (2 mg), and *Pm*PPA (2 mg). The reaction mixture was incubated at 37°C with periodic inversion of the inversion of the reaction vessel. Progress was monitored by TLC (7:3:2 EtOH, NH₄OH, H₂O) and when compete conversion was observed, the enzymes were removed by precipitation facilitated by the addition of EtOH (11 mL). The mixture was placed at -20°C for 2 hrs and the insoluble material was removed by centrifugation. The crude product was purified using Bio-Gel P2 size exclusion chromatography. Product containing fraction were concentrated and freeze-dried to yield trisaccharide **40** (142 mg, 87%) as white, fluffy solid. **40** (142 mg, 0.19 mmol) was dissolved in *t*-BuOH/H₂O (1.9 mL, 1:1 v/v) containing Pd/C (7 mg). The reaction was mixed under a hydrogen atmosphere at room temperature and the progress of the reaction was monitored by ESI-MS. The catalyst was removed using a 0.2 µm PALL Acrodisc syringe filter and the solution was freeze-dried to produce a white, fluffy solid (**41**). **41** was dissolved in DI water (9.5 mL) containing sodium bicarbonate (4.5 mmol). In a separate vial, Fmoc-OSu (128 mg, 0.38 mmol) was dissolved in ACN (9.5 mL), and to this vial was added, dropwise, **41** with vigorous mixing. The reaction progress was monitored by ESI-MS and after all starting material was converted to the desired product, the reaction was reduced *en vacuo* and purified by preparative HPLC (Agilent 1200, Eclipse XDB-C18, 21.2 x 250 mm, 7 µm. A solvent = 50 mM ammonium bicarbonate; B = ACN, λ = 262 m) with a linear gradient of 10→60% B over 30 mins, flow rate 20 mL/min. Compound containing fractions were freeze-dried to yield Fmoc-protected trisaccharide **42** (100 mg, 56% over 2-steps) as a white, fluffy solid.

### 3. Enzymatic synthesis of GD3-azido derivatives

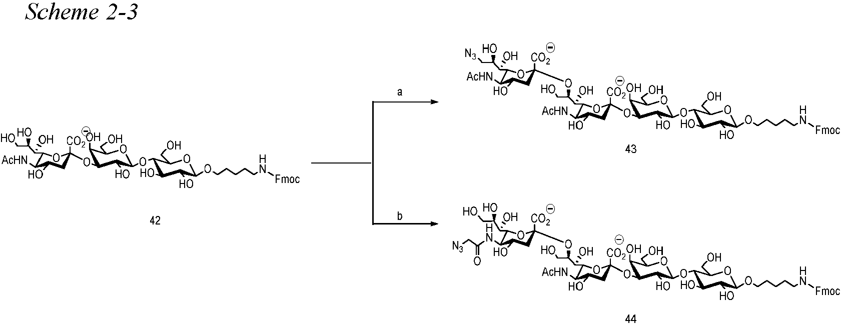

The following conditions were used in the reactions shown in Scheme 2-3: a) CSTII, CMP-Neu5Ac-9-N₃; b) CSTII, CMP-Neu5Az.

More specifically, **42** (50 mg, 53 µmol) was dissolved in Tris Buffer (5.3 mL, 100 mM, pH 8.5) containing MgCl₂ (10 mM), CMP-Neu5Ac-9-N₃ or CMP-Neu5Az (106 µmol), CSTIIΔ32^{I53S} (1 mg), and CIAP (53 µL, 1kU stock solution).⁶ The reaction was incubated at 37°C and monitored by ESI-MS. When no further product conversion was observed, the reaction mixture was filtered through a PALL Nanosep^{®} Centrifuge spin filter (3k MWCO) and the filtrate purified by Bio-Gel P2 size-exclusion chromatography. Product containing fractions were freeze-dried to provide desired tetrasaccharides **43** (40 mg, 59%) and **44** (44 mg, 65%) as a white, fluffy solid.

### 4. General protocol for α2,8-azido-sialic acid reduction

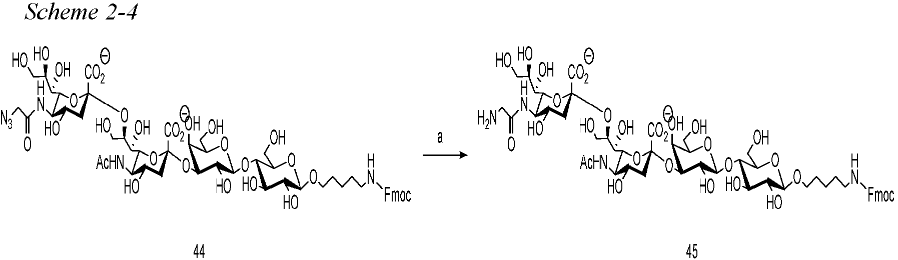

The following condition was used in the reaction shown in Scheme 2-4: Zn, AcOH, H₂O.

More specifically, **44** (30 mg, 23.5 µmol) was dissolved in H₂O (3 mL) and to this solution was added acid-treated Zn (s) (60 mg). The mixture was placed in an ice bath and to the cooled solution was added acetic acid (100 µL). The reaction was monitored by ESI-MS and when all starting material was converted to the product, the reaction was quenched with saturated ammonium bicarbonate (pH 7). The reaction was filtered with a 0.2 µm PALL Acrodisc syringe filter and purified by preparative HPLC (Agilent 1200, Eclipse XDB-C18, 21.2 x 250 mm, 7 µm. A solvent = 50 mM ammonium bicarbonate; B = ACN, λ = 262 m) with a linear gradient of 10→60% B over 30 mins, flow rate 20 mL/min. Compound containing fractions were freeze-dried to yield Fmoc-protected trisaccharide **45** (20 mg, 69%) as a white, fluffy solid.

### 5. General protocol for Fmoc deprotection

The deprotection of the D-series compounds was generally carried out according to Scheme 1-13, above.

### II. The Library of D-Compounds

Tetrasaccharide (**45** or **46,** 1mg, ~0.8 µmol) was dissolved in DMF (100 µL) containing diisopropylethylamine (DIPEA, 4 µmol). To this solution was added acyl chloride (1.6 µmol) and the reaction was mixed using a vortex for 30 mins. The reaction was monitored by ESI, and when complete conversion to the product was achieved, the product was purified by HPLC chromatography under the following conditions: Agilent 1200 HPLC, Eclipse XDB-C8, 4.6 x 250 mm, 5 µm analytical column, A solvent = 50 mM ammonium bicarbonate; B = ACN, λ = 262 m) with a linear gradient of 10→60% B over 30 mins, flow rate 1 mL/min. Fractions containing the desired product were freeze-dried to yield the desired product as a white, fluffy solid.

The following compounds of the D-series were prepared using the terminal modifying fragments listed in Table 1, above. The identities of the compounds were confirmed using ¹H-NMR spectrometry. R in he D-series compounds listed below is

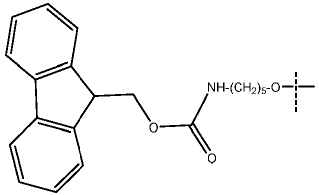

Compound **D101** (0.15 µmol) was prepared using the general procedure.

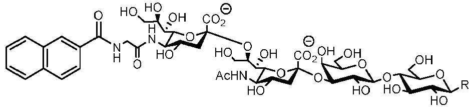

Compound **D102** (0.15 µmol) was prepared using the general procedure

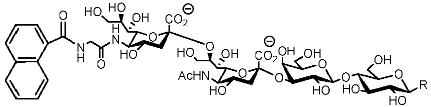

Compound **D103** (0.15 µmol) was prepared using the general procedure

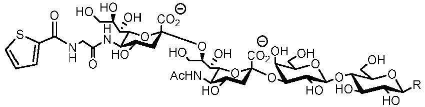

Compound **D104** (0.15 µmol) was prepared using the general procedure

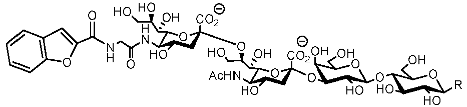

Compound **D105** (0.15 µmol) was prepared using the general procedure

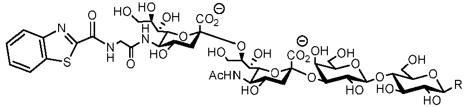

Compound **D106** (0.15 µmol) was prepared using the general procedure

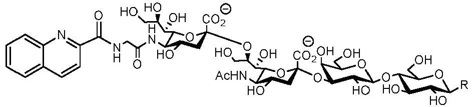

Compound **D107** (0.15 µmol) was prepared using the general procedure

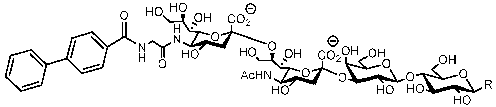

Compound **D108** (0.15 µmol) was prepared using the general procedure

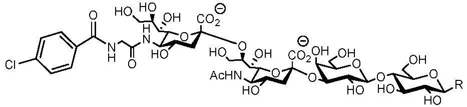

Compound **D109** (0.15 µmol) was prepared using the general procedure

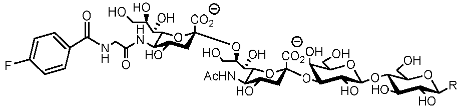

Compound D110 (0.15 µmol) was prepared using the general procedure

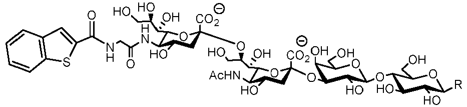

Compound **D111** (0.15 µmol) was prepared using the general procedure

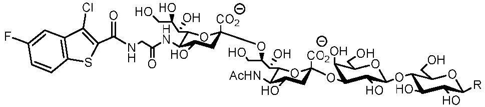

Compound **D112** (0.15 µmol) was prepared using the general procedure

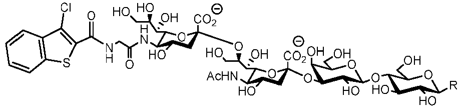

Compound **D113** (0.15 µmol) was prepared using the general procedure

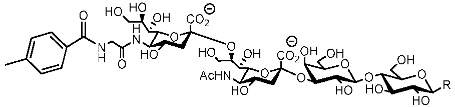

Compound **D114** (0.15 µmol) was prepared using the general procedure

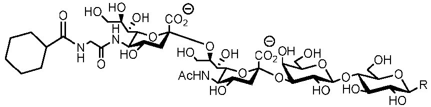

Compound **D115** (0.15 µmol) was prepared using the general procedure

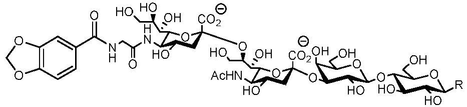

Compound **D116** (0.15 µmol) was prepared using the general procedure

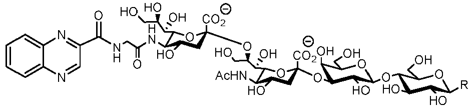

Compound **D117** (0.15 µmol) was prepared using the general procedure

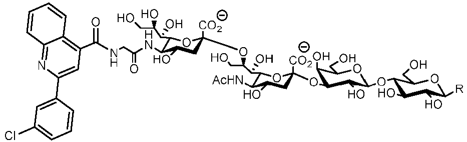

Compound **D118** (0.15 µmol) was prepared using the general procedure

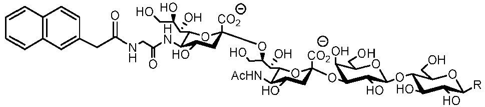

Compound **D119** (0.15 µmol) was prepared using the general procedure

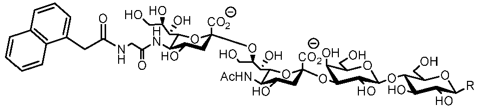

Compound **D120** (0.15 µmol) was prepared using the general procedure

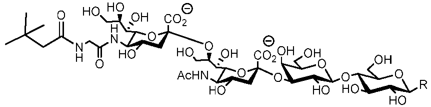

Compound **D121** (0.15 µmol) was prepared using the general procedure

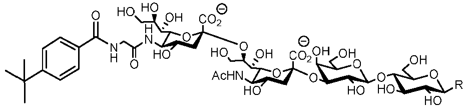

Compound **D122** (0.15 µmol) was prepared using the general procedure

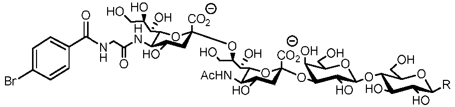

Compound **D123** (0.15 µmol) was prepared using the general procedure

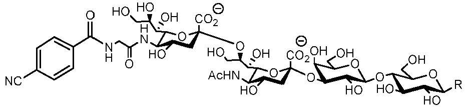

Compound **D124** (0.15 µmol) was prepared using the general procedure

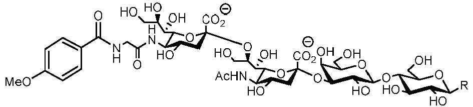

Compound **D125** (0.15 µmol) was prepared using the general procedure

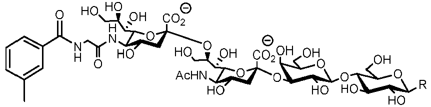

Compound **D126** (0.15 µmol) was prepared using the general procedure

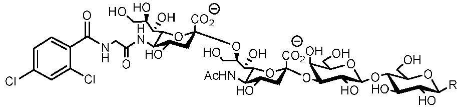

Compound **D127** (0.15 µmol) was prepared using the general procedure

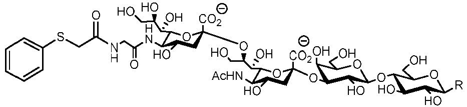

Compound **D128** (0.15 µmol) was prepared using the general procedure

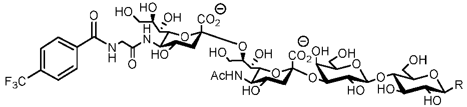

Compound **D129** (0.15 µmol) was prepared using the general procedure

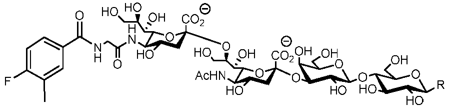

Compound **D130** (0.15 µmol) was prepared using the general procedure

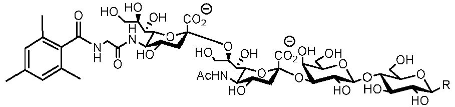

Compound **D131** (0.15 µmol) was prepared using the general procedure

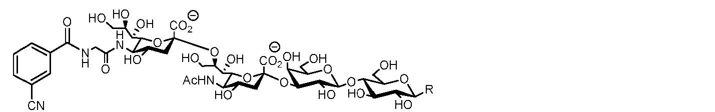

Compound **D201** (0.15 µmol) was prepared using the general procedure

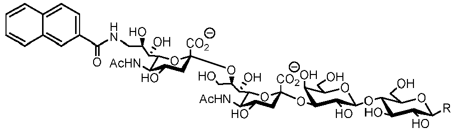

Compound **D202** (0.15 µmol) was prepared using the general procedure

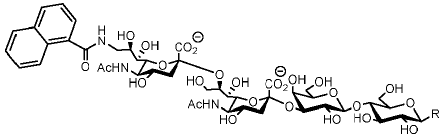

Compound **D203** (0.15 µmol) was prepared using the general procedure

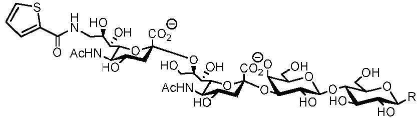

Compound **D204** (0.15 µmol) was prepared using the general procedure

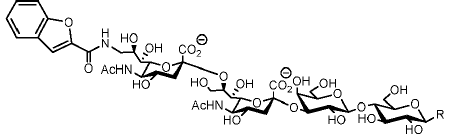

Compound **D205** (0.15 µmol) was prepared using the general procedure

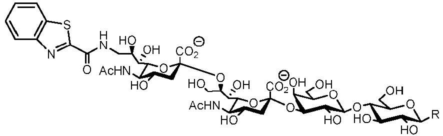

Compound **D206** (0.15 µmol) was prepared using the general procedure

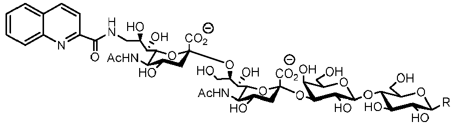

Compound **D207** (0.15 µmol) was prepared using the general procedure

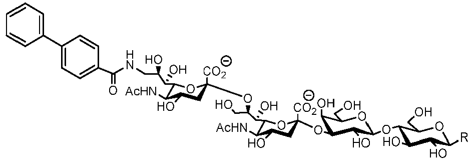

Compound **D208** (0.15 µmol) was prepared using the general procedure

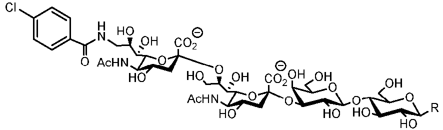

Compound **D209** (0.15 µmol) was prepared using the general procedure

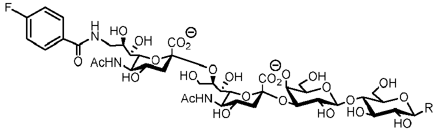

Compound **D210** (0.15 µmol) was prepared using the general procedure

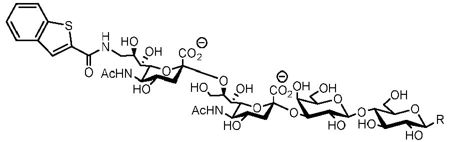

Compound **D211** (0.15 µmol) was prepared using the general procedure

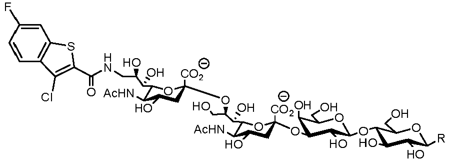

Compound **D212** (0.15 µmol) was prepared using the general procedure

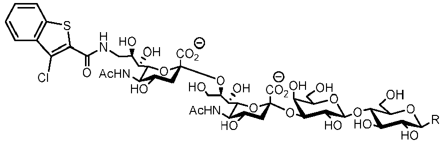

Compound **D213** (0.15 µmol) was prepared using the general procedure

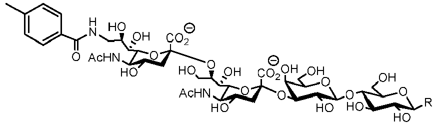

Compound **D214** (0.15 µmol) was prepared using the general procedure

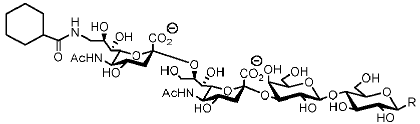

Compound **D215** (0.15 µmol) was prepared using the general procedure

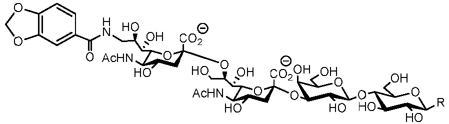

Compound **D216** (0.15 µmol) was prepared using the general procedure

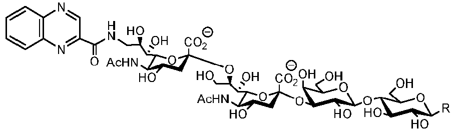

Compound **D217** (0.15 µmol) was prepared using the general procedure

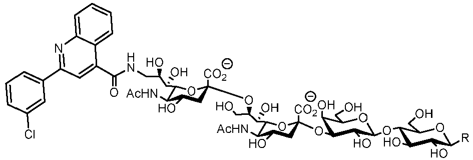

Compound **D218** (0.15 µmol) was prepared using the general procedure

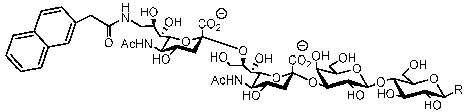

Compound **D219** (0.15 µmol) was prepared using the general procedure

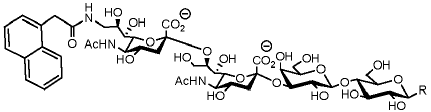

Compound **D220** (0.15 µmol) was prepared using the general procedure

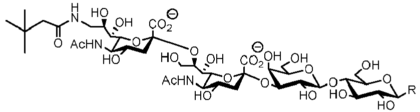

Compound **D221** (0.15 µmol) was prepared using the general procedure

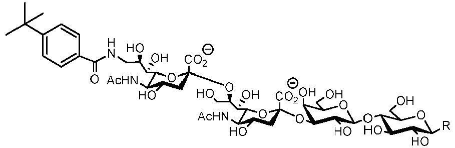

Compound **D222** (0.15 µmol) was prepared using the general procedure

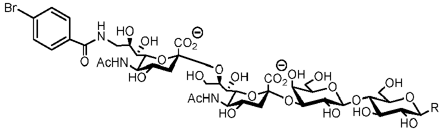

Compound **D223** (0.15 µmol) was prepared using the general procedure

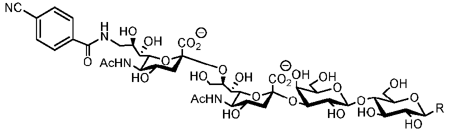

Compound **D224** (0.15 µmol) was prepared using the general procedure

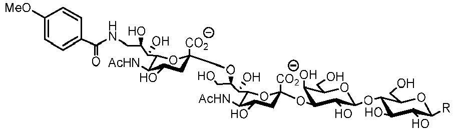

Compound **D225** (0.15 µmol) was prepared using the general procedure

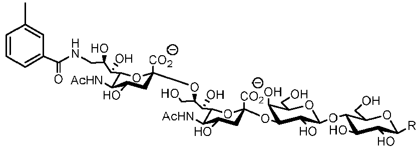

Compound **D226** (0.15 µmol) was prepared using the general procedure

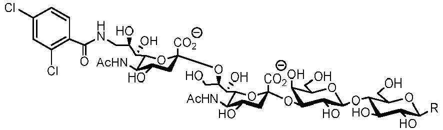

Compound **D227** (0.15 µmol) was prepared using the general procedure

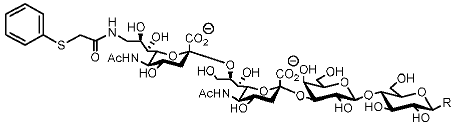

Compound **D228** (0.15 µmol) was prepared using the general procedure

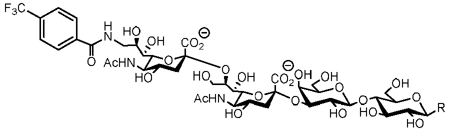

Compound **D229** (0.15 µmol) was prepared using the general procedure

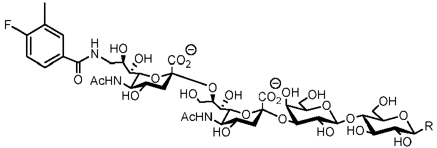

Compound **D230** (0.15 µmol) was prepared using the general procedure

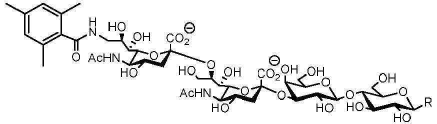

Compound **D231** (0.15 µmol) was prepared using the general procedure

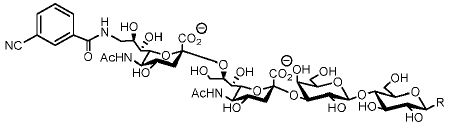

### Example 3: Conjugation of Polymers and Glycans

The following synthetic schemes are used to conjugate glycans and polymers. In these schemes, the glycan is partially represented using only the carbohydrate unit directly bonded to the linker group. Additional manufacturing protocols are provided in Example 8.

### Synthetic Example 3-1: Preparation of a Glycan Bearing a Click Reagent

### Synthetic Scheme 3-1

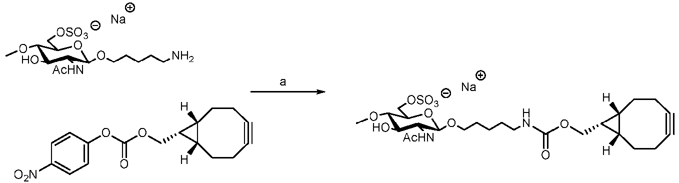

### Synthetic Scheme 3-2

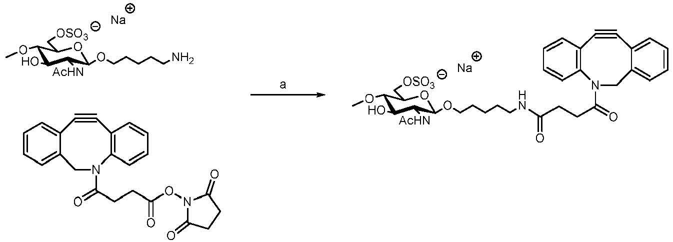

### Synthetic Scheme 3-3

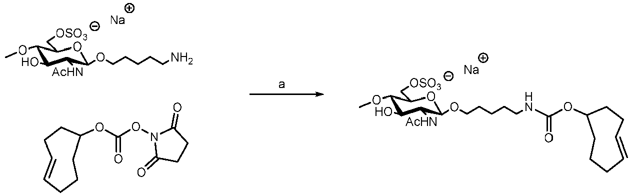

### Synthetic Scheme 3-4

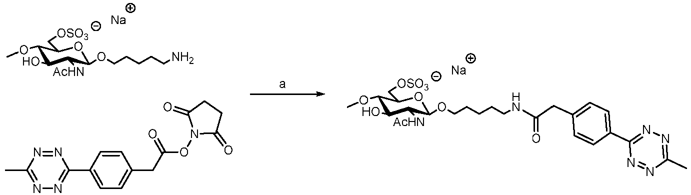

### Synthetic Scheme 3-5

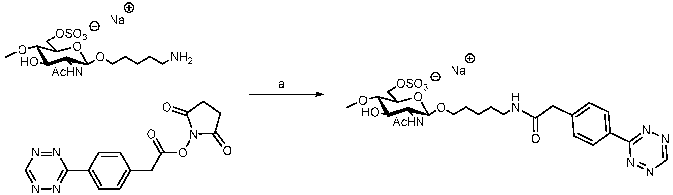

### Synthetic Scheme 3-6

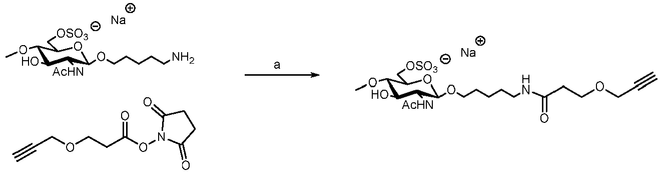

For each of the Synthetic Schemes 3-1 through 3-6, the anomeric linker is modified by a corresponding click reagent using the following general protocol. Amine terminating glycan (1 eq.) and click reagent (5 eq.) are dissolved in DMF such that the final glycan concentration is 10 mM. To this solution is added diisopropylethylamine (DIPEA, 5 eq) and the reaction is incubated at 37°C until no further starting material is observed by ESI-MS. The reaction is purified by P-2 size exclusion chromatography eluting with 0.1 M ammonium bicarbonate. The product containing fractions are pooled and the solvent is removed by lyophilization.

A skilled person would understand that many other click chemistry reagents are commercially available and can be employed depending on the need, availability, and the desirability of the final product. For example, with respect to Synthetic Scheme 3-1, any one of the following commercially avaibale (from such vendors as Broadpharma, Sigma-Millipore, or Fischer Scientific) click reagents can be used:

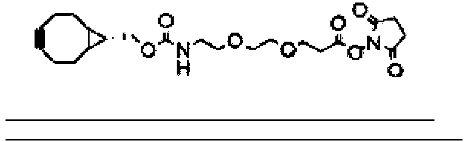

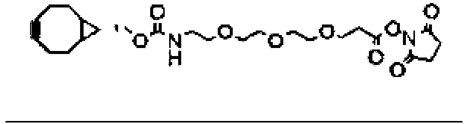

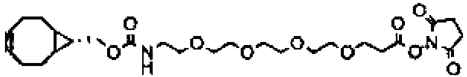

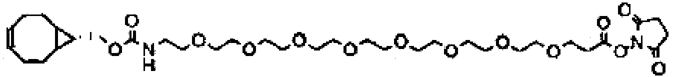

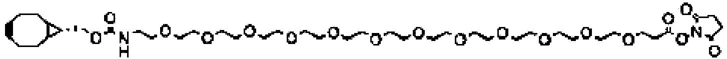

### Synthetic Example 3-2: Production of Exemplary Nanoparticles using polymers with DBCO.

Nanoparticles are prepared by the emulsion method. A representative example of nanoparticle production includes the following. PLGA (10k)-PEG(5k)-COOH and PLGA (10k)-PEG(5k)-DBCO are weighed at a weight ratio of 3:1 and are dissolved in mixture of organic solvents. The concentration of the polymer is 37mg/mL in organic solvent (59.5% ethyl acetate and 40.5% benzyl alcohol). 10mL of the polymer solution is added to 40mL of an aqueous phase consisting of 0 or 0.1% Tween 80 in water and homogenized using a Rotastator to form a coarse emulsion. The coarse emulsion is further homogenized to a fine nanoemulsion using a microfluidizer with 3 passes. The nanoemulsion is then quenched by addition to 450mL of cold water to harden the nanoparticles. The quenched nanoparticle solution is then concentrated and washed by tangential flow filtration to remove the organic solvent. The concentration of polymeric nanoparticles is determined by evaporating water from a known volume of nanoparticle solution. The nanoparticle solution is then prepared for conjugation to the oligoglycan ligands.

### Synthetic Example 3-3: Ligand-Azide Conjugation to Core DBCO Nanoparticle Surface via SPAAC Chemistry

Purified N3-ligand prepared as described above is conjugated to the surface of purified PLGA-DBCO nanoparticles via a SPAAC copper-free click chemistry protocol. Briefly, the nanoparticles with DBCO are incubated with the ligand-azide at 4 °C or room temperature or at 37°C overnight. Upon completion of the conjugation reaction, the nanoparticles are washed using TFF to remove unreacted components, and the buffer is replaced with an appropriate isotonic solution such as 10% Sucrose. The nanoparticle solution is then sterile filtered via a 0.2µm filter.

### Synthetic Example 3-4: Additional Production of Exemplary Nanoparticles by Preconjugation of ligand-azide to DBCO-polymer

Nanoparticles are prepared by the emulsion method. A representative example of nanoparticle production is described below. 10 mg of the ligand-azide is dissolved in 0.6mL of DMSO or DMF or Acetonitrile or some appropriare solvent. The dissolved ligand solution is added to 15 mg of PLGA (10k)-PEG (5k)-DBCO (dissolved in an organic solvent mixture of ethyl acetate:benzyl alcohol). The mixture is stirred and mixed overnight to allow for conjugation of the ligand-azide to the PLGA-PEG-DBCO via the azide-DBCO click chemistry coupling. After mixing overnight, 285 mg PLGA (10k)-PEG(5k)-COOH is added to this reaction mixture and the solution was mixed/stirred until it was visually clear.

The final volume of the organic solvents is 3.03 mL with 2.43 mL of ethyl acetate:benzyl alcohol at a volume ratio of 60:40, and 0.6mL of DMSO. In order to introduce a fluorescence moiety into the nanoparticles, a polymer, fluorescein-PLGA can be used. The total fraction of the fluorescein polymer in the total polymer used for preparing the nanoparticles was 1% by weight, i.e., 3 mg of PLGA-fluorescein was also added to the polymer solution.

This polymer solution is added to 27 mL of cold water (saturated with ethyl acetate) and homogenized using a IKA T-18 Rotastator to form a coarse emulsion. The coarse emulsion is further homogenized to a fine nanoemulsion using a microfluidizer (Microfluidics LM10) with 3 passes. The nanoemulsion is then quenched by addition to 270 mL of cold water to harden the nanoparticles. The quenched nanoparticle solution is then concentrated and washed using cold water by tangential flow filtration (KR2i TFF, Repligen) to remove the organic solvents and unreacted ligand-azide. The concentration of polymeric nanoparticles is determined by evaporating water from a known volume of nanoparticle solution. Sucrose is added to the nanoparticle solution at 10% wt/wt and filtered using a 0.2 µm Millipore syringe filter. The nanoparticle solution is then frozen at -20°C. The size of the nanoparticles is measured using Dynamic Light Scattering using a Malvern Zetasizer. Dynamic light scattering techniques use the constant random thermal motion of particles and molecules called Brownian motion to measure the size. The particles diffuse at a speed related to their size, smaller particles diffusing faster than larger particles. The diffusion speed is measured from the speckle pattern produced by illuminating the particles with a laser. The fluctuations in the scattering intensity at a specific angle is detected using a sensitive photodiode detector. The intensity changes are analysed with a digital autocorrelator to generate a correlation function. This curve is analysed to give the size and the size distribution of the particles. The nanoparticles are diluted to a concentration of approximately 0.1-1.0 mg/mL using clean water and measured in the Zetasizer. Each value generated is an average of 3 readings.

### Synthetic Example 3-5: Conjugation of glycans to polymers by amide coupling

### Synthetic Scheme 3-7

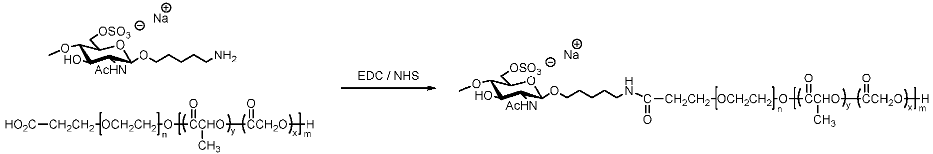

Carboxylic Acid terminating PLGA or PLGA-PEG is dissolved in DCM to a final concentration of 5 mM. The solution is mixed at room temperature, and to this sample is added EDC and NHS or Sulfo-NHS to provide a final concentration of each reagent to 25 mM. The solution is stirred at room temperature for one hour to yield to desired NHS or sulfo-NHS activated ester. The activated ester is precipitated by addition of MeOH (10x reaction volume) and collected by centrifugation (2,000 g for 10 mins). The activated PLGA-PEG-NHS pellet is redissolved in DCM and precipitated in MeOH three total times to ensure complete removal for residual EDC and NHS or sulfo-NHS. Amide formation is facilitated by dissolving the amine containing glycan and the activated PLGA in a mixture of organic solvents to facilitate the reaction between the amine component of the glycan-amine and the NHS group on PLGA-PEG-NHS. The choice of the organic solvents can be optimized for each glycan-amine and PLGA-PEG-NHS system. For example, the glycan is typically dissolved in DMSO or DMF with the addition of small amount of water if necessary. The PLGA-PEG-NHS block copolymer is dissolved in DMSO or DMF. The glycan and PLGA-PEG-NHS solutions should be clear and then mixed to initiate the reaction. N, N-Diisopropylethylamine (DIPEA) is added to this reaction mixture to enable the coupling of PLGA-PEG and glycans. The reaction is carried out for at least 4 hours or more. After the reaction has proceeded to completion, the organic solvent mixture is emulsified into an aqueous phase to yield the nanoemulsion. Unreacted components of the reaction are expected to be removed during the fabrication of the nanoparticles. The aqueous phase used during the formation of the nanoemlusions can be slightly basic with a pH or > 7.2 to assist in hydrolysis of unreacted NHS groups to COOH.

### Synthetic Example 3-6: Conjugation of glycans to polymers by azide/alkyne click coupling

### Synthetic Scheme 3-8

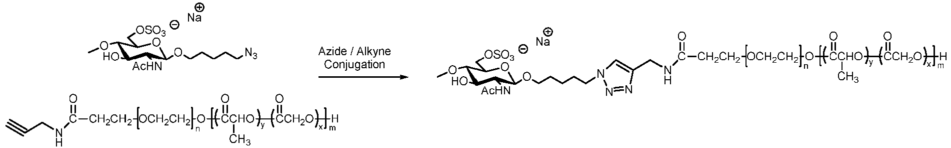

Azide containing glycan (> 1.2 eq) and alkyne containing polymer (1 eq) are mixed in a solvent system containing DMSO or DMF, benzyl alcohol and ethyl acetate. The reaction is carried out for at least 4 hours until completion. Unreacted alkyne groups in the polymer can be capped by addition of excess amounts of azide containing molecules. After the reaction has proceeded to completion, the organic solvent mixture is emulsified into an aqueous phase to yield the nanoemulsion. Unreacted components of the reaction are expected to be removed during the fabrication of the nanoparticles.

### Synthetic Example 3-7: Conjugation of glycans to polymers by Copper-free click coupling

### Synthetic Scheme 3-9A

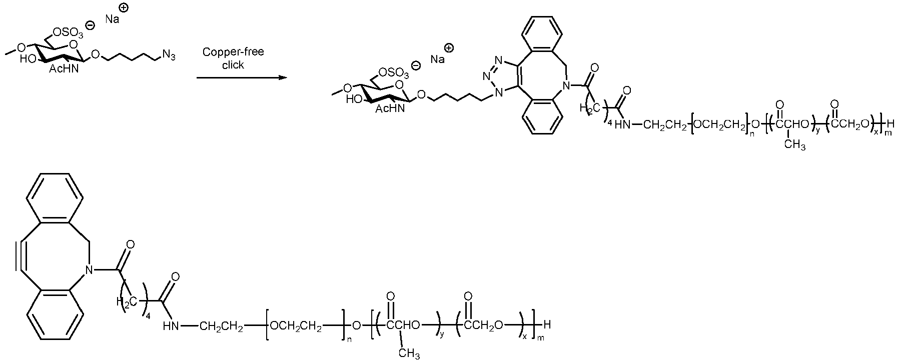

### Synthetic Scheme 3-9B

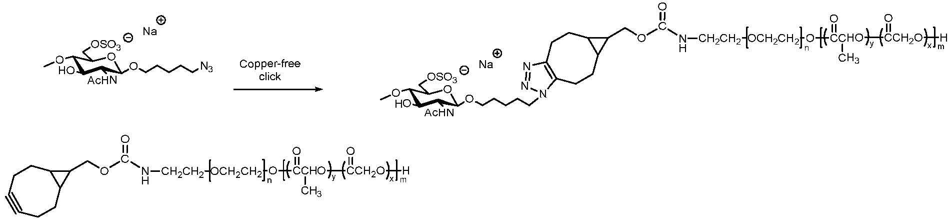

### Synthetic Scheme 3-9C

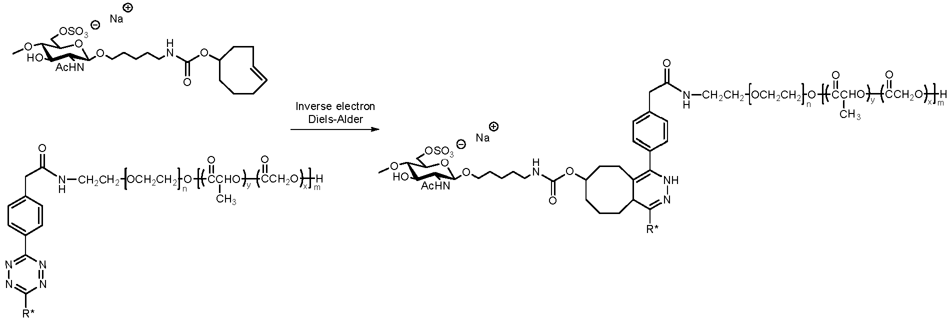

Azide containing glycan (1 eq) and cyclooctyne containing polymer (1 eq) are mixed in a solvent system containing DMSO or DMF, benzyl alcohol and ethyl acetate. The reaction is carried out for at least 4 hours until completion. Unreacted alkyne groups in the polymer can be capped by addition of excess amounts of azide containing molecules. After the reaction has proceeded to completion, the organic solvent mixture is emulsified into an aqueous phase to yield the nanoemulsion. Unreacted components of the reaction are expected to be removed during the fabrication of the nanoparticles.

### Synthetic Scheme 3-9D

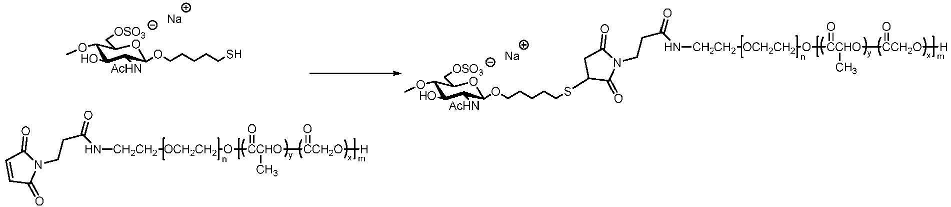

Thiol containing glycan (1 eq) and maleimide containing polymer (1 eq) are mixed in a solvent system containing DMSO or DMF, benzyl alcohol and ethyl acetate. The reaction is carried out for at least 4 hours until completion. Unreacted maleimide groups in the polymer can be capped by addition of excess amounts of thiol containing molecules such as cysteine. After the reaction has proceeded to completion, the organic solvent mixture is emulsified into an aqueous phase to yield the nanoemulsion. Unreacted components of the reaction are expected to be removed during the fabrication of the nanoparticles.

The above processes for fabricating nanoparticles can be applied to various combinations of coupling chemistries including maleimide-thiol or cysteine, NHS and amine, click chemistries etc.

### Example 4: Microarray Printing and Screening

The compounds described herein were initially tested for binding to Siglec targets using microarray printing techniques described below.

All compounds were printed on NHS-ester activated glass slides (NEXTERION^{®} Slide H, Schott Inc.) using a Scienion sciFLEXARRAYER S3 non-contact microarray equipped with a Scienion PDC80 nozzle (Scienion Inc.). Individual samples were dissolved in sodium phosphate buffer (50 µL, 0.225 M, pH 8.5) at a concentration of 100 µM and were printed in replicates of 10 with spot volume ~ 400 pL, at 20 °C and 50% humidity. Each slide has 24 subarrays in a 3x8 layout. After printing, slides were incubated in a humidity chamber for 8 h and then blocked for 30 min with a 5mM ethanolamine in a Tris buffer (pH 9.0, 50 mM) at 40 °C. Blocked slides were rinsed with DI water, spun dry, and kept in a desiccator at room temperature for future use.

Screening was performed by incubating the slides with a protein solution for a certain amount of time followed by washing and drying. The buffers used in screening were TSM buffer (TSM, 20 mM Tris·Cl, pH 7.4, 150 mM NaCl, 2 mM CaCl₂, and 2 mM MgCl₂), TSM binding buffer (TSMBB, TSM buffer with 0.05% Tween-20 and 1% BSA) and TSM washing buffer (TSMWB, TSM buffer with 0.05% Tween-20). A typical washing procedure included sequentially dipping the glass slide in TSM wash buffer (2 min, containing 0.05 % Tween 20), TSM buffer (2 min) and, water (2 x 2 min), followed by spun dry.

For the screening of the Fc tagged Siglec -2, 3, -5, -6, -7 and -9, the slides were incubated with Siglec-2 (Fc tagged, R&D system cat. # 1968-SL-050, 1 µg/mL in TSMBB), Siglec-3 (Fc tagged, R&D system cat. # 1137-SL-050, 1 µg/mL in TSMBB), Siglec-5 (Fc tagged, R&D system cat. # 1072-SL-050, 20 µg/mL in TSMBB), Siglec-6 (Fc tagged, R&D system cat. # 2859-SL-050, 1 µg/mL in TSMBB), Siglec 7 (Fc tagged, R&D system cat. # 1138-SL-050, 20, 10, and 5lµg/mL in TSMBB) or Siglec 9 (Fc tagged, R&D system, 10, 3, 1 µg/mL in TSMBB) for 1 h, followed by washing and incubating with a solution of goat anti-human IgG antibody (Alexa Fluor 647 conjugated, Jackson ImmunoResearch,10 µg/mL) for 30 min. For the screening of strep-tagged enterovirus protein B3/1013, A2/2018, B2/039, A4/P4, and hemagglutinins of H5VN, H3N8, the slides were incubated with the protein (50 µg/mL in TSMBB) for 1 h, followed by washing and incubating with a solution of anti-Strep tag antibody (StrepMAB-Classic Oyster 645, IBA Lifesciences 2-1555-050, 10 µg/mL) for 30 min. For the screening of MERS spike protein, the slides were incubated with a pre-mixed mixture of MERS spike protein (Fc tagged, 50 µg/mL) and pAb-LS nanoparticle (50 µg/mL) for 1h, followed by washing and incubating with a solution of goat anti-human IgG antibody (Alexa Fluor 647 conjugated, Jackson ImmunoResearch,10 µg/mL) for 30 min. After washing and drying, the slides were scanned using a GenePix 4000B microarray scanner (Molecular Devices) at the appropriate excitation wavelength with a resolution of 5 µm. Various gains and PMT values were employed for the scanning to ensure all signals were within the linear range of the scanner's detector and there were no saturation of signals. The images were analyzed using GenePix Pro 7 software (version 7.2.29.2, Molecular Devices). The data was analyzed with an Excel macro (http://zenodo.org/record/5146251). The highest and the lowest value of the total fluorescence intensity of the replicates were removed and the remaining values were used to calculate the mean value and standard deviation.

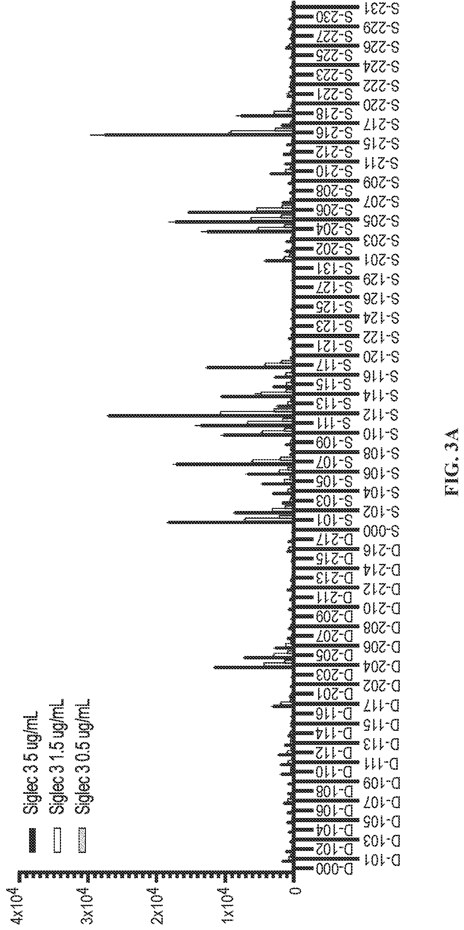

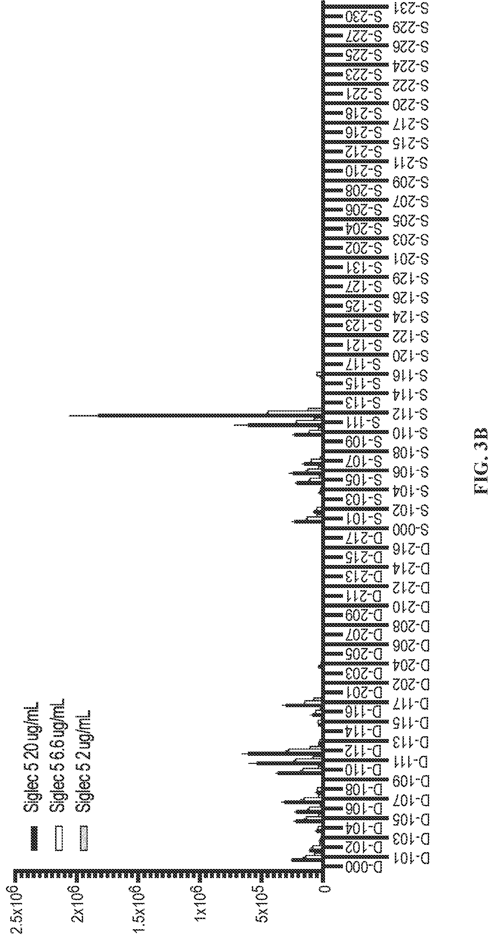

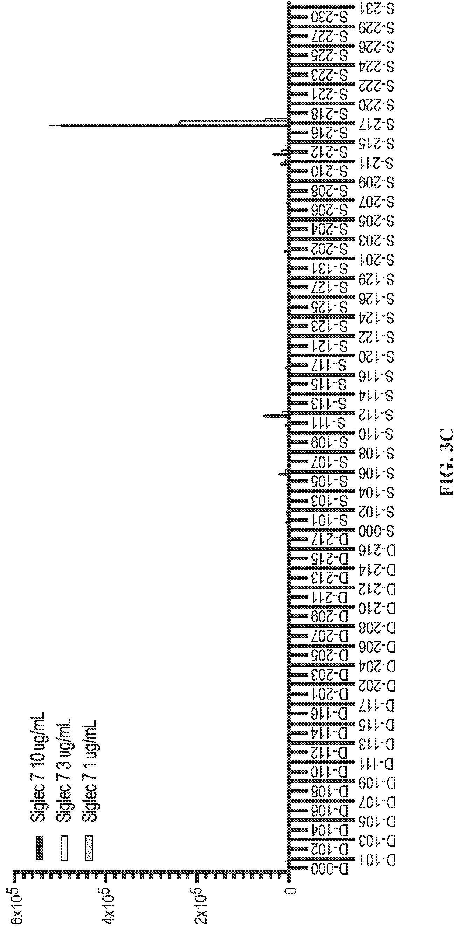

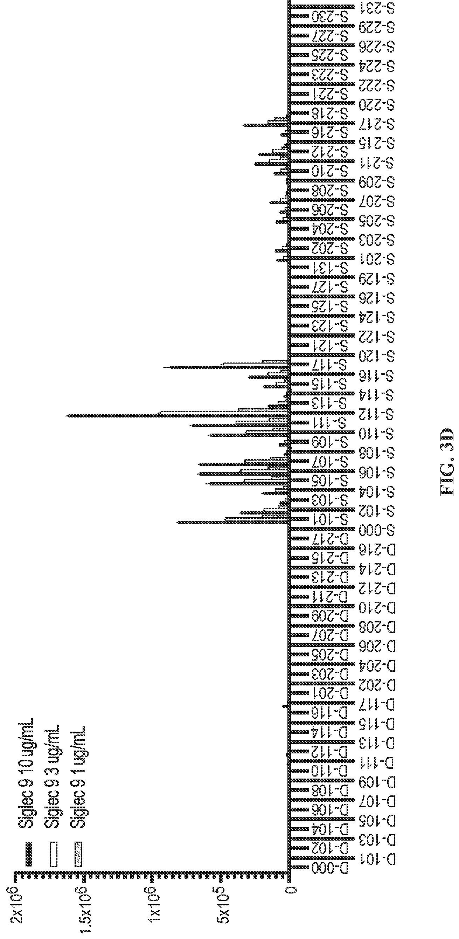

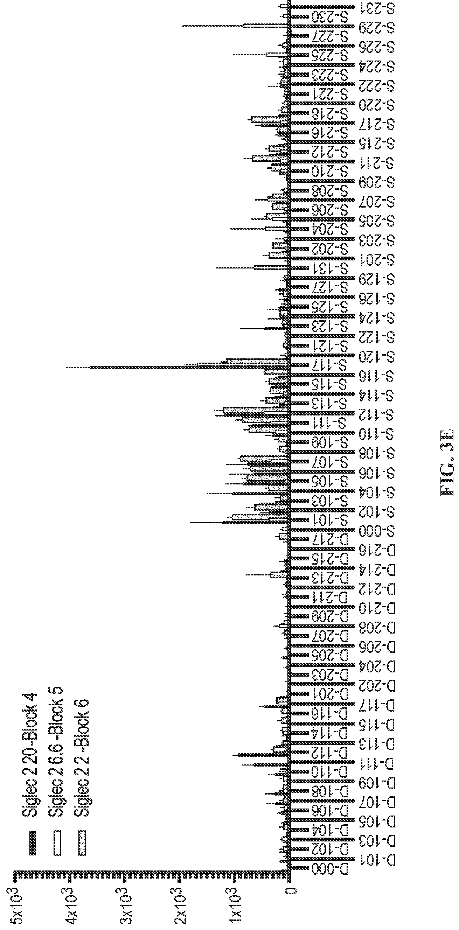

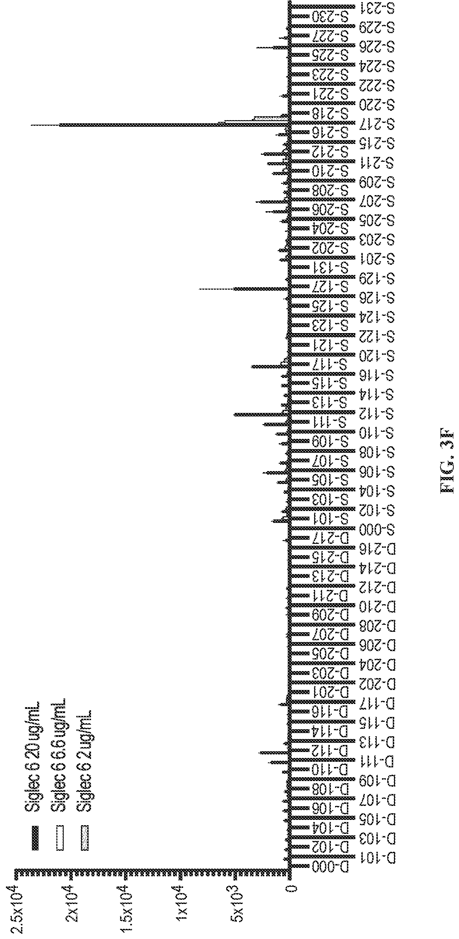

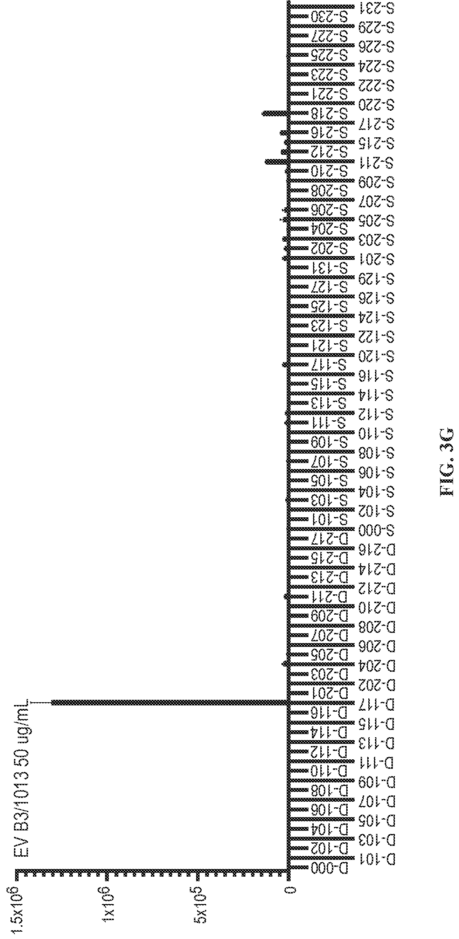

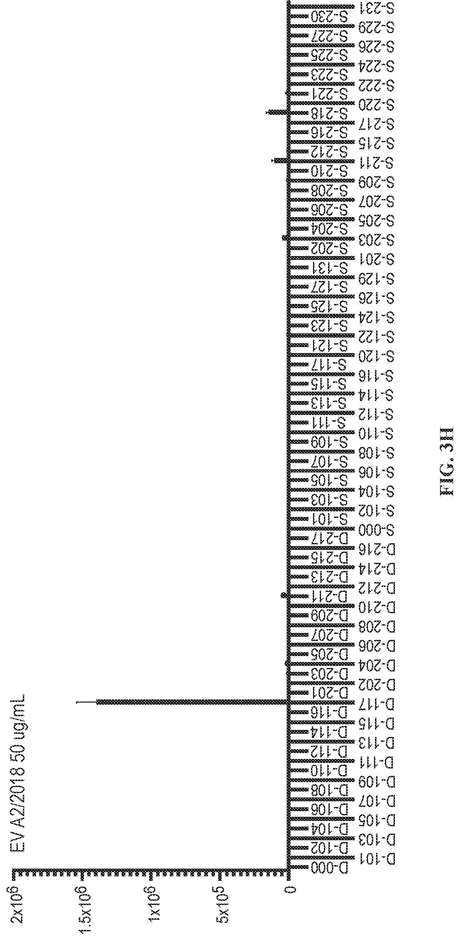

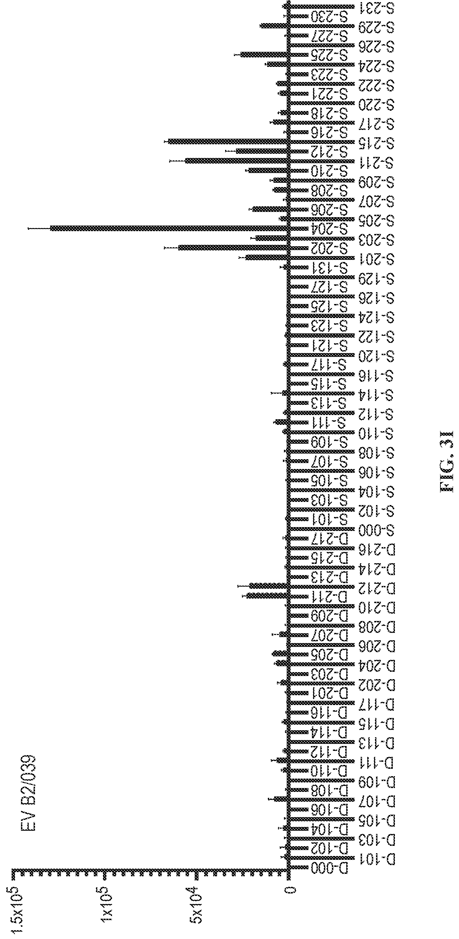

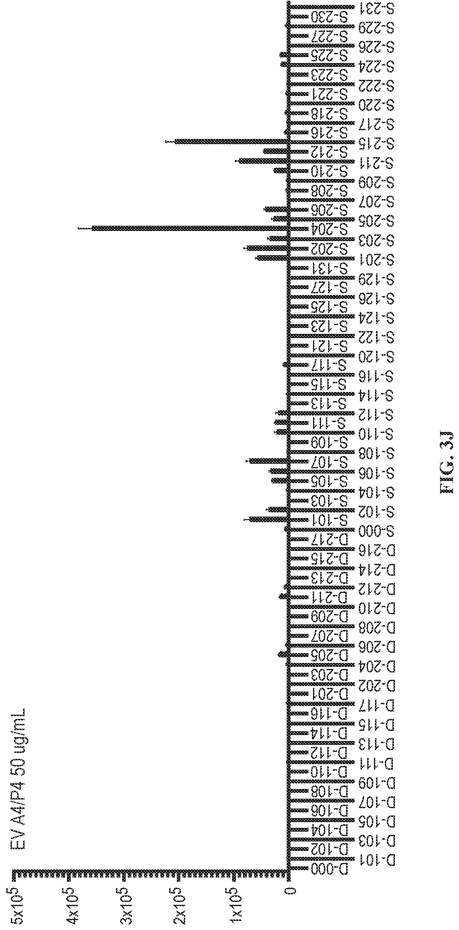

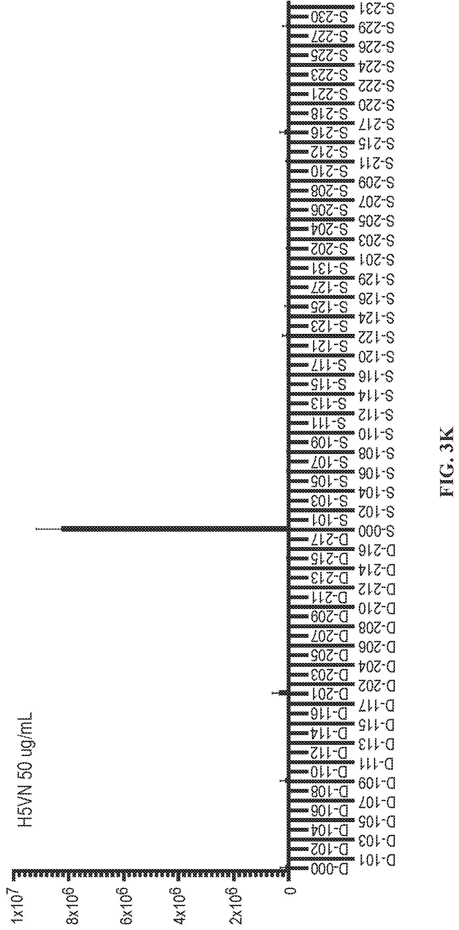

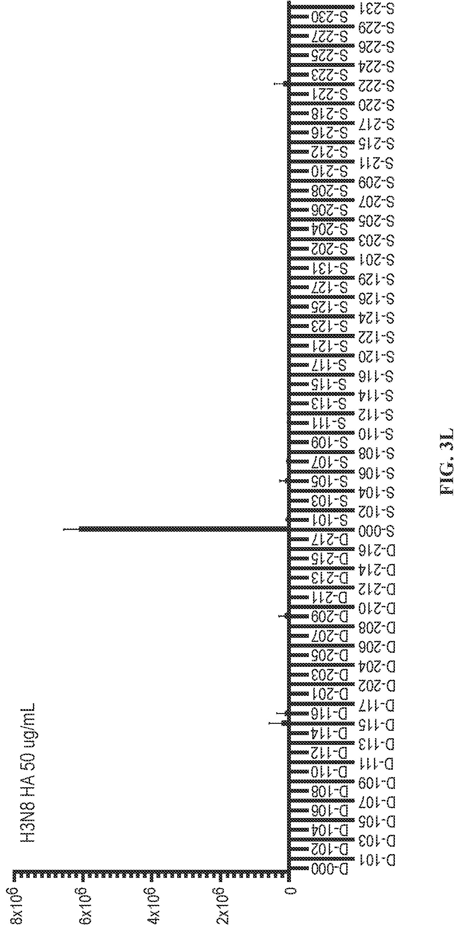

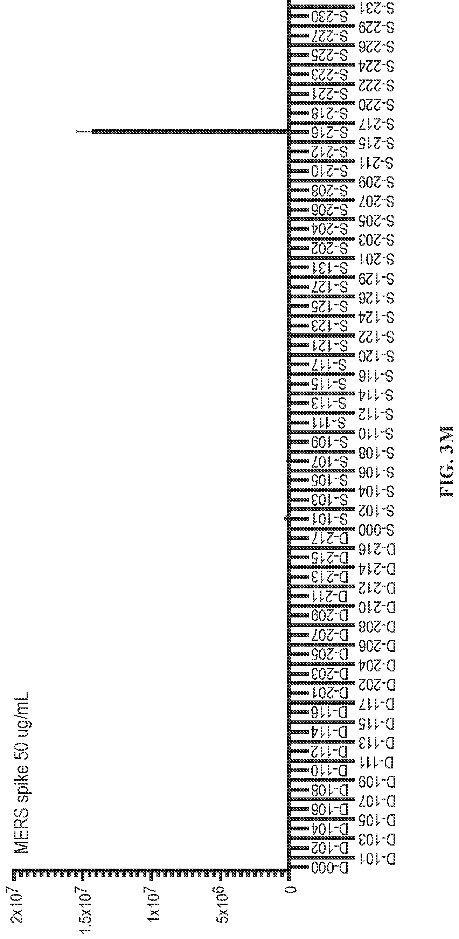

The results are shown in FIGs. 3A through 3M (vertical axis corresponds to fluorescent intensity measured in relative fluorescent unit (RFU)).

### Example 5: Surface Plasmon Resonance Analysis of Binding to Siglecs

The K_{d} of binding of the compounds of the present invention is determined using Surface Plasmon Resonance (SPR) technique.

### Surface / Sample Conjugation:

All analyses are performed on a GE Biacore T100 machine. To a Series S CM5 chip (Cytiva No. 29149603) is coated streptavidin (2,000 response units, Thermo Fisher Scientific No.434301) using standard EDC/NHS amide conjugation chemistry (Flow cell 1, 2, 3, and 4). All flow cells are subsequently blocked using ethanolamine and the chip surface is stabilized by flowing (10 µL/min) a degassed solution of PBS-P (10 mM Na₂HPO₄, 1.8 mM KH₂PO₄, 137 mM NaCl, 2.7 mM KCl, and 0.5% surfactant P20 (Cytiva No. BR100054), pH 7.4) overnight.

To the stabilized baseline is introduced biotinylated compounds dissolved in DI water to a concentration of 50 µg/mL (Volume total = 200 µL). Using the T100 software in manual mode, the 50 µg/mL solution of biotinylated disialoside is introduced to flow cell 2 at a flow rate of 30 µL/min for 90 seconds followed by PBS-P for 90 seconds at a flow rate of 30 µL/min. This process is repeated three times, after which, no additional biotin conjugation is observed.

### Siglec-3 Screening

Lyophylized hFc-Siglec-3 (R&D Systems No. 1137-SL-050) is reconstituted in PBS-P buffer to a concentration of 200 µg/mL and allowed to equilibrate on ice for 30 mins prior to analysis. Binding assays are set up to measure the interaction between siglec-3 and biotinylated sialosides by subtracting the response units measured from flow cell 2 and 1. This analysis is accomplished by introducing siglec-3 at three different concentrations: 200 µg/mL, 100 µg/mL, and 50 µg/mL using the following parameters:
a. Flow rate = 30 µL/min; b. Contact time = 60 s; c. Dissociation time 90 s; d. Stabilization time = 30 s.

### Siglec-5 Screening

Lyophylized hFc-Siglec-5 (R&D Systems No. 1072-SL-050) is reconstituted in PBS-P buffer to a concentration of 200 µg/mL and allowed to equilibrate on ice for 30 mins prior to analysis. Binding assays are set up to measure the interaction between siglec-3 and biotinylated sialosides by subtracting the response units measured from flow cell 2 and 1. This analysis is accomplished by introducing siglec-5 at three different concentrations: 200 µg/mL, 100 µg/mL, and 50 µg/mL using the following parameters:
a. Flow rate = 30 µL/min; b. Contact time = 60 s; c. Dissociation time 90 s; d. Stabilization time = 30 s.

### Siglec-7 Screening

Lyophylized hFc-Siglec-7 (R&D Systems No. 1138-SL-050) is reconstituted in PBS-P buffer to a concentration of 200 µg/mL and allowed to equilibrate on ice for 30 mins prior to analysis. Binding assays are set up to measure the interaction between siglec-7 and biotinylated sialosides by subtracting the response units measured from flow cell 2 and 1. This analysis is accomplished by introducing siglec-7 at three different concentrations: 200 µg/mL, 100 µg/mL, and 50 µg/mL using the following parameters:
a. Flow rate = 30 µL/min; b. Contact time = 60 s; c. Dissociation time 90 s; d. Stabilization time = 30 s.

### Siglec-9 Screening

Lyophylized hFc-Siglec-9 (R&D Systems No. 1139-SL-050) is reconstituted in PBS-P buffer to a concentration of 200 µg/mL and allowed to equilibrate on ice for 30 mins prior to analysis. Binding assays are set up to measure the interaction between siglec-9 and biotinylated sialosides by subtracting the response units measured from flow cell 2 and 1. This analysis is accomplished by introducing siglec-9 at a concentration of 100 µg/mL, 50 µg/mL, and 25 µg/mL using the following parameters:
a. Flow rate = 30 µL/min; b. Contact time = 60 s; c. Dissociation time 90 s; d. Stabilization time = 30 s.

### Siglec-E Screening

Lyophylized mFc-Siglec-E (R&D Systems No. 5806-SL-050) is reconstituted in PBS-P buffer to a concentration of 200 µg/mL and allowed to equilibrate on ice for 30 mins prior to analysis. Parallel binding assays are set up to measure the interaction between Siglec-E and biotinylated sialosides by subtracting the response units measured from flow cell 2 and 1. This analysis is accomplished by introducing Siglec-E at a concentration of 100 µg/mL, 50 µg/mL, and 25µg/mL using the following parameters: a. Flow rate = 30 µL/min; b. Contact time = 60 s; c. Dissociation time 90 s; d. Stabilization time = 30 s.

### Example 6A: S-Compounds Demonstrate Binding Selectivity to Siglec-9

The K_{d} values of binding of S-compounds to Siglec-3, Siglec-7, Siglec-9, and Siglec-11 were measured using biolayer interferometry (BLI) analysis.

Briefly, all analyses were performed on an Octet RED384 instrument. To high precision streptavidin (SAX) tips (ForteBio #18-5117) specific biotinylated library members were conjugated by dipping the SAX tip into a 50 µg/mL PBS-P solution of each sample for 5 mins with a rotation value of 1000 RPMs. After sample conjugation, the tip was stabilized by immersing it in PBS-P (no ligands) for 2 mins with a rotation value of 1000 RPMs.

### Screening SIXX series compounds against Siglec-3, -7, -9, and -11

Lyophylized (h/m)Fc-Siglecs purchased from R&D Systems were reconstituted in PBS-P buffer to a concentration of 10 µg/mL and allowed to equilibrate on ice for 30 mins prior to analysis. Binding assays were set up using the following parameters:
a. Association (10 µg/mL Siglec in PBS-P) = 5 mins at 1000 RPMs
b. Dissociation (PBS-P) = 5 mins at 1000 RPMs
c. NaCl (1 M) wash = 1 min at 1000 RPMs
d. Baseline regeneration (PBS-P) = 1 min at 1000 RPMs

### Screening S112, S212, and S217 against Siglec-7

Lyophylized hFc-Siglec-7s purchased from R&D Systems was reconstituted in PBS-P buffer to a concentration of 10 µg/mL and allowed to equilibrate on ice for 30 mins prior to analysis. Binding assays were set up using the following parameters:
a. Association (10 µg/mL Siglec in PBS-P) = 2 mins at 1000 RPMs
b. Dissociation (PBS-P) = 3 mins at 1000 RPMs
c. NaCl (1 M) wash = 1 min at 1000 RPMs
d. Baseline regeneration (PBS-P) = 1 min at 1000 RPMs

The following compounds were tested: 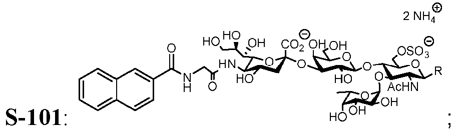 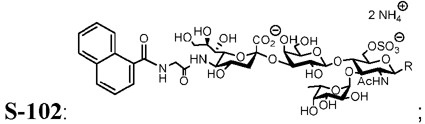 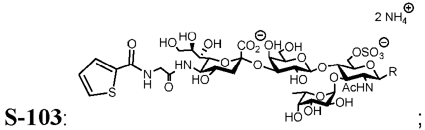 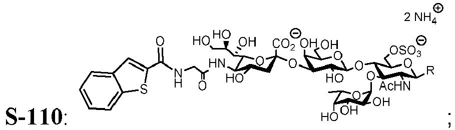 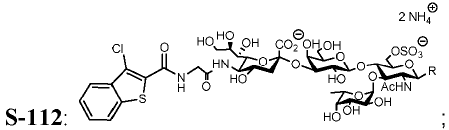 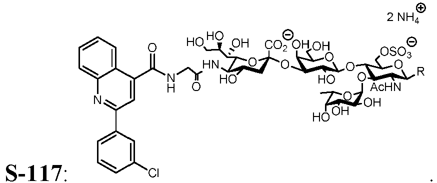 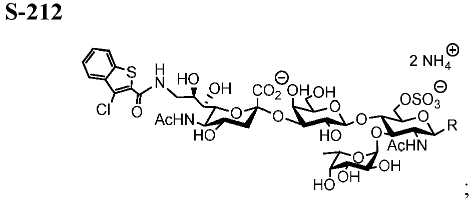 and

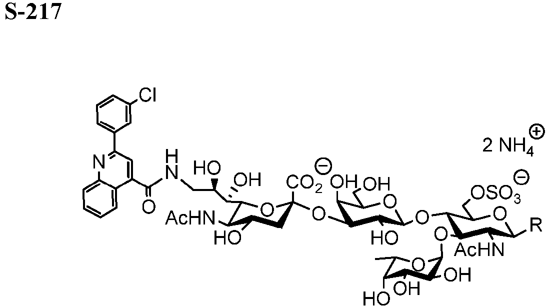

The natural ligand of Siglec-9, 6-sulfosialyl Lewis^{X}, also known as compound S000, 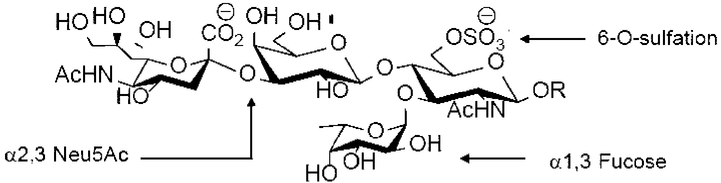 was used as a positive control.

In each of the compounds depicted above, R was a moiety represented by the following structural formula:

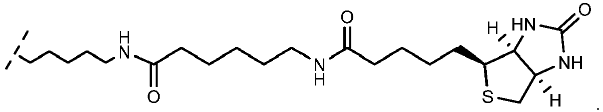

For example, the positive control, 6-sulfosialyl Lewis^{X}, was represented by the following structural formula:

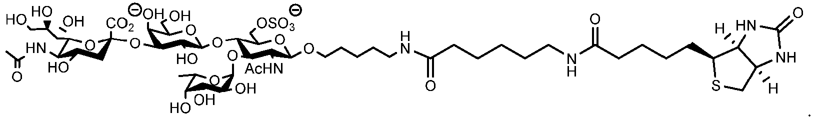

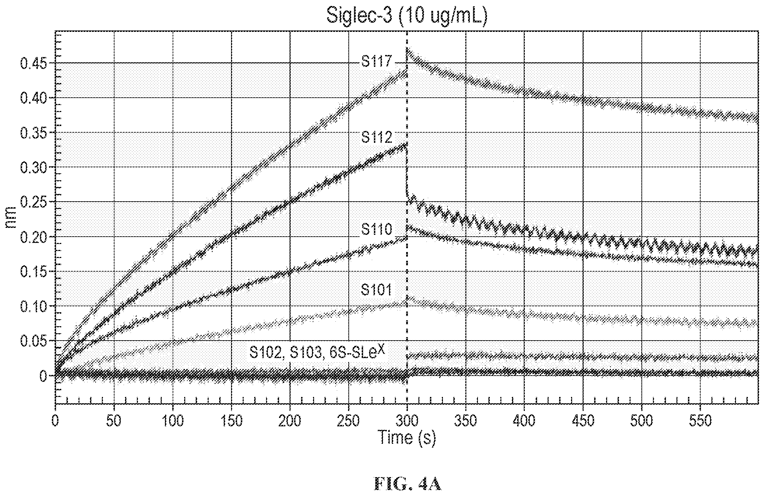

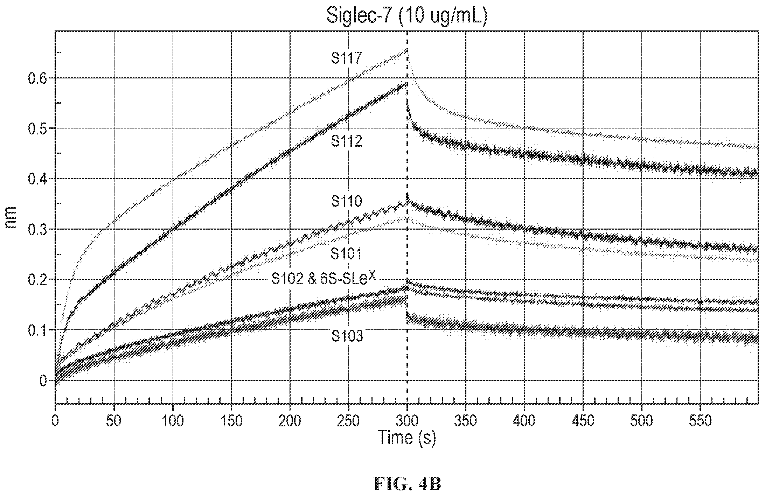

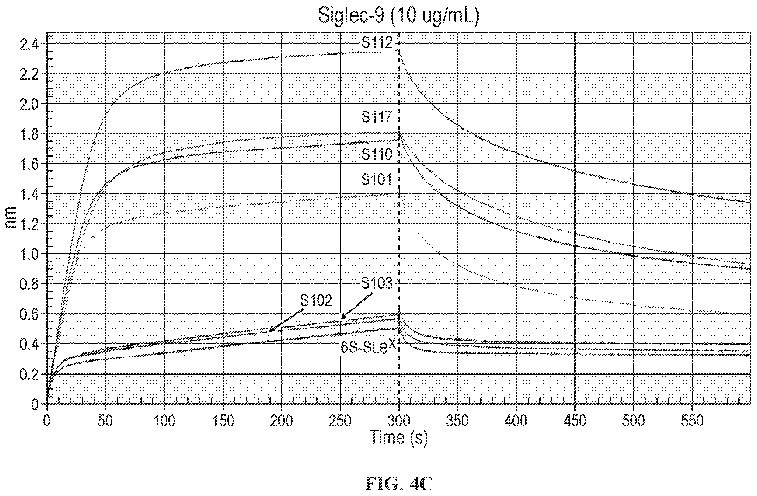

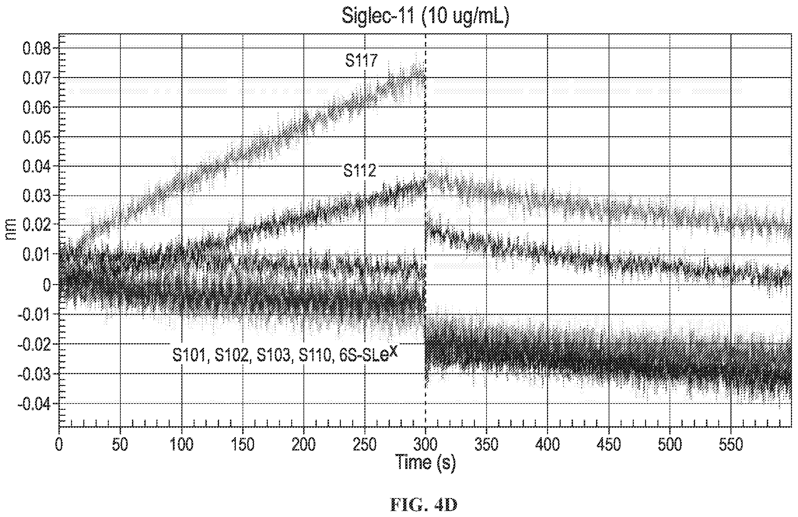

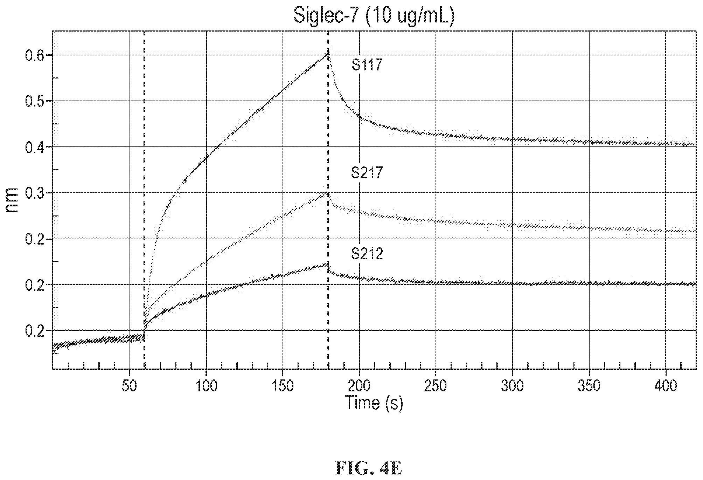

The results of the binding experiments are shown in the sensograms reproduced in FIG. 4A through FIG. 4E. In the plots shown, binding affinity (response) is measured as a a wavelength shift (nm) in the interference pattern as a function of time, for a fixed concetration of the Siglec target. The response is proportional to the number of ligand molecules bound to the surface of a biosensor.

The data illustrates the higher binding affinity of certain tested compounds to Siglec-9 when compared to the other Siglecs examined (Siglec-3, -7, and -11).

### Example 6B: Compound D-102 Demonstrates Enhanced Binding to Siglec-7

The strength of bidning of certain D-series compounds to Siglec 7 was tested according to the same BLI protocol as described above in Example 6A.

The following compounds were tested:

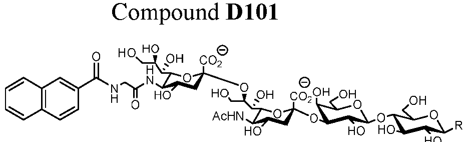

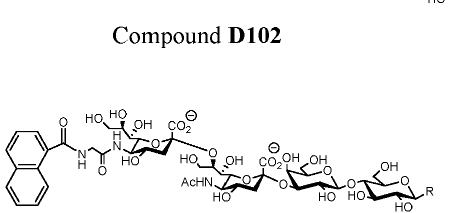

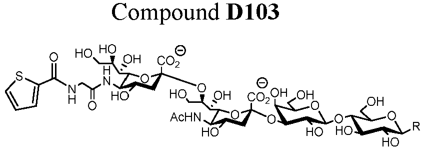

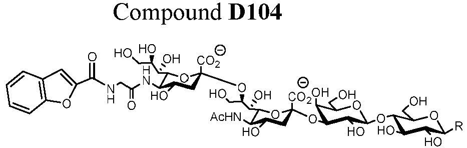

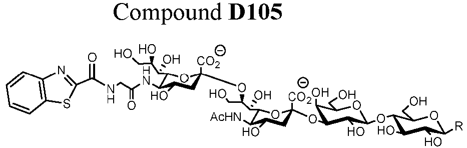

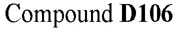

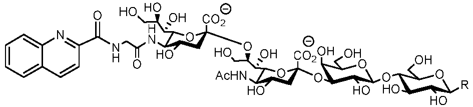

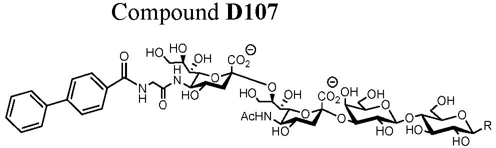

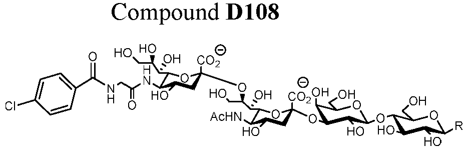

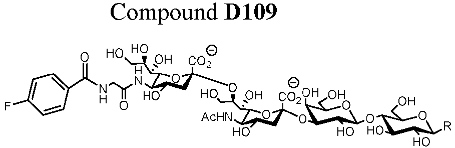

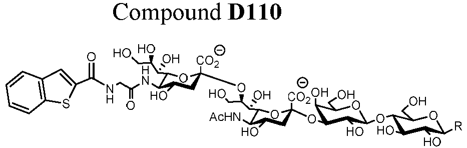

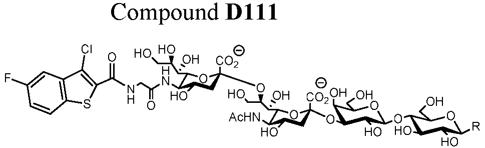

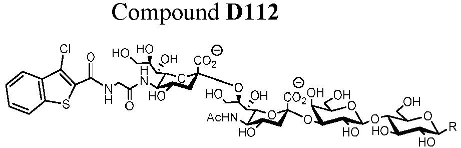

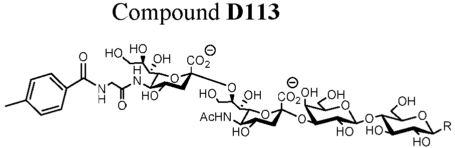

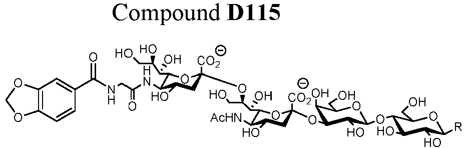

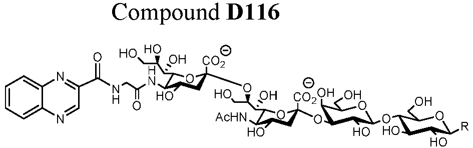

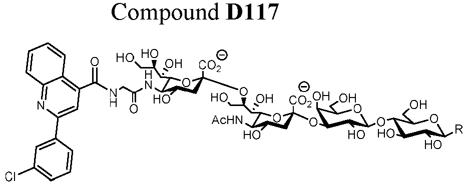

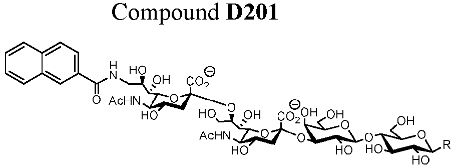

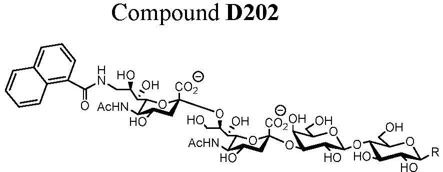

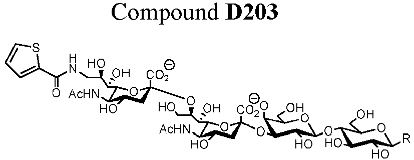

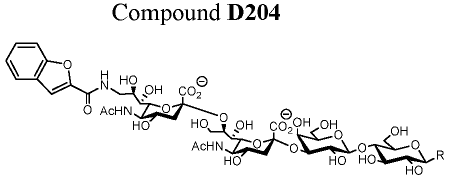

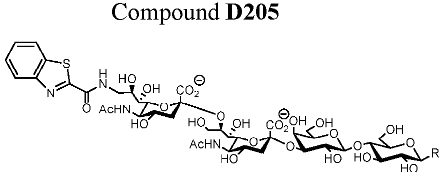

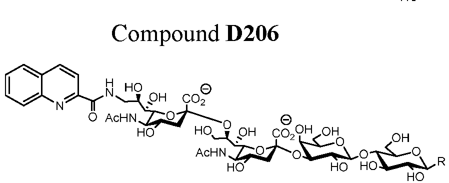

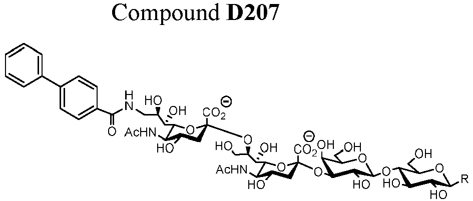

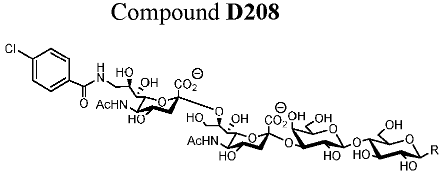

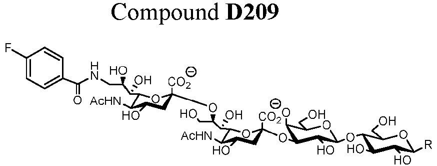

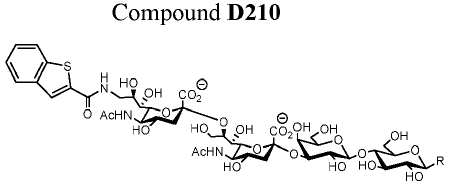

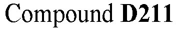

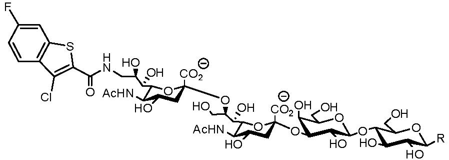

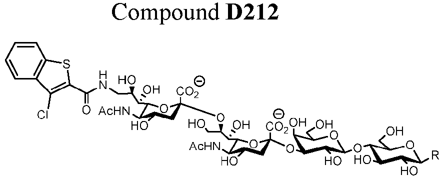

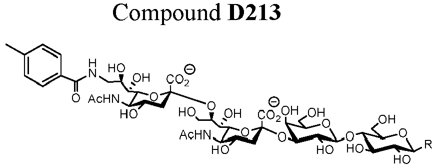

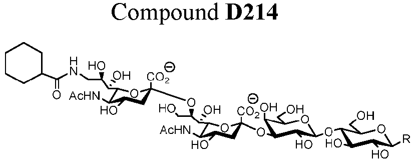

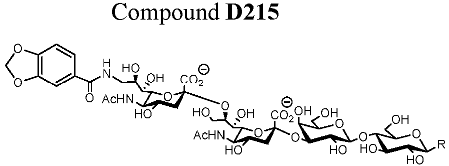

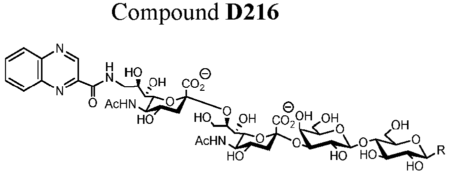

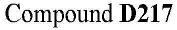

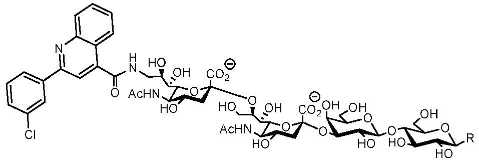

The natural ligand to Siglec 7, known as compound D000, was used as a positive control:

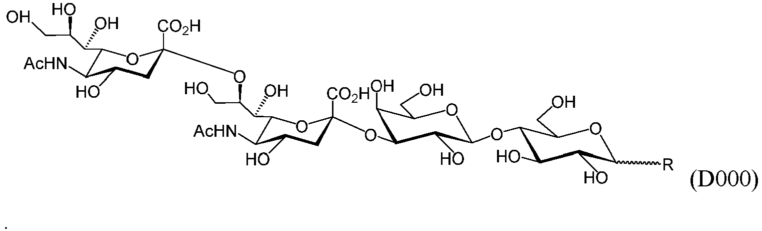

In each of the compounds depicted above, R was a moiety represented by the following structural formula:

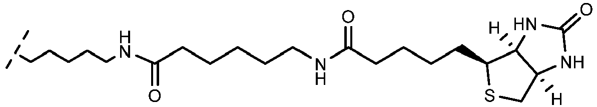

The data demonstrates that compound D102 exhibits approximately 4-fold higher affinity to Siglec 7 than its natural ligand, compound D000. Other tested D-series compounds did not show a marked improvement in binding.

### Example 7: Methods of Testing the Siglec Ligands

The Siglec ligands described herein (modified oligosaccharides as well as the modified oligosaccharides conjugated to polymers that form nanoparticles) can be screened and tested for biological activity in *in vitro* and *in vivo* models.

### 1. In vivo models

### Lung fibrosis

1) Bleomycin-Induced Pulmonary Fibrosis Model in Mice
2) Silica-Induced Lung Fibrosis Model in Mice:
   a) Route of delivery: oral pharyngeal route or osmotic pump; b) Test article administration: oral, IP, IV, IM, SC, inhalation and osmotic pumps; c) Treatment regimen: Therapeutic or Prophylactic.

### Liver /kidney fibrosis/Cardiac

1) CCl4-Model of Liver Fibrosis in Mice or 2) NI-F mouse model for liver and kidney fibrosis.

### Retinal disease

1) Ocular fibrosis, or 2) Diabetic retinopathy - mouse ocular model for oxygen induced retinopathy.

### Pulmonary

1) ARDS or 2) Acute lung injury.

### Sepsis

### Oncology

### 2. In vitro assays

1) Fibroblast cell culture, 2) NK cells cell assays, 3)Macrophages assays, 4) Neutrophils cell assays, 5) Fibrocyte cell assays.

### 3. Protocol for Isolating Human Neutrophils from Whole Blood using EasySep using magnetic sorting

1) Transfer 10 ml of whole blood to 50ml conical tube.
2) Supplement with EDTA (1mM final) if no EDTA-anticoagulant was used.
3) Vortex magnetic beads and isolation cocktail before use.
4) Add 50ul/ml of isolation cocktail to the sample.
5) Add 50ul/ml of magnetic beads to the sample.
6) Mix and incubate at RT for 5 min.
7) Top up sample tube to 50 ml with FACS buffer. Mix by gently inverting 2-3 times
8) Incubate without lid at RT for 10min in EasySep magnet.
9) Carefully pipette the enriched cell suspension into a new tube.
10) Add same volume of magnetic beads to the new tube and incubate 5 min RT
11) Place the tube without lid in the magnetic rack for 5 min RT.
12) Transfer cell suspension to a new tube and place on magnet for 10 min RT
13) Carefully pipette the enriched cell suspension into a new tube and spin at 400g 5min.
14) Resuspend the cell pellet in 5 ml R10 medium and count.

### 4. Verification of purity of isolated neutrophils

1) Transfer 500 000 cells to 5 ml FACS tubes
2) Spin down 400x 5 min and resuspend in FACS buffer
3) Add Fc blocker 1ul/100ul cells.
4) Incubate RT for 20 min.
5) Spin down cells at 400x 5min and resuspend in 200 ul FACS buffer
6) Stain cells with antibodies 1ul/100ul cells for 20 min RT.
7) Spin down cells at 400x 5min and resuspend in 500 ul FACS buffer
8) Wash cells using FACS buffer and resuspend in 500ul.
9) Analyze cells on MACSQuant machine for CD45/CD166b/CD16 expression

### 5. Preparation of medium

Use sterile techniques to prepare R10 Medium (RPMI1640 + 10% FBS + 1x P/S). The following example is for preparing 500 mL of medium. If preparing other volumes, adjust accordingly.
1) Thaw FBS and P/S in water bath.
2) Add 50 mL of 100% FBS and 5 ml of 100x P/S
3) Invert bottle 4-5 times.
4) Store medium in 4C

Use sterile techniques to prepare FACS Medium (PBS + 2% FBS + 1mM EDTA). The following example is for preparing 500 mL of medium. If preparing other volumes, adjust accordingly.
1) Thaw FBS in water bath.
2) Add 10 mL of 100% FBS and 550 ul of 1M EDTA
3) Invert bottle 4-5 times.
4) Store medium in 4C

### 6. Establishing the protocol for Fibrocyte Cell Culture

Isolating Human Monocytes from Leukopak using EasySep (Easy 50 method)
1. Prepare 40mL of the sample from Leukopak (Cat. No. Cr70500.2) at a concentration of 5x10^7 cells/mL
2. Add sample to 50mL conical tube.
3. Add 2mL of 50ul/ml of isolation cocktail to the sample.
4. Mix and incubate at RT for 5min
5. Vortex Magnetic particles for 30s before adding 2ml to sample
6. Mix and incubate at RT for 5min
7. Top up sample tube to 50ml with medium. Mix by gently pipetting up and down 2-3 times
8. Incubate tube at RT for 10min in EasySep magnet

### 7. Preparation of ImmunoCult-SF Macrophage Differentiation Medium

Use sterile techniques to prepare ImmunoCult-SF Macrophage Differentiation Medium (ImmunoCult-SF Macrophage Medium + Human Recombinant M-CSF). The following example is for preparing 10 mL of medium. If preparing other volumes, adjust accordingly.
1) Thaw ImmunoCult-SF Macrophage Medium at room temperature (15 - 25°C) until just thawed. Mix thoroughly. NOTE: If not used immediately, aliquot and store at -20°C. Do not exceed the shelf life of the medium. After thawing aliquots, use immediately or store at 2 - 8°C for up to 4 weeks. Do not re-freeze.
2) Add 100 µL of 5 µg/mL Human Recombinant M-CSF to 10 mL of ImmunoCult-SF Macrophage Medium (final concentration 50 ng/mL). Mix thoroughly. NOTE: If not used immediately, store ImmunoCult-SF Macrophage Differentiation Medium at 2 - 8°C for up to 1 week.

### 8. Establishing the protocol for Fibrocyte Cell Culture

a) Fibrocytes are generated by cell culture using Serum-free medium.
b) Serum-free fibrocyte medium consists of RPMI 1640, supplemented with 1% L-glutamine, 1% Penicillin-Streptomycin, 1% Hepes buffer, 1% liquid media supplement (ITS+3), 1% non-essential amino acids and 1% sodium pyruvate.
c) All cell cultures were performed at 5% CO2 in a humidified incubator.
d) PBMC (10^6/ml) or CD14 cells 05 (2 x 10^5/ml) are resuspended in SF medium, cultured in either 8 well glass chamber slides or 24 well plastic plates for 5 days.
e) At day 4 the non-adherent cells are washed out and half the medium is removed and replaced with fresh medium.
f) The number of fibrocytes was determined by counting triplicate wells with three fields of view per well.

### 9. Establishing the protocol for Normal Human Lung Fibroblast (NHLF) Cell Culture

i. Warm medium in a 37°C water bath.
ii. Wipe the outside of the vial of cells with 70% ethanol or isopropanol.
iii. In a biosafety cabinet, twist the cap a quarter-turn to relieve internal pressure and then retighten.
iv. Quickly thaw cells in a 37°C water bath by gently shaking the vial. Do not submerge the vial. Remove the vial when only a small frozen cell pellet remains. Do not vortex cells.
v. Wipe the outside of the vial with 70% ethanol or isopropanol.
vi. Dilute cells 1 in 10 with warmed medium.
vii. Centrifuge the cell suspension at 300 x g for 10 minutes at room temperature (15 - 25°C).
viii. Carefully remove the supernatant with a pipette, leaving a small amount of medium to ensure the cell pellet is not disturbed. Resuspend the cell pellet by gently flicking the tube.
ix. Gently add medium to the tube and seed in T-25 flasks for 2-3 days.

### Establishing a Subculture

i. Change media in cells every 2-3 days. Centrifuge at 125 x g for 5 mins, then resuspend in fresh media.
ii. Split cells when they reach approx. 90% confluency.
iii. The cells are stimulated with recombinant (r) TGF-β1 (rTGF-β, 10 ng/ml) and for 48hr at 37°C in 5% CO2.
iv. Save supernatant and run ELISA

### Example 8: Study 1 of Nanoparticle Formulations: Nanoparticles Decorated with Exemplary Ligands Modulate Siglec-mediated Signaling

### 1. Summary

S101, S103, S105, S106, S107, S110, S112, S212 and S217 ligands nanoparticle (NP) conjugates were formulated using PLGA-PEG block polymer using either Click chemistry or NHS chemistry (See FIG. 1). The formulation batches S212-NP01, S217-NP01 and S112-NP01 were prepared using NHS chemistry. The formulation batches S101-NP01, S103-NP01, S105-NP01, S106-NP01, S107-NP01, S110-NP01, S112- NP02 were prepared using click chemistry. See FIGs. 6A-6F for the chemical structures of the NP conjugates.

### 2. Manufacturing of Nanoparticle formulation using NHS chemistry (S212-NP01, S217-NP01 and S112-NP01)

100 mg PLGA(10K)-PEG(5K)-NHS (available from Nansoft Polymers at the URL https://www.nanosoftpolymers.coml) was dissolved in 29.5% DMF (0.295 ml), followed by the addition of 30% benzyl alcohol (0.300 ml) and 30% ethyl acetate (0.300 ml) (polymer solution). 3 mg ligand (S212-amine or S217-amine or S112-amine) dissolved in 5% water (0.050 ml) for injection (WFI) and 5% DMF (0.050 ml). Then this ligand solution was mixed with polymer solution, followed by addition of 0.5% DIPEA (0.005 ml). Solution was stirred overnight at room temperature protected from light (organic phase). Under homogenization (9600 rpm), the organic phase was injected into 9 ml pre-cooled WFI containing 8% ethyl acetate (aqueous phase). The sample was then sonicated at 100% power for 30 sec using an Ultrasonic probe. The fine suspension was then quenched with 10x quantity of pre-cooled WFI. After 30 min incubation on ice, the formulation was concentrated and purified using a TFF system. Concentrate was collected and sucrose was added up to the 10% solution. pH was adjusted to around 7 using 0.1 M sodium hydroxide solution. The final product was filtered using 0.2 micron filter. Particle size and PDI were measured using a Malvern Zetasizer Pro. The final Formulation was stored at -20°C.

The blank control for the amine-conjugated nanoparticles was prepared according the same protocol, omitting the step of the ligand attachment.

### 3. Manufacturing of Nanoparticle formulation using Click chemistry (S101-NP01, S103-NP01, S105-NP01, S106-NP01, S107-NP01, S110-NP01, S112-NP02)

90 mg of PLGA(10K)-PEG(5K)-COOH and 10 mg of PLGA-(10K)-PEG(5K)-DBCO (available from Nanosoft Polymers at the URL https://www.nanosoftpolymers.com/) were dissolved in 30% DMF (0.300 ml), followed by the addition of 30% benzyl alcohol (0.300 ml) and 30% ml ethyl acetate (0.300 ml) (polymer solution). 3 mg ligand (S112-azide) was dissolved in 5% water for injection (WFI) (0.050 ml) and 5% DMF (0.050 ml). Then this ligand solution was mixed with polymer solution. The solution was stirred overnight at room temperature protected from light (organic phase). After homogenization (9600 rpm), the organic phase was injected into 9 ml pre-cooled WFI containing 8% ethyl acetate (aqueous phase). The sample was then sonicated at 100% power for 30 sec using an Ultrasonic probe. The fine suspension was then quenched with 10x quantity of pre-cooled WFI. After 30 min incubation on ice, the formulation was concentrated and purified using a TFF system. Concentrate was collected and sucrose was added up to the 10% solution. pH was adjusted to around 7 using 0.1 M sodium hydroxide solution. The final product was filtered using 0.2 micron filter. Particle size and PDI were measured using a Malvern Zetasizer Pro. The final Formulation was stored at -20°C.

The blank control for the azide-conjugated nanoparticles was prepared according the same protocol, omitting the step of the ligand attachment.

The structures of the polymer-ligand conjugates as well as the polymers used for "blank" formulations (positive control) are shown in FIG. 6A through FIG. 6F. S101-azide, S103-azide, S105-azide, S106-azide, S107-azide, S110-azide and S112-azide were conjugated to polymer blend of PLGA-PEG-COOH and PLGA-PEG-DBCO, resulting in formation of a triazole linkage between both reactants to yield conjugation. S112-amine, S212-amine and S217-amine were conjugated to PLGA-PEG-NHS polymer, resulting in formation of an amide bond between both reactants to conjugation.

### 4. Characterization of Nanoparticle formulations

Results are summarized in the following table.

**Table 2: Nanoparticle formulation using NHS chemistry**

| **Batch number** | **S212-NP01** | **S217-NP01** | **S112-NP01** | **AT-07-NP11** |
|---|---|---|---|---|
| **Ligand** | S212 | S217 | S112 | Blank |
| **Conjugation chemistry** | NHS chemistry | | | |
| **Polymer:Drug (mg)** | 100:3 | 100:2.6 | 100:3 | 100 |
| **Appearance** | Clear | Clear | Clear | Translucent |
| **Particle size, nm** | 114.3 | 120.3 | 87.1 | 64 |
| **PDI** | 0.23 | 0.23 | 0.26 | 0.12 |
| **pH** | 6.13 | 6.22 | 7.23 | N/A |
| **Total solids mg/ml** | 8.25 | 11.17 | 5.55 | 12 |
| **ligand concentration on NPs** | N/A | N/A | 0.140 mg/mL | N/A |
| **Loading efficiency*** | N/A | N/A | 83.8% | N/A |

**Table 3: Nanoparticle formulation using Click chemistry**

| **Batch #** | **S101-NP01** | **S103-NP01** | **S105-NP01** | **S106-NP01** | **S107-NP01** | **S110-NP01** | **S112-NP02** | **AT-07-NP23** |
|---|---|---|---|---|---|---|---|---|
| **Ligand** | S101 | S103 | S105 | S106 | S107 | S110 | S112 | Blank |
| **Conjugation** | Click chemistry | | | | | | | |
| **Polymer:Drug (mg)** | 100:3 | | | | | | | 100 |
| **Total solids mg/ml** | 18.85 | 16.78 | 12.91 | 12.69 | 11.88 | 12.93 | 8.58 | 10.35 |
| **pH** | 6.84 | 6.92 | 6.77 | 6.48 | 6.44 | 6.80 | 6.73 | 6.55 |
| **Particle size,nm** | 101.3 | 114.2 | 113.4 | 118.4 | 117.6 | 124.3 | 133.6 | 107.5 |
| **PDI** | 0.29 | 0.26 | 0.21 | 0.26 | 0.24 | 0.23 | 0.15 | 0.31 |
| **Ligand concentration on NPs UV absorbance** | N/A | N/A | N/A | N/A | 0.374 mg/ml | N/A | 0.108 mg/mL | N/A |
| **Loading effici ency*** | N/A | N/A | N/A | N/A | 104% | N/A | 40% | N/A |
| **Appearance** | Translucent | Translucent | Translucent | Translucent | Translucent | Translucent | Translucent | Translucent |
| **Endotoxin (EU /ml)** | 24.97 | 0.27 | 7.84 | 13.39 | 10.0 | 1.76 | 15.15 | N/A |

The term "loading efficiency" in Tables 1 and 2 refers to the % of ligand (referred to herein as a sialic acid-containing ligand) conjugated to polymer. It is computed as a ratio of the actual drug loading to the target (theoretical) drug loading. The target drug loading is calculated as the ratio of the weight of the ligand to the weight of the polymer. The method for measuring of the actual drug loading can be selected by a person of ordinary skill based on the chemical structure of the ligand.

For example, the measurement of the actual drug load can begin with measuring "total solids," *i.e.* the amount (usually, in milligrams) of all polymers, including polymer molecules conjugated to the ligands described herein as well as unconjugated polymers in a unit volume of a nanoparticle formulation. In one example, a UV spectroscopy can be used at 230 nm. After a standard curve is obtained by a serial dilution of a polymer solution, the UV absorbance of the sample at 230 nm is measured and compared to the standard curve to obtain the weight of the "total solids" in a unit volume of the formulation.

Next, the amount of ligands in a unit volume of a sample is measured. For the ligands that absorb in the UV spectrum, such as S107 or S112, UV spectroscopy can be used. The S112 ligand has a λmax at 280nm; S107 ligand has a λmax at 260nm. After a standard curve was obtained by a serial dilution of solutions of the free ligands S107 and S112, the UV absorbance of a sample of the S107-conjugated nanoparticle was measured at 260 nm, whereas the UV absorbance of a sample of the S112-conjugated nanoparticle was measured at 280 nm. Both absorbance values were compared to the relevant standard curve to obtain the weight of the "ligand" in a unit volume of the formulation.

The ratio of the two values, usually expressed in µg of ligand per mg of total solid, is the actual drug load.

Using the methods described herein, it is possible to achieve the values of actual drug load from 1 to 1,000 µg of ligand/1 mg total solid. The amount of ligand can be about 1 µg, about 2 µg, about 3 µg, about 4 µg, about 5 µg, about 6 µg, about 7 µg, about 8 µg, about 9 µg, about 10 µg, about 11 µg, about 12 µg, about 13 µg, about 14 µg, about 15 µg, about 16 µg, about 17 µg, about 18 µg, about 19 µg, about 20 µg, about 21 µg, about 22 µg, about 23 µg, about 24 µg, about 25 µg, about 26 µg, about 27 µg, about 28 µg, about 29 µg, about 30 µg, about 31 µg, about 32 µg, about 33 µg, about 34 µg, about 35 µg, about 36 µg, about 37 µg, about 38 µg, about 39 µg, about 40 µg, about 41 µg, about 42 µg, about 43 µg, about 44 µg, about 45 µg, about 46 µg, about 47 µg, about 48 µg, about 49 µg, about 50 µg, about 60 µg, about 70 µg, about 80 µg, about 90 µg, about 100 µg, about 150 µg, about 200 µg, about 250 µg, about 300 µg, about 350 µg, about 400 µg, about 450 µg, about 500 µg, about 550 µg, about 600 µg, about 650 µg, about 700 µg, about 750 µg, about 800 µg, about 850 µg, about 900 µg, about 950 µg or about 1000 µg. Additionally, the amount of ligand can range from about 5 µg to about 500 µg, such as about 10 µg to about 250 µg, such as about 20 µg to about 100 µg.

The nanoparticles comprise a polyethylene-glycol/polylactic-glycolic acid core decorated with synthetically modified sialic acids on its surface. Without being bound to any particular theory, it is believed that the nanoparticle core provides a stable yet biodegradable scaffold for the presentation of the synthetically modified ligands of the Siglec receptors present on immune cells. Further, it is believed that the presentation, at a relatively high density, of these ligands to the immune cells augments the potency of the anti-inflammatory signaling through enhancement of *avidity,* that is, strong cell binding through a multiplicity of weak interactions. Finally, it is believed that synthetic ligands have an increased *affinity* to the targeted Siglec receptor, which, in turn, augments the potency of the nanoparticle in anti-inflammatory signaling. Sialic acid-activated signaling is considered to be a mechanism by which immune cells recognize "self". It is postulated that enhanced *affinity* of the synthetic ligands, when compared to sialic acid, toward their Siglec receptors, and enhanced *avidity,* through presentation of a multiplicity of Siglec ligands on the surface of the nanoparticle operate in tandem to generate a therapeutically tractable anti-inflammatory strategy.

### 5. Non-toxicity profile of exemplary nanoparticles with respect to macrophages

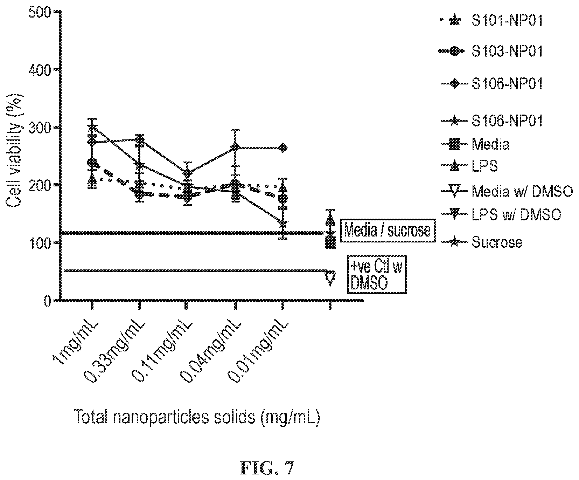

In order to determine the non-toxicity profile of S101-NP-01, S103-NP-01, S106-NP-01, S107-NP-01 nanoparticles, an in vitro cytotoxicity assay on THP-1 monocyte derived macrophages was conducted. THP-1 cells were seeded in a 96 well plates at a density of 100x10³ cells/200ul in each well. THP-1 monocyte differentiated macrophages were treated with 0.01 mg/mL - 1 mg/mL of the nanoparticles for 24 hours. Cells were differentiated using 10 ng/mL phorbol-12-myristate-13-acetate (PMA) and activated using lipopolysaccharide (LPS) at 1µg/mL. The cells were incubated for 24 hours with 0.01mg/mL - 1 mg/mL dose of exemplary nanoparticles and control nanoparticles lacking a ligand. After 24 hours, cells were washed and incubated with 1 mg/mL of MTT for 3-4 h at 37 °C. The resulting formazan crystals were dissolved in 100 µl of MTT solubilization buffer and the absorbance were measured at 570 nm using a Spectra iMax plate reader, the values were compared to the control cells. Graphs represent percent cell viability when compared to control untreated cells. The results, shown in FIG. 7, demonstrate that these nanoparticles are non-toxic to THP-1 derived macrophages.

### 6. Affect of Nanoparticles on LPS-activated Macrophages

### Derivation of THP-1 LPS-activated macrophages

The THP-1 cells used in these experiments are a "monocyte-like" cell line derived from a one-year-old boy with leukemia (Tsuchiya, 1980). The cells express complement 3 (C3) and Fc receptors. They are phagocytic (for both latex beads and sensitized erythrocytes and others) but lack surface and cytoplasmic immunoglobulin. The cells are weakly responsive to toll-like receptor agonists in their undifferentiated state but become more responsive after differentiation. Cells were grown in Roswell Park Memorial Institute (RPMI) culture medium that had been supplemented with 20% fetal bovine serum (FBS; Gibco, 10438026). The initial seeding and incubation were conducted in T-25 flasks for 2-3 days. At 48 hours before use the differentiation of THP-1 cells to monocytes were induced with 10 ng/mL, phorbol 12-myristate 12-acetate (PMA) in serum-free RPMI. The exact function of the PMA is not known but it is believed to mimic signaling molecules that are inserted in the inner face of the plasma membrane and stimulate the protein kinase C pathway. Thus, once added it cannot be washed away and differentiation is terminal. After an additional 48 hours, the differentiated cells were adherent and ready for activation with 1 µg/mL lipopolysaccharide (LPS). The cells can be evaluated for bioactivity using western blot for SHP-1 phosphorylation, cellular binding using IHC. Supernatants were used for ELISA based cytokine release assays. (Abbreviations used: PMA = phorbol 12-myristate 13-acetate; LPS = lipopolysaccharides.)

### General Procedure

R&D huELISA kits for TNF-α, IL-6, and VEGF were used and followed manufacture's protocol. Cytokine levels were determined by coating a 96 well plate with capture antibody diluted in 1x phosphate buffered saline (PBS) overnight. All steps were carried out at room temperature. The wells were washed three times in 1X PBS and 1% Polyoxyethylene sorbitan monolaurate (Tween 20) before being blocked for one hour with 1% BSA dissolved in 1xPBS. The washing step was repeated and 50µL of treated cell supernatants or standards based on the manufacturer's protocol were added to the wells and left for 2 hours. Subsequently supernatant was aspirated out, the wells washed 3 times and 50 uL of detection antibody diluted in 1% BSA in 1XPBS was added for 2 hours. Wells were washed three times and Horse Radish Peroxidase (HRP) conjugated antibody was added at 1: 200 dilutions in 1% B SA in 1xPBS for 20 minutes. The plates were covered in aluminum foil. Once wells were washed, 3,3',5,5'-tetramethylbenzidine (TMIB) was added for 20 minutes and protected from light. 1M hydrochloric acid was added to halt the reaction and absorbance read on a plate reader at 450 nM.

### Nanoparticles modulate TNF-alpha production in LPS-activated macrophages.

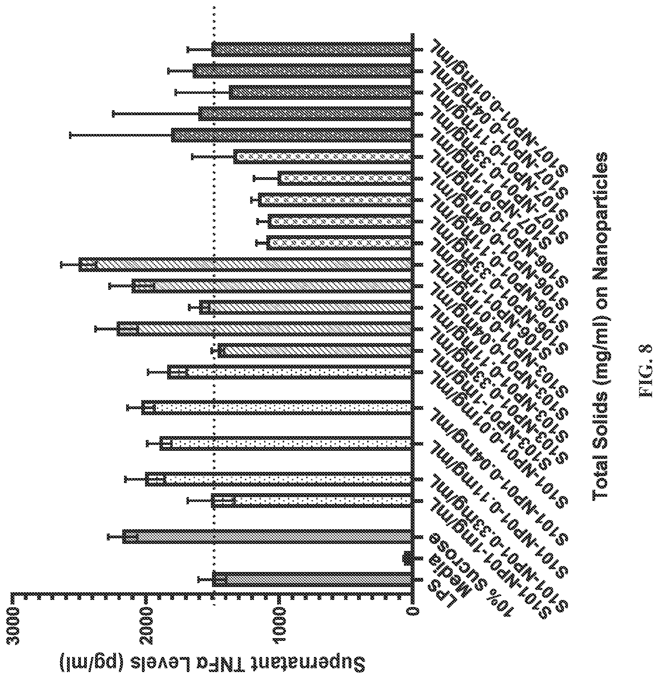

The effect of nanoparticles on TNF-alpha in LPS-activated macrophages was assessed as follows: THP-1 cells were seeded in a 24 well plates at a density of 500,000 cells/500 ul media in each well. Cells were differentiated using 10ng/ml of Phorbol-12-myristate-13-acetate (PMA) and activated using lipopolysaccharide (LPS) at 1µg/mL. The cells were incubated for 24 hours with nanoparticles, LPS alone, Media alone and 10% Sucrose as controls. After 24 hours, supernatants were collected and assayed for Hu TNF-alpha by ELISA (R&D systems). FIG. 8 reflects the results; graphs show a trend in suppression of TNF-alpha protein levels in LPS activated cells 24 hrs post treatment in S106-NP01 when compared to LPS alone group.

The data shown in FIG. 8 demonstrates that S106 ligand, when presented on a scaffold PLGA-PEG nanoparticle S106-NP01, has the ability to suppress key proinflammatory cytokine TNF-alpha. Each ligand had a characteristic feature of either suppression or activation of inflammatory markers found on the tested macrophages. This also indicates polarization of M1 macrophages to M2c resolution macrophages as identified by suppression of proinflammatory mediators. TNF-α can be widely targeted for inflammatory indications.

### Nanoparticles modulate IL-6 production in LPS-activated macrophages.

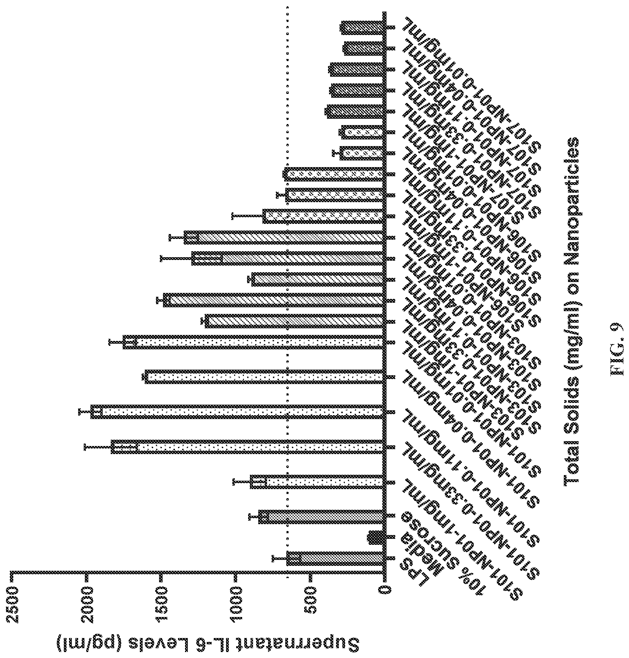

The effect of exemplary nanoparticles on IL-6 in LPS-activated macrophages was assessed as follows: THP-1 cells were seeded in a 24 well plates at a density of 500,000 cells/500 ul media in each well. Cells were differentiated using 10ng/ml of Phorbol-12-myristate-13-acetate (PMA) and activated using lipopolysaccharide (LPS) at 1µg/mL. The cells were incubated for 24 hours with nanoparticles, LPS alone, Media alone and 10% Sucrose as controls. After 24 hours, supernatants were collected and assayed for Hu IL-6 by ELISA (R&D systems). FIG. 9 reflects the results; graphs show a trend in suppression of IL-6 protein levels in LPS activated cells 24 hrs post treatment in S107-NP01 when compared to LPS alone group.

The data in FIG. 9 shows that S107 ligand when presented on a scaffold PLGA-PEG nanoparticle S107-NP01 has the ability to suppress key proinflammatory cytokine IL-6. Each ligand had a characteristic feature to suppress or activate inflammatory markers, which is a very important factor in inflammatory diseases. This also indicates suppression of highly activated M1 macrophages by these S107-NP01 nanoparticles as compared to other formulations and LPS alone.

### Nanoparticles modulate VEGF production in LPS-activated macrophages.

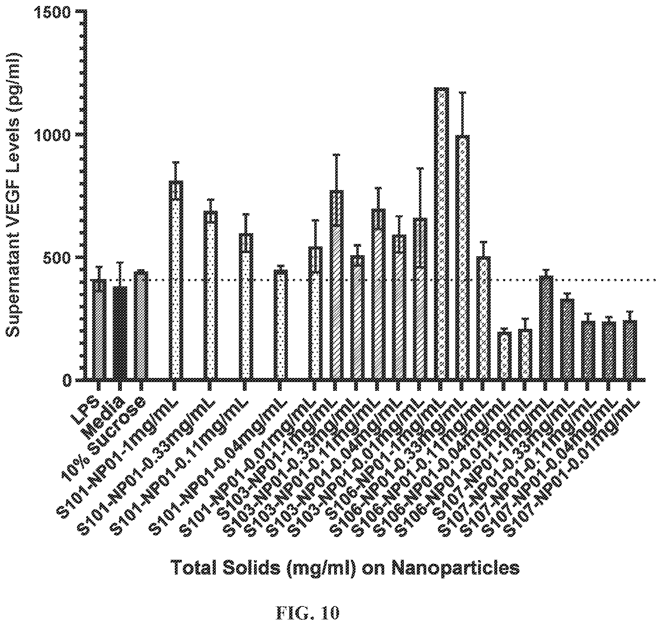

Effect of nanoparticles on VEGF in LPS-activated macrophages was assessed as follows: THP-1 cells were seeded in a 24 well plates at a density of 500,000 cells/500 ul media in each well. Cells were differentiated using 10ng/ml of Phorbol-12-myristate-13-acetate (PMA) and activated using lipopolysaccharide (LPS) at 1 µg/mL. The cells were incubated for 24 hours with nanoparticles, LPS alone, Media alone and 10% Sucrose as controls. After 24 hours, supernatants were collected and assayed for Hu VEGF by ELISA (R&D systems). FIG. 10 reflects the results; graphs show a trend in suppression of VEGF protein levels in LPS activated cells 24 hrs post treatment in S107-NP01 when compared to LPS alone group.

The data shown in FIG. 10 demonstrates that S107 ligand when presented on a scaffold PLGA-PEG nanoparticle S107-NP01 has the ability to suppress key proinflammatory cytokine VEGF. Vascular endothelial growth factor (VEGF)-VEGF receptor (R) system is extensively involved in angiogenesis. Suppression of VEGF is an important part of many inflammatory diseases, mainly involving macrophages/monocytes. This suppression by S107NP-01 could potentially be involved in polarization of M1 macrophages to M2c resolution macrophages.

### 7. Fibrosis differentiation assay on S-series ligands Nanoparticles

### General protocol

Fibrocytes were generated from monocyte isolated from PBMCs by using the EasySep kit (Stem cell) according to manufacture's instructions. CD14+ monocytes were cultured in serum-free medium (RPMI 1640, supplemented with 1% L-glutamine, 1% Penicillin-Streptomycin, 1% Hepes buffer, 1% liquid media supplement (ITS+3), 1% non-essential amino acids and 1% sodium pyruvate) at 2 x 10^5/mL in 96 well plate for 4 days. At day 4 the non-adherent cells were washed out and half the medium removed and replaced with the same volume of media alone or containing the following nano-formulations (NPs) with the ligands: S105-NP01, S106-NP01, S107-NP01, S110-NP01, S112-NP01, S112-NP02, at 0.5 and 0.25 mg/mL. AT-07-NP23 blank nanoparticles (azide linked) at 0.5 and 0.25 mg/mL, 10% sucrose and serum amyloid P (SAP) 1 ug/mL were used as controls. The monocyte/fibrocyte cells were incubated for another 24 h. The number of differentiated fibrocytes was determined by counting fibrocytes and monocytes by duplicate wells with three fields of view per well and images were analyzed on ImageJ by using cell counter plug in. Differentiated fibrocytes are spindly shaped cells, while monocytes have a round circular shape. A total N=6-9 images per condition were analyzed.

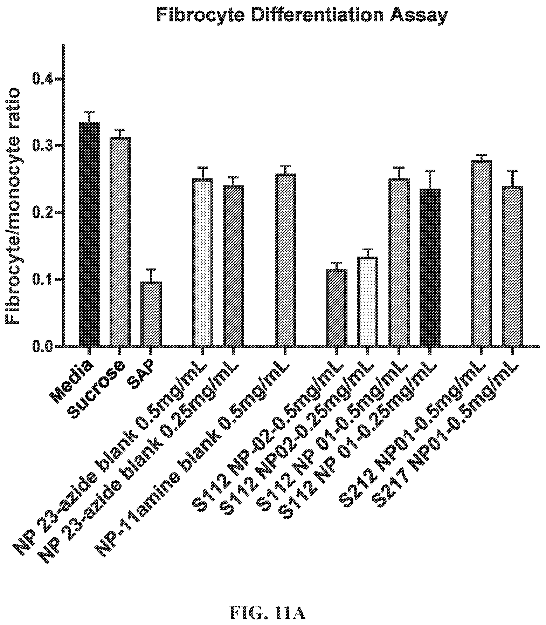

The results shown in FIG. 11(A) indicate that S112-NP02 (azide linked) significantly inhibited monocyte differentiation at 0.5 and 0.25 mg/mL compared to sucrose control and the blanks. Moreover, the inhibition of fibrocyte differentiation observed for S112-NP02 (azide linked) is similar to SAP, a well described anti-fibrotic drug (Pilling D, et al., 2018). Šídák's multiple comparisons test p***-0.0001.

The graph shown in FIG. 11(A) suggests that the ratio of fibrocytes/monocytes conversion is significantly reduced in S112NP-02 treated cells when compared to AT-07-NP23 (blank azide) treated cells and also exhibited a dose response, with p value <0.0001 using Dunnett's multiple comparisons test. This suppression was very similar to that of Serum amyloid protein (SAP) a positive control in preventing fibrocyte differentiation. Further suggesting that the presentation of S112 ligand on a nanoparticle S112-NP02 involves a multivalency representation and significantly inhibits the differentiation of monocytes to fibrocytes.

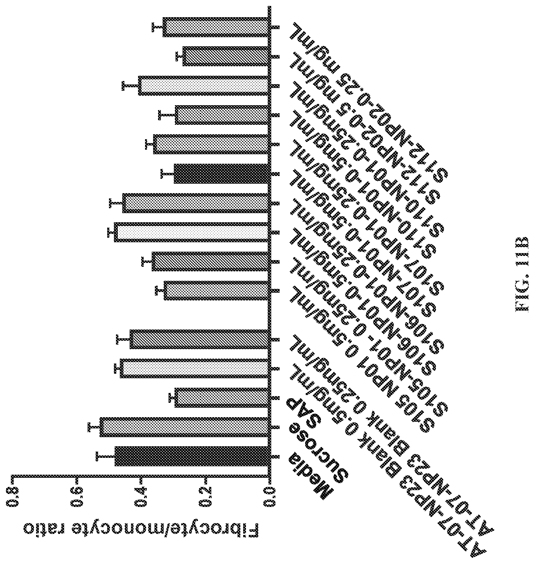

The plot shown in FIG. 11(B) shows fibrocyte differentiation rates determined for the NPs S105-NP01, S106- NP01, S107-NP01, S110-NP01, S112-NP02, AT-07-NP-23 blank azide, sucrose and SAP controls by analyzing 6-8 images from duplicated wells using ImageJ software.

The data in FIG. 11(B) suggests the ratio of fibrocytes/monocytes conversion is significantly reduced in S105-NP01, S107-NP01, S110-NP01 and S112-NP02 when compared to AT-07-NP23 Blank azide. All these nanoparticles exhibited a dose response, with p value <0.001 using Dunnett's multiple comparisons test toward AT-NP23 Blank Azide. This suppression was very similar to that of Serum amyloid protein (SAP) a positive control in preventing fibrocyte differentiation. The use of the nanoparticles described herein can be exploited in myelofibrosis disease where monocytes and fibrocyte involvement is extensively explored (Verstovsek, S., et al 2012, 2018, Manshouri T etal., 2019, Veletic, I., et al., 2018) The data indicates that the presentation of S112 ligand on a nanoparticle S112 NP-02 is a multivalency (i.e. the multiplicity of presentation of ligands on the NP surface) that significantly inhibits the differentiation of monocytes to fibrocytes.

### 8. Suppression of ROS in Neutrophils

Neutrophil granulocytes are the most abundant leukocytes of the innate immune system representing the first line of defense against invading pathogens. Myeloperoxidase MPO is a heme-based peroxidase enzyme found at high levels in the primary granules of neutrophils. MPO enzymatic activity has been shown in mouse models of Diabtetic retinopathy (Binett, et al., 2020). Along with ocular indication, role of neutrophils has been established in acute respiratory distress syndrome (ARDS), which is a disease characterized by accumulation of fluid and inflammatory cells in the airspace of the lungs. This accumulation of fluid and cells results in a compromise of the lung's ability to deliver oxygen to the bloodstream. There are several identified causes of this disease, although the most common is sepsis, implying that there is a relationship between ARDS and uncontrolled inflammation. NETosis is a unique form of programmed cell death that neutrophils undergo when exposed to certain agents such as phorbol myristate acetate (PMA), bacterial LPS (Fuchs et al., 2007). These agonists activate NADPH oxidase (NOX), which generates reactive oxygen species (ROS) and subsequently activates mitogen-activated protein kinases. Hence the sialic acid receptor Siglec-9 is highly expressed on both macrophages and neutrophils, the Siglec 9 ligand are excellent candidate therapeutics for targeting these disease.

Neutrophils were isolated from the blood of healthy controls using EasySep Human Neutrophil Isolation Kit (StemCell Technologies Catalog # 19359. Human PMNs were rested for 1hr prior to treatment with phorbol myristate acetate (PMA) (100nM) following the manufacturer's instructions. Following PMA treatment, cells were incubated at different concentrations of nanoparticles S101-NP01, S103-NP01, S105-NP01, S106-NP01, S107-NP01, S112-NP02 at 37C and supernatants were collected at different time points. ROS activity was assessed using manufacturer's instructions from Promega ROS-Glo H2O2 (lytic protocol, Catalogue #G8820) at 1 hr post treatment.

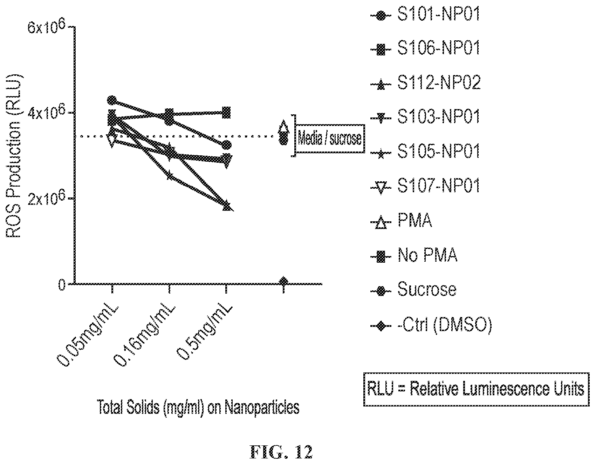

FIG. 12 illustrates the veffect on ROS production in neutrophils isolated from whole blood and treated with PMA(100mM) at different concentrations (0.05mg/mL to 0.5mg/mL) of nanoparticles S101-NP01, S103-NP01, S105-NP01,S106-NP01, S107-NP01, S112-NP02. The data in FIG. 12 indicates a trend in a dose dependent suppression of ROS activity 1 hr post treatment with S112-NP02 and S105-NP01 at 0.5mg/mL when compared to media with PMA positive control.

The data shown in FIG. 12 strongly suggests that the suppression of ROS in a dose depending manner using S112 NP-02 and S105 NP01 in neutrophils could be useful in atherapeutic approach aimed at reducing NETosis.

### 9. MPO (Myeloperoxidase) levels post PMA treatment on neutrophils at different time points

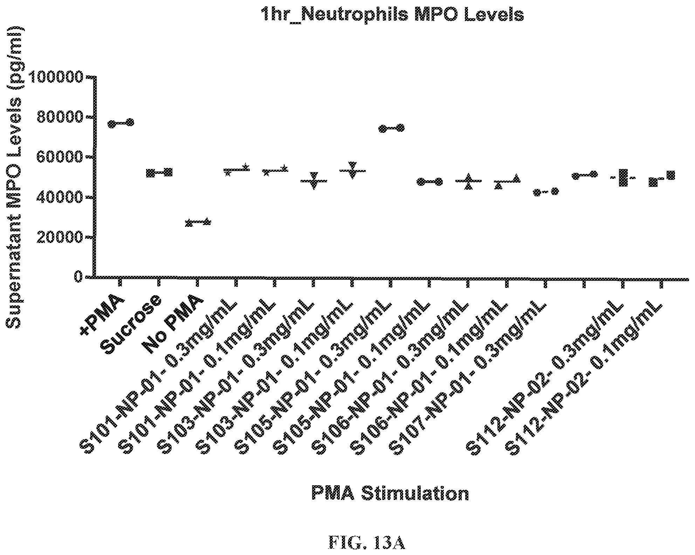

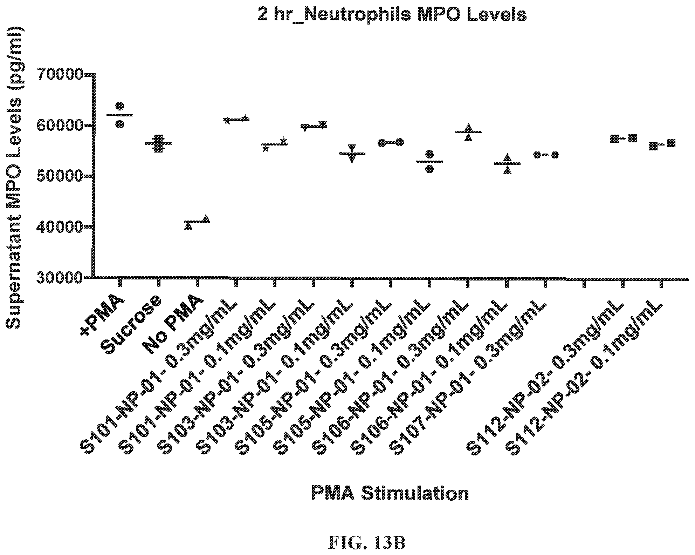

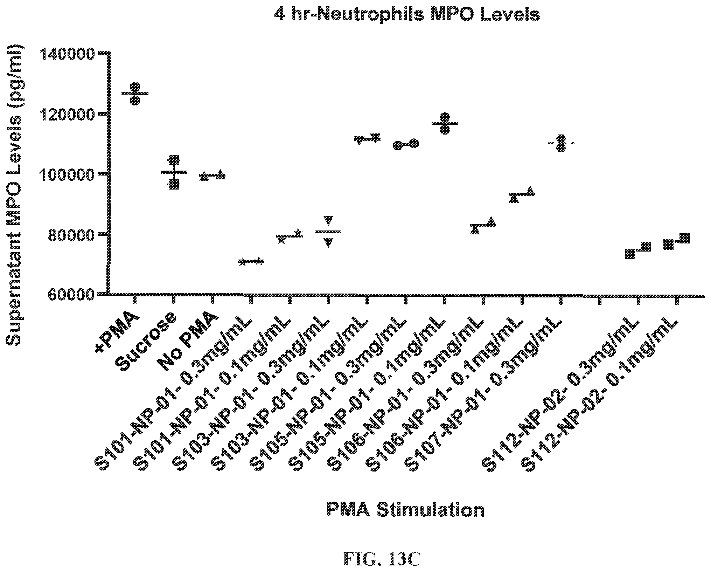

Effect on MPO production in neutrophils isolated from whole blood and treated with PMA (100mM) at different concentrations (0.3mg/mL to 0.1mg/mL) of nanoparticles S101-NP01, S103-NP01, S105-NP01 S106-NP01, S107-NP01, S112-NP02 is illustrated in FIGs. 13(A), 13(B), and 13(C). (In each of FIGs. 13(A)-13(C), the two points corresponding to a concetration value represent the number of sample repeats. This experiment was performed in duplicates. The line between the two points represents standard error of mean between the two duplicates.) Supernatants were isolated at different time points 1 hr (A), 2hr(B), 4 hr (C). Below graphs indicate that only 4 hrs post treatment had a significant suppression of MPO compared to sucrose treatment 0.5mg/mL when compared to media with PMA positive control.

The graphs shown in FIGs. 13(A) through 13(C) suggest that at treatment with nanoparticles (A) 1 hr post treatment of PMA with neutrophils and (B) 2 hrs post treatment does not suppress the MPO enzyme levels (MPO is a neutrophil marker and a constituent of NETs), while (C) at 4 hrs post treatment, S101-NP01, S112-NP02 exhibited a very highly significant dose dependent suppression with the p value of <0.0001. S103-NP01, S106-NP01, had a moderate suppression with the p = 0.0002 at high concentrations and S107-NP01 had a lower suppression with p = 0.01 (all p values computed by Dunnett's multiple comparisons test). S105-NP01 did not exhibit suppression of MPO 4 hrs post treatment.

It is believed that presentation of the above-described ligands to the immune cells augments the potency of the anti-inflammatory signaling through enhancement of *avidity.* At the same time, the synthetic ligands have an increased *affinity* to the individual Siglec receptor (compared to the sialic acid), which also enhances the anti-inflammatory potency of the nanoparticles. The data presented in FIGs. 13(A) through 13(C) clearly indicates the role of the siglecs in target engagement and eliciting functional response. MPO is a key neutrophil marker in NETosis and suppression of MPO is a potential target for neutrophil-mediated disease indications.

### 10. Role of phagocytosis and involvement of Siglecs in modulating innate and adaptive immunity

Phagocytosis is a specific form of endocytosis where cells internalize solid matter, thereby eliminating cellular debris and pathogens. Although most cells are capable of phagocytosis, the phagocytes in the immune system including neutrophils, macrophages and immature dendritic cells, play a key role in this process. In these cells, phagocytosis is a mechanism by which microorganisms can be contained, killed and processed for antigen presentation; it represents an important facet of the innate immune response, and plays an essential role in initiating the adaptive immune response. Siglecs that can bind trans-ligands, such as Sialoadhesin, allow cell-cell interactions to take place. These glycan-Siglec interactions allow cells to bind one another. Sialoadhesin lacks a cytosolic ITIM or a positive residue to bind ITAM-containing adaptors and so is thought not to influence signalling. Studies show that this protein is involved in phagocytosis of bacteria that contain highly sialylated glycan structures such as the lipopolysaccharide of Neisseria meningitidis. Binding to these structures allows the macrophage to phagocytose these bacteria, clearing the system of pathogens.

### Siglec ligand nanoparticles affect phagocytosis in vitro.

Cells (immortalized cell lines BV-2 cells (mouse microglia cell line) and RAW 264.7 (murine macrophages)) were plated on black wall 96 well plates, rested for 24 hours, then treated for 24 hours (n=2-8 wells/condition), before adding LysoBrite Dye and incubating at 37C for 1hr and washing twice. Plates were read by fluorescent spectrophotometer (ex: 475 nm/em: 500-550nm), then cells were fixed (2% PFA for 30min), stored at 4C, and images were captured (FITC channel) on an inverted Zeiss microscope.

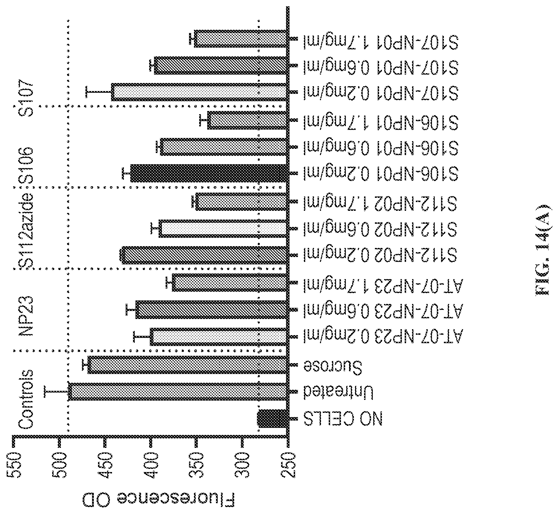

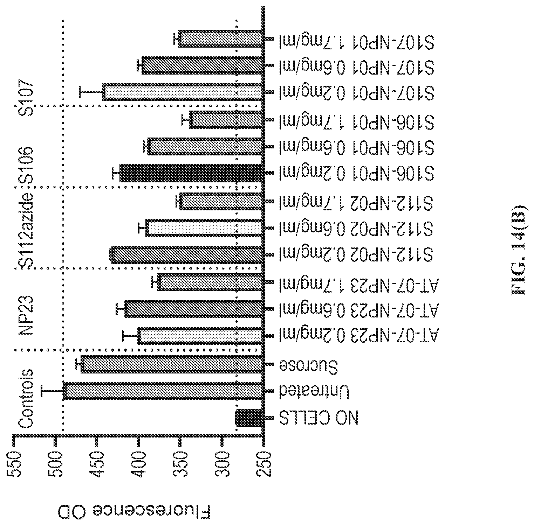

The results are shown in FIG. 14(A) for microglia cells and FIG. 14(B) for macrophages.

Results shown in FIG. 14(A) indicate that BV-2 cells (mouse microglia cell line) after treatment with: media (untreated control), sucrose (buffer control), AT-07-NP-23 (blank NP control), had higher fluorescence indicative of phagocytosis, while S106-NP01, S107-NP01 and S112-NP02 showed lower intensity of fluorescence suggesting these ligands inhibit phagocytosis.

FIG. 14(B): Quantification of LysoBrite labeled RAW cells (mouse macrophage cell line) by fluorescent spectrophotometer after treatment with LPS plus: media, sucrose, AT-07-NP-23, S106-NP01, S107-NP01, or S110-NP01.

Results shown in FIG. 14(B) indicate that RAW cells (mouse macrophages cell line) after treatment with: media (untreated control), sucrose (buffer control), AT-07-NP-23 (blank NP control), had higher fluorescence indicative of phagocytosis, while S106-NP01, S107-NP01 and S110-NP01 has lower intensity of fluorescence suggesting these ligands inhibit phagocytosis

In summary, in comparison to untreated and sucrose controls, all siglec ligand nanoparticles tested appear to inhibit phagocytosis in a dose-dependent manner. Although AT-07-NP23 blank NP control also weakly inhibits phagocytosis, treatment with S112-NP02, S106-NP01, S107-NP01, or S110-NP01 appears to be more effective than AT-07-NP23. Suppression of phagocytosis may be harnessed to reduce phagocytosis of neurons, synapses, and intact myelin in multiple neurodegenerative diseases, including: amyotrophic lateral sclerosis, multiple sclerosis, Parkinson's disease, Alzheimer's disease (Late), Huntington's disease, CNS injury (chronic).

### Example 9: Study 2 of Nanoparticle Formulations: Nanoparticles Decorated with Exemplary Ligands Bind to Siglecs

### 1. METHODS

### 1.1. Formulation of NHS- and DBCO-functionalised PLGA-PEG nanoparticles

Nanoparticles were synthesised in 20 mg batches consisting of either (1) 15 mg PLGA 502H (acid) (Sigma) plus 5 mg PLGA-PEG-NHS (Nanosoft Polymers), (2) 15 mg PLGA 502H plus 5 mg PLGA-PEG-DBCO (Nanosoft Polymers), (3) 17.5 mg PLGA 502H plus 2.5 mg PLGA-PEG-DBCO or (4) 10 mg PLGA 502H plus 10 mg PLGA-PEG-DBCO. Polymers were dissolved in 1 mL of dichloromethane (DCM) to form the organic phase and then injected into an aqueous phase comprising 7 mL of 2.5% w/v polyvinyl alcohol (PVA) in 50 mM 2-(N-morpholino) ethanesulfonic acid hydrate buffer at pH 5 (MES), under moderate stirring on ice. The emulsion was subjected to probe sonication in pulse mode (3 sec on followed by 2 sec off, for a total of 90 sec) on ice using a Model 120 sonic dismembrator (Fisher Scientific) set at an amplitude of 50% and left stirring overnight at room temperature to facilitate DCM evaporation. Nanoparticles were then washed by repeated centrifugation (3x) at 17,000 x g for 20 min at 4°C. Between each centrifugation step, nanoparticle pellets were resuspended in PBS by probe sonication in pulse mode (1 sec on followed by 1 sec off, for a total of 10 sec) at an amplitude of 35%.

### 1.2. Conjugation of amine- and azide-labelled ligands to NHS- and DBCO-functionalised PLGA-PEG nanoparticles

Conjugation reactions were set up by adding 100 µg of amine- or azide-functionalised ligand (20 µL of 5 mg/mL stock in H₂O) to 1 mg of NHS- or DBCO-functionalised nanoparticles, respectively, which were resuspended in 1 mL of PBS. Following overnight incubation at 4°C under gentle rotation, nanoparticles were centrifuged at 12,000 x g for 20 min at 4°C and supernatants containing excess non-conjugated ligand were discarded. Nanoparticles were finally resuspended at the desired concentration in PBS by probe sonication in pulse mode (1 sec on followed by 1 sec off, for a total of 10 sec) at an amplitude of 20% prior to downstream studies.

### 1.3. Characterisation of nanoparticles

Nanoparticles (formulated as described in sections 1.1. and 1.2.) were resuspended at 100 µg polymer/mL in PBS prior to analysis of size and polydispersity index (PDI) by dynamic light scattering (DLS) using a NanoBrook Omni (Brookhaven Instruments Corporation).

### 1.4. Conjugation of biotin-labelled ligands to FluoSpheres^{™} NeutrAvidin^{™}-labelled microspheres

Yellow-green fluorescent 0.2 µm FluoSpheres^{™} NeutrAvidin^{™}-labelled microspheres (Thermo Fisher Scientific) were placed in an ultrasonic bath for 15 mins and 100 µg (10 µL of 1% solids stock) aliquots were then dispensed into 1.5 mL Eppendorf tubes. Microspheres were washed by adding 1 mL of BlockAid^{™} blocking solution (Thermo Fisher Scientific) to each tube, followed by centrifugation at 20,000 x g for 15 min at 4°C. After discarding the supernatants, a further 1 mL BlockAid^{™} was added to each tube and microsphere pellets were resuspended by probe sonication in pulse mode (1 sec on followed by 1 sec off, for a total of 10 sec) at an amplitude of 35%. Conjugation reactions were set up by adding 10 µg of biotinylated ligand (5 µL of 2 mg/mL stock or 10 µL of 1 mg/mL stock in H₂O) to each tube. Following overnight incubation at 4°C under gentle rotation, microspheres were centrifuged at 20,000 x g for 15 min at 4°C and supernatants containing excess non-conjugated ligand were discarded. Microspheres were finally resuspended at the desired concentration in BSA/PBS by probe sonication in pulse mode (1 sec on followed by 1 sec off, for a total of 10 sec) at an amplitude of 20% prior to downstream studies.

### 1.5. Cell surface binding of Siglec-9 Fc +/- PLGA-based nanoformulations

Siglec-9 Fc chimera protein (R&D Systems; 5 µL of 50 µg/mL stock) was precomplexed with PE-conjugated anti-IgG Fc antibody (Thermo Fisher Scientific; 1 µL of 500 µg/mL stock) in 94 µL of 0.5% BSA/PBS for 15 mins at 4°C in darkness. Nanoformulations (50 µL of 2 mg polymer/mL stock in 0.5% BSA/PBS) were then incubated with the above for 15 mins at 4°C in darkness, followed by addition of PANC-1 cells (50 µL of 5 x 10⁶ cells/mL in 0.5% BSA/PBS) for a further 30 mins at 4°C in darkness. Cells were washed by adding 1 mL of 0.5% BSA/PBS to each tube and then centrifuged at 500 x g for 5 mins at 4°C, after which the supernatants were discarded. Cells were washed again as above and finally resuspended in 0.5% BSA/PBS prior to analysis of PE fluorescence on a BD Accuri C6 Plus flow cytometer. A minimum of 10,000 events per sample were acquired.

### 1.6. Cell surface binding of Siglec-9 Fc +/- FluoSpheres^{™} microspheres

A similar protocol was applied as described in **section 1.5.** with some minor modifications. Briefly, Siglec-9 Fc chimera protein (2.5 µL of 100 µg/mL stock) was precomplexed with PE-conjugated anti-IgG Fc antibody (1 µL of 500 µg/mL stock) in 46.5 µL of 0.5% BSA/PBS for 15 mins at 4°C in darkness. Microspheres (150 µL of 0.666 mg solids/mL stock in 0.5% BSA/PBS) were then incubated with the above for 15 mins at 4°C in darkness. PANC-1 cells (3 x 10⁵) were resuspended in the above mixture of reagents and incubated for a further 30 mins at 4°C in darkness. Cells were washed and PE fluorescence was analysed as described in **section 1.5.**

### 1.7. Fluorescence-linked immunosorbent assay (FLISA) analysis of Siglec-9 Fc binding by FluoSpheres^{™} microspheres

High-binding black 96-well microplates (Greiner Bio-One) were coated with 0.5 µg/mL Siglec-9 Fc chimera protein or control IgG1 Fc (R&D Systems) in PBS (100 µL per well) and incubated at 4°C overnight. Wells were washed (3x) by immersion in 0.1% Tween 20 in PBS (PBST) and non-specific binding sites were blocked with 1% BSA/PBS (blocking buffer; 150 µL per well) for 1 h at room temperature, after which washing was repeated as before. Fluorescent microspheres (100 µL of 0.25 mg solids/mL stock in blocking buffer per well) were then added for 2 h at room temperature in darkness. Finally, wells were washed (3x), bound microspheres were dissolved in a 1:1 volume ratio of ACN:DMSO (50 µL per well) and fluorescence was measured at 487/20ₑₓ and 528/20ₑₘ using a Cytation 5 plate reader (BioTek).

### 1.8. Cell surface binding of Siglec-9-PE +/- PLGA-based nanoformulations

HEK293T cells (2.5 x 10⁵) stably transduced with Siglec-9-encoding lentiviral particles were resuspended in 67.5 µL of 0.5% BSA/PBS. Cells were then incubated with nanoformulations (75 µL of 10 mg polymer/mL stock in 0.5% BSA/PBS) for 30 mins at 4°C, after which PE-conjugated anti-Siglec-9 antibody (R&D Systems; 7.5 µL of 10 µg/mL stock) was added for a further 30 mins at 4°C in darkness. Cells were washed by adding 1 mL of 0.5% BSA/PBS to each tube and then centrifuged at 500 x g for 5 mins at 4°C, after which the supernatants were discarded. Cells were washed again as above and finally resuspended in 0.5% BSA/PBS prior to analysis of PE fluorescence on a BD Accuri C6 Plus flow cytometer. A minimum of 10,000 events per sample were acquired.

### 2. RESULTS

### 2.1. Cell surface binding of recombinant Siglec-9 Fc

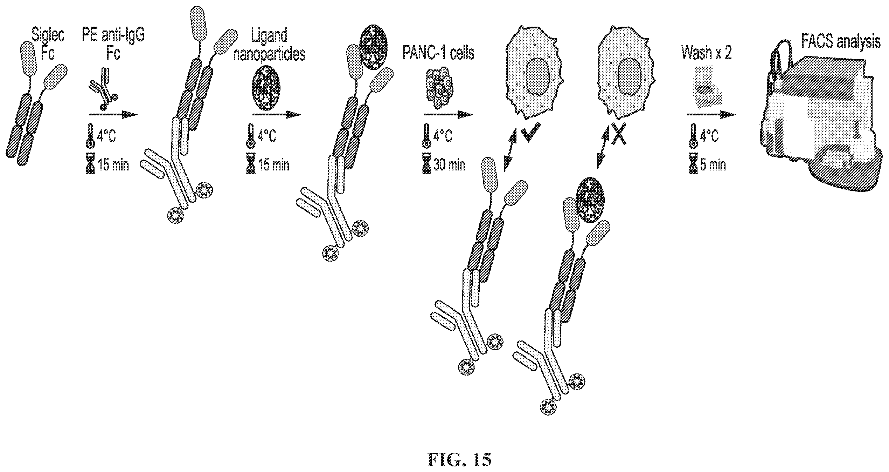

A flow cytometry assay was employed to assess whether nanoformulated ligands were capable of disrupting binding of Siglec-9 Fc to its cognate sialylated ligands expressed on the surface of tumour cells (FIG. 15).

In the schematic of flow cytometry assay setup shown in FIG. 15, Siglec-9 Fc was pre-complexed with PE-labelled anti-IgG Fc for 15 mins at 4°C, followed by the addition of nanoparticles (either PLGA-based nanoparticles described in **section 1.1. and** 1.2. or FluoSpheres^{™} microspheres described in **section** 1.4.) functionalised with S series ligands for a further 15 mins at 4°C. Complexes were then incubated with PANC-1 cells for 30 mins at 4°C. Excess reagents were removed by washing and PE fluorescence was assessed via flow cytometry.

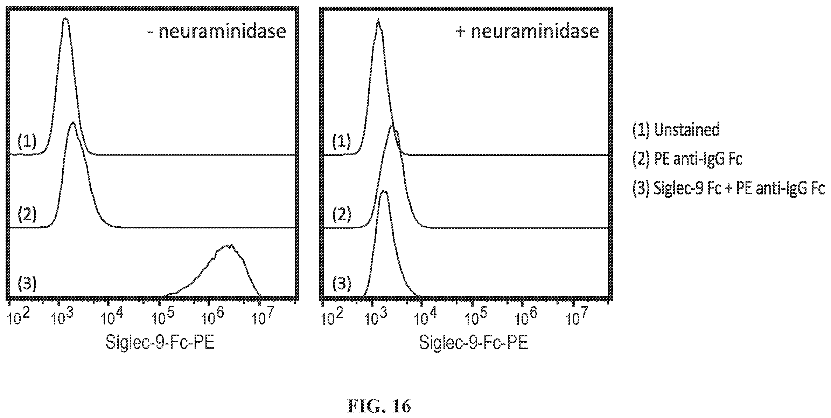

The PANC-1 pancreatic line was considered an appropriate model for these studies based on literature demonstrating high surface expression of Siglec-9 ligands on these cells (*see* Rodriguez E, Boelaars K, Brown K, Eveline Li RJ, Kruijssen L, Bruijns SCM, van Ee T, Schetters STT, Crommentuijn MHW, van der Horst JC, van Grieken NCT, van Vliet SJ, Kazemier G, Giovannetti E, Garcia-Vallejo JJ & van Kooyk Y. Sialic acids in pancreatic cancer cells drive tumour-associated macrophage differentiation via the Siglec receptors Siglec-7 and Siglec-9. Nat Commun 2021, 12(1), 1270). In agreement with these findings, Siglec-9 Fc bound to PANC-1 cells as indicated by an increase in PE fluorescence compared to unstained cells and cells stained with PE anti-IgG Fc alone (FIG. 16, - neuraminidase). Moreover, this was abrogated upon pre-treatment of cells with neuraminidase, confirming that Siglec-9 Fc binding to PANC-1 cells was sialic acid-dependent (FIG. 16, + neuraminidase).

FIG. 16 shows the results of the flow cytometry analysis of Siglec-9 Fc binding to PANC-1 cells +/- neuraminidase. PANC-1 cells were either left untreated (- neuraminidase) or incubated with neuraminidase (+ neuraminidase) to cleave sialic acids from the cell surface. Cells were then incubated with PE-labelled Siglec-9 Fc. PE fluorescence was assessed by flow cytometry as a readout of Siglec-9 Fc binding to the PANC-1 cell surface

### 2.1.1. Cell surface binding of recombinant Siglec-9 Fc +/- PLGA-based nanoparticles

This assay was then employed to assess the impact of nanoformulated ligands on Siglec-9 Fc binding to PANC-1 cells. PLGA-based nanoparticles were tested initially, which were synthesised using a polymer blend of 75% PLGA 502H and either 25% PLGA-PEG-NHS or 25% PLGA-PEG-DBCO, enabling conjugation to amine- or azide-labelled S112 ligand, respectively according to Scheme 100 below:

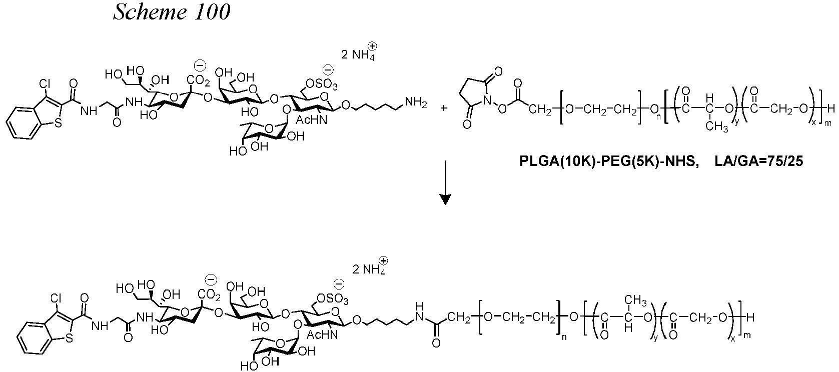

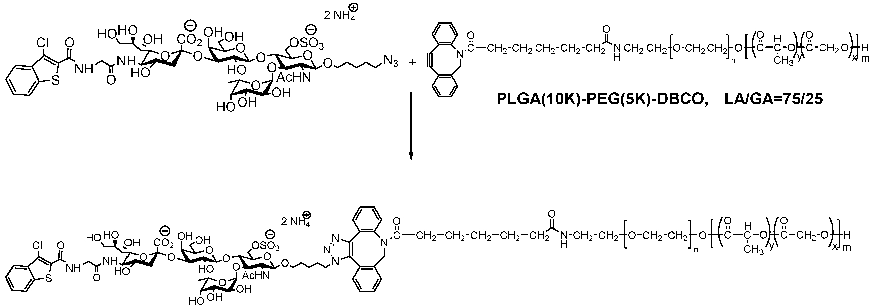

In Scheme 100, S112-amine was conjugated to nanoparticles comprised of a polymer blend of PLGA 502H (i.e., an acid-terminated PLGA, not illustrated) and PLGA-PEG-NHS, resulting in formation of an amide bond between both reactants to yield S112-amine nanoparticle. S112-azide was conjugated to nanoparticles comprised of a polymer blend of PLGA 502H (not illustrated) and PLGA-PEG-DBCO, resulting in formation of a triazole linkage between both reactants to yield S112-azide NP.

Physicochemical characterisation of these PLGA-based nanoparticles revealed similar diameters for both S112-amine nanoparticles and S112-azide nanoparticles, at 185.21 ± 3.75 nm and 198.81 ± 5.88 nm, respectively (**Table 5**). Likewise, the corresponding control non-targeted nanoparticles showed comparable diameters of 183.20 ± 3.91 nm and 196.50 ± 6.99 nm for NHS- and DBCO-functionalised nanoparticles, respectively. Moreover, the size distributions of all nanoformulations were highly monodisperse as indicated by low PDI values of <0.1.

**Table 5. Physicochemical characteristics of PLGA-based nanoparticles.**

| **Nanoformulation** | **Size (nm) (mean ± SD)** | **PDI (mean ± SD)** |
|---|---|---|
| S112-amine NP | 185.21 ± 3.75 | 0.05 ± 0.04 |
| Control NHS NP | 183.20 ± 3.91 | 0.06 ± 0.04 |
| S112-azide NP | 198.81 ± 5.88 | 0.07 ± 0.04 |
| Control DBCO NP | 196.50 ± 6.99 | 0.07 ± 0.02 |

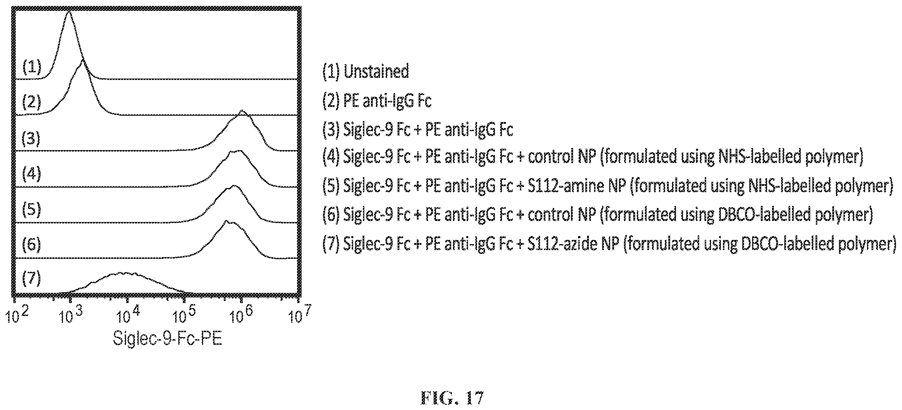

Referring to FIG. 17, binding of Siglec-9 Fc to PANC-1 cells was inhibited by S112-azide nanoparticles (**histogram (7)**), as indicated by a reduction in PE fluorescence compared to cells stained with PE-labelled Siglec-9 Fc in the absence of nanoparticles (**histogram (3)**). This indicates that S112-azide nanoparticles engage Siglec-9 Fc, thus impeding binding of the latter to PANC-1 cells. In contrast however, S112-amine nanoparticles did not affect Siglec-9 Fc binding to PANC-1 cells (**histogram (5)),** with PE fluorescence remaining comparable to that observed for cells stained with PE-labelled Siglec-9 Fc alone (**histogram (3)).** Likewise, Siglec-9 Fc binding to PANC-1 cells was also not affected by control nanoparticles (**histograms (4) and (6)),** which were formulated using the same polymer blends as S112-amine and S112-azide nanoparticles, although not subsequently conjugated to S112 ligand. Collectively, these findings highlight the superior Siglec-9 binding ability of S112 nanoparticles formulated via click chemistry compared to NHS-amine coupling.

FIG. 17 shows the flow cytometry analysis of Siglec-9 Fc binding to PANC-1 cells +/- PLGA-based nanoparticles. PE-labelled Siglec-9 Fc was incubated with PLGA-based nanoparticles, followed by the addition of PANC-1 cells. PE fluorescence was assessed by flow cytometry as a readout of Siglec-9 Fc binding to the PANC-1 cell surface.

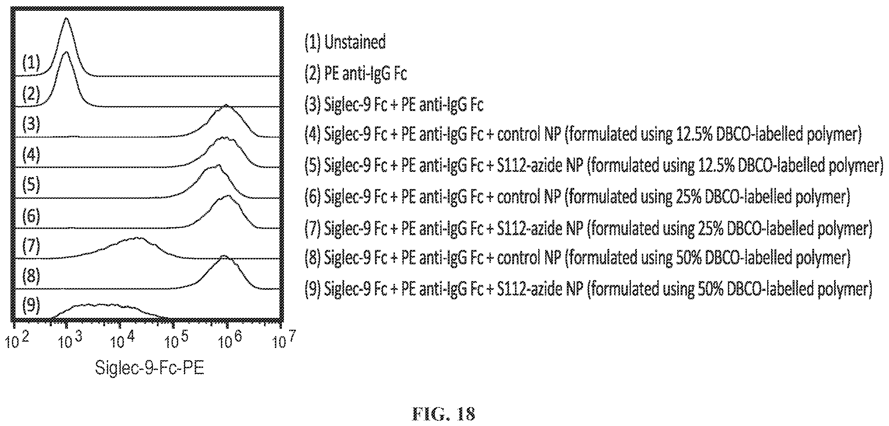

Referring to FIG. 18, these studies were next extended to assess the impact of varying ligand densities on the surface of S112-azide nanoparticles. This was achieved by formulating nanoparticles with differing amounts of the PLGA-PEG-DBCO polymer required for S112-azide coupling, either: (1) 12.5% PLGA-PEG-DBCO blended with 87.5% PLGA 502H, (2) 25% PLGA-PEG-DBCO blended with 75% PLGA 502H or (3) 50% PLGA-PEG-DBCO blended with 50% PLGA 502H. Binding of Siglec-9 Fc to PANC-1 cells was progressively inhibited with each stepwise increase in the % of PLGA-PEG-DBCO polymer incorporated within the nanoformulation (histograms (5), (7), and (9)). This suggests that ligand loading plays a role in determining the affinity of the interaction between Siglec-9 Fc and S112-azide nanoparticles.

FIG. 18 shows the results of the flow cytometry analysis of Siglec-9 Fc binding to PANC-1 cells +/- PLGA-based nanoparticles. PE-labelled Siglec-9 Fc was incubated with PLGA-based nanoparticles incorporating various amounts of the PLGA-PEG-DBCO polymer required for S112-azide coupling, followed by the addition of PANC-1 cells. PE fluorescence was assessed by flow cytometry as a readout of Siglec-9 Fc binding to the PANC-1 cell surface.

### 2.1.2. Cell surface binding of recombinant Siglec-9 Fc +/- FluoSpheres^{™} microspheres

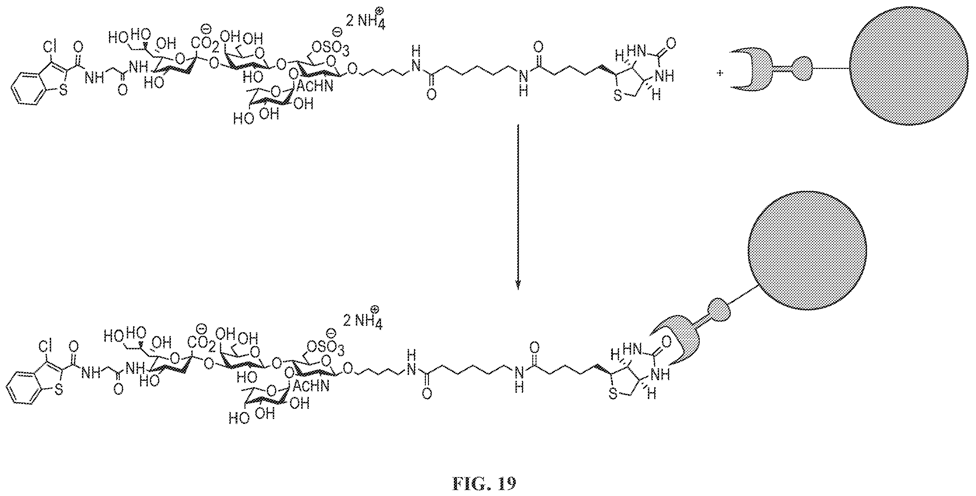

In addition, the studies described in **section 2.1.1.** were also replicated using an alternative nanoscaffold, whereby S series ligands were synthesised in biotinylated format (See structures below) and then coupled to FluoSpheres^{™} NeutrAvidin^{™}-labelled microspheres (see FIG. 19, which shows S112-biotin NP as an example; all other biotinylated ligands were coupled to FluoSpheres^{™} NeutrAvidin^{™}-labelled microspheres in the same manner). These are commercially available polystyrene beads of a similar size range to the PLGA-based nanoparticles employed in the previous **section 2.1.1.,** with a nominal bead diameter of 0.2 µm.

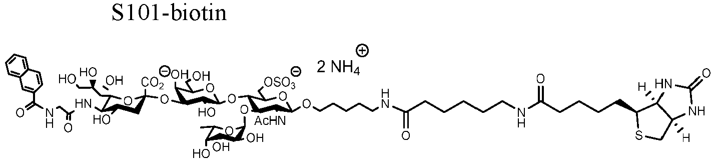

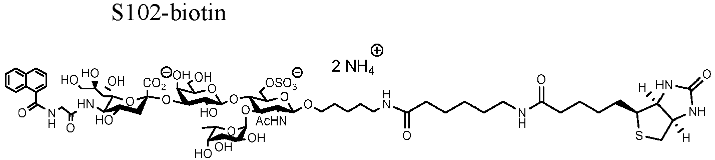

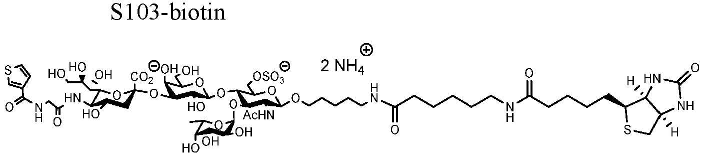

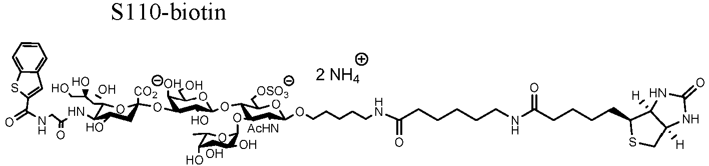

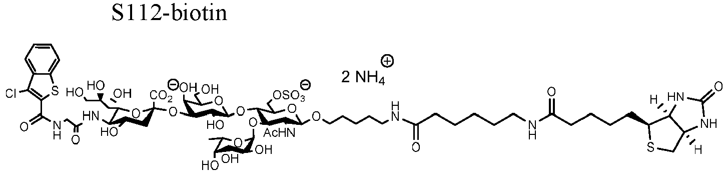

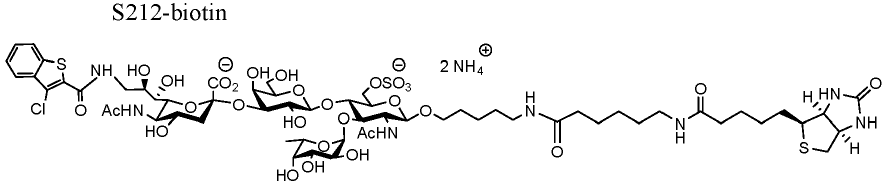

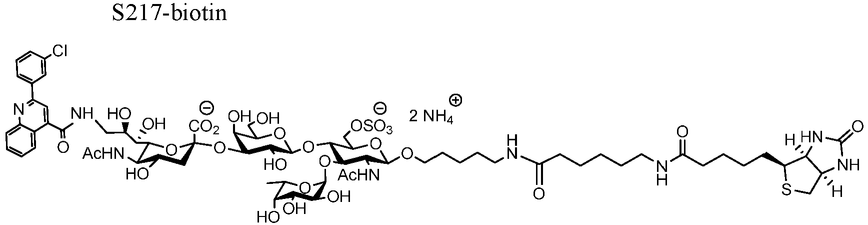

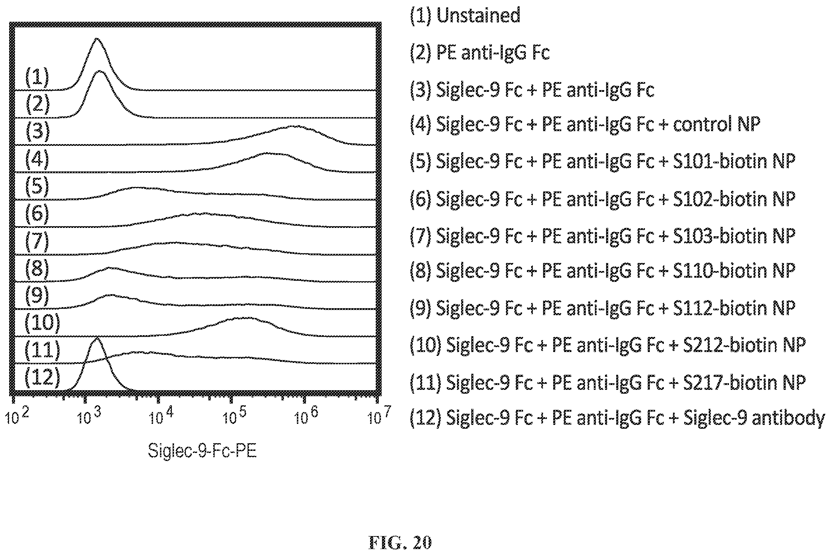

Referring to FIG. 20, binding of PE-labelled Siglec-9 Fc to PANC-1 cells was observed (histogram (3)) and could be markedly inhibited by S112-biotin nanoparticles (histogram (9)). Similarly, microsphere formulations of the other S series ligands also inhibited Siglec-9 Fc binding to PANC-1 cells to differing extents, with the most pronounced reduction in binding seen with S101-biotin nanoparticles (histogram (5)), S110-biotin nanoparticles (histogram (8)) and S217-biotin nanoparticles (histogram (11)). These findings indicate that microsphere formulations of all S series ligands tested are capable of binding to Siglec-9 Fc, albeit with varying affinities.

FIG. 20 shows the results of the flow cytometry analysis of Siglec-9 Fc binding to PANC-1 cells +/- FluoSpheres^{™} microspheres. PE-labelled Siglec-9 Fc was incubated with microsphere formulations of various S series ligands (or Siglec-9 antibody clone 191240 as a positive control), followed by the addition of PANC-1 cells. PE fluorescence was assessed by flow cytometry as a readout of Siglec-9 Fc binding to the PANC-1 cell surface.

### 2.2. Binding of FluoSpheres^{™} microspheres to recombinant Siglec-9 Fc immobilised on microtiter wells

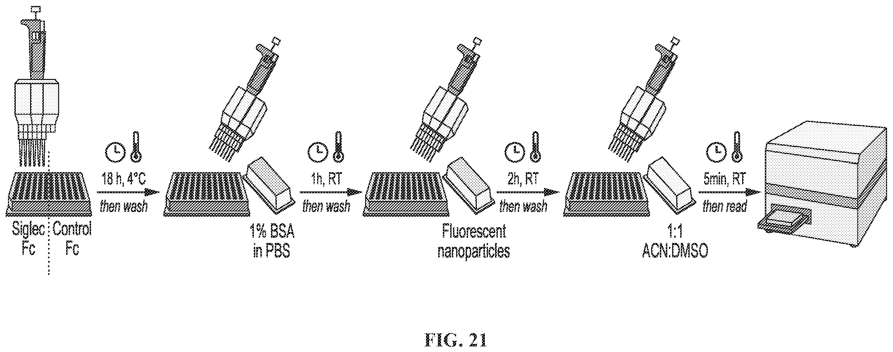

FLISA was next employed to assess binding of the microsphere formulations described in the previous **section 2.1.2.** (i.e. biotinylated S series ligands coupled to FluoSpheres^{™} NeutrAvidin^{™}-labelled microspheres) to Siglec-9 Fc immobilised on a microtiter plate (see FIG. 21). All microsphere formulations incorporated a fluorescent dye, enabling simple measurement of fluorescence as a readout of their binding to immobilised Siglec-9 Fc.

FIG. 21 shows a schematic of FLISA setup. Microtiter plate wells were coated with Siglec-9 Fc or control IgG1 Fc overnight and then washed on the following day. After blocking wells with BSA, a further wash step was performed prior to the addition of fluorescent microsphere formulations of S series ligands. Wells were then washed again and fluorescence was measured as a readout of microsphere binding to Siglec-9 Fc.

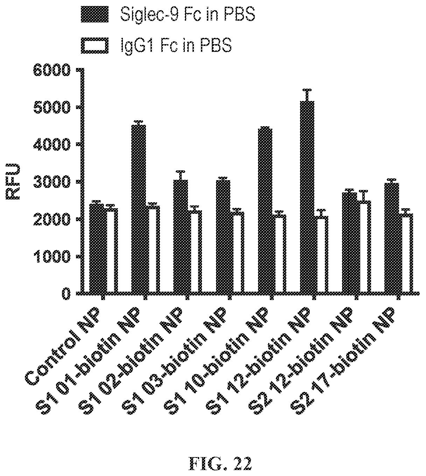

Referring to FIG. 22, all microsphere formulations tested showed a similar level of background binding to IgG1 Fc (FIG. 22, white bars). However, clear differences were observed in the binding of the microsphere formulations to Siglec-9 Fc (FIG. 22, black bars). In particular, S101-biotin NP, S 110-biotin NP and S 112-biotin NP showed superior binding to Siglec-9 Fc. Binding of S102-biotin NP, S103-biotin NP, S212-biotin NP and S217-biotin NP to Siglec-9 Fc was comparatively lower, although still exceeded that observed for control NP that were not functionalised with any ligand.

FIG. 22 is a bar plot showing the results of the FLISA analysis of FluoSpheres^{™} microspheres binding to immobilised Siglec-9 Fc. Fluorescent microsphere formulations of various S series ligands were added to microtiter plate wells coated with Siglec-9 Fc or control IgG1 Fc (FIG. 22, white bars). Wells were then washed and fluorescence was measured as a readout of microsphere binding to Siglec-9 Fc. Data presented as mean + SEM. RFU = relative fluorescence units.

### 2.3. Cell surface binding of Siglec-9-PE antibody +/- PLGA-based nanoparticles

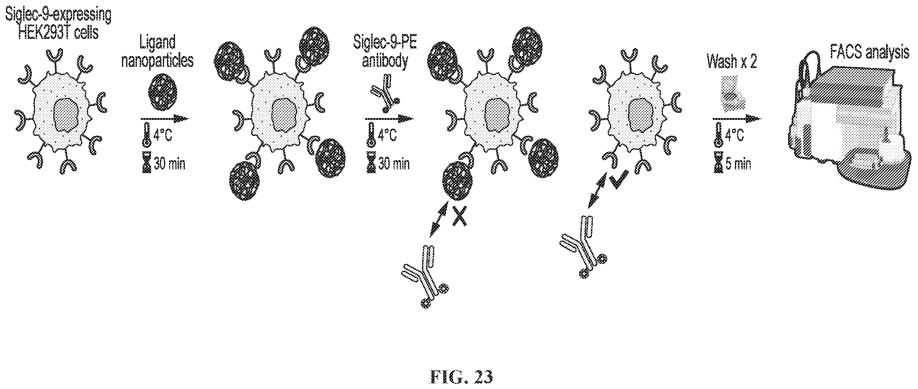

Referring to FIG. 23, in contrast to previous assays where a recombinant Siglec-9 Fc protein was employed, the next studies aimed to investigate whether PLGA-based nanoparticles were similarly capable of binding to cell surface-expressed Siglec-9.

FIG. 23 is a schematic diagram of binding assay setup with Siglec-9-expressing HEK293T cells. Cells were initially pre-incubated with or without S112-azide nanoparticles for 30 mins at 4°C, followed by the addition of a PE-labelled anti-Siglec-9 antibody for a further 30 mins at 4°C. Excess reagents were removed by washing and PE fluorescence was assessed via flow cytometry.

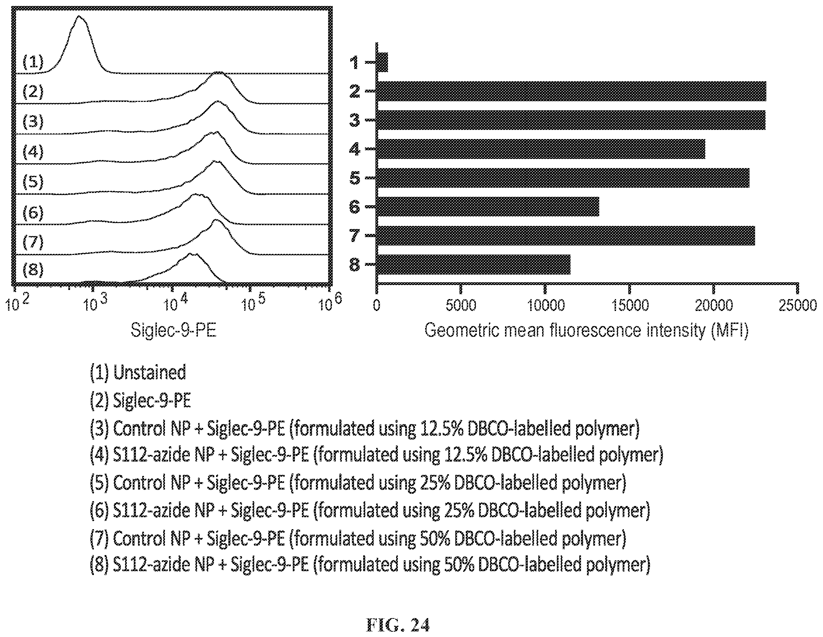

Referring to FIG. 24, for these studies, a cell model was developed via stable transduction of the HEK293T line with lentiviral particles encoding for Siglec-9. Surface expression of Siglec-9 was confirmed by flow cytometry, where an increase in fluorescence was observed upon staining cells with a PE-conjugated Siglec-9 antibody (histogram/bar (2)). To assess whether S112-azide nanoparticles were capable of binding to surface Siglec-9, the cells were first pre-incubated with nanoparticles, prior to staining with the PE-conjugated antibody as before to detect uncomplexed receptor remaining on the cell surface. Pre-incubation of cells with S112-azide nanoparticles led to a reduction in PE fluorescence, which became more pronounced with each increment in the % of PLGA-PEG-DBCO polymer within the nanoformulation (histograms/bars (4), (6) and (8)). In contrast, PE fluorescence was not affected by control non-targeted nanoparticles, remaining similar to levels observed on cells that were stained with Siglec-9 antibody alone without any nanoparticle pre-incubation (histograms/bars (3), (5) and (7)). These findings suggest that S112-azide nanoparticles engage Siglec-9 on the HEK293T cell surface, thereby obstructing binding of the PE-tagged antibody to the receptor. Moreover, the results further highlight the importance of ligand density in dictating the strength of the interaction between Siglec-9 and S 112-azide nanoparticles.

FIG. 24 shows the results of the flow cytometry analysis of Siglec-9-PE binding to Siglec-9-expressing HEK293T cells +/- PLGA-based nanoparticles. Cells were pre-incubated with PLGA-based nanoparticles for 30 mins at 4°C, after which PE-conjugated anti-Siglec-9 antibody was added for a further 30 mins at 4°C. Cells were then washed and PE fluorescence was assessed by flow cytometry.

### Example 10: Example Siglec Ligands

Table 4, below, provides structural formulas of exemplary Siglec ligands of the present disclosure.

For any of the structural formulas listed in Table 4, the variable R can take any one of the following values:
a) -O-(CH₂)₅-NH₂,
b) -O-(CH₂)₅-NH-Fmoc, where Fmoc is the fluorenylmethyloxycarbonyl protecting groups of the structural formula: 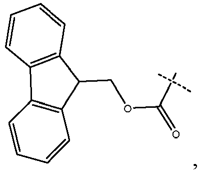
c) -O-(CH₂)₅-N₃, or
d) -O-(CH₂)₅-NH-biotin, where the term "biotin" refers to the moiety represented by the following structural formula: 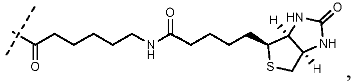
wherein the dash line denotes the point of attachment to the -NH- moiety.

Throughout this disclosure, the compounds of Table 4 may be referred to as "amines," "azides," "Fmoc," or "biotin." For example, S101-amine refers to the compound S101, as shown in Table 4, where R = -O-(CH₂)₅-NH₂. Likewise, S101-azide refers to the compound S101 as shown in Table 4, where R = -O-(CH₂)₅-N₃, and S101-Fmoc, where R = -O-(CH₂)₅-NH-Fmoc.

Although the moiety R is shown in an equatorial position, it is understood that because moiety R is attached to the anomeric carbon, it can occupy either equatorial or axial position. Additionally, although the structural formulas of the compounds in Table 4 are shown as ionized conjugate bases of carboxylic and/or sulfonic acids, sometimes with a metal counterion, it is understood that the listed compounds can exist in the form of an acid or any salt thereof, for example, a pharmaceutically acceptable salt.

**Table 4**

| **Compound** | **Structure** |
|---|---|
| | ***S-series*** |
| **S101** | 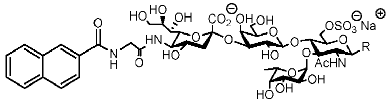 |
| **S102** | 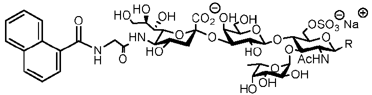 |
| **S103** | 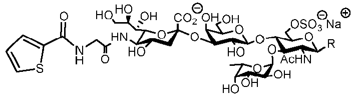 |
| **S104** | 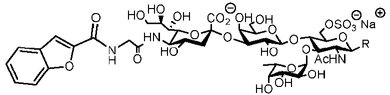 |
| **S105** | 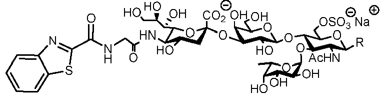 |
| **S106** | 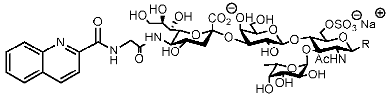 |
| **S107** | 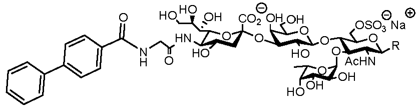 |
| **S108** | 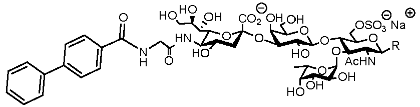 |
| **S109** | 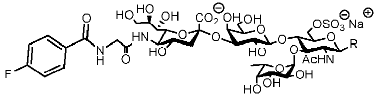 |
| **S110** | 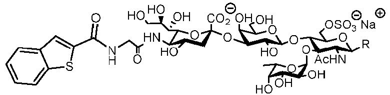 |
| **S111** | 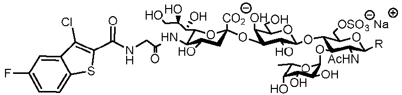 |
| **S112** | 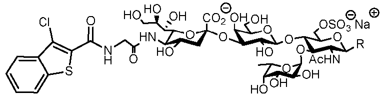 |
| **S113** | 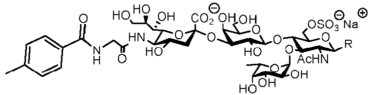 |
| **S114** | 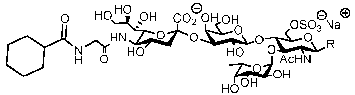 |
| **S115** | 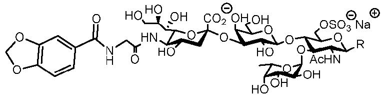 |
| **S116** | 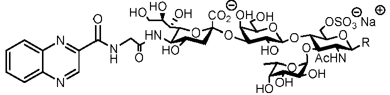 |
| **S117** | 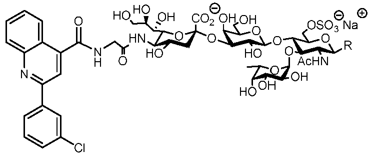 |
| **S118** | 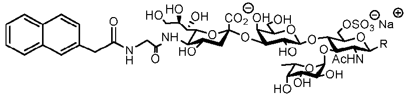 |
| **S119** | 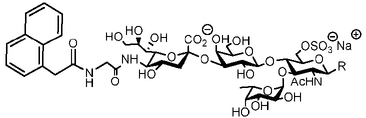 |
| **S120** | 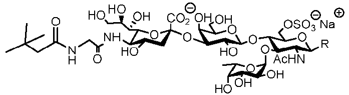 |
| **S121** | 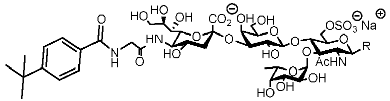 |
| **S122** | 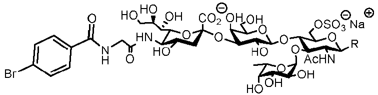 |
| **S123** | 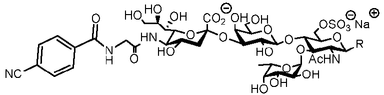 |
| **S124** | 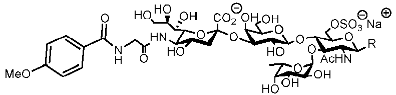 |
| **S125** | 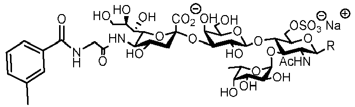 |
| **S126** | 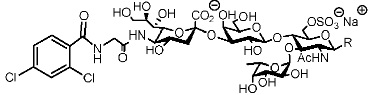 |
| **S127** | 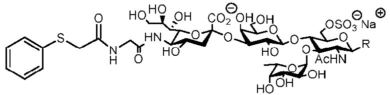 |
| **S128** | 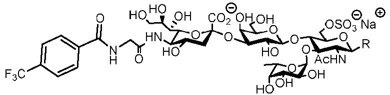 |
| **S129** | 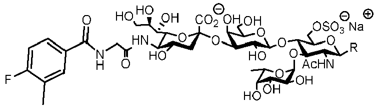 |
| **S130** | 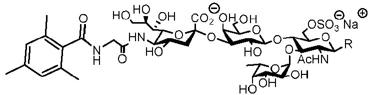 |
| **S131** | 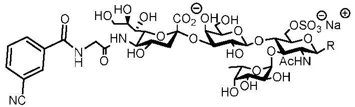 |
| **S201** | 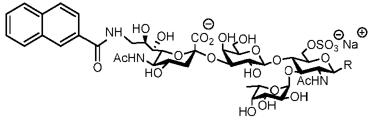 |
| **S202** | 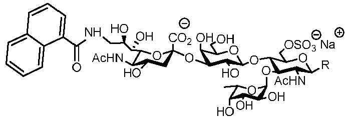 |
| **S203** | 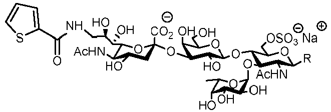 |
| **S204** | 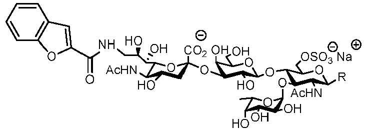 |
| **S205** | 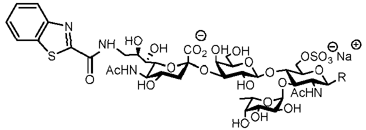 |
| **S206** | 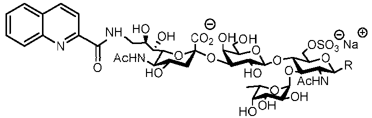 |
| **S207** | 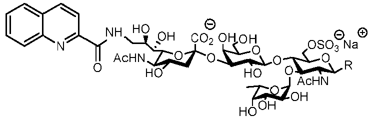 |
| **S208** | 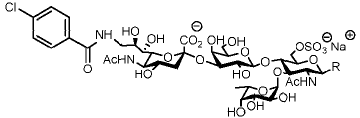 |
| **S209** | 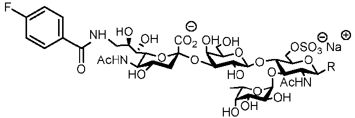 |
| **S210** | 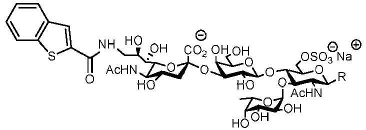 |
| **S211** | 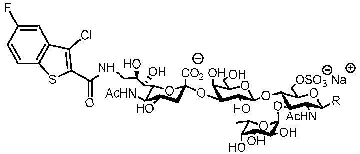 |
| **S212** | 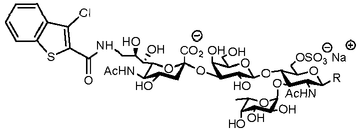 |
| **S213** | 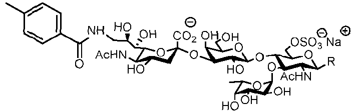 |
| **S214** | 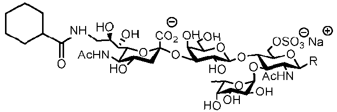 |
| **S215** | 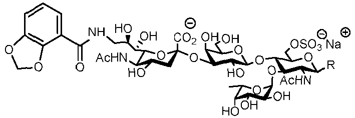 |
| **S216** | 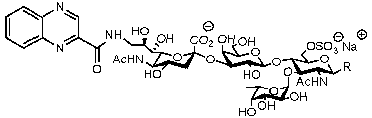 |
| **S217** | 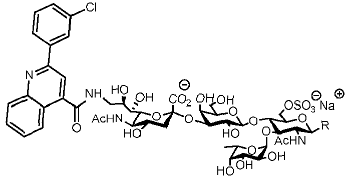 |
| **S218** | 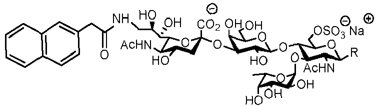 |
| **S219** | 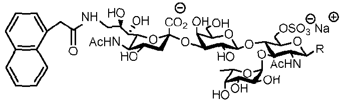 |
| **S220** | 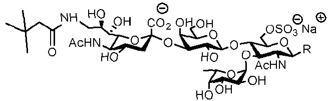 |
| **S221** | 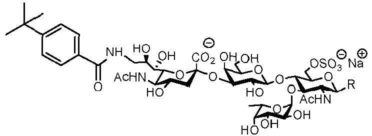 |
| **S222** | 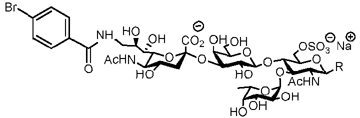 |
| **S223** | 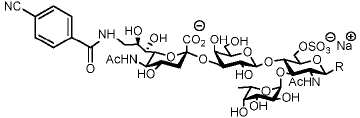 |
| **S224** | 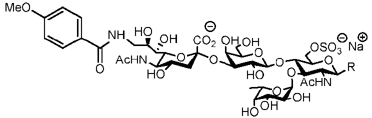 |
| **S225** | 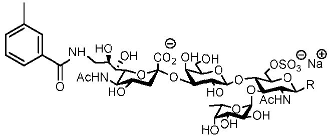 |
| **S226** | 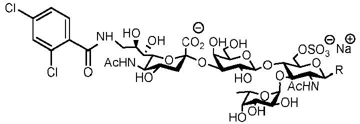 |
| **S227** | 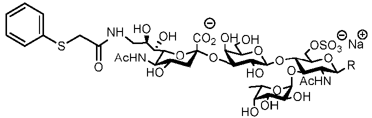 |
| **S228** | 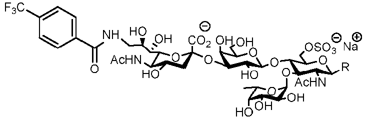 |
| **S229** | 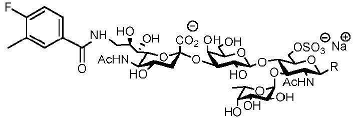 |
| **S230** | 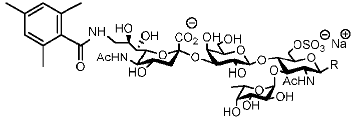 |
| **S231** | 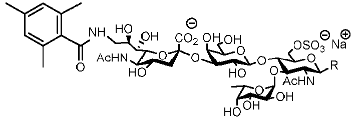 |

| | ***D-series*** |
|---|---|
| **D101** | 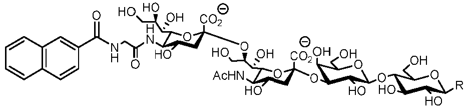 |
| **D102** | 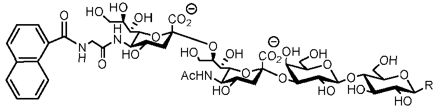 |
| **D103** | 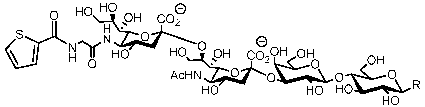 |
| **D104** | 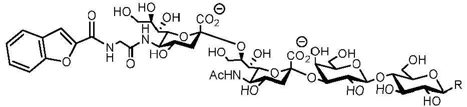 |
| **D105** | 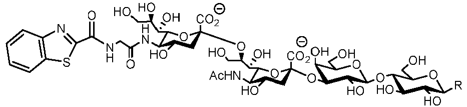 |
| **D106** | 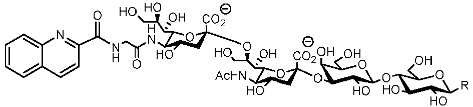 |
| **D107** | 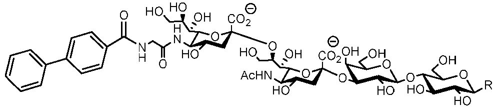 |
| **D108** | 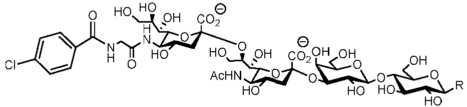 |
| **D109** | 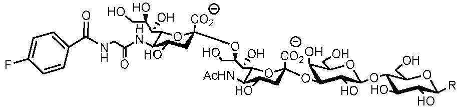 |
| **D110** | 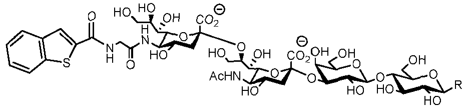 |
| **D111** | 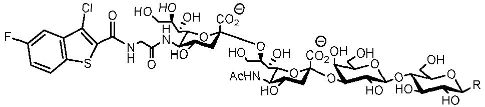 |
| **D112** | 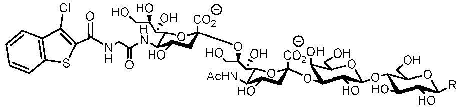 |
| **D113** | 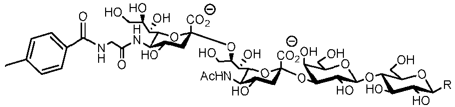 |
| **D114** | 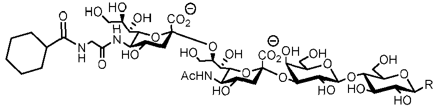 |
| **D115** | 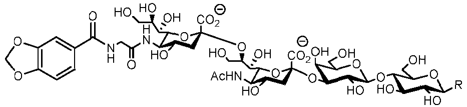 |
| **D116** | 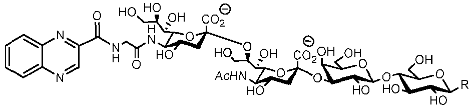 |
| **D117** | 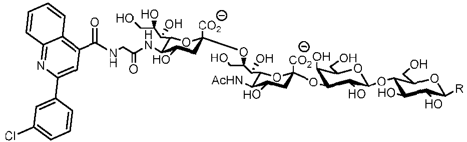 |
| **D118** | 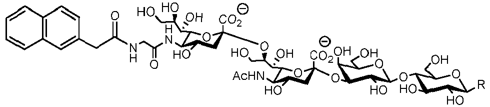 |
| **D119** | 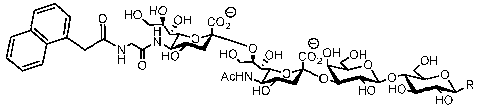 |
| **D120** | 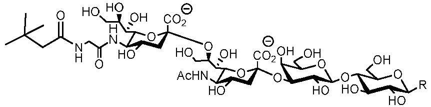 |
| **D121** | 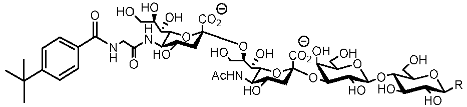 |
| **D122** | 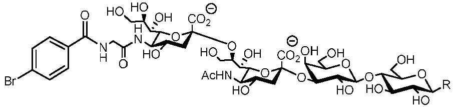 |
| **D123** | 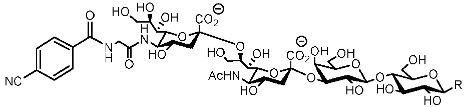 |
| **D124** | 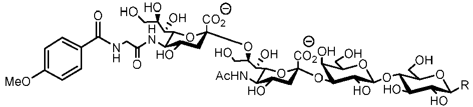 |
| **D125** | 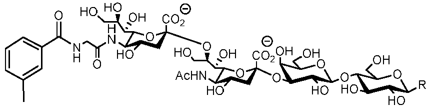 |
| **D126** | 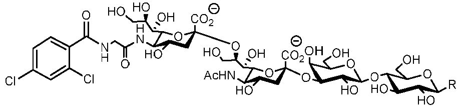 |
| **D127** | 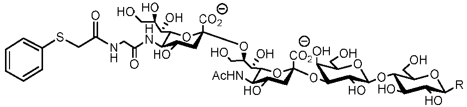 |
| **D128** | 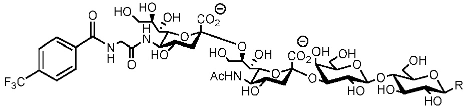 |
| **D129** | 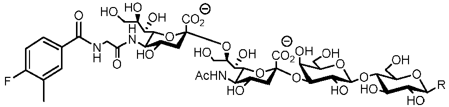 |
| **D130** | 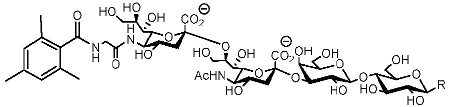 |
| **D131** | 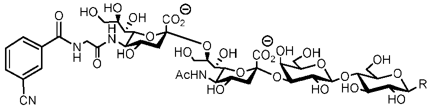 |
| **D201** | 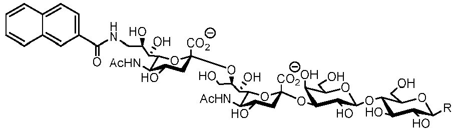 |
| **D202** | 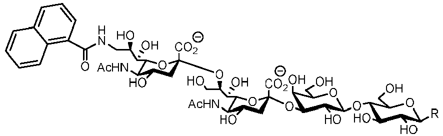 |
| **D203** | 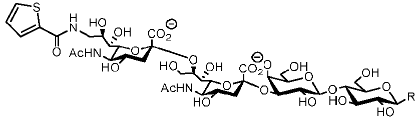 |
| **D204** | 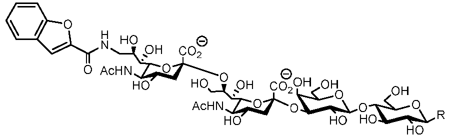 |
| **D205** | 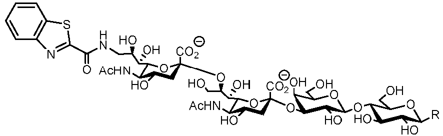 |
| **D206** | 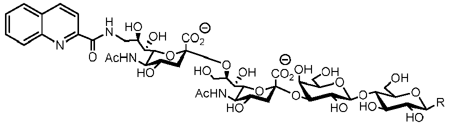 |
| **D207** | 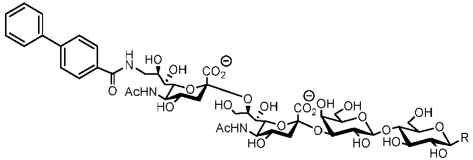 |
| **D208** | 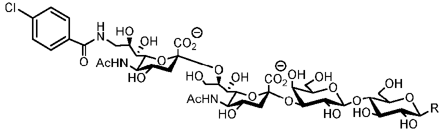 |
| **D209** | 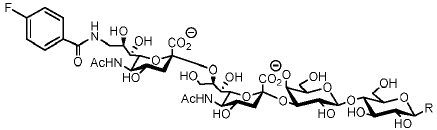 |
| **D210** | 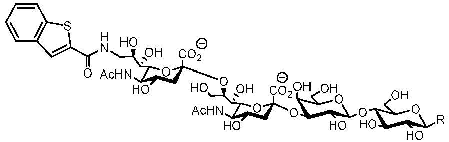 |
| **D211** | 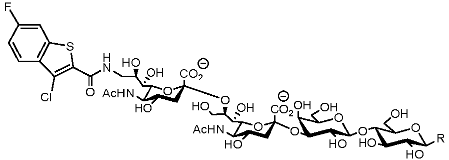 |
| **D212** | 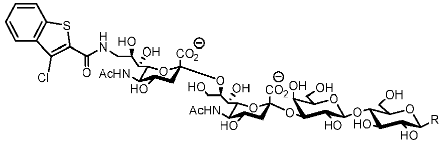 |
| **D213** | 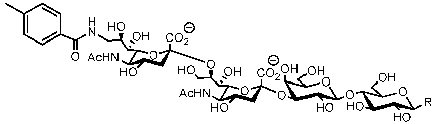 |
| **D214** | 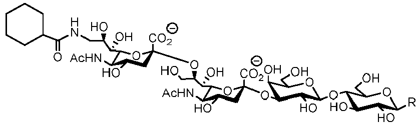 |
| **D215** | 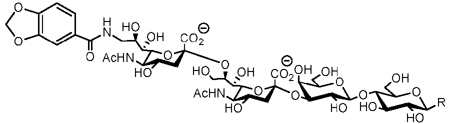 |
| **D216** | 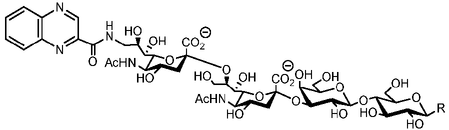 |
| **D217** | 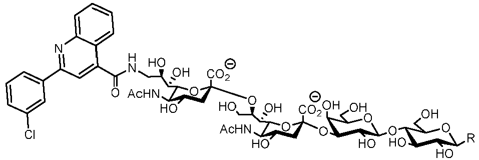 |
| **D218** | 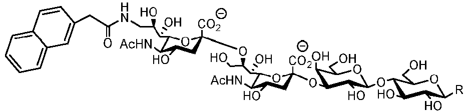 |
| **D219** | 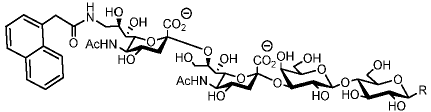 |
| **D220** | 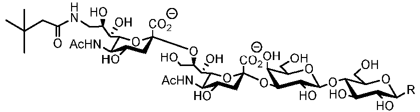 |
| **D221** | 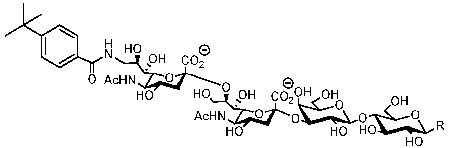 |
| **D222** | 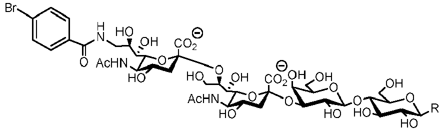 |
| **D223** | 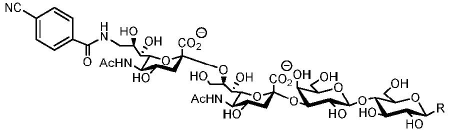 |
| **D224** | 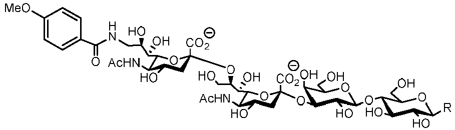 |
| **D225** | 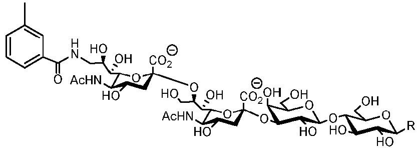 |
| **D226** | 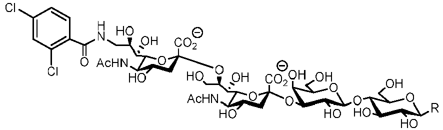 |
| **D227** | 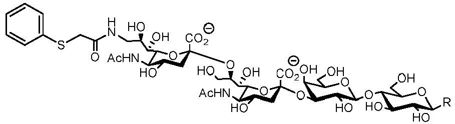 |
| **D228** | 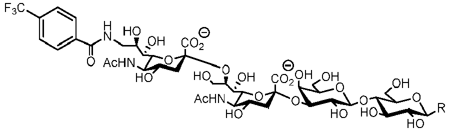 |
| **D229** | 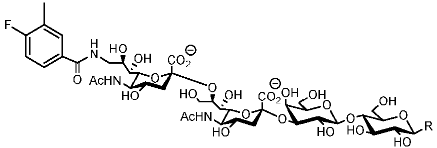 |
| **D230** | 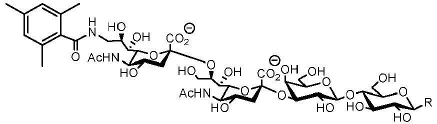 |
| **D231** | 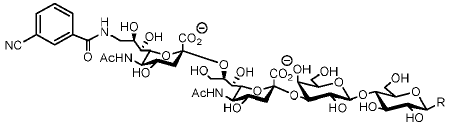 |

## Claims

1. A compound represented by any one of the following structural formulas: 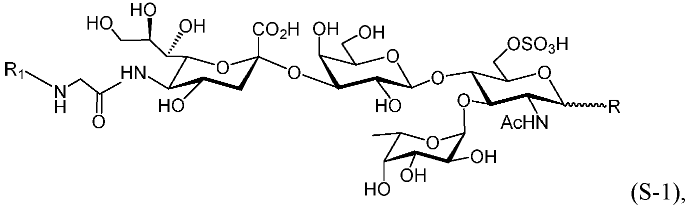
or 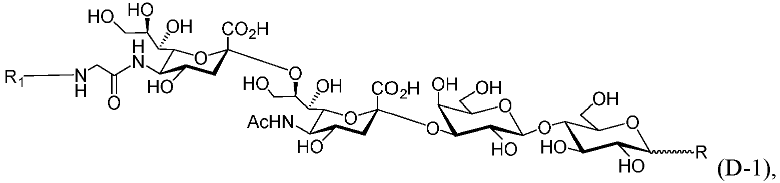
or a pharmaceutically acceptable salt thereof,
wherein:
R₁, for each occurrence independently, is -C(O)-A, wherein A is a C₁-C₆ alkyl, a C₆-C₁₈ aryl, a (C₆-C₁₈)aryl(C₁-C₃)alkyl, a 5-18-member heteroaryl, a (5-18-member)heteroaryl(C₁-C₃)alkyl, a C₃-C₈ cycloalkyl, a (C₃-C₈)cycloalkyl(C₁-C₃)alkyl, a 5-8-member heterocycloalkyl, or a (5-8-member)heterocycloalkyl(C₁-C₃)alkyl;
wherein one or two carbon atoms within the alkyl portion of A is optionally, each independently, replaced with a heteroatom selected from N, O, or S;
and wherein A is optionally substituted with 1 to 3 R¹¹ groups, each said R¹¹ group independently selected from a C₁-C₆ alkyl, a C₁-C₆ haloalkyl, a C₁-C₆ alkoxy, a halogen, a C₆₋C₁₂ aryl, a 5-12-member heteroaryl and cyano, or two groups R¹¹, taken together with the atoms to which they are attached, form a 5-7-member heterocyclyl having 1 to 3 heteroatoms selected from N, O, or S;
wherein R¹¹, each independently, is optionally substituted with 1 to 3 substituents selected from a halogen, a C₁-C₆ alkyl, a C₁-C₆ haloalkyl, a C₁-C₆ alkoxy, or cyano; and and further wherein:
R, for each occurrence independently, is -R^{L}-R^{F}, and wherein:
R^{L} is, for each occurrence independently:
-O-(C₁-C₁₂) alkylenyl-, -O-, -S-, -NR¹⁰⁰-, -S-(C₁-C₁₂) alkylenyl-, -NR¹⁰¹-(C₁-C₁₂) alkylenyl-, -NR^{101a}-O-(C₁-C₁₂)alkylenyl-; -O-(CH₂CH₂O)ₘ-, -O-(CH₂CH₂O)ₖ-(CH₂CH₂)-, -NR¹⁰²-X¹⁰⁰-(C₁-C₁₂) alkylenyl-, -NR^{102a}-NR^{102b}-C(O)-(C₁-C₁₂)alkylenyl, wherein R¹⁰⁰, R¹⁰¹, R^{101a}, R¹⁰², R^{102a}, and R^{102b}, each independently is H or a C₁-C₃ alkyl, and X¹⁰⁰ is -O- or -NH-, and wherein m and k, each independently, is an integer from 1 to 12;
R^{F}, for each occurrence independently, is:
H, a C₁-C₃ alkyl, -NH₂, -NH-Fmoc, -NH-Boc, -NH-CBz, -NH-Troc, -NH-TFA, a mono(C₁-C₃)alkylamino, a di(C₁-C₃ alkyl)amino;
-C(O)-R¹⁰³, wherein R¹⁰³ is -H, -OH, or a (C₁-C₃) alkyl;
-SH, a moiety represented by the following structural formula 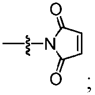 or a click chemistry reagent.

2. The compound of Claim 1, wherein:
(a) the click chemistry reagent comprises an azide, a C₂-C₃ alkyne, a tetrazine, a trans-cyclooctene, or a cyclooctyne, optionally wherein the click chemistry reagent is
(i) an azide, a C₂-C₃ alkyne, or a moiety represented by any one of the following structural formulas: 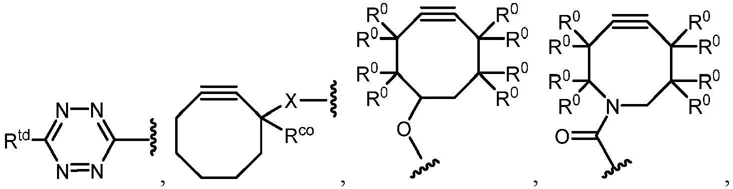 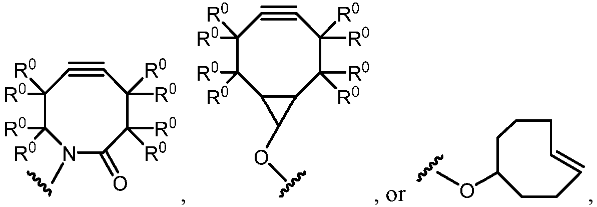 wherein: R^{co} is hydrogen or halogen; X is null or O; and R⁰ is in each case independently selected from hydrogen, halogen, C₁₋₈alkyl, C₁₋₈alkoxy, a C₆-C₁₂ aryl, 5-8 member heteroaryl, C₃₋₈cycloalkyl, or 3-8-member heterocyclyl; wherein any two or more R⁰ groups together with the atoms to which they are attached optionally form an unsaturated, saturated, or aromatic 5-8-member ring; and R^{td} is hydrogen, C₁₋₈ alkyl, C₁₋₈alkoxy, a C₆-C₁₂ aryl, a 5-8-member heteroaryl, C₃₋₈cycloalkyl, or a 3-8 member heterocyclyl, or
(ii) an azide, a C₂-C₃ alkyne, or any one of the moieties represented by the following structural formulas: 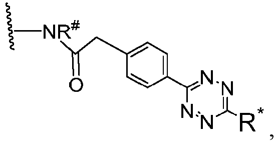 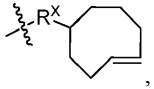 or 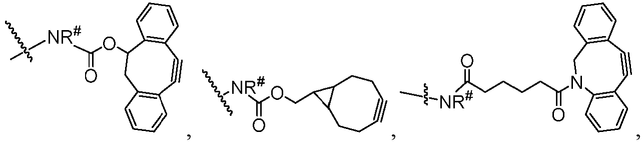 wherein R* is H or methyl, R^{#}, for each occurrence independently, is H or a C₁-C₃ alkyl, and R^{X} is -NH-C(O)O-; or
(b) R, for each occurrence independently, is -O-(CH₂)ₓ-NH₂ or -O-(CH₂)ₓ-NH-Fmoc, wherein x, for each occurrence independently, is an integer from 1 to 10.

3. The compound of Claim 1 or Claim 2, wherein:
(a) the cycloalkyl portion of moiety A is selected from a C₆-C₇ cycloalkyl;
the heterocycloalkyl portion of moiety A is selected from a 5-6-member heterocycloalkyl having 1 or 2 heteroatoms selected from N, O, or S;
the aryl portion of moiety A is selected from a phenyl or a naphthalenyl; and
the heteroaryl portion of moiety A is selected from a moiety represented by any of the following structural formulas: 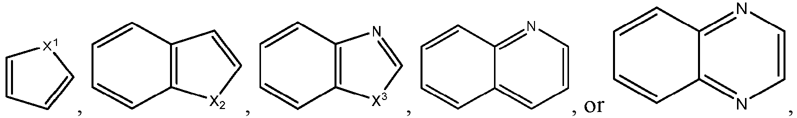 wherein X¹, X² and X³, each independently, is selected from NR^{H}, O, or S, wherein R^{H} is H or a C₁-C₃ alkyl; and further
wherein if present each R¹¹ is independently selected from cyano, a halogen, a phenyl, a halophenyl, a C₁-C₆ alkyl, a C₁-C₆ haloalkyl, a C₁-C₃ alkoxy, or two groups R¹¹, taken together with the atoms to which they are attached, form a [1,3]dioxolo group, optionally substituted with one or two methyl or ethyl groups; and/or
(b) R₁, for each occurrence independently, is selected from the moiety represented by the following structural formulas, wherein the wavy line represents the point of attachment of R₁ to the nitrogen atom:

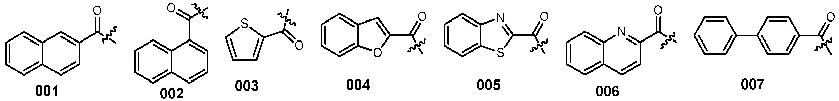

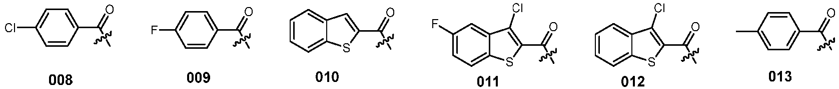

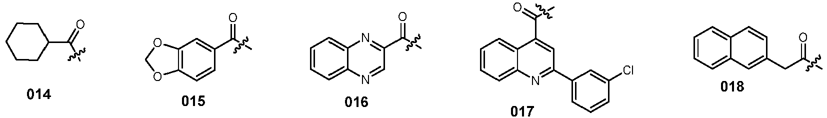

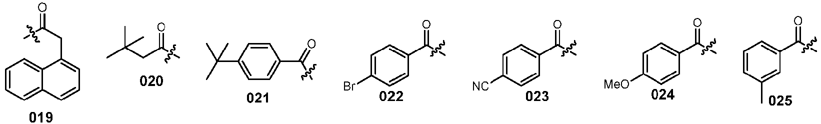

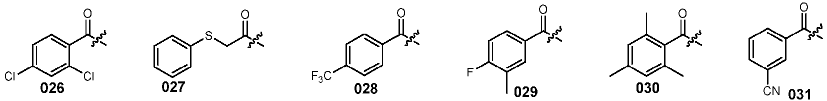

4. The compound of any one of Claims 1-3, wherein the compound is any one of the compounds S-101 through S-131, or D-101 through D-131, wherein the moiety R is:
(a) -O-(CH₂)₅-NH-Fmoc; or
(b) -O-(CH₂)₅-NH₂ or -O-(CH₂)₅-N₃,
| **Compound** | **Structure** |
|---|---|
| | ***S-series*** |
| **S101** | 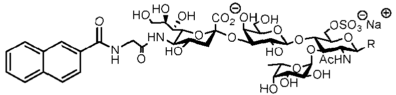 |
| **S102** | 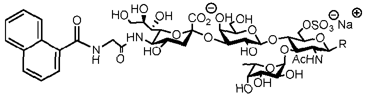 |
| **S103** | 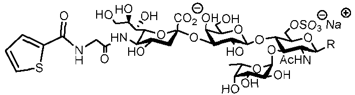 |
| **S104** | 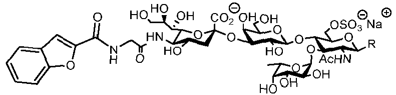 |
| **S105** | 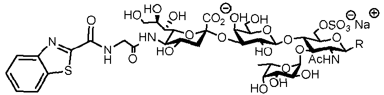 |
| **S106** | 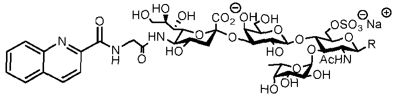 |
| **S107** | 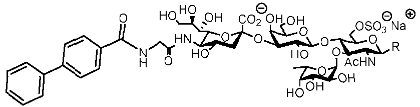 |
| **S108** | 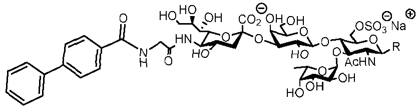 |
| **S109** | 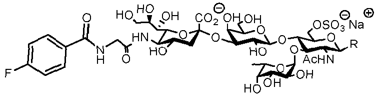 |
| **S110** | 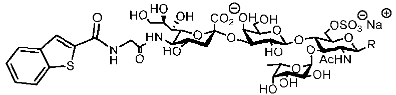 |
| **S111** | 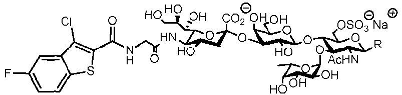 |
| **S112** | 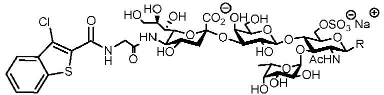 |
| **S113** | 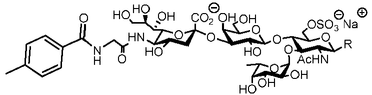 |
| **S114** | 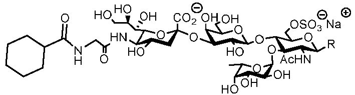 |
| **S115** | 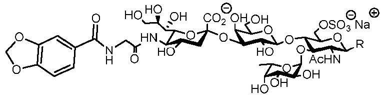 |
| **S116** | 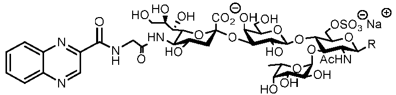 |
| **S117** | 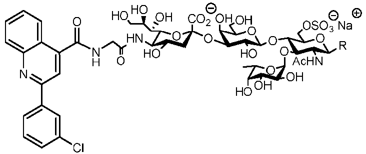 |
| **S118** | 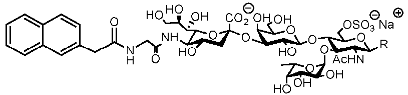 |
| **S119** | 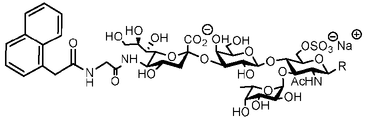 |
| **S120** | 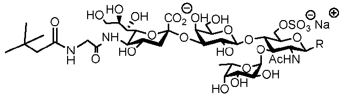 |
| **S121** | 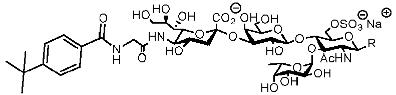 |
| **S122** | 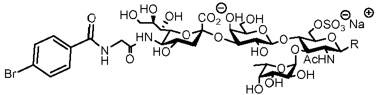 |
| **S123** | 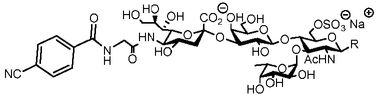 |
| **S124** | 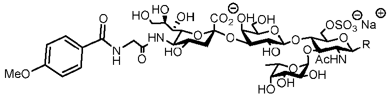 |
| **S125** | 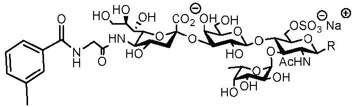 |
| **S126** | 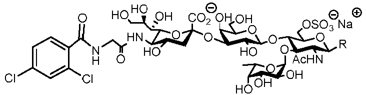 |
| **S127** | 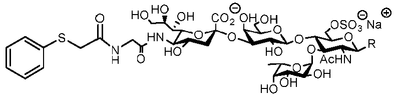 |
| **S128** | 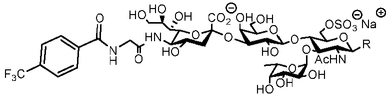 |
| **S129** | 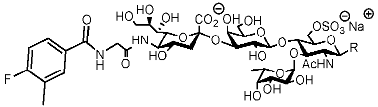 |
| **S130** | 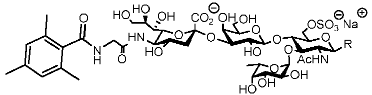 |
| **S131** | 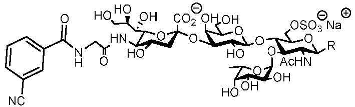 |
| | ***D-series*** |
|---|---|
| **D101** | 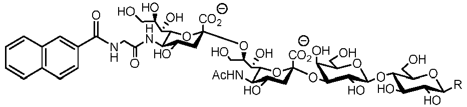 |
| **D102** | 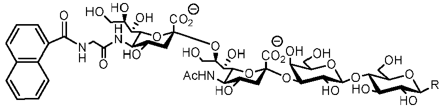 |
| **D103** | 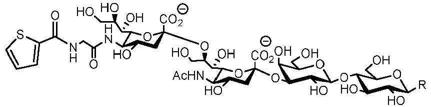 |
| **D104** | 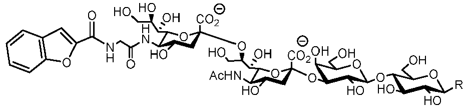 |
| **D105** | 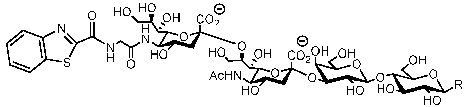 |
| **D106** | 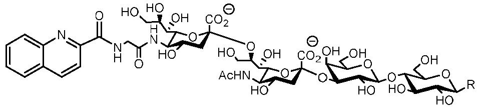 |
| **D107** | 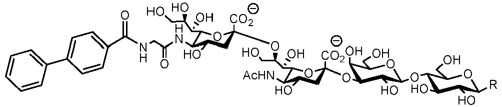 |
| **D108** | 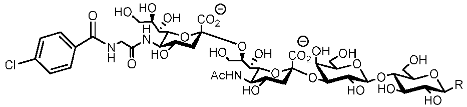 |
| **D109** | 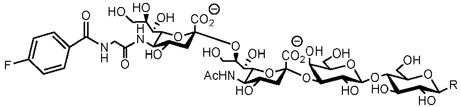 |
| **D110** | 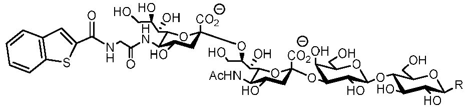 |
| **D111** | 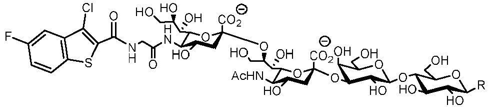 |
| **D112** | 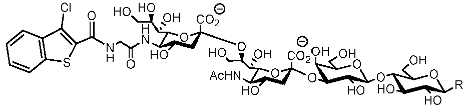 |
| **D113** | 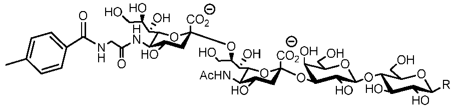 |
| **D114** | 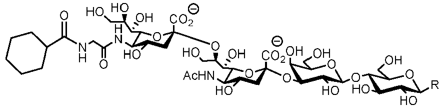 |
| **D115** | 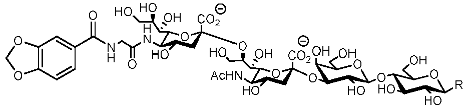 |
| **D116** | 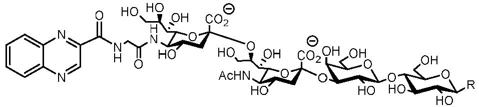 |
| **D117** | 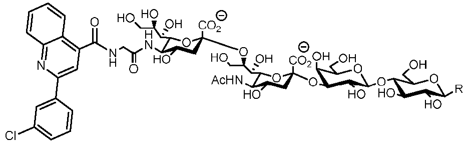 |
| **D118** | 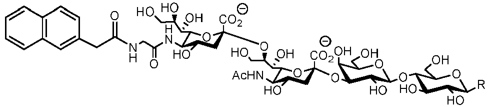 |
| **D119** | 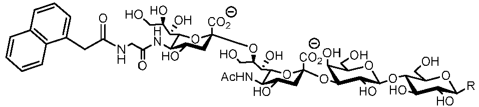 |
| **D120** | 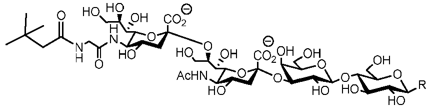 |
| **D121** | 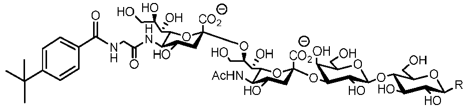 |
| **D122** | 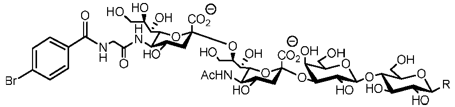 |
| **D123** | 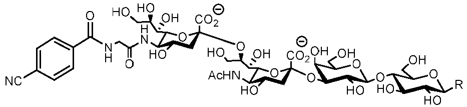 |
| **D124** | 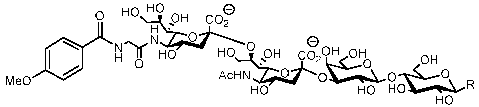 |
| **D125** | 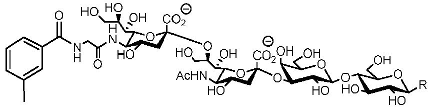 |
| **D126** | 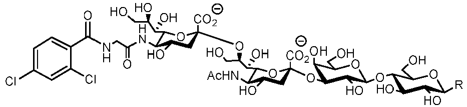 |
| **D127** | 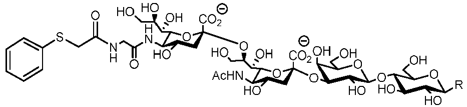 |
| **D128** | 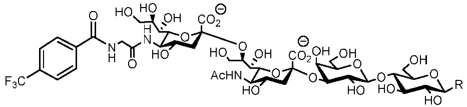 |
| **D129** | 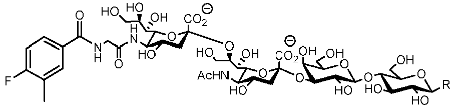 |
| **D130** | 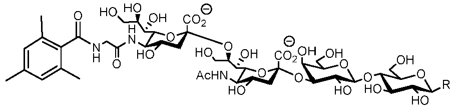 |
| **D131** | 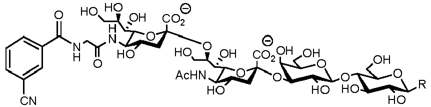 |

5. A compound according to Claim 1 represented by any one of the compounds S-101 through S-131, or D-101 through D-131, as defined in Claim 4, or a pharmaceutically acceptable salt thereof, wherein the variable R is -O-(CH₂)₅-NH₂, -O-(CH₂)₅-NH-Fmoc, -O-(CH₂)₅-N₃, or -O-(CH₂)₅-biotin.

6. The compound of Claim 1, wherein R, for each occurrence independently, is -O-(CH₂)ₓ-N₃, wherein x, for each occurrence independently, is an integer from 1 to 10.

7. A particle, comprising a compound represented by the following structural formula:
G-L-P,
wherein:
P is a biocompatible polymer;
L is a covalent linker; and
G is any one of the moieties represented by the following structural formulas: 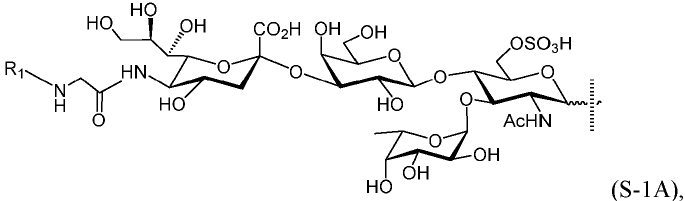 or 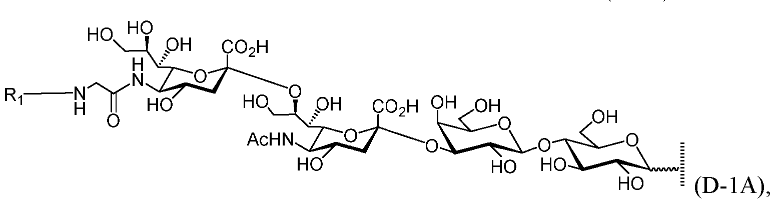 or a pharmaceutically acceptable salt thereof,
wherein:
the symbol 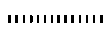 represents the point of attachment to L;
R₁, for each occurrence independently, is -C(O)-A, wherein A is a C₁-C₆ alkyl, a C₆-C₁₈ aryl, a (C₆-C₁₈)aryl(C₁-C₃)alkyl, a 5-18-member heteroaryl, a (5-18-member)heteroaryl(C₁-C₃)alkyl, a C₃-C₈ cycloalkyl, a (C₃-C₈)cycloalkyl(C₁-C₃)alkyl, a 5-8-member heterocycloalkyl, or a (5-8-member)heterocycloalkyl(C₁-C₃)alkyl;
wherein one or two carbon atoms within the alkyl portion of A is optionally, each independently, replaced with a heteroatom selected from N, O, or S;
and wherein A is optionally substituted with 1 to 3 R¹¹ groups, each said R¹¹ group independently selected from a C₁-C₆ alkyl, a C₁-C₆ haloalkyl, a C₁-C₆ alkoxy, a halogen, a C₆₋C₁₂ aryl, a 5-12-member heteroaryl and cyano, or two groups R¹¹, taken together with the atoms to which they are attached, form a 5-7-member heterocyclyl having 1 to 3 heteroatoms selected from N, O, or S;
wherein R¹¹, each independently, is optionally substituted with 1 to 3 substituents selected from a halogen, a C₁-C₆ alkyl, a C₁-C₆ haloalkyl, a C₁-C₆ alkoxy, or cyano,
wherein the biocompatible polymer comprises at least one of polyglycolic acid, poly(lactic acid), poly(lactic-co-glycolic acid), polycaprolactone, poly(3-hydroxybutyric acid), poly(ethylene glycol), polyethylene oxide, poly(ethylene glycol)-block-poly(propylene glycol)-block-poly(ethylene glycol) (Pluronic F127), polyoxyethylene-polyoxypropylene block copolymer (Pluronic F68), poloxamer, poly(hydroxymethylmethacrylate), polyvinyl alcohol, poly(vinylpyrrolidone), hyaluronic acid, heparin, heparin sulfate, polysialic acid and chitosan.

8. The particle of Claims 7, wherein
(a) the biocompatible polymer comprises a copolymer PLGA-PEG; or
(b) P is represented by the following structural formula: 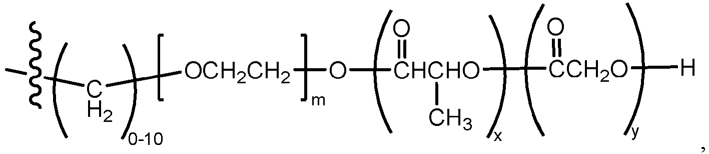 wherein the symbol 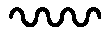 represents the point of attachment of the polymer to the linker L, and further wherein:
y is an integer from 0 to 1000, x is an integer from 0 to 1000, and m is an integer from 0 to 450, provided that x and y are not simultaneously 0, optionally wherein
(i) y is an integer from 0 to 500, x is an integer from 0 to 500, and m is an integer from 0 to 250 or
(ii) x is an integer from 90 to 140, y is an integer from 10 to 75 and m is an integer from 90 to 140.

9. The particle of Claim 7 or Claim 8, wherein the linker L:
(a) includes a portion that is a product of a click chemistry reaction; and/or
(b) comprises a portion represented by any one of the following structural formulas, wherein the symbols ------ and 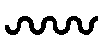 , each independently, represents the point of attachment to additional portions of the linker L, to P, or to G: 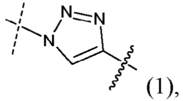 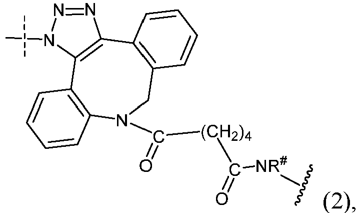 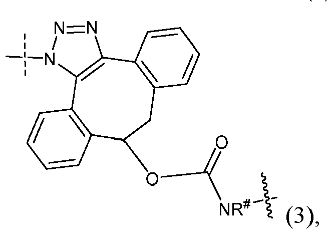 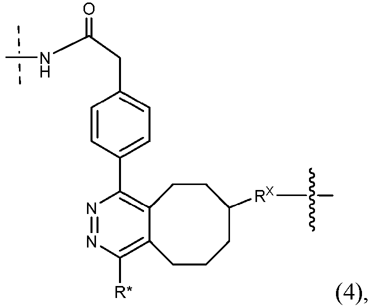 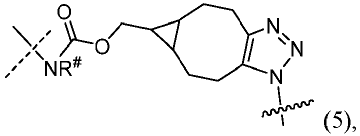 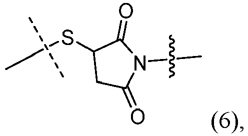 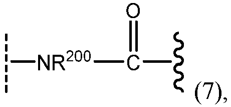
wherein R* is H or methyl, R^{#}, for each occurrence independently, is H or a C₁-C₃ alkyl, and R^{X} is -NH-C(O)O-, and R²⁰⁰ is an -H or a C₁-C₃ alkyl,
optionally wherein the additional portion of the linker is a -O-(C₁-C₁₂) alkylenyl-.

10. The particle of any one of Claims 7-9, wherein the P is a PLGA(10k)-PEG(5k).

11. A particle according to Claim 7 represented by any of the following structural formulas of a pharmaceutically acceptable salts thereof: 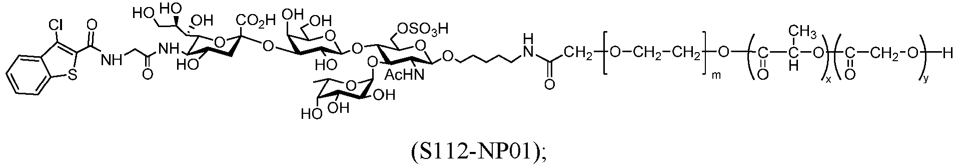 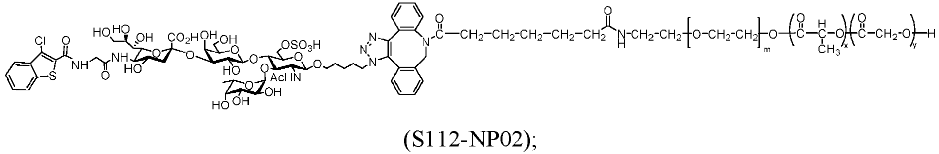 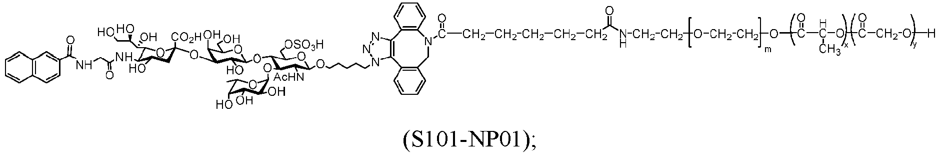 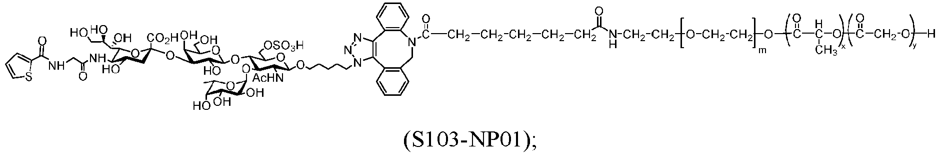 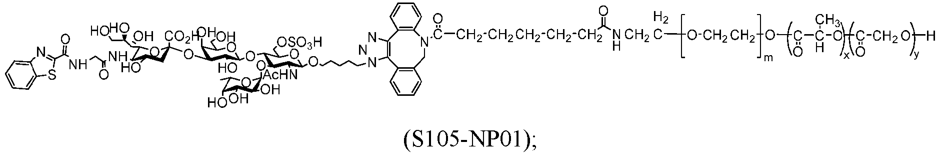 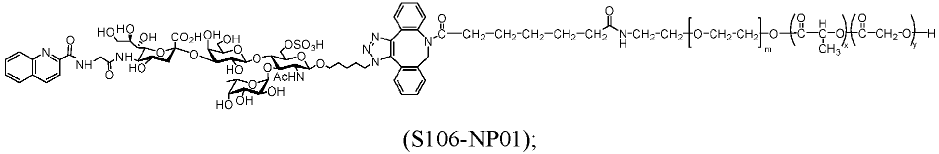 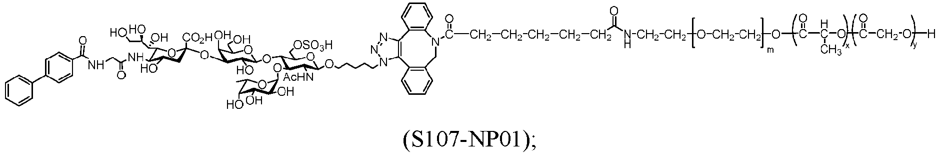 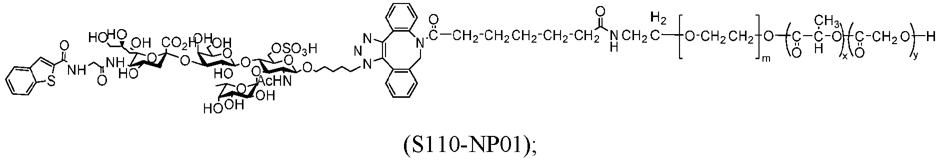 wherein, for each occurrence independently,
x is an integer from 90 to 140;
y is an ineteger from 10 to 75; and
m is an integer from 90 to 140.

12. The particle of Claim 7, wherein:
(a) the biocompatible polymer comprises at least one of polyglycolic acid, poly(lactic acid), poly(lactic-co-glycolic acid), polycaprolactone, poly(3-hydroxybutyric acid), polyethylene oxide, polyoxyethylene-polyoxypropylene block copolymer, poly(hydroxymethylmethacrylate), polyvinyl alcohol, poly(vinylpyrrolidone), hyaluronic acid, heparin, heparin sulfate, polysialic acid and chitosan;
(b) the biocompatible polymer comprises poly(L-lactic acid), poly(D-lactic acid), poly(D/L-lactic acid), a copolymer thereof, or a combination thereof;
(c) the biocompatible polymer comprises polyethylene oxide, polyoxyethylene-polyoxypropylene block copolymer, a copolymer thereof, or a combination thereof; or
(d) the particle further comprises one or more of:
an acid-terminated PLGA (PLGA-COOH);
an acid-terminated PLGA-PEG copolymer, wherein the acid moiety terminates the PEG block (PLGA-PEG-COOH);
an PLGA-PEG copolymer, wherein the PEG block is terminated with the moiety represented by the following structural formula: 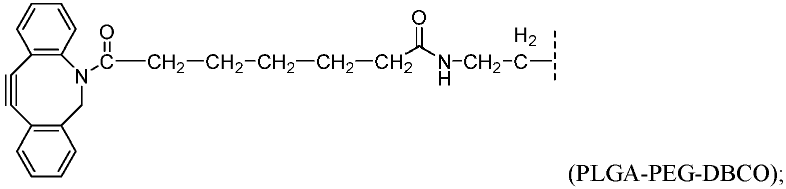 or
an PLGA-PEG copolymer, wherein the PEG block is terminated by the the moiety represented by the following structural formula: 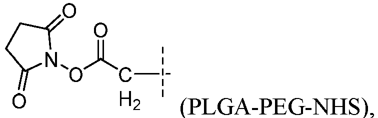 wherein the dash line denotes the point of attachment of the terminating moiety to PEG block, optionally wherein the particle comprises at least one of:
a blend of 75% by weight of PLGA-COOH and 25% by weight of PLGA-PEG-DBCO;
a blend of 75% by weight of PLGA-COOH and 25% by weight of PLGA-PEG-NHS;
a blend of 90% by weight of PLGA-PEG-COOH and 10% by weight of PLGA-PEG-DBCO; and
PLGA-PEG-NHS.

13. The particle of:
(a) any one of Claims 7-10, wherein the weight of G per unit weight of P (ligand density) is from 10 to 75 µg/mg; or
(b) any one of Claims 7-10 and 12, wherein the amount of G per unit weight of the particle is from 1µg/mg to 1000 µg/mg.

14. A compound of any one of Claims 1-4 and 6, or particles of any one of Claims 7-10, 12 and 13 or a pharmaceutically acceptable salt thereof, for use in a method of treating a disorder in a subject in need thereof, the method comprising:
administering to a subject an effective amount of a compound of any one of Claims 1-4 and 6, or particles of any one of Claims 7-10, 12 and 13 or a pharmaceutically acceptable salt threof, wherein the disorder is selected from a cancer, an ophthalmic disease, a fibrotic disease, a parasitic inflammation, a fungal inflammation, a viral inflammation, an autoimmune inflammation, a neurological inflammation, a neurological degeneration, a dermatologic inflammation, a renal inflammation, a cardiovascular disease, a gastrointestinal inflammation, or a rheumatic disease.

15. A pharmaceutical composition, comprising a compound of any one of Claims 1-4 or a particle of any one of Claims 7-10, or a pharmaceutically acceptable salt thereof, in a pharmaceutically acceptable carrier.

## Patentansprüche

1. Verbindung, dargestellt durch eine beliebige der folgenden Strukturformeln: 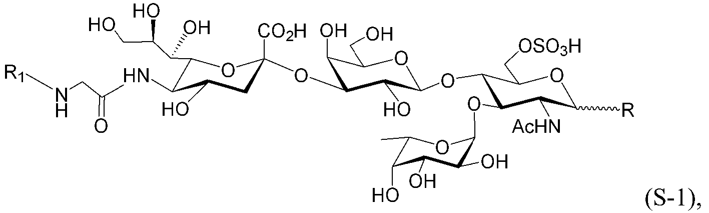
oder 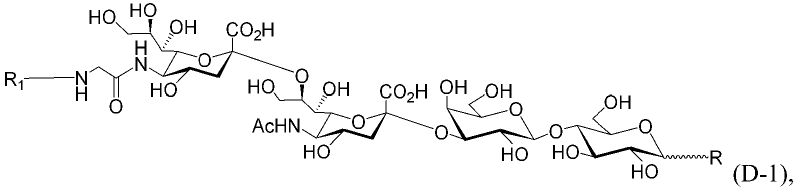
oder pharmazeutisch annehmbares Salz davon,
wobei:
R₁ für jedes Vorkommen unabhängig -C(O)-A ist, wobei A ein C₁-C₆-Alkyl, ein C₆-C₁₈-Aryl, ein (C₆-C₁₈)Aryl(C₁-C₃)alkyl, ein 5-18-gliedriges Heteroaryl, ein (5-18-gliedriges)Heteroaryl(C₁-C₃)alkyl, ein C₃-C₈-Cycloalkyl, ein (C₃-C₈)Cycloalkyl(C₁-C₃)alkyl, ein 5-8-gliedriges Heterocycloalkyl oder ein (5-8-gliedriges)Heterocycloalkyl(C₁-C₃)alkyl ist;
wobei ein oder zwei Kohlenstoffatome innerhalb des Alkylteils von A optional jeweils unabhängig durch ein Heteroatom ausgewählt aus N, O oder S ersetzt sind;
und wobei A optional substituiert ist mit 1 bis 3 R¹¹-Gruppen, jede R¹¹-Gruppe unabhängig ausgewählt aus einem C₁-C₆-Alkyl, einem C₁-C₆-Haloalkyl, einem C₁-C₆-Alkoxy, einem Halogen, einem C₆-C₁₂-Aryl, einem 5-12-gliedrigen Heteroaryl und Cyano, oder zwei Gruppen R¹¹ zusammengenommen mit den Atomen, an die sie gebunden sind, ein 5-7-gliedriges Heterocyclyl mit 1 bis 3 Heteroatomen ausgewählt aus N, O oder S bilden;
wobei R¹¹ jeweils unabhängig optional substituiert ist mit 1 bis 3 Substituenten ausgewählt aus einem Halogen, einem C₁-C₆-Alkyl, einem C₁-C₆-Haloalkyl, einem C₁-C₆-Alkoxy oder Cyano; und wobei ferner:
R für jedes Vorkommen unabhängig -R^{L}-R^{F} ist, und wobei:
R^{L} für jedes Vorkommen unabhängig wie folgt ist:
-O-(C₁-C₁₂)Alkylenyl-, -O-, -S-, -NR¹⁰⁰-, -S-(C₁-C₁₂)Alkylenyl-, -NR¹⁰¹-(C₁-C₁₂)Alkylenyl-, -NR^{101a}-O-(C₁-C₁₂)Alkylenyl-; -O-(CH₂CH₂O)ₘ-, -O-(CH₂CH₂O)ₖ-(CH₂CH₂)-, - NR¹⁰²-X¹⁰⁰-(C₁-C₁₂)Alkylenyl-, -NR^{102a}-NR^{102b}-C(O)-(C₁-C₁₂)Alkylenyl, wobei R¹⁰⁰, R¹⁰¹, R^{101a}, R¹⁰², R^{102a} und R^{102b} jeweils unabhängig H oder ein C₁-C₃-Alkyl sind, und X¹⁰⁰ -O- oder -NH- ist, und wobei m und k jeweils unabhängig eine ganze Zahl von 1 bis 12 ist;
R^{F} für jedes Vorkommen unabhängig wie folgt ist:
H, ein C₁-C₃-Alkyl, -NH₂, -NH-Fmoc, -NH-Boc, -NH-CBz, -NH-Troc, -NH-TFA, ein mono(C₁-C₃)Alkylamino, ein di(C₁-C₃-Alkyl)amino;
-C(O)-R¹⁰³, wobei R¹⁰³ -H, -OH oder ein (C₁-C₃)Alkyl ist;
-SH, eine Einheit dargestellt durch die folgende Strukturformel 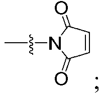 oder ein Click-Chemie-Reagens.

2. Verbindung nach Anspruch 1, wobei:
(a) das Click-Chemie-Reagens ein Azid, ein C₂-C₃-Alkin, ein Tetrazin, ein Transcycloocten oder ein Cyclooctin umfasst, wobei optional das Click-Chemie-Reagens wie folgt ist:
(i) ein Azid, ein C₂-C₃-Alkin oder eine Einheit dargestellt durch eine beliebige der folgenden Strukturformeln: 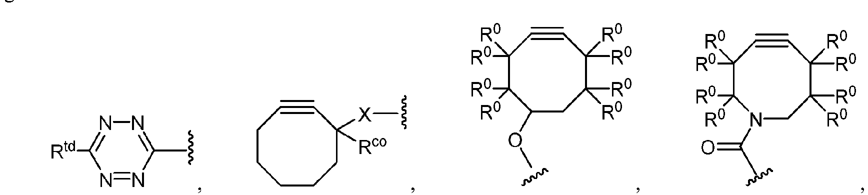 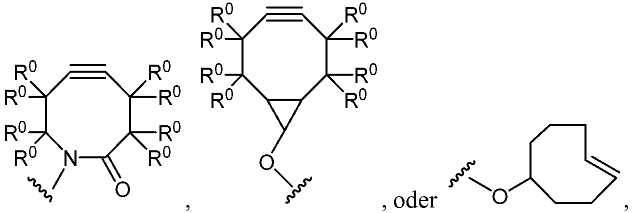 wobei: R^{co} Wasserstoff oder Halogen ist; X null oder O ist; und R⁰ in jedem Fall unabhängig ausgewählt ist aus Wasserstoff, Halogen, C₁₋₈-Alkyl, C₁₋₈-Alkoxy, einem C₆-C₁₂-Aryl, 5-8-gliedrigem Heteroaryl, C₃₋₈-Cycloalkyl oder 3-8-gliedrigem Heterocyclyl; wobei beliebige zwei oder mehr R⁰-Gruppen zusammen mit den Atomen, an die sie gebunden sind, optional einen ungesättigten, gesättigten oder aromatischen 5-8-gliedrigen Ring bilden; und R^{td} Wasserstoff, C₁₋₈-Alkyl, C₁₋₈-Alkoxy, ein C₆-C₁₂-Aryl, ein 5-8-gliedriges Heteroaryl, ein C₃₋₈-Cycloalkyl oder ein 3-8-gliedriges Heterocyclyl ist, oder
(ii) ein Azid, ein C₂-C₃-Alkin oder eine beliebige der Einheiten dargestellt durch die folgenden Strukturformeln: 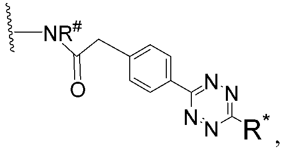 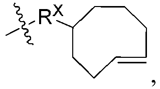 oder 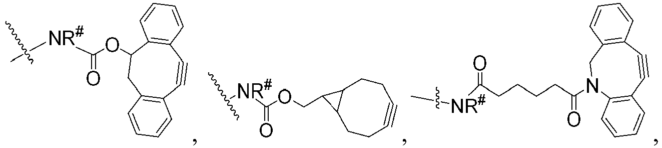 wobei R* H oder Methyl ist, R^{#} für jedes Vorkommen unabhängig H oder ein C₁-C₃-Alkyl ist und R^{X} -NH-C(O)O- ist; oder
(b) R für jedes Vorkommen unabhängig -O-(CH₂)ₓ-NH₂ oder -O-(CH₂)ₓ-NH-Fmoc ist, wobei x für jedes Vorkommen unabhängig eine ganze Zahl von 1 bis 10 ist.

3. Verbindung nach Anspruch 1 oder Anspruch 2, wobei:
(a) der Cycloalkylteil der Einheit A ausgewählt ist aus einem C₆-C₇-Cycloalkyl;
der Heterocycloalkylteil der Einheit A ausgewählt ist aus einem 5-6-gliedrigen Heterocycloalkyl mit 1 oder 2 Heteroatomen ausgewählt aus N, O oder S;
der Arylteil der Einheit A ausgewählt ist aus einem Phenyl oder einem Naphthalenyl; und
der Heteroarylteil der Einheit A ausgewählt ist aus einer Einheit dargestellt durch eine beliebige der folgenden Strukturformeln: 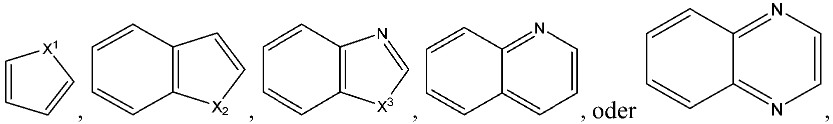
wobei X¹, X² und X³ jeweils unabhängig ausgewählt sind aus NR^{H}, O oder S, wobei R^{H} H oder ein C₁-C₃-Alkyl ist; und ferner
wobei, falls vorhanden, jedes R¹¹ unabhängig ausgewählt ist aus Cyano, einem Halogen, einem Phenyl, einem Halophenyl, einem C₁-C₆-Alkyl, einem C₁-C₆-Haloalkyl, einem C₁-C₃-Alkoxy, oder zwei Gruppen R¹¹ zusammengenommen mit den Atomen, an die sie gebunden sind, eine [1,3]Dioxolo-Gruppe bilden, optional substituiert mit einer oder zwei Methyl- oder Ethylgruppen; und/oder
(b) R₁ für jedes Vorkommen unabhängig ausgewählt ist aus der Einheit dargestellt durch die folgenden Strukturformeln, wobei die Wellenlinie den Bindungspunkt von R₁ an das Stickstoffatom darstellt:

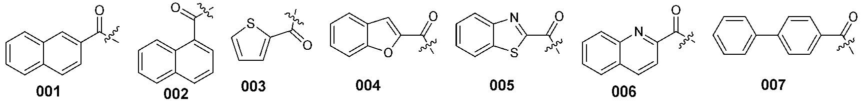

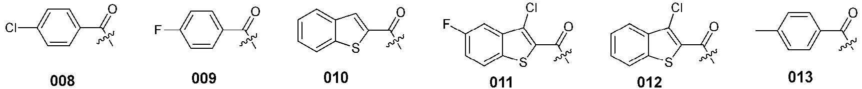

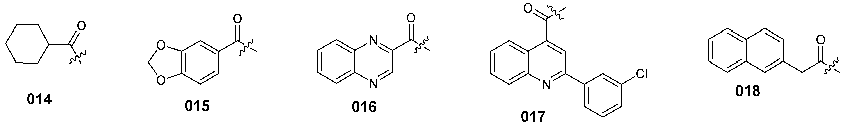

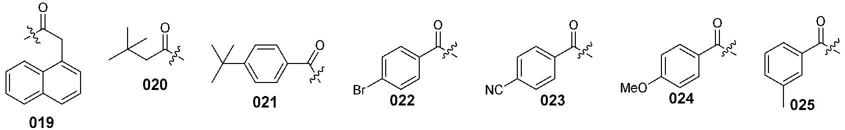

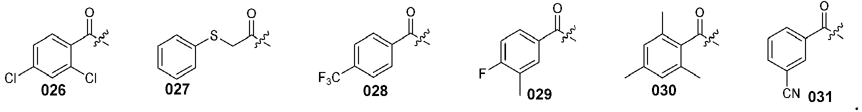

4. Verbindung nach einem der Ansprüche 1-3, wobei die Verbindung eine beliebige der Verbindungen S-101 bis S-131 oder D-101 bis D-131 ist, wobei die Einheit R wie folgt ist:
(a) -O-(CH₂)₅-NH-Fmoc; oder
(b) -O-(CH₂)₅-NH₂ oder -O-(CH₂)₅-N₃,
| **Verbindung** | **Struktur** |
|---|---|
| | ***S-Reihe*** |
| **S101** | 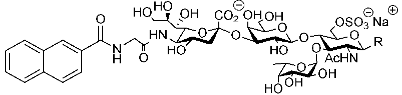 |
| **S102** | 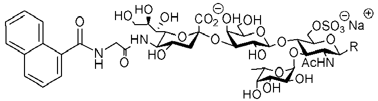 |
| **S103** | 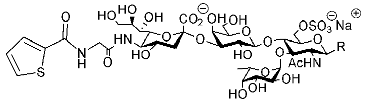 |
| **S104** | 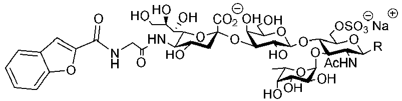 |
| **S105** | 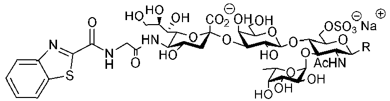 |
| **S106** | 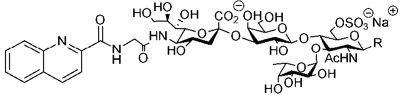 |
| **S107** | 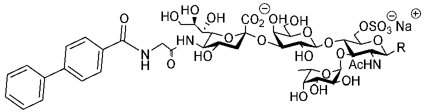 |
| **S108** | 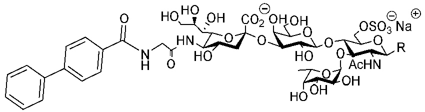 |
| **S109** | 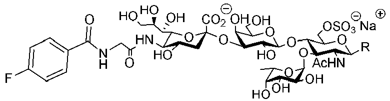 |
| **S110** | 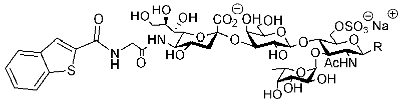 |
| **S111** | 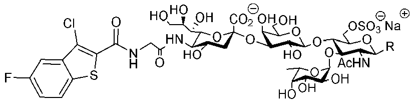 |
| **S112** | 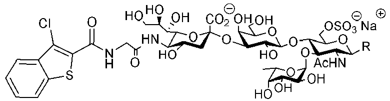 |
| **S113** | 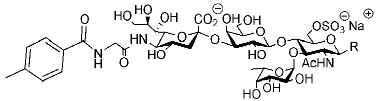 |
| **S114** | 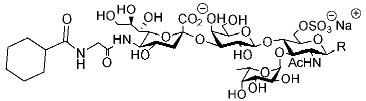 |
| **S115** | 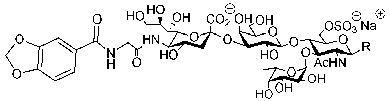 |
| **S116** | 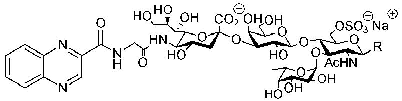 |
| **S117** | 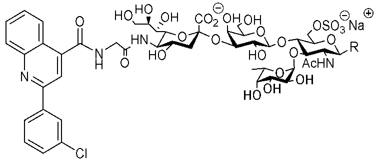 |
| **S118** | 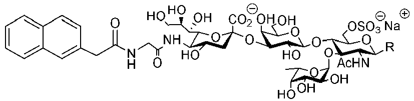 |
| | ***D-Reihe*** |
|---|---|
| **Verbindung** | **Struktur** |
| **S119** | 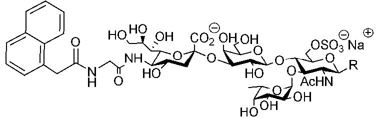 |
| **S120** | 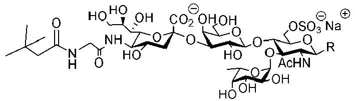 |
| **S121** | 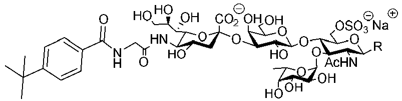 |
| **S122** | 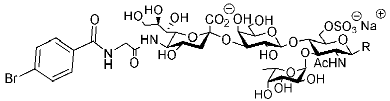 |
| **S123** | 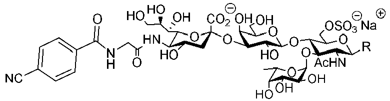 |
| **S124** | 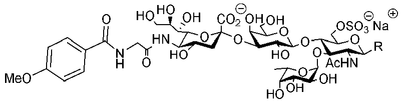 |
| **S125** | 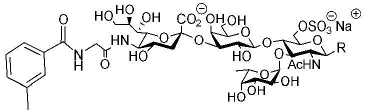 |
| **S126** | 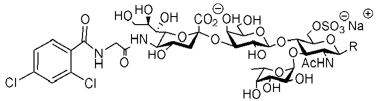 |
| **S127** | 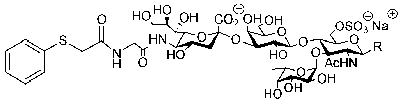 |
| **S128** | 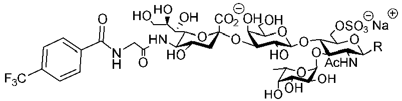 |
| **S129** | 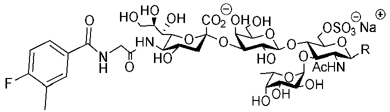 |
| **S130** | 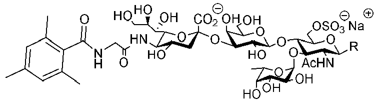 |
| **S131** | 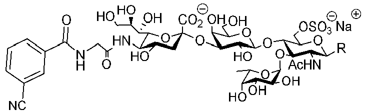 |
| | |
|---|---|
| **D101** | 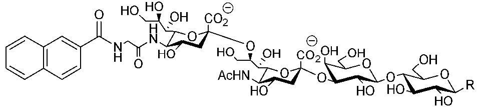 |
| **D102** | 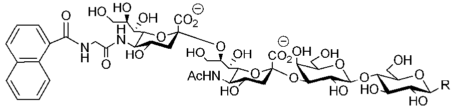 |
| **D103** | 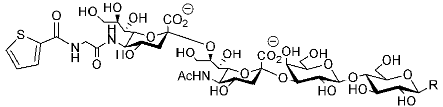 |
| **D104** | 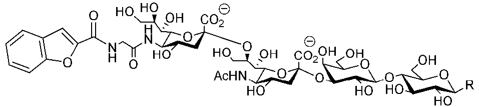 |
| **D105** | 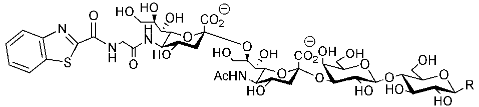 |
| **D106** | 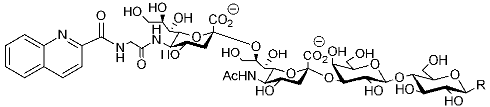 |
| **D107** | 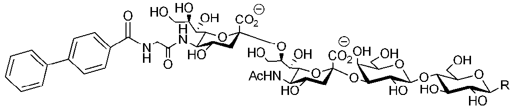 |
| **D108** | 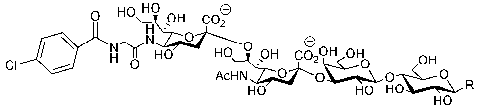 |
| **D109** | 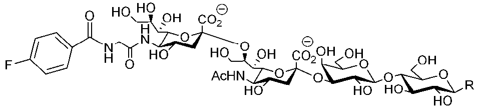 |
| **D110** | 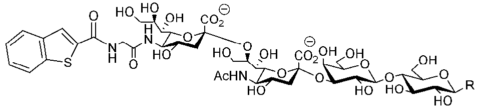 |
| **D111** | 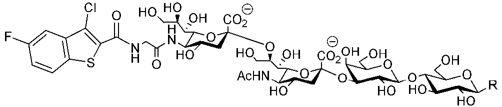 |
| **D112** | 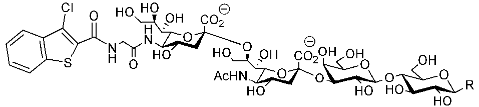 |
| **D113** | 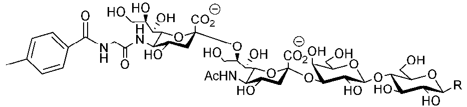 |
| **D114** | 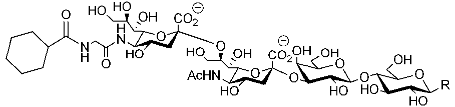 |
| **D115** | 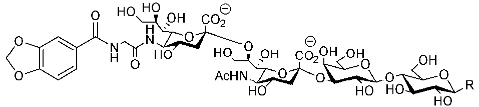 |
| **D116** | 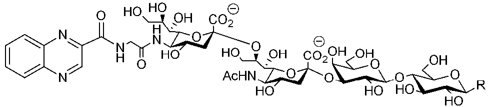 |
| **D117** | 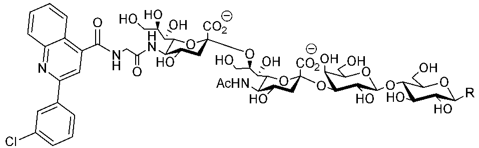 |
| **D118** | 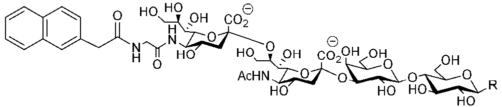 |
| **D119** | 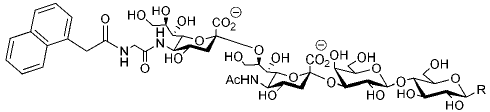 |
| **D120** | 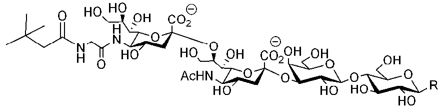 |
| **D121** | 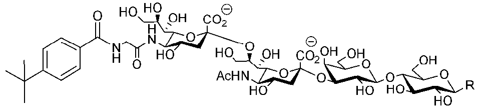 |
| **D122** | 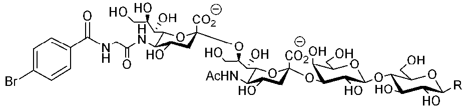 |
| **D123** | 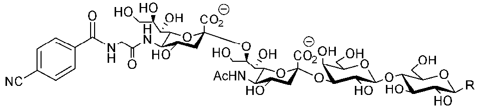 |
| **D124** | 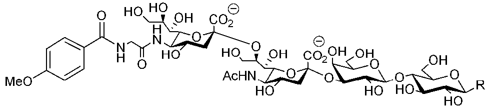 |
| **D125** | 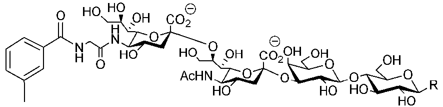 |
| **D126** | 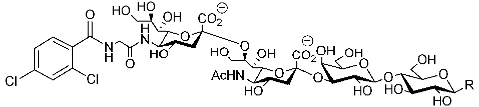 |
| **D127** | 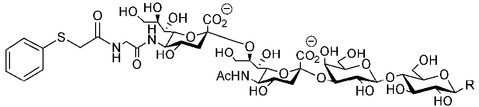 |
| **D128** | 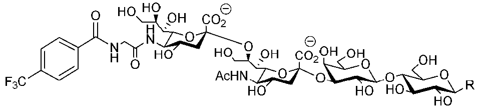 |
| **D129** | 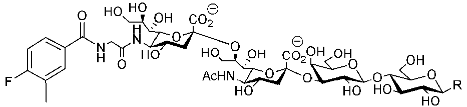 |
| **D130** | 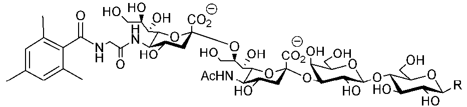 |
| **D131** | 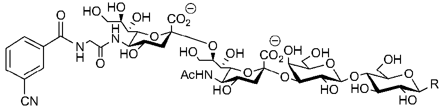 |

5. Verbindung nach Anspruch 1, dargestellt durch eine beliebige der Verbindungen S-101 bis S-131 oder D-101 bis D-131, wie in Anspruch 4 definiert, oder ein pharmazeutisch annehmbares Salz davon, wobei die Variable R -O-(CH₂)₅-NH₂, -O-(CH₂)₅-NH-Fmoc, -O-(CH₂)₅-N₃ oder -O-(CH₂)₅-Biotin ist.

6. Verbindung nach Anspruch 1, wobei R für jedes Vorkommen unabhängig -O-(CH₂)ₓ-N₃ ist, wobei x für jedes Vorkommen unabhängig eine ganze Zahl von 1 bis 10 ist.

7. Partikel, umfassend eine Verbindung dargestellt durch die folgende Strukturformel:
G-L-P,
wobei:
P ein biokompatibles Polymer ist;
L ein kovalenter Linker ist; und
G eine beliebige der Einheiten dargestellt durch die folgenden Strukturformeln ist: 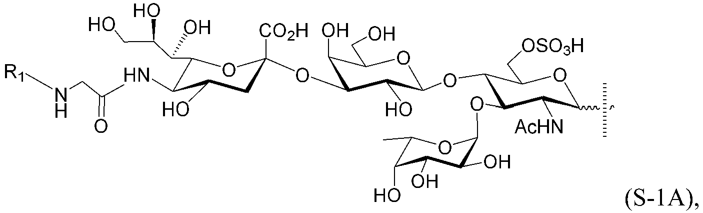 oder 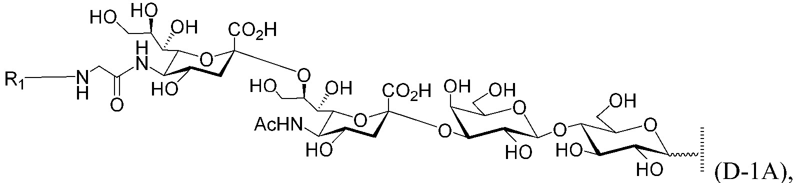
oder pharmazeutisch annehmbares Salz davon,
wobei:
das Symbol 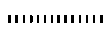 den Bindungspunkt an L darstellt;
R₁ für jedes Vorkommen unabhängig -C(O)-A ist, wobei A ein C₁-C₆-Alkyl, ein C₆-C₁₈-Aryl, ein (C₆-C₁₈)Aryl(C₁-C₃)alkyl, ein 5-18-gliedriges Heteroaryl, ein (5-18-gliedriges)Heteroaryl(C₁-C₃)alkyl, ein C₃-C₈-Cycloalkyl, ein (C₃-C₈)Cycloalkyl(C₁-C₃)alkyl, ein 5-8-gliedriges Heterocycloalkyl oder ein (5-8-gliedriges)Heterocycloalkyl(C₁-C₃)alkyl ist;
wobei ein oder zwei Kohlenstoffatome innerhalb des Alkylteils von A optional jeweils unabhängig durch ein Heteroatom ausgewählt aus N, O oder S ersetzt sind;
und wobei A optional substituiert ist mit 1 bis 3 R¹¹-Gruppen, jede R¹¹-Gruppe unabhängig ausgewählt aus einem C₁-C₆-Alkyl, einem C₁-C₆-Haloalkyl, einem C₁-C₆-Alkoxy, einem Halogen, einem C₆-C₁₂-Aryl, einem 5-12-gliedrigen Heteroaryl und Cyano, oder zwei Gruppen R¹¹ zusammengenommen mit den Atomen, an die sie gebunden sind, ein 5-7-gliedriges Heterocyclyl mit 1 bis 3 Heteroatomen ausgewählt aus N, O oder S bilden;
wobei R¹¹ jeweils unabhängig optional substituiert ist mit 1 bis 3 Substituenten ausgewählt aus einem Halogen, einem C₁-C₆-Alkyl, einem C₁-C₆-Haloalkyl, einem C₁-C₆-Alkoxy oder Cyano, wobei das biokompatible Polymer zumindest eines von Polyglycolsäure, Poly(milchsäure), Poly(milch-co-glycolsäure), Polycaprolacton, Poly(3-hydroxybuttersäure), Poly(ethylenglycol), Polyethylenoxid, Poly(ethylenglycol)-Block-Poly(propylenglycol)-Block-Poly(ethylenglycol) (Pluronic F127), Polyoxyethylenpolyoxypropylen-Block-Copolymer (Pluronic F68), Poloxamer, Poly(hydroxymethylmethacrylat), Polyvinylalkohol, Poly(vinylpyrrolidon), Hyaluronsäure, Heparin, Heparinsulfat, Polysialsäure und Chitosan umfasst.

8. Partikel nach Anspruch 7, wobei
(a) das biokompatible Polymer ein Copolymer PLGA-PEG umfasst; oder
(b) P dargestellt ist durch die folgende Strukturformel: 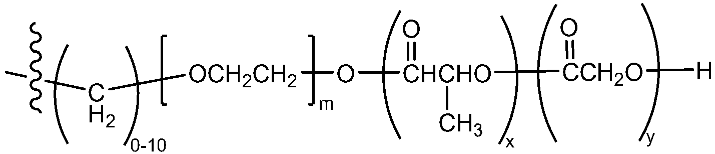 wobei das Symbol 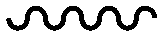 den Bindungspunkt des Polymers an den Linker L darstellt, und wobei ferner:
y eine ganze Zahl von 0 bis 1000 ist, x eine ganze Zahl von 0 bis 1000 ist und m eine ganze Zahl von 0 bis 450 ist, vorausgesetzt, dass x und y nicht gleichzeitig 0 sind, wobei optional
(i) y eine ganze Zahl von 0 bis 500 ist, x eine ganze Zahl von 0 bis 500 ist und m eine ganze Zahl von 0 bis 250 ist, oder
(ii) x eine ganze Zahl von 90 bis 140 ist, y eine ganze Zahl von 10 bis 75 ist und m eine ganze Zahl von 90 bis 140 ist.

9. Partikel nach Anspruch 7 oder Anspruch 8, wobei der Linker L:
(a) einen Teil beinhaltet, der ein Produkt von Click-Chemie-Reaktion ist; und/oder
(b) einen Teil dargestellt durch eine beliebige der folgenden Strukturformeln umfasst, wobei die Symbole ------ und 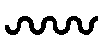 jeweils unabhängig den Bindungspunkt an zusätzliche Teile des Linkers L, an P oder an G darstellen: 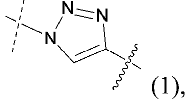 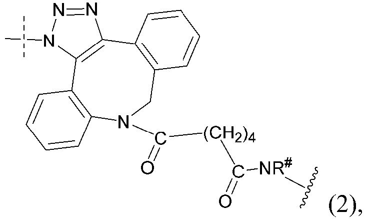 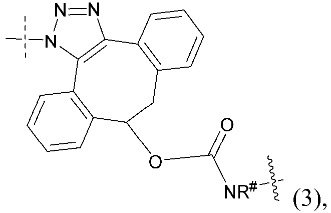 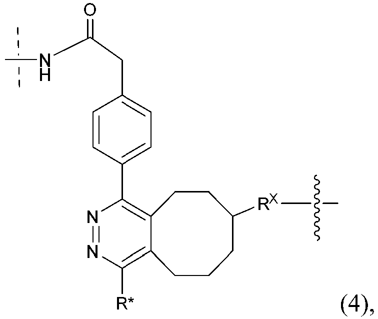 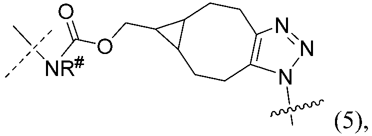 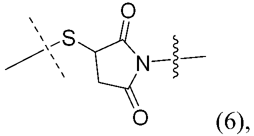 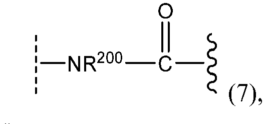
wobei R* H oder Methyl ist, R^{#} für jedes Vorkommen unabhängig H oder ein C₁-C₃-Alkyl ist und R^{x} -NH-C(O)O- ist, und R²⁰⁰ ein -H oder ein C₁-C₃-Alkyl ist,
wobei optional der zusätzliche Teil des Linkers ein -O-(C₁-C₁₂)Alkylenyl- ist.

10. Partikel nach einem der Ansprüche 7-9, wobei das P ein PLGA(10k)-PEG(5k) ist.

11. Partikel nach Anspruch 7, dargestellt durch eine beliebige der folgenden Strukturformeln eines pharmazeutisch annehmbaren Salzes davon: 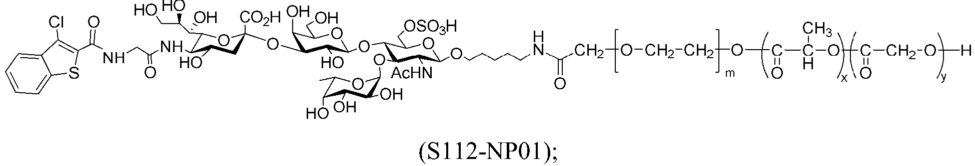 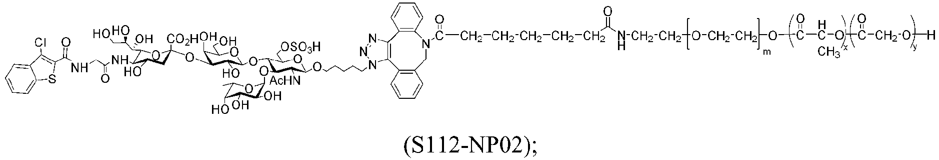 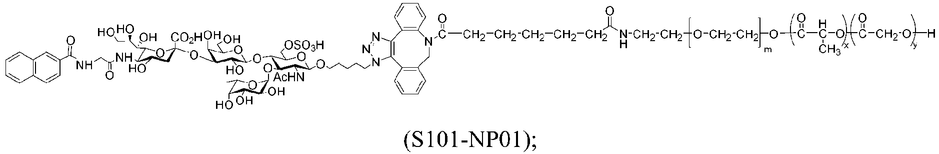 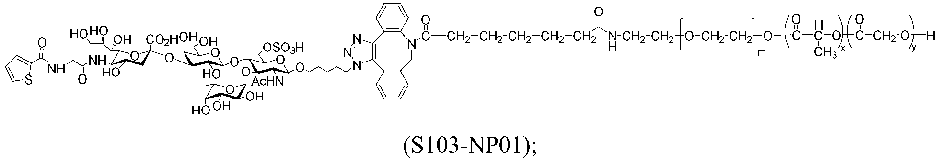 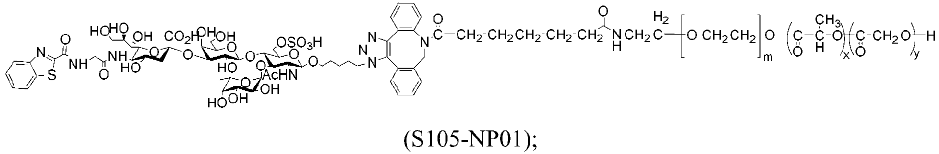 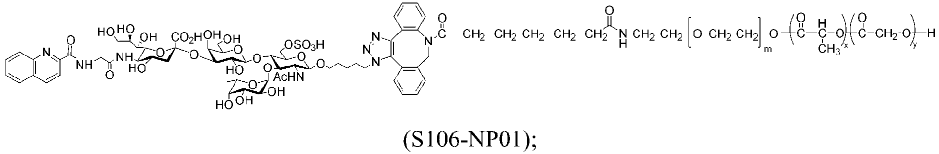 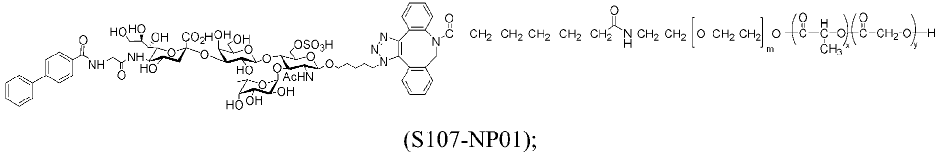 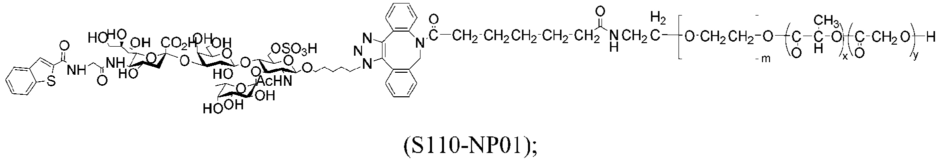
wobei für jedes Vorkommen unabhängig
x eine ganze Zahl von 90 bis 140 ist;
y eine ganze Zahl von 10 bis 75 ist; und
m eine ganze Zahl von 90 bis 140 ist.

12. Partikel nach Anspruch 7, wobei:
(a) das biokompatible Polymer zumindest eines von Polyglycolsäure, Poly(milchsäure), Poly(milch-co-glycolsäure), Polycaprolacton, Poly(3-hydroxybuttersäure), Polyethylenoxid, Polyoxyethylen-Polyoxypropylen-Block-Copolymer, Poly(hydroxymethylmethacrylat), Polyvinylalkohol, Poly(vinylpyrrolidon), Hyaluronsäure, Heparin, Heparinsulfat, Polysialsäure und Chitosan umfasst;
(b) das biokompatible Polymer Poly(L-milchsäure), Poly(D-milchsäure), Poly(D/L-milchsäure), ein Copolymer davon oder eine Kombination davon umfasst;
(c) das biokompatible Polymer Polyethylenoxid, Polyoxyethylenpolyoxypropylen-Block-Copolymer, ein Copolymer davon oder eine Kombination davon umfasst; oder
(d) das Partikel ferner eines oder mehrere von Folgendem umfasst:
einem säureterminierten PLGA (PLGA-COOH);
einem säureterminierten PLGA-PEG-Copolymer, wobei die Säureeinheit den PEG-Block (PLGA-PEG-COOH) terminiert;
einem PLGA-PEG-Copolymer, wobei der PEG-Block mit der Einheit dargestellt durch die folgende Strukturformel terminiert ist: 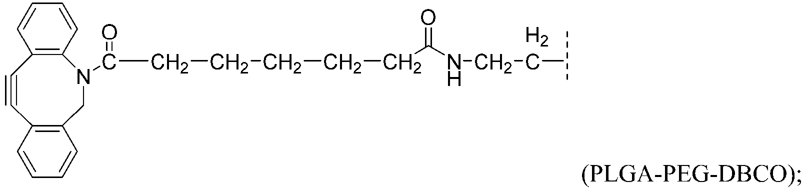 oder
einem PLGA-PEG-Copolymer, wobei der PEG-Block durch die Einheit dargestellt durch die folgende Strukturformel terminiert ist: 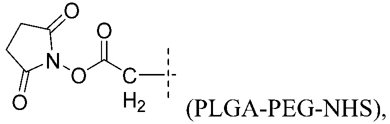
wobei die gestrichelte Linie den Bindungspunkt der terminierenden Einheit an PEG-Block bezeichnet, wobei optional das Partikel zumindest eines von Folgendem umfasst:
einer Mischung aus 75 Gewichts-% PLGA-COOH und 25 Gewichts-% PLGA-PEG-DBCO;
einer Mischung aus 75 Gewichts-% PLGA-COOH und 25 Gewichts-% PLGA-PEG-NHS;
einer Mischung aus 90 Gewichts-% PLGA-PEG-COOH und 10 Gewichts-% PLGA-PEG-DBCO; und
PLGA-PEG-NHS.

13. Partikel nach:
(a) einem der Ansprüche 7-10, wobei das Gewicht von G pro Gewichtseinheit von P (Ligandendichte) von 10 bis 75 µg/mg ist; oder
(b) einem der Ansprüche 7-10 und 12, wobei die Menge von G pro Gewichtseinheit des Partikels von 1 µg/mg bis 1000 µg/mg ist.

14. Verbindung nach einem der Ansprüche 1-4 und 6 oder Partikel nach einem der Ansprüche 7-10, 12 und 13 oder pharmazeutisch annehmbares Salz davon zur Verwendung in einem Verfahren zum Behandeln einer Störung bei einem Subjekt, das dessen bedarf, wobei das Verfahren Folgendes umfasst:
Verabreichen einer wirksamen Menge einer Verbindung nach einem der Ansprüche 1-4 und 6, oder von Partikeln nach einem der Ansprüche 7-10, 12 und 13 oder eines pharmazeutisch annehmbares Salzes davon an ein Subjekt, wobei die Störung ausgewählt ist aus einem Krebs, einer ophthalmischen Erkrankung, einer fibrotischen Erkrankung, einer parasitären Entzündung, einer Pilzentzündung, einer viralen Entzündung, einer autoimmunen Entzündung, einer neurologischen Entzündung, einer neurologischen Degeneration, einer dermatologischen Entzündung, einer renalen Entzündung, einer kardiovaskulären Erkrankung, einer gastrointestinalen Entzündung oder einer rheumatischen Erkrankung.

15. Pharmazeutische Zusammensetzung, umfassend eine Verbindung nach einem der Ansprüche 1-4 oder ein Partikel nach einem der Ansprüche 7-10 oder ein pharmazeutisch annehmbares Salz davon in einem pharmazeutisch annehmbaren Träger.

## Revendications

1. Composé représenté par l'une quelconque des formules structurales suivantes : 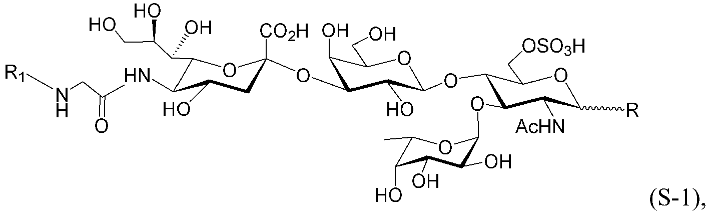 ou 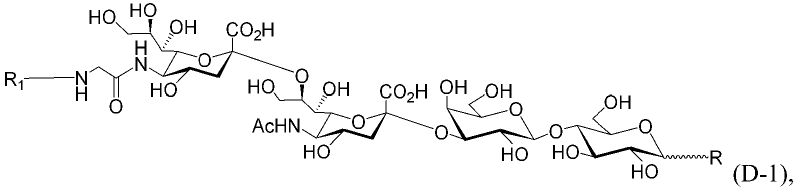
ou sel pharmaceutiquement acceptable de celui-ci,
dans lesquelles :
R₁, indépendamment pour chaque occurrence, représente un groupe -C(O)-A, dans lequel A représente un groupe C₁-C₆ alkyle, un groupe C₆-C₁₈ aryle, un groupe (C₆-C₁₈)aryl(C₁-C₃)alkyle, un groupe hétéroaryle de 5 à 18 chaînons, un groupe (5 à 18 chaînons)hétéroaryl(C₁-C₃)alkyle, un groupe C₃-C₈ cycloalkyle, un groupe (C₃-C₈)cycloalkyl(C₁-C₃)alkyle, un groupe hétérocycloalkyle de 5 à 8 chaînons ou un groupe (5 à 8 chaînons)hétérocycloalkyl(C₁-C₃)alkyle ;
dans lequel un ou deux atomes de carbone dans la partie alkyle de A sont éventuellement, chacun indépendamment, remplacés par un hétéroatome choisi parmi un atome N, O ou S ;
et dans lequel A est éventuellement substitué par 1 à 3 groupes R¹¹, chacun desdits groupes R¹¹ étant indépendamment choisi parmi un groupe C₁-C₆ alkyle, un groupe C₁-C₆ halogénoalkyle, un groupe C₁-C₆ alcoxy, un atome d'halogène, un groupe C₆-C₁₂ aryle, un groupe hétéroaryle de 5 à 12 chaînons et un groupe cyano, ou deux groupes R¹¹, pris ensemble avec les atomes auxquels ils sont fixés, forment un groupe hétérocyclyle de 5 à 7 chaînons comportant 1 à 3 hétéroatomes choisis parmi un atome N, O ou S ;
dans lesquelles R¹¹, chacun indépendamment, est éventuellement substitué par 1 à 3 substituants choisis parmi un atome d'halogène, un groupe C₁-C₆ alkyle, un groupe C₁-C₆ halogénoalkyle, un groupe C₁-C₆ alcoxy ou un groupe cyano ; et en outre dans lesquelles :
R, indépendamment pour chaque occurrence, représente un groupe -R^{L}-R^{F}, et dans lesquelles :
R^{L} représente, indépendamment pour chaque occurrence :
les groupes -O-(C₁-C₁₂)alkylényl-, -O-, -S-, -NR¹⁰⁰-, -S-(C₁-C₁₂)alkylényl-, -NR¹⁰¹-(C₁-C₁₂)alkylényl-, -NR^{101a}-O-(C₁-C₁₂)alkylényl- ; -O-(CH₂CH₂O)ₘ-, -O-(CH₂CH₂O)ₖ-(CH₂CH₂)- , - NR¹⁰²-X¹⁰⁰-(C₁-C₁₂)alkylényl-, -NR^{102a}-NR^{102b}-C(O)-(C₁-C₁₂)alkylényle, dans lesquels R¹⁰⁰, R¹⁰¹, R^{101a}, R¹⁰², R^{102a} et R^{102b}, représentent chacun indépendamment un atome H ou un groupe C₁-C₃ alkyle, et X¹⁰⁰représente un groupe -O- ou -NH-, et dans lesquels m et k, chacun indépendamment, représente un entier de 1 à 12 ;
R^{F}, indépendamment pour chaque occurrence, représente :
un atome H, les groupes C₁-C₃ alkyle, -NH₂, -NH-Fmoc, -NH-Boc, -NH-CBz, -NH-Troc, -NH-TFA, un mono(C₁-C₃)alkylamino, un di(C₁-C₃ alkyl)amino ;
-C(O)-R¹⁰³, dans lequel R¹⁰³ représente un groupe -H, -OH ou un groupe (C₁-C₃) alkyle ;
-SH, un groupement représenté par la formule structurale suivante 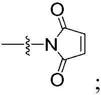 ou un réactif de chimie clic.

2. Composé selon la revendication 1 :
(a) ledit réactif de chimie clic comprenant un groupe azoture, un groupe C₂-C₃ alcyne, un groupe tétrazine, un groupe transcyclooctène ou un groupe cyclooctyne, éventuellement ledit réactif de chimie clic étant
(i) un groupe azoture, un groupe C₂-C₃ alcyne ou un groupement représenté par l'une quelconque des formules structurales suivantes : 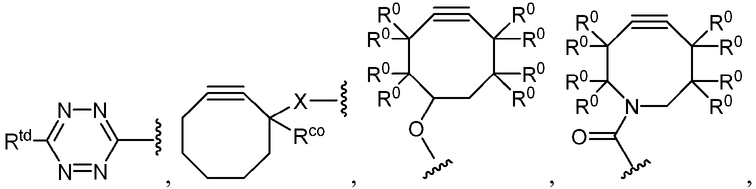 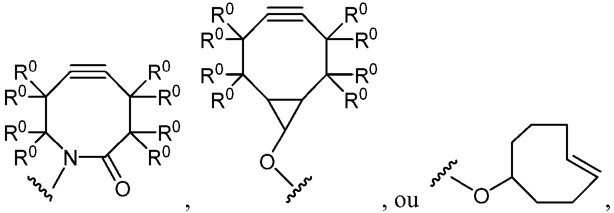 dans lesquelles : R^{co} représente un atome d'hydrogène ou un atome d'halogène ; X est nul ou représente un atome O ; et R⁰ est dans chaque cas indépendamment choisi parmi un atome d'hydrogène, un atome d'halogène, un groupe C₁₋₈ alkyle, un groupe C₁₋₃ alcoxy, un groupe C₆-C₁₂ aryle, un groupe hétéroaryle de 5 à 8 chaînons, un groupe C₃₋₈ cycloalkyle ou un groupe hétérocyclyle de 3 à 8 chaînons ; dans lesquelles deux groupes R⁰ quelconques ou plus avec les atomes auxquels ils sont liés forment éventuellement un cycle insaturé, saturé ou aromatique de 5 à 8 chaînons ; et R^{td} représente un atome d'hydrogène, un groupe C₁₋₈ alkyle, un groupe C₁₋₈ alcoxy, un groupe C₆-C₁₂ aryle, un groupe hétéroaryle de 5 à 8 chaînons, un groupe cycloalkyle ou un groupe hétérocyclyle de 3 à 8 chaînons, ou
(ii) un groupe azoture, un groupe C₂-C₃ alcyne, ou l'un quelconque des groupements représentés par les formules structurales suivantes : 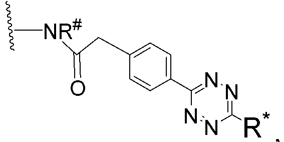 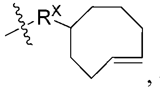 ou 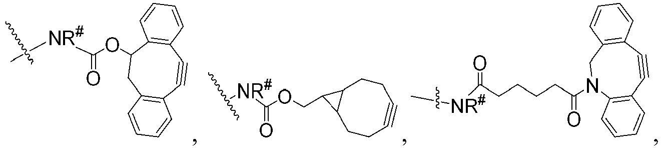 dans lesquelles R* représente un atome H ou un groupe méthyle, R^{#}, indépendamment pour chaque occurrence, représente un atome H ou un groupe C₁-C₃ alkyle, et R^{X} représente un groupe -NH-C(O)O- ; ou
(b) R, indépendamment pour chaque occurrence, représente un groupe -O-(CH₂)ₓ-NH₂ ou -O-(CH₂)ₓ-NH-Fmoc, dans lequel x, indépendamment pour chaque occurrence, représente un entier de 1 à 10.

3. Composé selon la revendication 1 ou la revendication 2 :
(a) ladite partie cycloalkyle du groupement A étant choisie parmi un groupe C₆-C₇ cycloalkyle ;
ladite partie hétérocycloalkyle du groupement A étant choisie parmi un groupe hétérocycloalkyle de 5 à 6 chaînons comportant 1 ou 2 hétéroatomes choisis parmi un atome N, O ou S ;
ladite partie aryle du groupement A étant choisie parmi un groupe phényle ou un groupe naphtalényle ; et
ladite partie hétéroaryle du groupement A étant choisie parmi un groupement représenté par l'une quelconque des formules structurales suivantes : 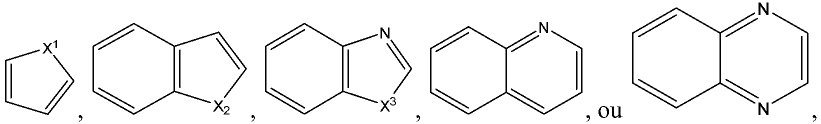
dans lesquelles X¹, X² et X³, chacun indépendamment, sont choisis parmi un groupe NR^{H}, un atome O ou S, dans laquelle R^{H} représente un atome H ou un groupe C₁-C₃ alkyle ; et en outre
dans lesquelles, s'ils sont présents, chaque R¹¹ est indépendamment choisi parmi un groupe cyano, un atome d'halogène, un groupe phényle, un groupe halogénophényle, un groupe C₁-C₆ alkyle, un groupe C₁-C₆ halogénoalkyle, un groupe C₁-C₃ alcoxy ou deux groupes R¹¹, pris ensemble avec les atomes auxquels ils sont fixés, forment un groupe [1,3]dioxolo, éventuellement substitué par un ou deux groupes méthyle ou éthyle ; et/ou
(b) R₁, indépendamment pour chaque occurrence, étant choisi parmi le groupement représenté par les formules structurales suivantes, dans lesquelles la ligne ondulée représente le point de fixation de R₁ à l'atome d'azote :

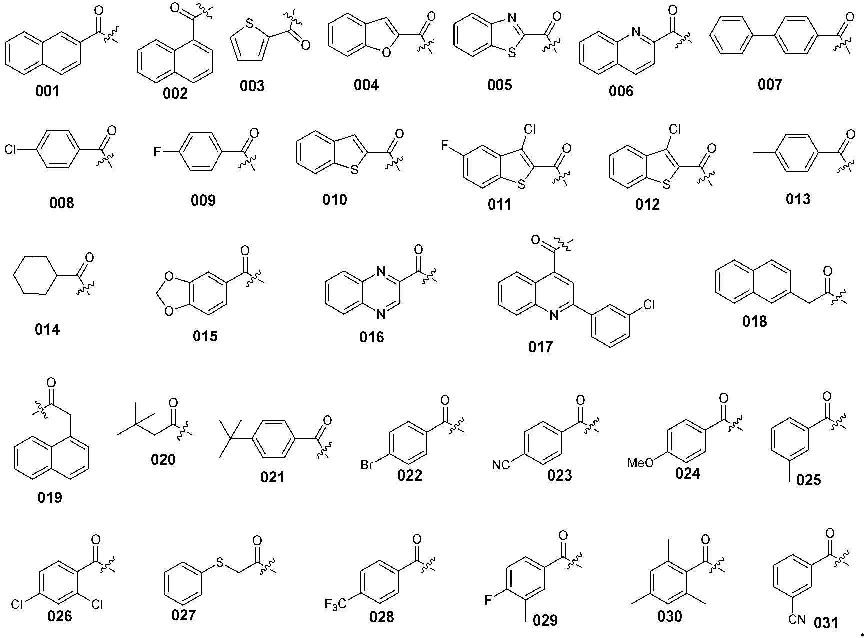

4. Composé selon l'une quelconque des revendications 1 à 3, ledit composé étant l'un quelconque des composés S-101 à S-131, ou D-101 à D-131, ledit groupement R représentant :
(a) un groupe -O-(CH₂)₅-NH-Fmoc ; ou
(b) un groupe -O-(CH₂)₅-NH₂ ou -O-(CH₂)₅-N₃,
| **Composé** | **Structure** |
|---|---|
| | ***Série S*** |
| **S101** | 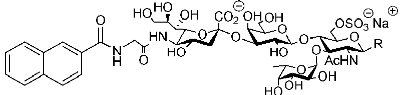 |
| **S102** | 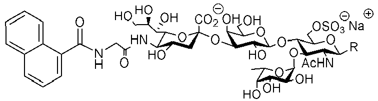 |
| **S103** | 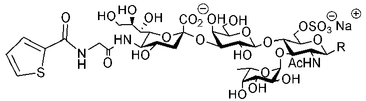 |
| **S104** | 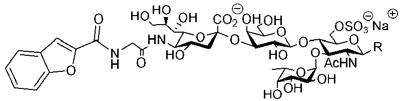 |
| **S105** | 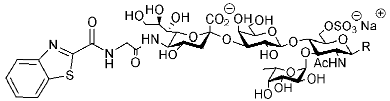 |
| **S106** | 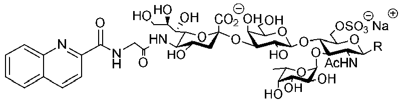 |
| **S107** | 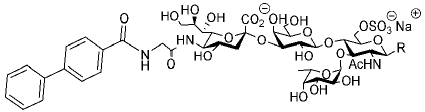 |
| **S108** | 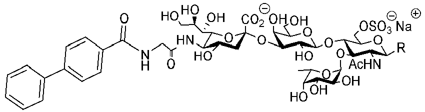 |
| **S109** | 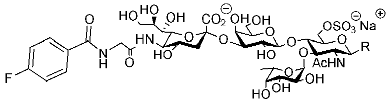 |
| **S110** | 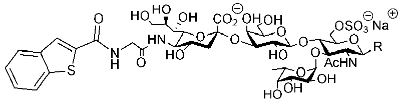 |
| **S111** | 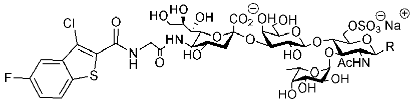 |
| **S112** | 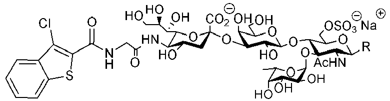 |
| **S113** | 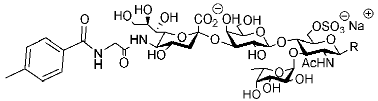 |
| **S114** | 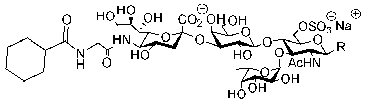 |
| **S115** | 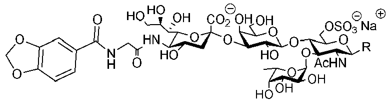 |
| **S116** | 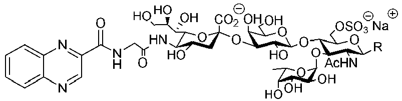 |
| **S117** | 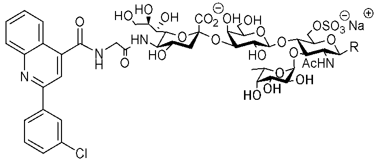 |
| **S118** | 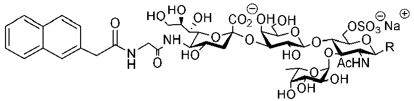 |
| **S119** | 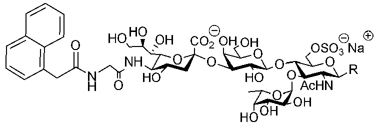 |
| **S120** | 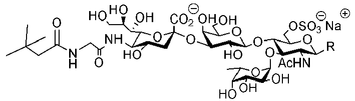 |
| **S121** | 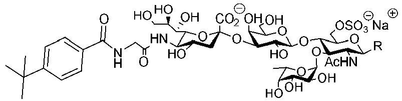 |
| **S122** | 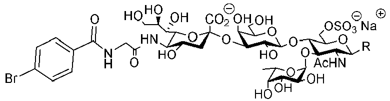 |
| **S123** | 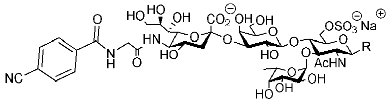 |
| **S124** | 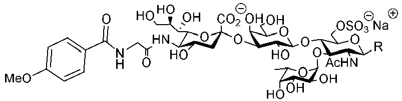 |
| **S125** | 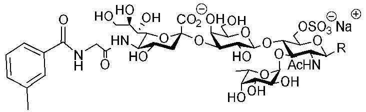 |
| **S126** | 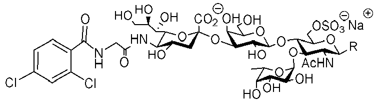 |
| **S127** | 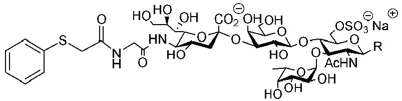 |
| **S128** | 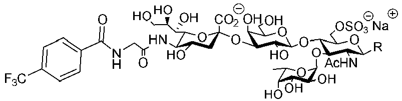 |
| **S129** | 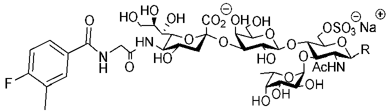 |
| **S130** | 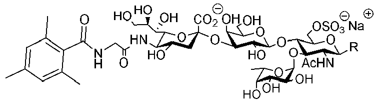 |
| **S131** | 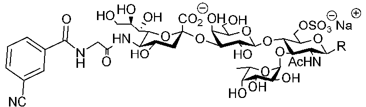 |
| | ***Série D*** |
|---|---|
| **D101** | 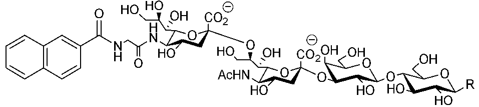 |
| **D102** | 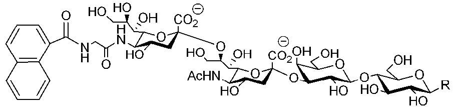 |
| **D103** | 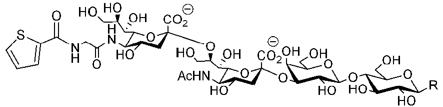 |
| **D104** | 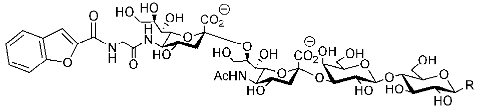 |
| **D105** | 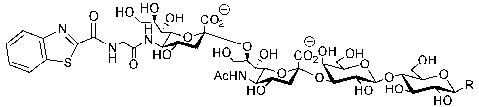 |
| **D106** | 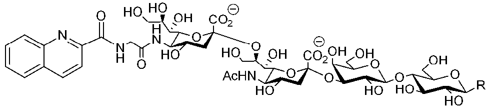 |
| **D107** | 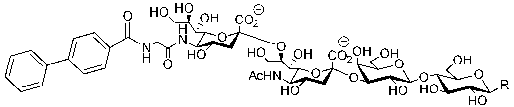 |
| **D108** | 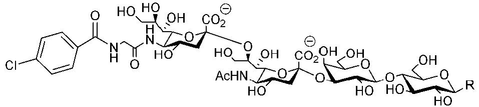 |
| **D109** | 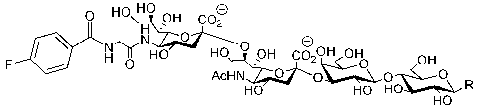 |
| **D110** | 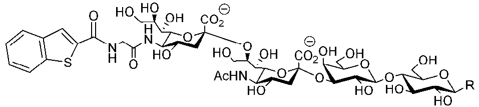 |
| **D111** | 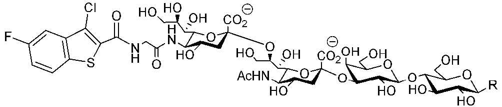 |
| **D112** | 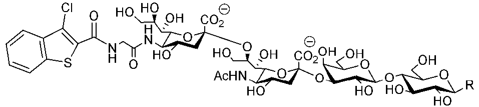 |
| **D113** | 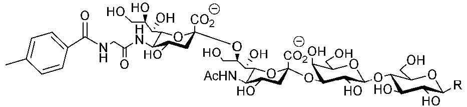 |
| **D114** | 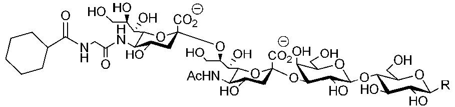 |
| **D115** | 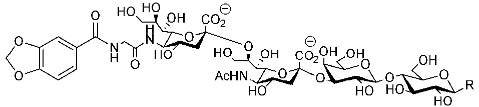 |
| **D116** | 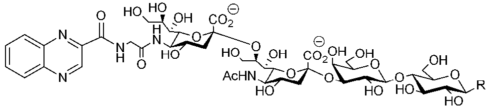 |
| **D117** | 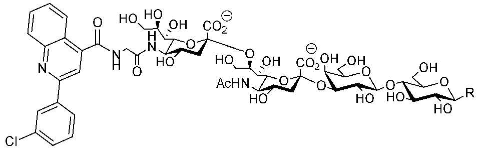 |
| **D118** | 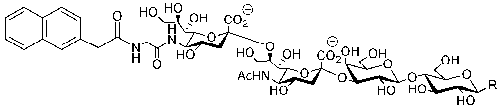 |
| **D119** | 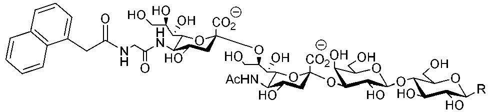 |
| **D120** | 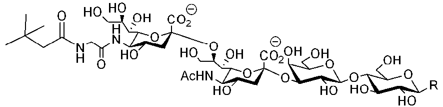 |
| **D121** | 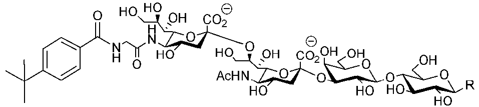 |
| **D122** | 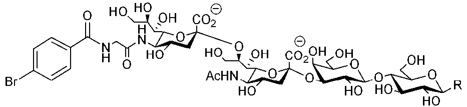 |
| **D123** | 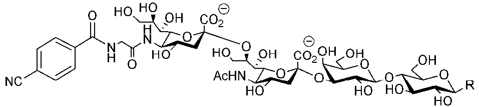 |
| **D124** | 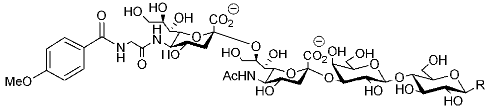 |
| **D125** | 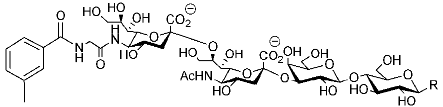 |
| **D126** | 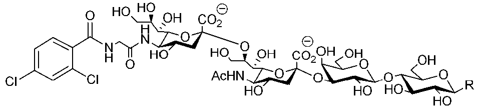 |
| **D127** | 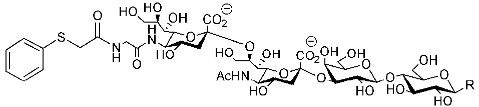 |
| **D128** | 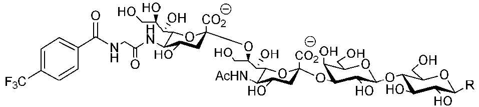 |
| **D129** | 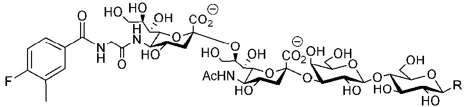 |
| **D130** | 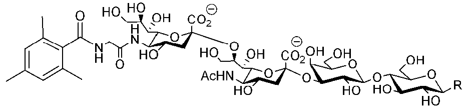 |
| **D131** | 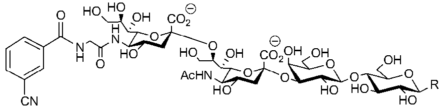 |
| | ***Série S*** |
|---|---|
| **S101** | 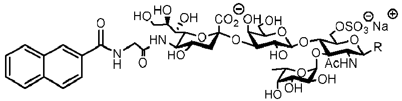 |
| **S102** | 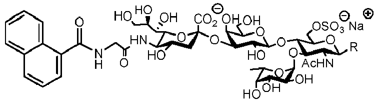 |
| **S103** | 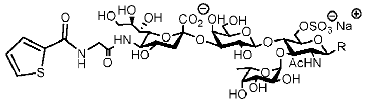 |
| **S104** | 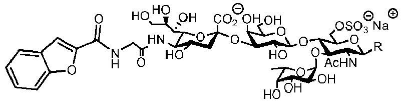 |
| **S105** | 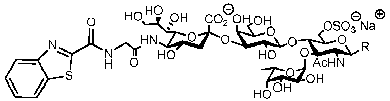 |
| **S106** | 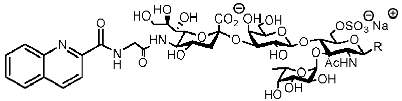 |
| **S107** | 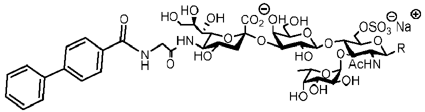 |
| **S108** | 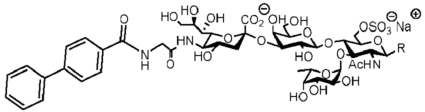 |
| **S109** | 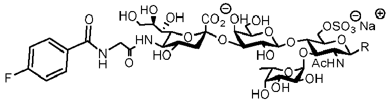 |
| **S110** | 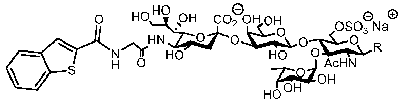 |
| **S111** | 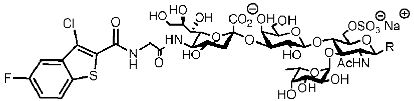 |
| **S112** | 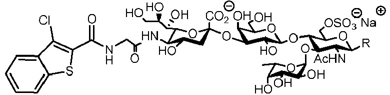 |
| **S113** | 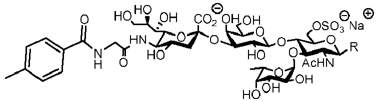 |
| **S114** | 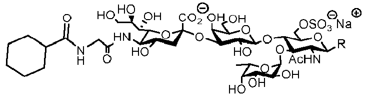 |
| **S115** | 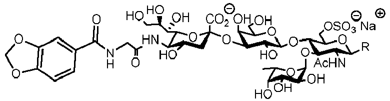 |
| **S116** | 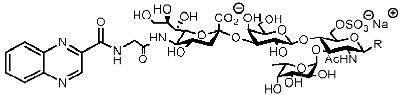 |
| **S117** | 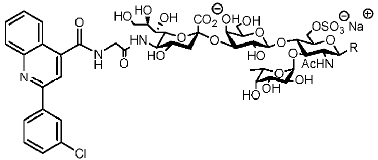 |
| **S118** | 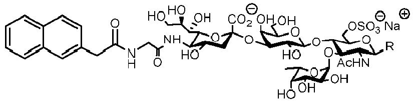 |
| **S119** | 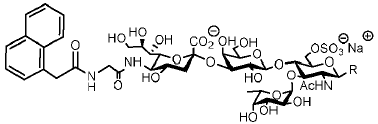 |
| **S120** | 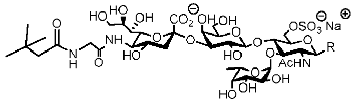 |
| **S121** | 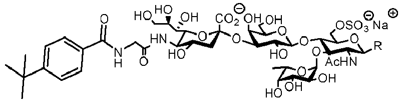 |
| **S122** | 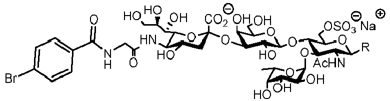 |
| **S123** | 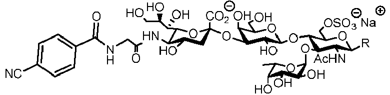 |
| **S124** | 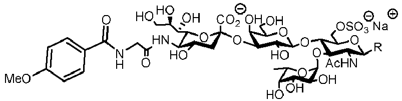 |
| **S125** | 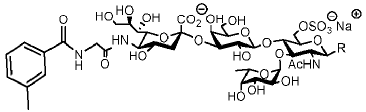 |
| **S126** | 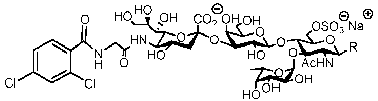 |
| **S127** | 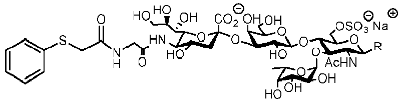 |
| **S128** | 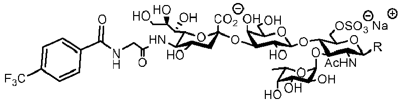 |
| **S129** | 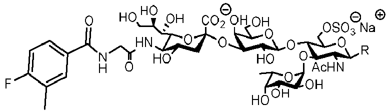 |
| **S130** | 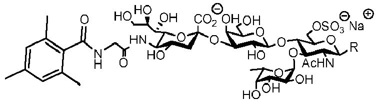 |
| **S131** | 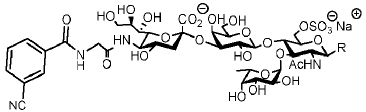 |
| | ***Série D*** |
|---|---|
| **D101** | 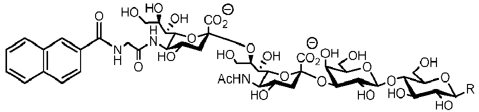 |
| **D102** | 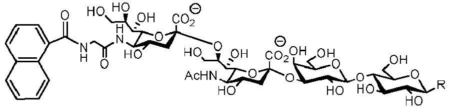 |
| **D103** | 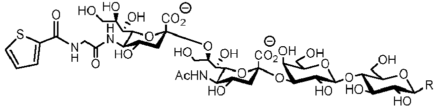 |
| **D104** | 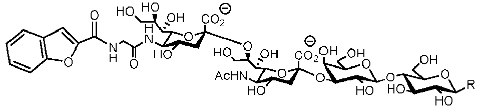 |
| **D105** | 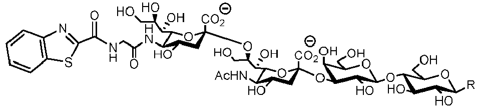 |
| **D106** | 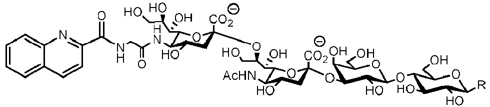 |
| **D107** | 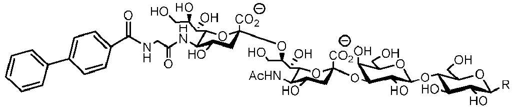 |
| **D108** | 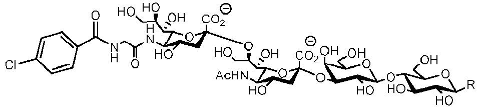 |
| **D109** | 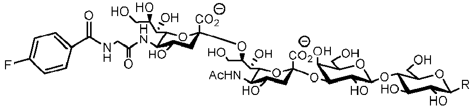 |
| **D110** | 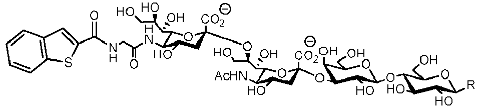 |
| **D111** | 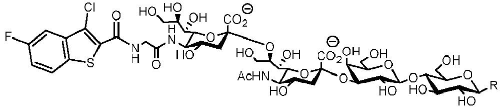 |
| **D112** | 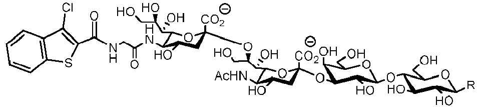 |
| **D113** | 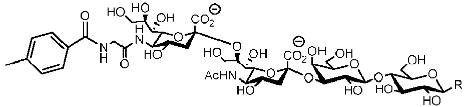 |
| **D114** | 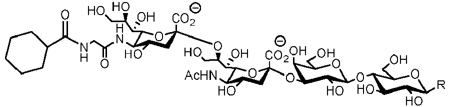 |
| **D115** | 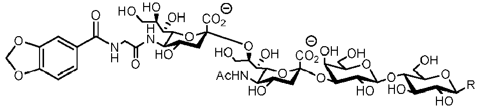 |
| **D116** | 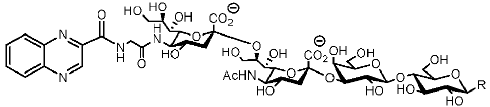 |
| **D117** | 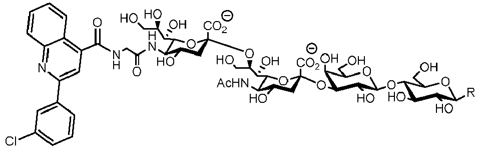 |
| **D118** | 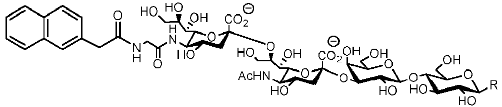 |
| **D119** | 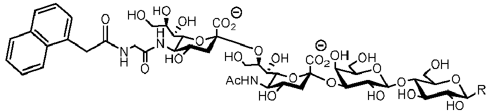 |
| **D120** | 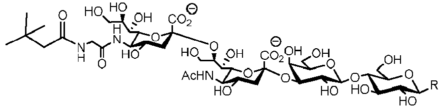 |
| **D121** | 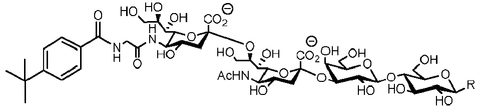 |
| **D122** | 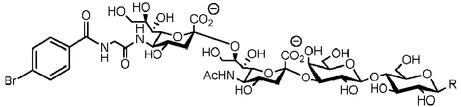 |
| **D123** | 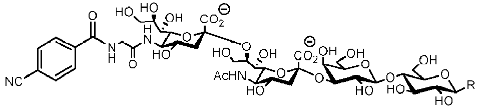 |
| **D124** | 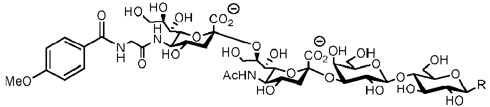 |

5. Composé selon la revendication 1 représenté par l'un quelconque des composés S-101 à S-131, ou D-101 à D-131, tel que défini dans la revendication 4, ou sel pharmaceutiquement acceptable de celui-ci, dans lequel la variable R représente un groupe -O-(CH₂)₅-NH₂, -O-(CH₂)₅-NH-Fmoc, -O-(CH₂)₅-N₃, ou -O-(CH₂)₅-biotine.

6. Composé selon la revendication 1, dans lequel R, indépendamment pour chaque occurrence, représente un groupe -O-(CH₂)ₓ-N₃, dans lequel x, indépendamment pour chaque occurrence, représente un entier de 1 à 10.

7. Particule, comprenant un composé représenté par la formule structurale suivante :
G-L-P,
dans laquelle :
P représente un polymère biocompatible ;
L représente un lieur covalent ; et
G représente l'un quelconque des groupements représentés par les formules structurales suivantes : 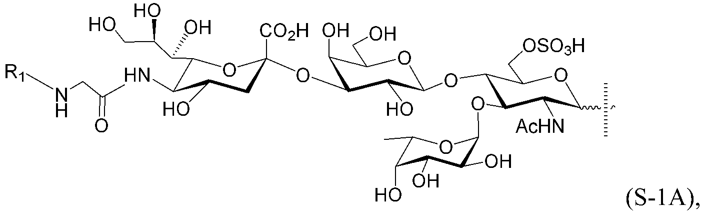 ou 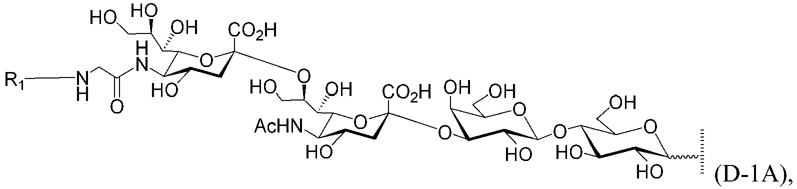
ou un sel pharmaceutiquement acceptable de celui-ci,
dans lesquelles :
le symbole 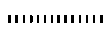 représente le point de fixation à L ;
R₁, indépendamment pour chaque occurrence, représente un groupe -C(O)-A, dans lequel A représente un groupe C₁-C₆ alkyle, un groupe C₆-C₁₈ aryle, un groupe (C₆-C₁₈)aryl(C₁-C₃)alkyle, un groupe hétéroaryle de 5 à 18 chaînons, un groupe (5 à 18 chaînons)hétéroaryl(C₁-C₃)alkyle, un groupe C₃-C₈ cycloalkyle, un groupe (C₃-C₈)cycloalkyl(C₁-C₃)alkyle, un groupe hétérocycloalkyle de 5 à 8 chaînons ou un groupe (5 à 8 chaînons)hétérocycloalkyl(C₁-C₃)alkyle ;
dans lequel un ou deux atomes de carbone dans la partie alkyle de A sont éventuellement, chacun indépendamment, remplacés par un hétéroatome choisi parmi un atome N, O ou S ;
et dans lequel A est éventuellement substitué par 1 à 3 groupes R¹¹, chacun desdits groupes R¹¹ étant indépendamment choisi parmi un groupe C₁-C₆ alkyle, un groupe C₁-C₆ halogénoalkyle, un groupe C₁-C₆ alcoxy, un atome d'halogène, un groupe C₆-C₁₂ aryle, un groupe hétéroaryle de 5 à 12 chaînons et un groupe cyano, ou deux groupes R¹¹, pris ensemble avec les atomes auxquels ils sont fixés, forment un groupe hétérocyclyle de 5 à 7 chaînons comportant 1 à 3 hétéroatomes choisis parmi un atome N, O ou S ;
dans lesquelles R¹¹, chacun indépendamment, est éventuellement substitué par 1 à 3 substituants choisis parmi un atome d'halogène, un groupe C₁-C₆ alkyle, un groupe C₁-C₆ halogénoalkyle, un groupe C₁-C₆ alcoxy ou un groupe cyano,
dans laquelle le polymère biocompatible comprend au moins un composé parmi l'acide polyglycolique, l'acide polylactique, l'acide poly(lactique-co-glycolique), la polycaprolactone, l'acide poly-3-hydroxybutyrique, le polyéthylène glycol, l'oxyde de polyéthylène, le polyéthylène glycol-bloc-polypropylène glycol-bloc-polyéthylène glycol (Pluronic F127), le copolymère séquencé polyoxyéthylène-polyoxypropylène (Pluronic F68), le poloxamère, le polyméthacrylate d'hydroxyméthyle, l'alcool polyvinylique, la polyvinylpyrrolidone, l'acide hyaluronique, l'héparine, le sulfate d'héparine, l'acide polysialique et le chitosane.

8. Particule selon la revendication 7,
(a) ledit polymère biocompatible comprenant un copolymère PLGA-PEG ; ou
(b) P étant représenté par la formule structurale suivante : 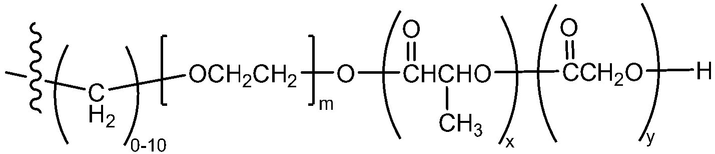 dans laquelle le symbole 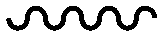 représente le point de fixation du polymère au lieur L, et en outre dans laquelle :
y représente un entier de 0 à 1000, x représente un entier de 0 à 1000, et m représente un entier de 0 à 450, à condition que x et y ne valent pas simultanément 0, éventuellement dans laquelle
(i) y représente un entier de 0 à 500, x représente un entier de 0 à 500, et m représente un entier de 0 à 250 ou
(ii) x représente un entier de 90 à 140, y représente un entier de 10 à 75 et m représente un entier de 90 à 140.

9. Particule selon la revendication 7 ou la revendication 8, ledit lieur L :
(a) comprenant une partie qui est le produit d'une réaction chimique clic ; et/ou
(b) comprenant une partie représentée par l'une quelconque des formules structurales suivantes, dans lesquelles les symboles ------ et 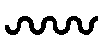 , indépendamment l'un de l'autre, représentent le point de fixation à des parties supplémentaires du lieur L, à P ou à G : 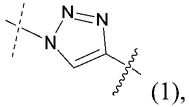 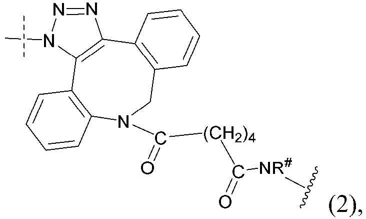 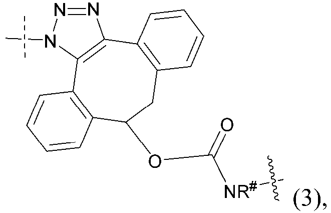 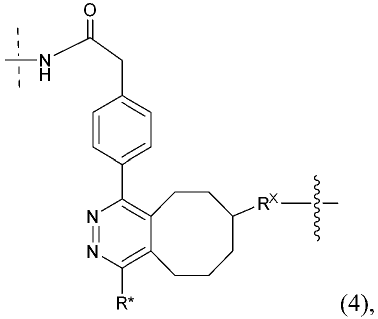 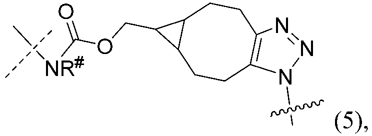 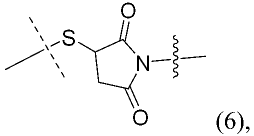 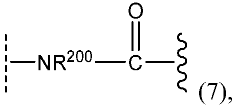
dans lesquelles R* représente un atome H ou un groupe méthyle, R^{#}, indépendamment pour chaque occurrence, représente un atome H ou un groupe C₁-C₃ alkyle, et R^{X} représente un groupe -NH-C(O)O-, et R²⁰⁰ représente un groupe -H ou un groupe C₁-C₃ alkyle,
éventuellement dans lesquelles la partie supplémentaire du lieur représente un groupe -O-(C₁-C₁₂)alkylényl-.

10. Particule selon l'une quelconque des revendications 7 à 9, P représentant un PLGA(10k)-PEG(5k).

11. Particule selon la revendication 7 représentée par l'une quelconque des formules structurales suivantes d'un sel pharmaceutiquement acceptable de celle-ci : 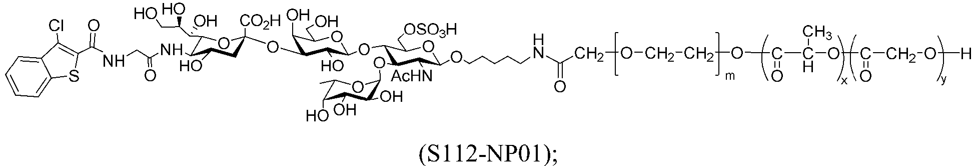 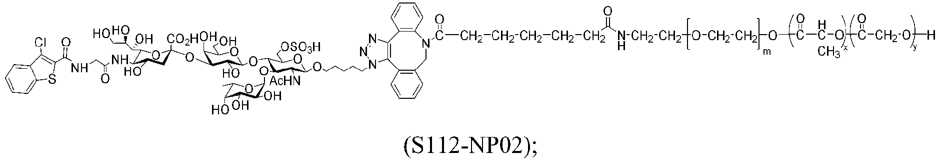 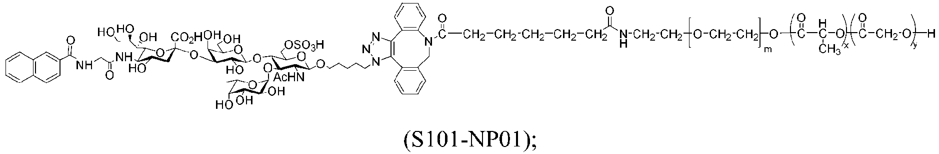 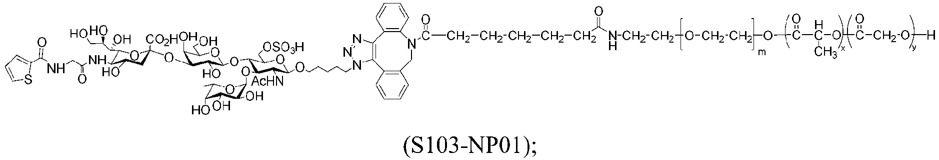 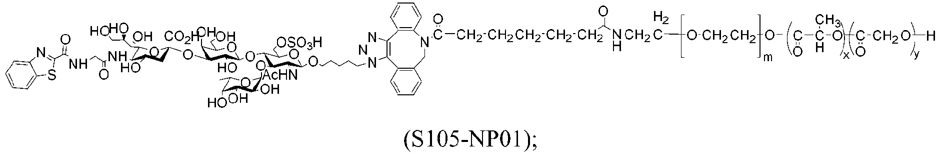 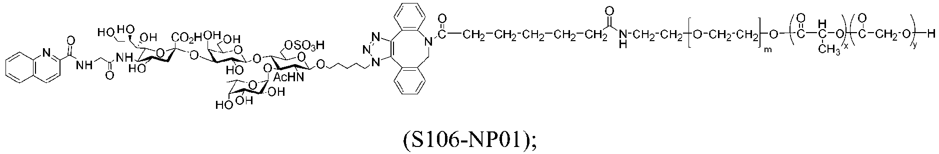 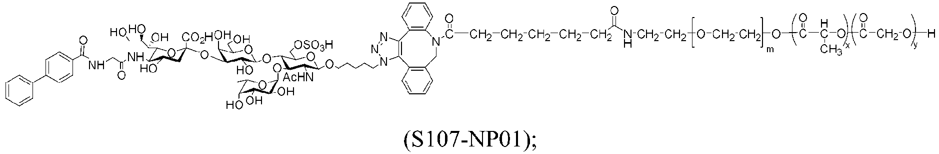 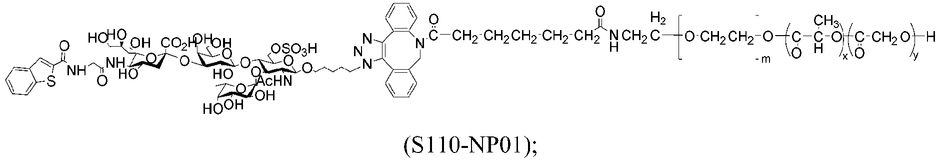
dans lesquelles, indépendamment pour chaque occurrence,
x représente un entier de 90 à 140 ;
y représente un entier de 10 à 75 ; et
m représente un entier de 90 à 140.

12. Particule selon la revendication 7 :
(a) ledit polymère biocompatible comprenant au moins un composé parmi l'acide polyglycolique, l'acide polylactique, l'acide poly(lactique-co-glycolique), la polycaprolactone, l'acide poly-3-hydroxybutyrique, l'oxyde de polyéthylène, le copolymère séquencé polyoxyéthylène-polyoxypropylène, le polyméthacrylate d'hydroxyméthyle, l'alcool polyvinylique, la polyvinylpyrrolidone, l'acide hyaluronique, l'héparine, le sulfate d'héparine, l'acide polysialique et le chitosane ;
(b) ledit polymère biocompatible comprenant de l'acide poly-L-lactique, de l'acide poly-D-lactique, de l'acide poly-D/L-lactique, un copolymère de ceux-ci, ou une combinaison de ceux-ci ;
(c) ledit polymère biocompatible comprenant de l'oxyde de polyéthylène, un copolymère séquencé de polyoxyéthylène-polyoxypropylène, un copolymère de ceux-ci, ou une combinaison de ceux-ci ; ou
(d) ladite particule comprenant en outre un ou plusieurs des composés suivants :
un PLGA à terminaison acide (PLGA-COOH) ;
un copolymère PLGA-PEG à terminaison acide, dans lequel le groupement acide termine le bloc de PEG (PLGA-PEG-COOH) ;
un copolymère PLGA-PEG, dans lequel le bloc PEG est terminé par le groupement représenté par la formule structurale suivante : 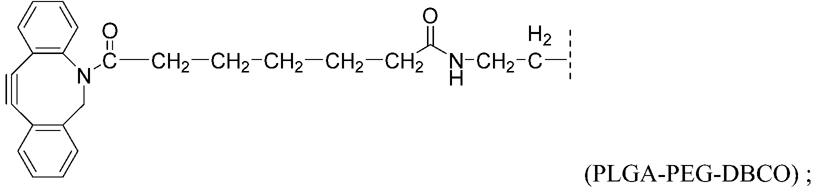 ou
un copolymère PLGA-PEG, dans lequel le bloc PEG est terminé par le groupement représenté par la formule structurale suivante : 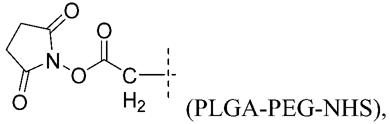
dans laquelle la ligne pointillée désigne le point de fixation du groupement de terminaison au bloc de PEG, éventuellement ladite particule comprenant au moins un mélange parmi :
un mélange de 75 % en poids de PLGA-COOH et 25 % en poids de PLGA-PEG-DBCO ;
un mélange de 75 % en poids de PLGA-COOH et 25 % en poids de PLGA-PEG-NHS ;
un mélange de 90 % en poids de PLGA-PEG-COOH et de 10 % en poids de PLGA-PEG-DBCO ; et
PLGA-PEG-NHS.

13. Particule selon :
(a) l'une quelconque des revendications 7 à 10, dans laquelle le poids de G par unité de poids de P (densité de ligand) représente de 10 à 75 µg/mg ; ou
(b) l'une quelconque des revendications 7 à 10 et 12, dans laquelle la quantité de G par unité de poids de la particule représente de 1 µg/mg à 1000 µg/mg.

14. Composé selon l'une quelconque des revendications 1 à 4 et 6, ou particules selon l'une quelconque des revendications 7 à 10, 12 et 13 ou sel pharmaceutiquement acceptable de celui-ci, destiné à être utilisé dans un procédé de traitement d'un trouble chez un sujet qui en a besoin, le procédé comprenant :
l'administration à un sujet d'une quantité efficace d'un composé selon l'une quelconque des revendications 1 à 4 et 6, ou de particules selon l'une quelconque des revendications 7 à 10, 12 et 13 ou d'un sel pharmaceutiquement acceptable de celles-ci, ledit trouble étant choisi parmi un cancer, une maladie ophtalmique, une maladie fibrotique, une inflammation parasitaire, une inflammation fongique, une inflammation virale, une inflammation auto-immune, une inflammation neurologique, une dégénérescence neurologique, une inflammation dermatologique, une inflammation rénale, une maladie cardiovasculaire, une inflammation gastro-intestinale ou une maladie rhumatismale.

15. Composition pharmaceutique, comprenant un composé selon l'une quelconque des revendications 1 à 4 ou une particule selon l'une quelconque des revendications 7 à 10, ou un sel pharmaceutiquement acceptable de celui-ci, dans un vecteur pharmaceutiquement acceptable.
